# EUROPEAN PATENT SPECIFICATION

(11) **EP 2 361 096 B1**
(45) Date of publication and mention of the grant of the patent: **02.01.2019**
(21) Application number: 09830696.2
(22) Date of filing: 24.11.2009
(51) Int. Cl.: A61K 39/395, A61P 37/06, C07K 16/24

(54) **IL-6-SPECIFIC ANTIBODY TO PREVENT OR TREAT CACHEXIA**
IL-6-SPEZIFISCHER ANTIKÖRPER ZUR PRÄVENTION BZW. BEHANDLUNG VON ABMAGERUNG
ANTICORPS IL-6 POUR PRÉVENIR OU TRAITER LA CACHEXIE

(30) Priority: 25.11.2008 US 117811 P; 24.02.2009 US 391717; 24.02.2009 US 391615; 25.11.2008 US 117861 P; 06.03.2009 US 399156; 05.02.2009 US 366567; 14.07.2009 US 502581; 25.11.2008 US 117839 P
(43) Date of publication of application: 31.08.2011
(73) Proprietor: AlderBio Holdings LLC, Las Vegas, NV 89109 (US)
(72) Inventor: SMITH, Jeffrey, T., L., Bellevue, WA 98004 (US); SCHATZMAN, Randall, C., Redmond, WA 98053 (US); LITTON, Mark, Seattle, WA 98119 (US); LATHAM, John, Seattle, WA 98119 (US)
(74) Representative: Boult Wade Tennant LLP
(86) International application number: PCT/US2009/006273
(87) International publication number: WO 2010/065078

(56) References cited:
- WO-A1-2004/039826
- WO-A2-2008/065378
- WO-A2-2008/144763
- US-A1- 2006 018 904
- US-A1- 2006 121 042
- US-A1- 2006 257 407
- US-A1- 2007 149 465
- US-A1- 2008 033 027
- US-A1- 2008 166 348
- EMILLE D ET AL: "ADMINISTRATION OF AN ANTI-INTERLEUKIN-6 MONOCLONAL ANTIBODY TO PATIENTS WITH ACQUIRED IMUNODEFICIENCY SYNDROME AND LYMPHOMA: EFFECT ON LYMPHOMA GROWTH AND ON B CLINICAL SYMPTOMS", BLOOD, AMERICAN SOCIETY OF HEMATOLOGY, US, vol. 84, no. 8, 15 October 1994 (1994-10-15), pages 2472-2479, XP001088120, ISSN: 0006-4971
- M. H. ZAKI ET AL: "Proc Am Soc Clin Oncol", 2003 ASCO ANNUAL MEETING - DEVELOPMENTAL THERAPEUTICS - CLINICAL PHARMACOLOGY AND IMMUNOTHERAPY, [Online] vol. 22, 697, 2003, XP055045899, Retrieved from the Internet: URL:http://www.asco.org/ASCOv2/Meetings/Ab stracts?&vmview=abst_detail_view&confID=23 &abstractID=100701> [retrieved on 2012-11-27]
- BATAILLE R ET AL: "Biologic effects of anti-interleukin-6 murine monoclonal antibody in advanced multiple myeloma", BLOOD, AMERICAN SOCIETY OF HEMATOLOGY, US, vol. 86, no. 2, 15 July 1995 (1995-07-15), pages 685-691, XP002448659, ISSN: 0006-4971
- G STRASSMANN ET AL: "Evidence for the involvement of interleukin 6 in experimental cancer cachexia.", JOURNAL OF CLINICAL INVESTIGATION, vol. 89, no. 5, 1 May 1992 (1992-05-01), pages 1681-1684, XP055045903, ISSN: 0021-9738, DOI: 10.1172/JCI115767
- PAUL R HINTON ET AL: "An engineered human IgG1 antibody with longer serum half-life", THE JOURNAL OF IMMUNOLOGY, THE AMERICAN ASSOCIATION OF IMMUNOLOGISTS, US, vol. 176, no. 1, 1 January 2006 (2006-01-01), pages 346-356, XP002484005, ISSN: 0022-1767
- STRASSMANN G ET AL: "INHIBITION OF EXPERIMENTAL CANCER CACHEXIA BY ANTI-CYTOKINE AND ANTI-CYTOKINE-RECEPTOR THERAPY", CYTOKINES AND MOLECULAR THERAPY, DUNITZ, LONDON, GB, vol. 1, no. 2, 1 January 1995 (1995-01-01) , pages 107-113, XP008043222, ISSN: 1355-6568
- KLEIN ET AL: "Murine anti-interleukin-6 monoclonal antibody therapy for a patient with plasma cell leukemia.", BLOOD, vol. 78, no. 5, 1 September 1991 (1991-09-01), pages 1198-1204, XP055045905, ISSN: 0006-4971
- KAORI FUJIMOTO-OUCHI ET AL: "Capecitabine improves cancer cachexia and normalizes IL-6 and PTHrP levels in mouse cancer cachexia models", CANCER CHEMOTHERAPY AND PHARMACOLOGY, SPRINGER, BERLIN, DE, vol. 59, no. 6, 29 September 2006 (2006-09-29), pages 807-815, XP019490080, ISSN: 1432-0843
- SUMIE TAMURA ET AL: "Involvement of human interleukin 6 in experimental cachexia induced by a human uterine cervical carcinoma xenograft.", CLINICAL CANCER RESEARCH, vol. 1, 1 January 1995 (1995-01-01), pages 1353-1358, XP055045909,

## Description

### BACKGROUND OF THE INVENTION

### Field of the Invention

This invention relates to novel anti-IL-6 antibodies and novel therapies and therapeutic protocols using anti-IL-6 antibodies for preventing or treating cachexia in a patient diagnosed with an IL-6 associated disorder, wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

This invention further pertains to novel methods of preventing or treating cachexiain a patient diagnosed with an IL-6 associated disorder, wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof using anti-IL-6 antibody Ab1, preferably aglycosylated and/or humanized and possessing an elimination half-life which is at least about 25 days.

### Description of Related Art

Known methods of addressing fatigue, weight loss and muscular weakness include regular routines of fitness and exercise, methods of conserving the patient's energy, and treatments that address anemia-induced fatigue and muscular weakness. Nevertheless, there remains a need in the art for methods and/or treatments that improve fatigue, weight loss and muscular weakness in a patient diagnosed with an IL-6 associated disorder, wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

Interleukin-6 (hereinafter "IL-6") (also known as interferon-β₂; B-cell differentiation factor; B-cell stimulatory factor-2; hepatocyte stimulatory factor; hybridoma growth factor; and plasmacytoma growth factor) is a multifunctional cytokine involved in numerous biological processes such as the regulation of the acute inflammatory response, the modulation of specific immune responses including Band T-cell differentiation, bone metabolism, thrombopoiesis, epidermal proliferation, menses, neuronal cell differentiation, neuroprotection, aging, cancer, and the inflammatory reaction occurring in Alzheimer's disease. *See* A. Papassotiropoulos, et al, Neurobiology of Aging, 22:863-871 (2001).

IL-6 is a member of a family of cytokines that promote cellular responses through a receptor complex consisting of at least one subunit of the signal-transducing glycoprotein gp130 and the IL-6 receptor ("IL-6R") (also known as gp80). The IL-6R may also be present in a soluble form ("sIL-6R"). IL-6 binds to IL-6R, which then dimerizes the signal-transducing receptor gp130. *See* Jones, SA, J. Immunology, 175:3463-3468 (2005).

In humans, the gene encoding IL-6 is organized in five exons and four introns, and maps to the short arm of chromosome 7 at 7p21. Translation of IL-6 RNA and post-translational processing result in the formation of a 21 to 28 kDa protein with 184 amino acids in its mature form. *See* A. Papassotiropoulos, et al, Neurobiology of Aging, 22:863-871 (2001).

As set forth in greater detail herein IL-6 is believed to play a role in the development of a multitude of diseases and disorders, including but not limited to fatigue, cachexia, autoimmune diseases, diseases of the skeletal system, cancer, heart disease, obesity, diabetes, asthma, Alzheimer's disease and multiple sclerosis. Due to the perceived involvement of IL-6 in a wide range of diseases and disorders, there remains a need in the art for compositions and methods useful for preventing or treating diseases associated with IL-6, as well as methods of screening to identify patients having diseases or disorders associated with IL-6. Particularly preferred anti-IL-6 compositions are those having minimal or minimizing adverse reactions when administered to the patient. Compositions or methods that reduce or inhibit diseases or disorders associated with IL-6 are beneficial to the patient in need thereof.

The function of IL-6 is not restricted to the immune response as it acts in hematopoiesis, thrombopoiesis, osteoclast formation, elicitation of hepatic acute phase response resulting in the elevation of C-reactive protein (CRP) and serum amyloid A (SAA) protein. It is known to be a growth factor for epidermal keratinocytes, renal mesangial cells, myeloma and plasmacytoma cells (Grossman et al., 1989 Prot Natl Acad Sci., 86, (16) 6367-6371; Horii et al., 1989, J Immunol, 143, 12, 3949-3955; Kawano et al., 1988, Nature 332, 6159, 83-85). IL-6 is produced by a wide range of cell types including monocytes/macrophages, fibroblasts, epidermal keratinocytes, vascular endothelial cells, renal messangial cells, glial cells, condrocytes, T and B-cells and some tumor cells (Akira et al, 1990, FASEB J., 4, 11, 2860-2867). Except for tumor cells that constitutively produce IL-6, normal cells do not express IL-6 unless appropriately stimulated.

Elevated IL-6 levels have been observed in many types of cancer, including breast cancer, leukemia, ovarian cancer, prostate cancer, pancreatic cancer, lymphoma, lung cancer, renal cell carcinoma, colorectal cancer, and multiple myeloma (*e.g.,* Chopra et al., 2004, MJAFI 60:45-49; Songur et al., 2004, Tumori 90:196-200; Blay et al., 1992, Cancer Research 52:3317-3322; Nikiteas et al., 2005, World J. Gasterenterol. 11:1639-1643; reviewed in Heikkila et al., 2008, Eur J Cancer, 44:937-945). As noted above, IL-6 is known or suspected to play a role in promoting proliferation or survival of at least some types of cancer. Moreover, some of these studies have demonstrated correlation between IL-6 levels and patient outcome. Together, these results suggest the possibility that inhibition of IL-6 can be therapeutically beneficial. Indeed, clinical studies (reviewed in Trikha et al., 2003, Clinical Cancer Research 9:4653-4665) have shown some improvement in patient outcomes due to administration of various anti-IL-6 antibodies, particularly in those cancers in which IL-6 plays a direct role promoting cancer cell proliferation or survival.

As noted above, IL-6 stimulates the hepatic acute phase response, resulting in increased production of CRP and elevated serum CRP levels. For this reason, C-reactive protein (CRP) has been reported to comprise a surrogate marker of IL-6 activity. Thus, elevated IL-6 activity can be detected through measurement of serum CRP. Conversely, effective suppression of IL-6 activity, e.g., through administration of a neutralizing anti-IL-6 antibody, can be detected by the resulting decrease in serum CRP levels.

A recent clinical trial demonstrated that administration of rosuvastatin to apparently healthy individuals having elevated CRP (greater than 2.0 mg/l) reduced their CRP levels by 37% and greatly decreased the incidence of myocardial infarction, stroke, arterial revascularization, hospitalization for unstable angina, or death from cardiovascular causes. Ridker et al., N Engl J Med. 2008 Nov 9 [Epub ahead of print].

In addition to its direct role in pathogenesis of some cancers and other diseases, chronically elevated IL-6 levels appear to adversely affect patient well-being and quality of life. For example, elevated IL-6 levels have been reported to be associated with cachexia and fever, and reduced serum albumin. Gauldie et al., 1987, PNAS 84:7251-7253; Heinric *et al.,* 1990, 265:621-636; Zamir et al., 1993, Metabolism 42:204-208; Zamir et al., 1992, Arch Surg, 127:170-174. Inhibition of IL-6 by a neutralizing antibody has been reported to ameliorate fever and cachexia in cancer patients, though improvement in these patients' serum albumin level has not been reported (Emille et al., 1994, Blood, 84:2472-2479; Blay et al., 1992, Cancer Research 52:3317-3322; Bataille et al., 1995, Blood, 86: 685-691).

Numerous studies have suggested that CRP is a valuable prognostic factor in cancer patients, with elevated CRP levels predicting poor outcome. *See*, *e.g*., Hefler et al, Clin Cancer Res, 2008 Feb 1;14(3):710-4; Nagaoka et al, Liver Int, 2007 Oct;27(8):1091-7; Heikkilä et al, J Epidemiol Community Health, 2007 Sep;61(9):824-33, Review; Hara et al, Anticancer Res, 2007 Jul-Aug;27(4C):3001-4; Polterauer et al, Gynecol Oncol, 2007 Oct;107(1):114-7, Epub 2007 Jul 6; Tingstedt et al, Scand J Gastroenterol, 2007 Jun;42(6):754-9; Suh et al, Support Care Cancer, 2007 Jun;15(6):613-20, Epub 2007 Jan 18; Gerhardt et al, World J Gastroenterol, 2006 Sep 14;12(34):5495-500; McArdle et al, Urol Int, 2006;77(2):127-9; Guillem et al, Dis Esophagus, 2005;18(3):146-50; Brown et al, Cancer, 2005 Jan 15;103(2):377-82. Decreased serum albumin (hypoalbuminemia) is also associated with increased morbidity and mortality in many critical illnesses, including cancers (*e.g*., Vigano et al., Arch Intern Med, 2000 Mar 27;160(6):861-8; Hauser et al., Support Care Cancer, 2006 Oct;14(10):999-1011; Seve et al., Cancer, 2006 Dec 1;107(11):2698-705). The apparent link between hypoalbuminemia and poor patient outcome suggests that restoring albumin levels through direct albumin infusion could promote patient survival, however, albumin infusion has not improved survival of patients with advanced cancer (Demirkazik et al., Proc Am Soc Clin Oncol 21: 2002 (abstr 2892)) or other critically ill patients groups (reviewed in Wilkes et al., Ann Intern Med, 2001 Aug 7;135(3):149-64).

The Glasgow Prognostic Score (GPS) is an inflammation-based prognostic score that combines levels of albumin (< 35 mg/L = 1 point) and CRP (> 10 mg/L = 1 point) (Forrest et al., Br J Cancer, 2004 May 4;90(9): 1704-6). Since its introduction in 2004, the Glasgow Prognostic Score has already been shown to have prognostic value as a predictor of mortality in numerous cancers, including gastro-esophageal cancer, non-small-cell lung cancer, colorectal cancer, breast cancer, ovarian cancer, bronchogenic cancer, and metastatic renal cancer (Forrest et al., Br J Cancer, 2004 May 4;90(9):1704-6; Sharma et al., Clin Colorectal Cancer, 2008 Sep;7(5):331-7; Sharma et al., Eur J Cancer, 2008 Jan;44(2):251-6; McMillan et al., Nutr Cancer, 2001;41(1-2):64-9; McMillan, Proc Nutr Soc, 2008 Aug;67(3):257-62; Ramsey et al., Cancer, 2007 Jan 15;109(2):205-12).

U.S. patent application publication no. 20080081041 (relating to treatment of cancer using an anti-IL-6 antibody) discloses that since IL-6 is associated with disease activity and since CRP is a surrogate marker of IL-6 activity, sustained suppression of CRP by neutralization of IL-6 by their anti-IL-6 antibody (CNTO 328, Zaki et al., Int J Cancer, 2004 Sep 10;111(4):592-5) may be assumed necessary to achieve biological activity. The same patent application indicates that the relationship between IL-6 and CRP in patients with benign and malignant prostate disease was previously examined by McArdle (McArdle et al. 2004 Br J Cancer 91(10):1755-1757). McArdle reportedly found no significant differences between the concentrations of IL-6 and CRP in the patients with benign disease compared with prostate cancer patients, in the cancer patients there was a significant increase in both IL-6 and CRP concentration with increasing tumor grade. The median serum CRP value for the 86 subjects with prostate cancer was 1.8 mg/L. Based thereon the inventors in this patent application postulate a proposed dose and schedule wherein 6 mg/kg of an anti-IL-6 antibody (CNTO 328) is administered every 2 weeks and allege that this is likely to achieve sustained suppression of CRP in subjects with metastatic HRPC.

IL-6 signaling is mediated by the Jak-Tyk family of cytoplasmic tyrosine kinases, including JAK1, JAK2, and JAK3 (reviewed in Murray J Immunol. 2007 Mar 1;178(5):2623-9). Sivash et al. report abrogation of IL-6-mediated JAK signaling by the cyclopentenone prostaglandin 15d-PGJ2 in oral squamous carcinoma cells. British Journal of Cancer (2004) 91, 1074-1080. These results suggest that inhibitors of JAK1, JAK2, or JAK3 could be employed as antagonists of IL-6.

Ulanova et al. report that inhibition of the nonreceptor protein tyrosine kinase Syk (using siRNA) decreased production of IL-6 by epithelial cells. Am J Physiol Lung Cell Mol Physiol. 2005 Mar;288(3):L497-507. These results suggest that an inhibitor of Syk could be employed as an antagonist of IL-6.

Kedar et al. report that treatment with thalidomide significantly reduced serum levels of CRP and IL-6 to normal or near normal levels in a substantial fraction of renal cell carcinoma patients. Int J Cancer. 2004 Jun 10;110(2):260-5. These results suggest that thalidomide, and possibly derivatives thereof, such as lenalidomide, may be useful antagonists of IL-6.

In addition, another published patent application, US 20070292420 teaches a Phase I dose escalating study using an anti-IL-6 (cCLB-8) antibody for treating refractory patients with advanced stage multiple myeloma (N=12) and indicate that this study demonstrated that some patients had disease stabilization. The application also reports that after discontinuation of treatment there was acceleration in the increase of M protein levels, suggesting disease re-bound after the withdrawal of therapy. Anti-IL-6 cCLB-8 antibody inhibited free circulating IL-6.

The application also indicates that this antibody trial resulted in no toxicity (except transient thrombocytopenia in two heavily pretreated patients) or allergic reactions were observed and that C-reactive protein (CRP) decreased below detection level in all patients. Their antibody (cCLB-8 antibody) reportedly possessed a circulating half-life of 17.8 days, and that there was no human anti-chimeric antibody (HACA) immune response observed (van Zaanen *et al.* 1998). They allege that the administration of CNTO 328 did not cause changes in blood pressure, pulse rate, temperature, hemoglobin, liver functions and renal functions. Except for transient thrombocytopenia in two heavily pretreated patients, no toxicity or allergic reactions allegedly were observed, and there was no human anti-chimeric antibody (HACA) immune response observed. Three patients in their study reportedly developed infection-related complications during therapy, however, a possible relation with anti-IL-6 cCLB-8 antibody was concluded by the inventors to be unlikely because infectious complications are reportedly common in end stage multiple myeloma and are a major cause of death. They conclude based on their results that this anti-IL-6 cCLB-8 antibody was safe in multiple myeloma patients.

As noted above, elevated IL-6 has been implicated in pathogenesis of cachexia, weakness, fatigue, and fever. Diseases and disorders associated with fatigue include, but are not limited to, general fatigue, exercise-induced fatigue, cancer-related fatigue, inflammatory disease-related fatigue and chronic fatigue syndrome. *See,* for example, Esper DH, et al, The cancer cachexia syndrome: a review of metabolic and clinical manifestations, Nutr Clin Pract., 2005 Aug;20 (4):369-76; Vgontzas AN, et al, IL-6 and its circadian secretion in humans, Neuroimmunomodulation, 2005;12(3):131-40; Robson-Ansley, PJ, et al, Acute interleukin-6 administration impairs athletic performance in healthy, trained male runners, Can J Appl Physiol., 2004 Aug;29(4):411-8; Shephard RJ., Cytokine responses to physical activity, with particular reference to IL-6: sources, actions, and clinical implications, Crit Rev Immunol., 2002;22(3):165-82; Arnold, MC, et al, Using an interleukin-6 challenge to evaluate neuropsychological performance in chronic fatigue syndrome, Psychol Med., 2002 Aug;32(6):1075-89; Kurzrock R., The role of cytokines in cancer-related fatigue, Cancer, 2001 Sep 15;92(6 Suppl): 1684-8; Nishimoto N, et al, Improvement in Castleman's disease by humanized anti-interleukin-6 receptor antibody therapy, Blood, 2000 Jan 1; 95 (1):56-61; Vgontzas AN, et al, Circadian interleukin-6 secretion and quantity and depth of sleep, J Clin Endocrinol Metab., 1999 Aug;84(8):2603-7; and Spath-Schwalbe E, et al, Acute effects of recombinant human interleukin 6 on endocrine and central nervous sleep functions in healthy men, J Clin Endocrinol Metab., 1998 May;83(5):1573-9.

Diseases and disorders associated with cachexia include, but are not limited to, cancer-related cachexia, cardiac-related cachexia, respiratory-related cachexia, renal-related cachexia and age-related cachexia. *See,* for example, Barton, BE., Interleukin-6 and new strategies for the treatment of cancer, hyperproliferative diseases and paraneoplastic syndromes, Expert Opin Ther Targets, 2005 Aug;9(4):737-52; Zaki MH, et al, CNTO 328, a monoclonal antibody to IL-6, inhibits human tumor-induced cachexia in nude mice, Int J Cancer, 2004 Sep 10;111(4):592-5; Trikha M, et al, Targeted anti-interleukin-6 monoclonal antibody therapy for cancer: a review of the rationale and clinical evidence, Clin Cancer Res., 2003 Oct 15;9(13):4653-65; Lelli G, et al, Treatment of the cancer anorexia-cachexia syndrome: a critical reappraisal, J Chemother., 2003 Jun;15(3):220-5; Argiles JM, et al, Cytokines in the pathogenesis of cancer cachexia, Curr Opin Clin Nutr Metab Care, 2003 Jul;6(4):401-6; Barton BE., IL-6-like cytokines and cancer cachexia: consequences of chronic inflammation, Immunol Res., 2001;23(1):41-58; Yamashita JI, et al, Medroxyprogesterone acetate and cancer cachexia: interleukin-6 involvement, Breast Cancer, 2000;7(2): 130-5; Yeh SS, et al, Geriatric cachexia: the role of cytokines, Am J Clin Nutr., 1999 Aug;70(2):183-97; Strassmann G, et al, Inhibition of experimental cancer cachexia by anti-cytokine and anti-cytokine-receptor therapy, Cytokines Mol Ther., 1995 Jun;1(2):107-13; Fujita J, et al, Anti-interleukin-6 receptor antibody prevents muscle atrophy in colon-26 adenocarcinoma-bearing mice with modulation of lysosomal and ATP-ubiquitin-dependent proteolytic pathways, Int J Cancer, 1996 Nov 27;68(5):637-43; Tsujinaka T, et al, Interleukin 6 receptor antibody inhibits muscle atrophy and modulates proteolytic systems in interleukin 6 transgenic mice, J Clin Invest., 1996 Jan 1;97(1):244-9; Emilie D, et al, Administration of an anti-interleukin-6 monoclonal antibody to patients with acquired immunodeficiency syndrome and lymphoma: effect on lymphoma growth and on B clinical Symptoms, Blood, 1994 Oct 15;84 (8):2472-9; and Strassmann G, et al, Evidence for the involvement of interleukin 6 in experimental cancer cachexia, J Clin Invest., 1992 May;89(5):1681-4.

Another cachexia-related disease is failure to thrive, also known as faltering growth, in which a child exhibits a rate of weight gain less than expected. Failure to thrive is typically defined as weight below the third percentile or a decrease in the percentile rank of 2 major growth parameters in a short period. Failure to thrive results from heterogeneous medical and psychosocial causes, and the cause sometimes eludes diagnosis. One recent study (totaling 34 patients) reported a statistically significant elevation in IL-6 levels in patients diagnosed with failure to thrive. Shaoul et al. J Pediatr Gastroenterol Nutr., 2003 Oct;37(4):487-91.

### BRIEF SUMMARY OF THE INVENTION

The present invention is an extension of Applicants' previous inventions directed to specific antibodies, humanized or chimeric or single chain antibodies and fragments thereof having binding specificity for IL-6, in particular antibodies having specific epitopic specificity and/or functional properties and novel therapies using these and other anti-IL-6 antibodies. One embodiment of the invention encompasses use of an anti-IL-6 antibody or antibody fragment in the manufacture of a medicament for use in a method of preventing or treating cachexia in a patient diagnosed with an IL-6 associated disorder,
wherein the administered antibody or fragment comprises:
(a) the VL CDRs in SEQ ID NO:4, 5 and 6 and the VH CDRs in SEQ ID NO: 7, 8 or 120 and 9, or
(b) a V_{H} polypeptide comprising the polypeptide depicted in SEQ ID NO: 3, 18, or 19, and further comprises a V_{L} polypeptide comprising the polypeptide depicted in SEQ ID NO: 2, or 20, and
wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof. In another embodiment the invention encompasses an antibody or antibody fragment for use in a method of preventing or treating cachexia in a patient diagnosed with an IL-6 associated disorder, wherein the antibody or fragment comprises:
(a) the V_{L} CDRs in SEQ ID NO:4, 5 and 6 and the V_{H} CDRs in SEQ ID NO: 7, 8 or 120 and 9, or
(b) a V_{H} polypeptide comprising the polypeptide depicted in SEQ ID NO: 3, 18, or 19, and further comprises a V_{L} polypeptide comprising the polypeptide depicted in SEQ ID NO: 2, or 20, and
wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

. Preferably the antibodies used in the invention will be aglycosylated. In more specific embodiments of the invention these antibodies will block gp130 activation and/or possess binding affinities (Kds) less than 50 picomolar and/or K_{off} values less than or equal to 10⁻⁴ S⁻¹.

In another embodiment of the invention the antibodies and humanized versions used will be derived from rabbit immune cells (B lymphocytes) and may be selected based on their homology (sequence identity) to human germ line sequences. These antibodies may require minimal or no sequence modifications, thereby facilitating retention of functional properties after humanization. In exemplary embodiments these humanized antibodies will comprise human frameworks which are highly homologous (possess high level of sequence identity) to that of a parent (e.g. rabbit) antibody as described infra.

In another embodiment of the invention the subject antibodies may be selected based on their activity in functional assays such as IL-6 driven T1165 proliferation assays, or IL-6 simulated HepG2 haptoglobin production assays.

There is described the administration of conjugates of anti-IL-6 antibodies and humanized, chimeric or single chain versions thereof and other binding fragments thereof conjugated to one or more functional or detectable moieties. There are described methods of making said humanized anti-IL-6 or anti-IL-6/IL-6R complex antibodies and binding fragments thereof. In one embodiment, binding fragments include, but are not limited to, Fab, Fab', F(ab')₂, Fv and scFv fragments.

There is described the production of anti-IL-6 antibodies in recombinant host cells, preferably diploid yeast such as diploid Pichia and other yeast strains.

The invention relates to methods of preventing or treating cachexia in a patient diagnosed with an IL-6 associated disorder, comprising administering to the patient an anti-IL-6 antibody or antibody fragment, whereby the patient's cachexia may be prevented or improved, and monitoring the patient to assess cachexia, , wherein the anti-IL-6 antibody is Ab1 and chimeric, humanized, single chain antibodies and fragments thereof (containing one or more CDRs of the afore-identified antibodies) that specifically bind IL-6, which preferably are aglycosylated. As discussed infra in a preferred exemplary embodiment the anti-IL-6 antibody will comprise a humanized antibody containing the CDRs of Ab1 and more preferably will comprise the variable heavy and light chain in SEQ ID NO:657 and SEQ ID NO:709 respectively and the constant regions in SEQ ID NO:588 and 586 respectively or variants thereof wherein one or more amino acids are modified by substitution or deletion without substantially disrupting IL-6 binding affinity.

In a preferred embodiment the humanized anti-IL-6 antibody will comprise the variable heavy and variable light chain sequences respectively contained in SEQ ID NO:657 and SEQ ID NO:709, and preferably further comprising the heavy chain and light chain constant regions respectively contained in SEQ ID NO:588 and SEQ ID NO:586. This embodiment also contemplates polynucleotides comprising, or alternatively consisting of, one or more of the nucleic acids encoding the variable heavy chain (SEQ ID NO: 700) and variable light chain (SEQ ID NO:723) sequences and the constant region heavy chain (SEQ ID NO: 589) and constant region light chain (SEQ ID NO:587) sequences.

In the invention, all of the CDRs in the anti-IL-6 antibody or antibody fragment are identical to the CDRs contained in an anti-IL-6 antibody comprising Ab1 and chimeric, humanized, single chain antibodies and fragments thereof (containing one or more CDRs of the afore-identified antibodies) that specifically bind IL-6, which preferably are aglycosylated.
of the antibodies utilised within the invention are Ab1, including rabbit and humanized forms thereof, as well fragments thereof. In the human clinical trials presented in the Examples, a humanized form of Ab1 was administered.

In the invention, all of the CDRs in the anti-IL-6 antibody or antibody fragment are identical to the CDRs contained in Ab1.

In an embodiment of the invention, the anti-IL-6 antibody or antibody fragment may be aglycosylated.

In an embodiment of the invention, the anti-IL-6 antibody or antibody fragment may contain an Fc region that has been modified to alter effector function, half-life, proteolysis, and/or glycosylation. Preferably the Fc region is modified to eliminate glycosylation.

In an embodiment of the invention, the anti-IL-6 antibody or antibody fragment may be a human, humanized, single chain or chimeric antibody.

In an embodiment of the invention, the anti-IL-6 antibody or antibody fragment may be a humanized antibody derived from a rabbit (parent) anti-IL-6 antibody.

In an embodiment of the invention, the framework regions (FRs) in the variable light region and the variable heavy regions of said anti-IL-6 antibody or antibody fragment respectively may be human FRs which are unmodified or which have been modified by the substitution of at most 2 or 3 human FR residues in the variable light or heavy chain region with the corresponding FR residues of the parent rabbit antibody, and the FRs may have been derived from human variable heavy and light chain antibody sequences which have been selected from a library of human germline antibody sequences based on their high level of homology to the corresponding rabbit variable heavy or light chain regions relative to other human germline antibody sequences contained in the library. As disclosed in detail infra in a preferred embodiment the antibody will comprise human FRs which are selected based on their high level of homology (degree of sequence identity) to that of the parent antibody that is humanized.

In an embodiment of the invention, the anti-IL-6 antibody or antibody fragment may be administered to the patient with a frequency at most once per period of approximately four weeks, approximately eight weeks, approximately twelve weeks, approximately sixteen weeks, approximately twenty weeks, or approximately twenty-four weeks.

In an embodiment of the invention, the patient's cachexia may remain improved for an entire period intervening two consecutive anti-IL-6 antibody administrations.

In an embodiment of the invention, the anti-IL-6 antibody or antibody fragment may comprise a VH polypeptide sequence comprising: SEQ ID NO: 3, 18, or 19; and may further comprise a VL polypeptide sequence comprising: SEQ ID NO: 2, or 20

In an embodiment of the invention, said anti-IL-6 antibody or antibody fragment may be humanized.

In an embodiment of the invention, said anti-IL-6 antibody or antibody fragment may be chimeric.

In an embodiment of the invention, said anti-IL-6 antibody or antibody fragment further may comprise a human Fc, e.g., an Fc region comprised of the variable heavy and light chain constant regions contained in SEQ ID NO:704 and 702.

In an embodiment of the invention, said human Fc may be derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19.

In an embodiment of the invention, the anti-IL-6 antibody or antibody fragment may have an elimination half-life of at least about 22 days, at least about 25 days, or at least about 30 days.

In an embodiment of the invention, the anti-IL-6 antibody or antibody fragment may be co-administered with a chemotherapy agent.

In an embodiment of the invention, the chemotherapy agent may be selected from VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof.

The anti-IL-6 antibody or antibody fragment which may be directly or indirectly attached to a detectable label or therapeutic agent.

In an embodiment of the invention, the anti-IL-6 antibody or antibody fragment may be Ab1 or a humanized, chimeric, single chain or fragment thereof comprising all of the CDRs of Ab1.

The disease or condition for treatment according to the invention is selected from AIDS malnutrition, lead exposure, malaria hypothyroidism, tuberculosis, hypopituitarism, neurasthenia, hypernatremia, hyponatremia, splenica, failure to thrive (faltering growth), or any combination thereof.

In an embodiment of the invention, the method may include administration of an antagonist of a cachexia-associated factor, weakness-associated factor, fatigue-associated factor, and/or fever-associated factor. The cachexia-associated factor, weakness-associated factor, fatigue-associated factor, and/or fever-associated factor may be selected from tumor necrosis factor-alpha, Interferon gamma, Interleukin 1 alpha, Interleukin 1 beta, Interleukin 6, proteolysis inducing factor, leukemia-inhibitory factor, or any combination thereof.

In an embodiment of the invention, the method may include administration of an anti-cachexia agent selected from cannabis, dronabinol (Marinol™), nabilone (Cesamet), cannabidiol, cannabichromene, tetrahydrocannabinol, Sativex, megestrol acetate, or any combination thereof.

In an embodiment of the invention, the method may include administration of an anti-nausea or antiemetic agent selected from 5-HT3 receptor antagonists, ajwain, alizapride, anticholinergics, antihistamines, aprepitant, benzodiazepines, cannabichromene, cannabidiol, cannabinoids, cannabis, casopitant, chlorpromazine, cyclizine, dexamethasone, dexamethasone, dimenhydrinate (Gravol™), diphenhydramine, dolasetron, domperidone, dopamine antagonists, doxylamine, dronabinol (Marinol™), droperidol, emetrol, ginger, granisetron, haloperidol, hydroxyzine, hyoscine, lorazepam, meclizine, metoclopramide, midazolam, muscimol, nabilone (Cesamet), nk1 receptor antagonists, ondansetron, palonosetron, peppermint, Phenergan, prochlorperazine, Promacot, promethazine, Pentazine, propofol, sativex, tetrahydrocannabinol, trimethobenzamide, tropisetron, nandrolone, stilbestrol, thalidomide, lenalidomide, ghrelin agonists, myostatin antagonists, anti-myostatin antibodies, selective androgen receptor modulators, selective estrogen receptor modulators, angiotensin All antagonists, beta two adenergic receptor agonists, beta three adenergic receptor agonists, or any combination thereof.

The patient's fever may be assessed by measurement of patient's body temperature.

The patient's body temperature may be measured prior to administration of the anti-IL-6 antibody, and the anti-IL-6 antibody or antibody fragment administered if the patient's body temperature is higher than about 38° F.

The patient's body temperature may be measured within 24 hours prior to administration of the anti-IL-6 antibody, and the anti-IL-6 antibody or antibody fragment administered if the patient's body temperature measurement indicates that a fever was present.

The patient's body weight may be measured prior to administration of the anti-IL-6 antibody, and the anti-IL-6 antibody or antibody fragment administered if the patient's weight has declined by greater than approximately 5% within approximately 30 days, or if the patient's lean body mass index is less than about 17 kg / m² (male patient) or less than about 14 kg / m² (female patient).

The patient's muscular strength may be measured prior to administration of the anti-IL-6 antibody, and the anti-IL-6 antibody or antibody fragment administered if the patient's muscular strength has declined by greater than approximately 20% within approximately 30 days.

The invention may result in a prolonged improvement in cachexia, weakness, fatigue, and/or fever in the patient.

The patient's body mass may be raised by approximately 1 kilogram within approximately 4 weeks of administration of the anti-IL-6 antibody or antibody fragment.

The patient's cachexia may be measurably improved within about 4 weeks of anti-IL-6 antibody administration.

The patient's cachexia may be assessed by measurement of the patient's total body mass, lean body mass, lean body mass index, and/or appendicular lean body mass.

The measurement of the patient's body mass may discount (subtract) the estimated weight of the patient's tumor(s) and/or extravascular fluid collection(s).

The patient's cachexia may remain measurably improved approximately 8 weeks after anti-IL-6 antibody administration.

The patient's weakness may be measurably improved within about 4 weeks of anti-IL-6 antibody administration.

The patient's weakness may be measured by the hand grip strength test.

The patient's hand grip strength may be improved by at least about 15%, or at least about 20%.

The patient's weakness may remain measurably improved approximately 8 weeks after anti-IL-6 antibody administration.

The patient's fatigue may be measurably improved within about 1 week of anti-IL-6 antibody administration.

The patient's fatigue may be measured by the FACIT-F FS test.

The patient's FACIT-F FS score may be improved by at least about 10 points.

The patient's fatigue may remain measurably improved approximately 8 weeks after anti-IL-6 antibody administration.

The patient's fever may be measurably improved within about 1 week of anti-IL-6 antibody administration.

The patient's fever may remain measurably improved approximately 8 weeks after anti-IL-6 antibody administration.

The patient's survivability may be improved.

The patient's quality of life may be improved.

### BRIEF DESCRIPTION OF THE SEVERAL VIEWS OF THE DRAWINGS

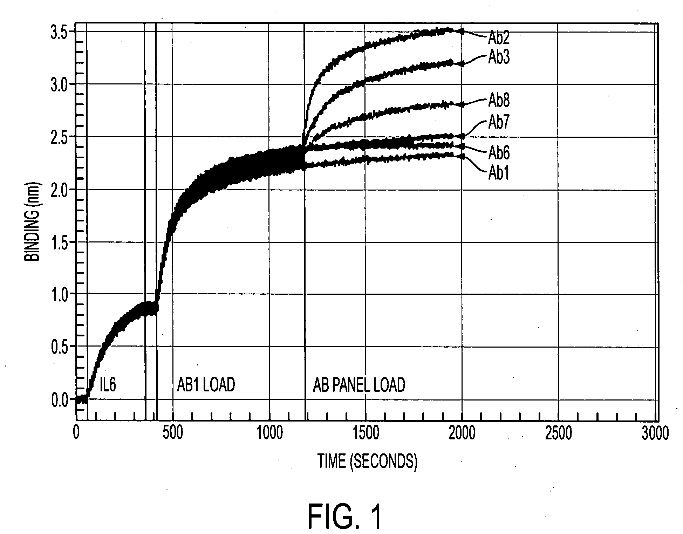

Fig. 1 shows that a variety of unique epitopes were recognized by the collection of anti-IL-6 antibodies prepared by the antibody selection protocol. Epitope variability was confirmed by antibody-IL-6 binding competition studies (ForteBio Octet).

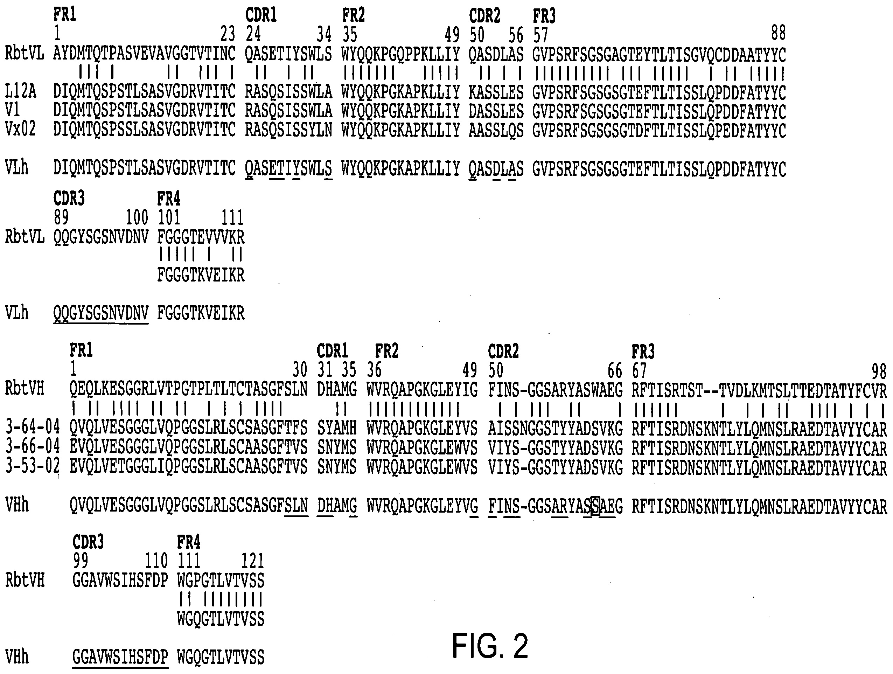

Fig. 2 shows alignments of variable light and variable heavy sequences between a rabbit antibody variable light and variable heavy sequences and homologous human sequences and the humanized sequences. Framework regions are identified FR1-FR4. Complementarity determining regions are identified as CDR1-CDR3. Amino acid residues are numbered as shown. The initial rabbit sequences are called RbtVL and RbtVH for the variable light and variable heavy sequences respectively. Three of the most similar human germline antibody sequences, spanning from Framework 1 through to the end of Framework 3, are aligned below the rabbit sequences. The human sequence that is considered the most similar to the rabbit sequence is shown first. In this example those most similar sequences are L12A for the light chain and 3-64-04 for the heavy chain. Human CDR3 sequences are not shown. The closest human Framework 4 sequence is aligned below the rabbit Framework 4 sequence. The vertical dashes indicate a residue where the rabbit residue is identical with one or more of the human residues at the same position. The bold residues indicate that the human residue at that position is identical to the rabbit residue at the same position. The final humanized sequences are called VLh and VHh for the variable light and variable heavy sequences respectively. The underlined residues indicate that the residue is the same as the rabbit residue at that position but different than the human residues at that position in the three aligned human sequences.
Fig. 3 demonstrates the high correlation between the IgG produced and antigen specificity for an exemplary IL-6 protocol. 9 of 11 wells showed specific IgG correlation with antigen recognition.
Fig. 4 provides the α-2-macroglobulin (A2M) dose response curve for antibody Ab1 administered intravenously at different doses one hour after a 100µg/kg s.c. dose of human IL-6.
Fig. 5 provides survival data for the antibody Ab1 progression groups versus control groups.
Fig. 6 provides additional survival data for the antibody Ab1 regression groups versus control groups.
Fig. 7 provides survival data for polyclonal human IgG at 10 mg/kg i.v. every three days (270-320 mg tumor size) versus antibody Ab1 at 10 mg/kg i.v. every three days (270-320 mg tumor size).
Fig. 8 provides survival data for polyclonal human IgG at 10 mg/kg i.v. every three days (400-527 mg tumor size) versus antibody Ab1 at 10 mg/kg i.v. every three days (400-527 mg tumor size).
Fig. 9 provides a pharmacokinetic profile of antibody Ab1 in cynomolgus monkey. Plasma levels of antibody Ab1 were quantitated through antigen capture ELISA. This protein displays a half life of between 12 and 17 days consistent with other full length humanized antibodies.
Fig. 10 (A-D) provides binding data for antibodies Ab4, Ab3, Ab8 and Ab2, respectively. Fig. 10E provides binding data for antibodies Ab1, Ab6 and Ab7.
Fig. 11 summarizes the binding data of Fig. 10 (A-E) in tabular form.
Fig. 12 presents the sequences of the 15 amino acid peptides used in the peptide mapping experiment of Example 14.
Fig. 13 presents the results of the blots prepared in Example 14.
Fig. 14 presents the results of the blots prepared in Example 14.
Fig. 15A shows affinity and binding kinetics of Ab1 for IL-6 of various species.
Fig. 15B demonstrates inhibition of IL-6 by Ab1 in the T1165 cell proliferation assay.
Fig. 16. shows the mean plasma concentration of Ab1 resulting from a single administration of Ab1 to healthy male subjects in several dosage groups.
Fig. 17 shows mean area under the plasma Ab1 concentration time curve (AUC) for the dosage groups shown in Fig. 16.
Fig. 18 shows mean peak plasma Ab1 concentration (Cₘₐₓ) for the dosage groups shown in Fig. 16.
Fig. 19 summarizes Ab1 pharmacokinetic measurements of the dosage groups shown in Fig. 16.
Fig. 20 shows the mean plasma concentration of Ab1 resulting from a single administration of Ab1 to patients with advanced cancer.
Fig. 21 illustrates the unprecedented elimination half-life of Ab1 compared with other anti-IL-6 antibodies.
Fig. 22 shows increased hemoglobin concentration following administration of Ab1 to patients with advanced cancer.
Fig. 23 shows mean plasma lipid concentrations following administration of Ab1 to patients with advanced cancer.
Fig. 24 shows mean neutrophil counts following administration of Ab1 to patients with advanced cancer.
Fig. 25 demonstrates suppression of serum CRP levels in healthy individuals.
Fig. 26 (A-B) demonstrates suppression of serum CRP levels in advanced cancer patients.
Fig. 27 shows prevention of weight loss by Ab1 in a mouse cancer cachexia model.
Fig. 28 shows the physical appearance of representative Ab1-treated and control mice in a cancer cachexia model.
Fig. 29 demonstrates that Ab1 promotes weight gain in advanced cancer patients.
Fig. 30 demonstrates that Ab1 reduces fatigue in advanced cancer patients.
Fig. 31 demonstrates that Ab1 promotes hand grip strength in advanced cancer patients.
Fig. 32 demonstrates that Ab1 suppresses an acute phase protein (Serum Amyloid A) in mice.
Fig. 33 demonstrates that Ab1 increase plasma albumin concentration in advanced cancer patients.

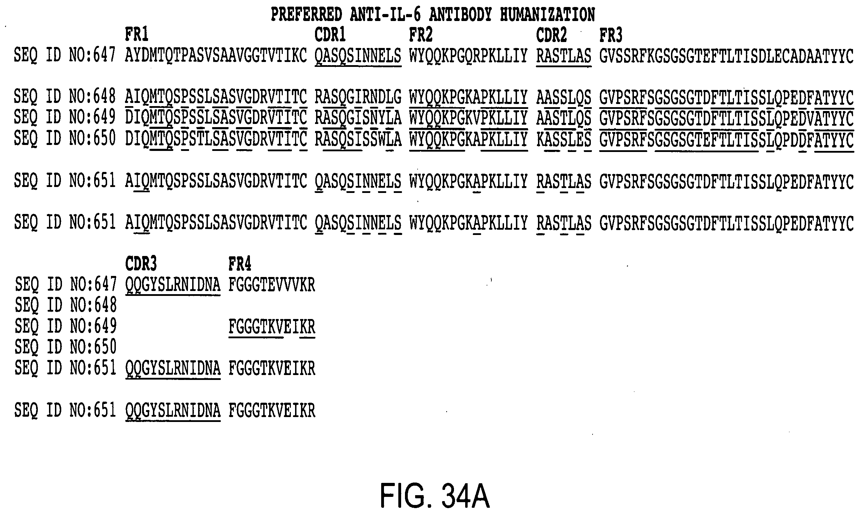

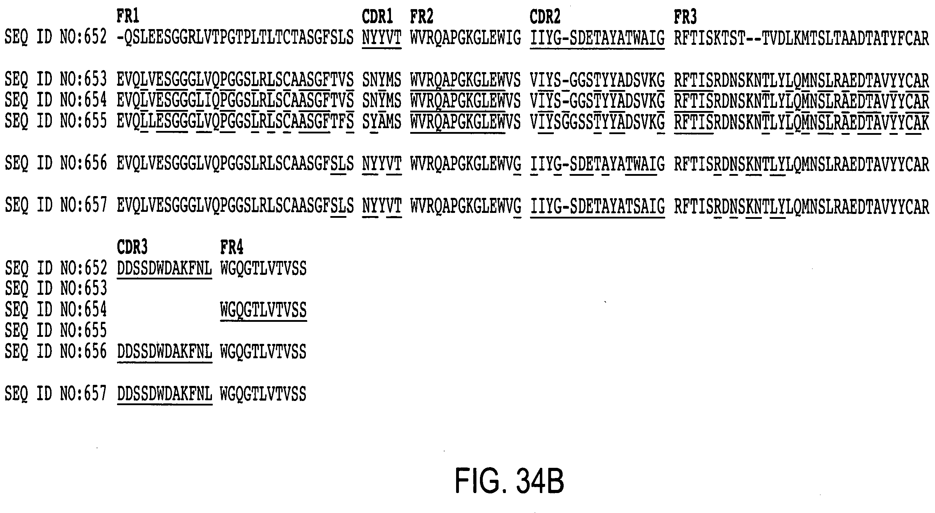

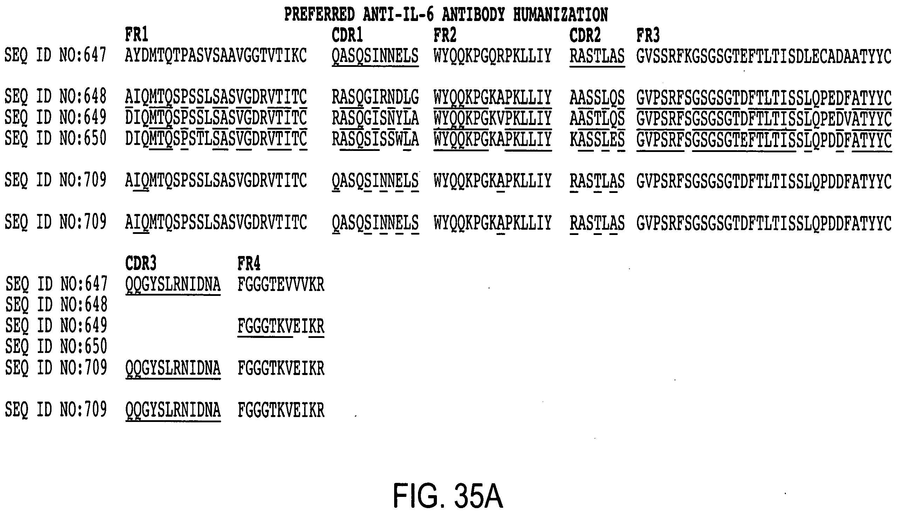

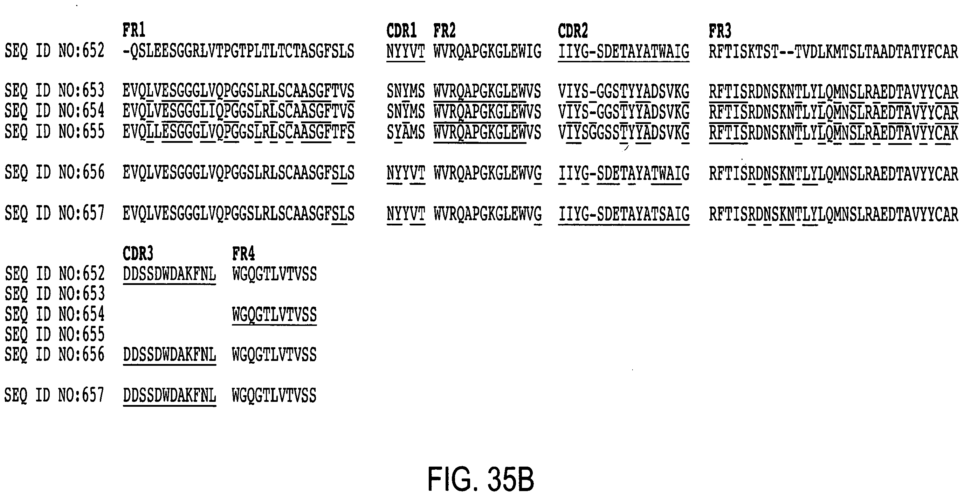

FIGs. 34 and 35 shows alignments between a rabbit antibody light and variable heavy sequences and homologous human sequences and the final humanized sequences. Framework regions are identified FR1-FR4. Complementarity determining regions are identified as CDR1-CDR3.
FIGS. 36 and 37 shows alignments between light and variable heavy sequences, respectively, of different forms of Ab1. Framework regions are identified FR1-FR4. Complementarity determining regions are identified as CDR1-CDR3. Sequence differences within the CDR regions highlighted.
Fig. 38 demonstrates that Ab1 increases mean hemoglobin at 80, 160 and 320 mg after 12 weeks of dosing.
Fig. 39 demonstrates mean change from baseline hemoglobin for the data presented in Fig. 38.
Fig. 40 demonstrates that Ab1 increases mean hemoglobin at 160 and 320 mg after 12 weeks of dosing in patients having baseline hemoglobin below 11 g/l.
Fig. 41 demonstrates that Ab1 increases mean hemoglobin at 80, 160 and 320 mg after 16 weeks of dosing.
Fig. 42 demonstrates the averaged weight change data from each dosage concentration group (placebo, 80 mg, 160 mg, and 320 mg) of the Ab1 monoclonal antibody over 12 weeks.
Fig. 43 demonstrates the averaged percent change in body weight from each dosage concentration group corresponding to Fig. 42.
Fig. 44 demonstrates the change in averaged lean body mass data for the dosage concentration groups corresponding to Fig. 42.
Fig. 45 demonstrates increases in the mean Facit-F FS subscale score for some of the dosage concentration groups in the patient population after dosing at 80, 160 and 320 mg after 8 weeks.
Fig. 46 demonstrates the change from baseline Facit-F FS subscale score corresponding to Fig. 45.

### DETAILED DESCRIPTION OF PREFERRED EMBODIMENTS

### Definitions

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

*Interleukin-6 (IL-6)*: As used herein, interleukin-6 (IL-6) encompasses not only the following 212 amino acid sequence available as GenBank Protein Accession No. NP_000591: MNSFSTSAFGPVAFSLGLLLVLPAAFPAPVPPGEDSKDVAAPHRQPLTSSERID KQIRYLDGISALRKETCNKSNMCESSKEALAENNLNLPKMAEKDGCFQSGFN EETCLVKIITGLLEFEVYLEYLQNRFESSEEQARAVQMSTKVLIQFLQKKAKN LDAITTPDPTTNASLLTKLQAQNQWLQDMTTHLILRSFKEFLQSSLRALRQM (SEQ ID NO: 1), but also any pre-pro, pro- and mature forms of this IL-6 amino acid sequence, as well as mutants and variants including allelic variants of this sequence.

*Disease or condition:* As used herein, "disease or condition" refers to a disease or condition that a patient has been diagnosed with or is suspected of having, particularly a disease or condition associated with elevated IL-6. A disease or condition encompasses, without limitation thereto, the side-effects of medications or treatments (such as radiation therapy), as well as idiopathic conditions characterized by symptoms that include elevated IL-6.

*Cachexia*: As used herein, cachexia, also known as wasting disease, refers to any disease marked especially by progressive emaciation, weakness, general ill health, malnutrition, loss of body mass, loss of muscle mass, or an accelerated loss of skeletal muscle in the context of a chronic inflammatory response (reviewed in Kotler, Ann Intern Med. 2000 Oct 17;133(8):622-34). Diseases and conditions in which cachexia is frequently observed include cancer, rheumatoid arthritis, AIDS, heart disease, dehydration, malnutrition, lead exposure, malaria, respiratory disease, old age, hypothyroidism, tuberculosis, hypopituitarism, neurasthenia, hypernatremia, hyponatremia, renal disease, splenica, ankylosing spondylitis, failure to thrive (faltering growth) and other diseases, particularly chronic diseases. Cachexia may also be idiopathic (arising from an uncertain cause). Weight assessment in a patient is understood to exclude growths or fluid accumulations, e.g. tumor weight, extravascular fluid accumulation, etc. Cachexia may be assessed by measurement of a patient's total body mass (exclusive of growths or fluid accumulations), total lean (fat-free) body mass, lean mass of the arms and legs (appendicular lean mass, e.g. measured using dual-energy x-ray absorptiometry or bioelectric impedance spectroscopy), and/or lean body mass index (lean body mass divided by the square of the patient's height). See Kotler, Ann Intern Med. 2000 Oct 17;133(8):622-34; Marcora et al., Rheumatology (Oxford). 2006 Nov;45(11):1385-8.

*Weakness*: As used herein, weakness refers physical fatigue, which typically manifests as a loss of muscle strength and/or endurance. Weakness may be central (affecting most or all of the muscles in the body) or peripheral (affecting a subset of muscles). Weakness includes "true weakness," in which a patient's muscles have a decrease in some measure of peak and/or sustained force output, and "perceived weakness," in which a patient perceives that a greater effort is required for performance of a task even though objectively measured strength remains nearly the same, and may be objectively measured or self-reported by the patient. For example, weakness may be objectively measured using the hand grip strength test (a medically recognized test for evaluating muscle strength), typically employing a handgrip dynamometer.

*Fatigue*: As used herein, fatigue refers to mental fatigue (for physical fatigue see "weakness"). Fatigue includes drowsiness (somnolence) and/or decreased attention. Fatigue may be measured using a variety of tests known in the art, such as the FACIT-F (Functional Assessment of Chronic Illness Therapy-Fatigue) test. See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.

*Fever*: As used herein, "fever" refers to a body temperature set-point that is elevated by at least 1 to 2 degrees Celsius. Fever is often associated with a subjective feeling of hypothermia exhibited as a cold sensation, shivering, increased heart rate and respiration rate by which the individual's body reaches the increased set-point. As is well understood in the medical arts, normal body temperature typically varies with activity level and time of day, with highest temperatures observed in the afternoon and early evening hours, and lowest temperatures observed during the second half of the sleep cycle, and temperature measurements may be influenced by external factors such as mouth breathing, consumption of food or beverage, smoking, or ambient temperature (depending on the type of measurement). Moreover, the normal temperature set point for individuals may vary by up to about 0.5 degrees Celsius, thus a medical professional may interpret an individual's temperature in view of these factors to diagnose whether a fever is present. Generally speaking, a fever is typically diagnosed by a core body temperature above 38.0 degrees Celsius, an oral temperature above 37.5 degrees Celsius, or an axillary temperature above 37.2 degrees Celsius.

*Improved*: As used herein, "improved," "improvement," and other grammatical variants, includes any beneficial change resulting from a treatment. A beneficial change is any way in which a patient's condition is better than it would have been in the absence of the treatment. "Improved" includes prevention of an undesired condition, slowing the rate at which a condition worsens, delaying the development of an undesired condition, and restoration to an essentially normal condition. For example, improvement in cachexia encompasses any increase in patient's mass, such as total body mass (excluding weight normally excluded during assessment of cachexia, e.g. tumor weight, extravascular fluid accumulation, etc.), lean body mass, and/or appendicular lean mass, as well as any delay or slowing in the rate of loss of mass, or prevention or slowing of loss of mass associated with a disease or condition with which the patient has been diagnosed. For another example, improvement in weakness encompasses any increase in patient's strength, as well as any delay or slowing in the rate of loss of strength, or prevention or slowing of loss of strength associated with a disease or condition with which the patient has been diagnosed. For yet another example, improvement in fatigue encompasses any decrease in patient's fatigue, as well as any delay or slowing in the rate of increase of fatigue, or prevention or slowing of increase in fatigue associated with a disease or condition with which the patient has been diagnosed. For still another example, improvement in fever encompasses any decrease in patient's fever, as well as any delay or slowing in the rate of increase in fever, or prevention or slowing of increase in fever associated with a disease or condition with which the patient has been diagnosed.

*C-Reactive Protein* (*CRP*): As used herein, C-Reactive Protein (CRP) encompasses not only the following 224 amino acid sequence available as GenBank Protein Accession No. NP_000558: MEKLLCFLVLTSLSHAFGQTDMSRKAFVFPKESDTSYVSLKAPLTKPLKAFTVCLHFYTELSSTRGYSIFSYATKRQDNEILIFWSKDIGYSFTVGGSEILFEVPEVTVAPVHICTSWESASGIVEFWVDGKPRVRKSLKKGYTVGAEASIILGQEQDSFGGNFEGSQSLVGDIGNVNMWDFVLSPDEINTIYLGGPFSPNVLNWRALKYEVQGEVFTKPQLWP (SEQ IDNO: 726), but also any pre-pro, pro- and mature forms of this CRP amino acid sequence, as well as mutants and variants including allelic variants of this sequence. CRP levels, e.g. in the serum, liver, tumor, or elsewhere in the body, can be readily measured using routine methods and commercially available reagents, e.g. ELISA, antibody test strip, immunoturbidimetry, rapid immunodiffusion, visual agglutination, Western blot, Northern blot, etc.

*Interleukin-6 receptor (IL-6R); also called IL-6 receptor alpha (IL-6RA)*: As used herein, "interleukin-6 receptor" ("IL-6R"; also "IL-6 receptor alpha" or "IL-6RA") encompasses not only the following 468 amino acid sequence available as Swiss-Prot Protein Accession No. P08887: MLAVGCALLAALLAAPGAALAPRRCPAQEVARGVLTSLPGDSVTLTCPGVEPEDNATVHWVLRKPAAGSHPSRWAGMRRLLLRSVQLHDSGNYSCYRAGRPAGTVHLLVDVPPEEPQLSCFRKSPLSNVVCEWGPRSTPSLTTKAVLLVRKFQNSPAEDFQEPCQYSQESQKFSCQLAVPEGDSSFYIVSMCASSVGSKFSKTQTFQGCGILQPDPPANITVTAVARNPRWLSVTWQDPHSWNSSFYRLRFELRYRAERSKTFTTWMVKDLQHHCVIHDAWSGLRHVVQLRAQEEFGQGEWSEWSPEAMGTPWTESRSPPAENEVSTPMQALTTNKDDDNILFRDSANATSLPVQDSSSVPLPTFLVAGGSLAFGGSLAFGTLLCIAIVLRFKKTWKLRALKEGKTSMHPPYSLGQLVPERPRPTPVLVPLISPPVSPSSLGSDNTSSHNRPDARDPRSPYDISNTDYFFPR (SEQ)ID NO: 727), but also any pre-pro, pro- and mature forms of this amino acid sequence, as well as mutants and variants including allelic variants of this sequence.

*gp130:* As used herein, gp130 (also called Interleukin-6 receptor subunit beta) encompasses not only the following 918 precursor amino acid sequence available as Swiss-Prot Protein Accession No. P40189: MLTLQTWVVQALFIFLTTESTGELLDPCGYISPESPVVQLHSNFTAVCVLKEKCMDYFHVNANYIVQKTNHFTIPKEQYTIINRTASSVTFTDIASLNIQLTCNILTFGQLEQNVYGITIISGLPPEKPKNLSCIVNEGKKMRCEWDGGRETHLETNFTLKSEWATHKFADCKAKRDTPTSCTVDYSTVYFNIEVWEAENALGKVTSDHINFDPVYKVKPNPPHNLSVINSEELSSILKLTWTNPSIKSVIILKYNIQYRTDASTWSQIPPEDTASTRSSFTVQDLKPFTEYVFRIRCMKEDGKGYWSDWSEEASGITYEDRPSKAPSFWYKIDPSHTQGYRTVQLVWKTLPPFEANGKILDYEVTLTRWKSHLQNYTVNATKLTVNLTNDYLATLTVRNLVGKSDAAVLTIPACDFQATHPVMDLKAFPKDNMLWVEWTTPRESVKKYILEWCVLSDKAPCITDWQQEDGTVHRTYLRGNLAESKCYLITVTPVYADGPGSPESIKAYLKQAPPSKGPTVRTKKVGKNEAVLEWDQLPVDVQNGFIRNYTIFYRTIIGNETAVNVDSSHTEYTLS SLTSDTLYMVRMAAYTDEGGKDGPEFTFTTPKFAQGEIEAIVVPVCLAFLLTTLLGVLFCFNKRDLIKKHIWPNVPDPSKSHIAQWSPHTPPRHNFNSKDQMYSDGNFTDVSVVEIEANDKKPFPEDLKSLDLFKKEKINTEGHSSGIGGSSCMSSSRPSISSSDENESSQNTSSTVQNTSSTVQYSTVVHSGYRHQVPSVQVFSRSESTQPLLDSEERPEDLQLVDHVDGGDGILPRQQYFKQNCSQHESSPDISHFERSKQVSSVNEEDFVRLKQQISDHISQSCGSQMKMFQEVSAADAFGPGTEGQVERFETVGMEAATDEGMPKSYLPQTVRQGGYMPQ (SEQ ID NO: 728), but also any pre-pro, pro- and mature forms of this amino acid sequence, such as the mature form encoded by amino acids 23 through 918 of the sequence shown, as well as mutants and variants including allelic variants of this sequence.

*Glasgow Prognostic Score* (*GPS*): As used herein, Glasgow Prognostic Score (GPS) refers to an inflammation-based prognostic score that awards one point for a serum albumin level less than < 35 mg/L and one point for a CRP level above 10 mg/L. Thus, a GPS of 0 indicates normal albumin and CRP, a GPS of 1 indicates reduced albumin or elevated CRP, and a GPS of 2 indicates both reduced albumin and elevated CRP.

*Effective amount*: As used herein, "effective amount," "amount effective to," and "amount of X effective to", refer to an amount of an active ingredient that is effective to relieve or reduce to some extent one or more of the symptoms of the disease in need of treatment, or to retard initiation of clinical markers or symptoms of a disease in need of prevention, when the compound is administered. Thus, an effective amount refers to an amount of the active ingredient which exhibit effects such as (i) reversing the rate of progress of a disease; (ii) inhibiting to some extent further progress of the disease; and/or, (iii) relieving to some extent (or, preferably, eliminating) one or more symptoms associated with the disease. The effective amount may be empirically determined by experimenting with the compounds concerned in known in vivo and in vitro model systems for a disease in need of treatment. The context in which the phrase "effective amount" is used may indicate a particular desired effect. For example, "an amount of an anti-IL-6 antibody effective to reduce weakness" and similar phrases refer to an amount of anti-IL-6 antibody that, when administered to a subject, will cause a measurable decrease in weakness as determined by the hand grip strength test. Similarly, "an amount of an anti-IL-6 antibody effective to increase weight" and similar phrases refer to an amount of anti-IL-6 antibody that, when administered to a subject, will cause a measurable increase in a patient's weight. An effective amount will vary according to the weight, sex, age and medical history of the individual, as well as the severity of the patient's condition(s), the type of disease(s), and mode of administration,. An effective amount may be readily determined using routine experimentation, e.g., by titration (administration of increasing dosages until an effective dosage is found) and/or by reference to amounts that were effective for prior patients. Generally, the anti-IL-6 antibodies used in the present invention will be administered in dosages ranging between about 0.1 mg/kg and about 20 mg/kg of the patient's body-weight.

*Prolonged improvement in cachexia*: As used herein, "prolonged improvement in cachexia" refers to a measureable improvement patient's body mass, lean body mass, appendicular lean body mass, and/or lean body mass index, relative to the initial level (i.e. the level at a time before treatment begins) that is detectable within about 4 weeks and remains improved for a prolonged duration, e.g. at least about 35 days, at least about 40 days, at least about 50 days, at least about 60 days, at least about 70 days, at least about 11 weeks, or at least about 12 weeks from when the treatment begins.

*Prolonged improvement in weakness*: As used herein, "prolonged improvement in weakness" refers to a measureable improvement in muscular strength, relative to the initial level (i.e. the level at a time before treatment begins) that is detectable within about 2 weeks and remains improved for a prolonged duration, e.g. at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged improvement in fatigue*: As used herein, "prolonged improvement in fatigue" refers to a measureable improvement in fatigue, relative to the initial level (i.e. the level at a time before treatment begins) that is detectable within about 1 week and remains improved for a prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged improvement in fever*: As used herein, "prolonged improvement in fever" refers to a measureable decrease in fever (e.g. peak temperature or amount of time that temperature is elevated), relative to the initial level (i.e. the level at a time before treatment begins) that is detectable within about 1 week and remains improved for a prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Mating competent yeast species:* This is intended to broadly encompass any diploid or tetraploid yeast which can be grown in culture. Such species of yeast may exist in a haploid, diploid, or tetraploid form. The cells of a given ploidy may, under appropriate conditions, proliferate for indefinite number of generations in that form. Diploid cells can also sporulate to form haploid cells. Sequential mating can result in tetraploid strains through further mating or fusion of diploid strains. Diploid or polyploidal yeast cells are preferably produced by mating or spheroplast fusion.

The mating competent yeast may be a member of the *Saccharomycetaceae* family, which includes the genera *Arxiozyma*; *Ascobotryozyma*; *Citeromyces*; *Debaryomyces*; *Dekkera*; *Eremothecium; Issatchenkia*; *Kazachstania*; *Kluyveromyces; Kodamaea*; *Lodderomyces; Pachysolen*; *Pichia; Saccharomyces; Saturnispora*; *Tetrapisispora*; *Torulaspora*; *Williopsis*; and *Zygosaccharomyces.* Other types of yeast potentially useful in the invention include *Yarrowia, Rhodosporidium, Candida, Hansenula, Filobasium, Filobasidellla, Sporidiobolus, Bullera, Leucosporidium* and *Filobasidella.*

The mating competent yeast may be a member of the genus *Pichia.* such as *Pichia pastoris*, *Pichia methanolica,* and *Hansenula polymorpha* (*Pichia angusta*).

*Haploid Yeast Cell*: A cell having a single copy of each gene of its normal genomic (chromosomal) complement.

*Polyploid Yeast Cell*: A cell having more than one copy of its normal genomic (chromosomal) complement.

*Diploid Yeast Cell*: A cell having two copies (alleles) of essentially every gene of its normal genomic complement, typically formed by the process of fusion (mating) of two haploid cells.

*Tetraploid Yeast Cell*: A cell having four copies (alleles) of essentially every gene of its normal genomic complement, typically formed by the process of fusion (mating) of two haploid cells. Tetraploids may carry two, three, four, or more different expression cassettes. Such tetraploids might be obtained in *S. cerevisiae* by selective mating homozygotic heterothallic a/a and alpha/alpha diploids and in *Pichia* by sequential mating of haploids to obtain auxotrophic diploids. For example, a [met his] haploid can be mated with [ade his] haploid to obtain diploid [his]; and a [met arg] haploid can be mated with [ade arg] haploid to obtain diploid [arg]; then the diploid [his] x diploid [arg] to obtain a tetraploid prototroph. It will be understood by those of skill in the art that reference to the benefits and uses of diploid cells may also apply to tetraploid cells.

*Yeast Mating*: The process by which two haploid yeast cells naturally fuse to form one diploid yeast cell.

*Meiosis*: The process by which a diploid yeast cell undergoes reductive division to form four haploid spore products. Each spore may then germinate and form a haploid vegetatively growing cell line.

*Selectable Marker*: A selectable marker is a gene or gene fragment that confers a growth phenotype (physical growth characteristic) on a cell receiving that gene as, for example through a transformation event. The selectable marker allows that cell to survive and grow in a selective growth medium under conditions in which cells that do not receive that selectable marker gene cannot grow. Selectable marker genes generally fall into several types, including positive selectable marker genes such as a gene that confers on a cell resistance to an antibiotic or other drug, temperature when two ts mutants are crossed or a ts mutant is transformed; negative selectable marker genes such as a biosynthetic gene that confers on a cell the ability to grow in a medium without a specific nutrient needed by all cells that do not have that biosynthetic gene, or a mutagenized biosynthetic gene that confers on a cell inability to grow by cells that do not have the wild type gene. Suitable markers include but are not limited to: ZEO; G418; LYS3; MET1; MET3a; ADE1; ADE3 and URA3.

*Expression Vector*: These DNA vectors contain elements that facilitate manipulation for the expression of a foreign protein within the target host cell. Conveniently, manipulation of sequences and production of DNA for transformation is first performed in a bacterial host, *e.g. E. coli,* and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, (b) complement auxotrophic deficiencies, or (c) supply critical nutrients not available from complex media. Exemplary vectors and methods for transformation of yeast are described, for example, in Burke, D., Dawson, D., & Stearns, T. (2000). Methods in yeast genetics: a Cold Spring Harbor Laboratory course manual. Plainview, N.Y.: Cold Spring Harbor Laboratory Press.

Expression vectors will further include yeast specific sequences, including a selectable auxotrophic or drug marker for identifying transformed yeast strains. A drug marker may further be used to amplify copy number of the vector in a yeast host cell.

The polypeptide coding sequence of interest is operably linked to transcriptional and translational regulatory sequences that provide for expression of the polypeptide in yeast cells. These vector components may include, but are not limited to, one or more of the following: an enhancer element, a promoter, and a transcription termination sequence. Sequences for the secretion of the polypeptide may also be included, e.g. a signal sequence. A yeast origin of replication is optional, as expression vectors are often integrated into the yeast genome.

A polypeptide of interest may be operably linked, or fused, to sequences providing for optimized secretion of the polypeptide from yeast diploid cells.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites or alternatively via a PCR/recombination method familiar to those skilled in the art (Gateway^{R} Technology; Invitrogen, Carlsbad California). If such sites do not exist, the synthetic oligonucleotide adapters or linkers are used in accordance with conventional practice.

Promoters are untranslated sequences located upstream (5') to the start codon of a structural gene (generally within about 100 to 1000 bp) that control the transcription and translation of particular nucleic acid sequences to which they are operably linked. Such promoters fall into several classes: inducible, constitutive, and repressible promoters (that increase levels of transcription in response to absence of a repressor). Inducible promoters may initiate increased levels of transcription from DNA under their control in response to some change in culture conditions, e.g., the presence or absence of a nutrient or a change in temperature.

The yeast promoter fragment may also serve as the site for homologous recombination and integration of the expression vector into the same site in the yeast genome; alternatively a selectable marker is used as the site for homologous recombination. *Pichia* transformation is described in Cregg et al. (1985) Mol. Cell. Biol. 5:3376-3385.

Examples of suitable promoters from *Pichia* include the AOX1 and promoter (Cregg et al. (1989) Mol. Cell. Biol. 9:1316-1323); ICL1 promoter (Menendez et al. (2003) Yeast 20(13):1097-108); glyceraldehyde-3-phosphate dehydrogenase promoter (GAP) (Waterham et al. (1997) Gene 186(1):37-44); and FLD1 promoter (Shen et al. (1998) Gene 216(1):93-102). The *GAP* promoter is a strong constitutive promoter and the AOX and FLD1 promoters are inducible.

Other yeast promoters include ADH1, alcohol dehydrogenase II, GAL4, PHO3, PHO5, Pyk, and chimeric promoters derived therefrom. Additionally, non-yeast promoters may be used in the invention such as mammalian, insect, plant, reptile, amphibian, viral, and avian promoters. Most typically the promoter will comprise a mammalian promoter (potentially endogenous to the expressed genes) or will comprise a yeast or viral promoter that provides for efficient transcription in yeast systems.

The polypeptides of interest may be produced recombinantly not only directly, but also as a fusion polypeptide with a heterologous polypeptide, *e.g.* a signal sequence or other polypeptide having a specific cleavage site at the N-terminus of the mature protein or polypeptide. In general, the signal sequence may be a component of the vector, or it may be a part of the polypeptide coding sequence that is inserted into the vector. The heterologous signal sequence selected preferably is one that is recognized and processed through one of the standard pathways available within the host cell. The *S. cerevisiae* alpha factor pre-pro signal has proven effective in the secretion of a variety of recombinant proteins from *P. pastoris.* Other yeast signal sequences include the alpha mating factor signal sequence, the invertase signal sequence, and signal sequences derived from other secreted yeast polypeptides. Additionally, these signal peptide sequences may be engineered to provide for enhanced secretion in diploid yeast expression systems. Other secretion signals of interest also include mammalian signal sequences, which may be heterologous to the protein being secreted, or may be a native sequence for the protein being secreted. Signal sequences include pre-peptide sequences, and in some instances may include propeptide sequences. Many such signal sequences are known in the art, including the signal sequences found on immunoglobulin chains, e.g., K28 preprotoxin sequence, PHA-E, FACE, human MCP-1, human serum albumin signal sequences, human Ig heavy chain, and human Ig light chain. For example, see Hashimoto et. al. Protein Eng 11(2) 75 (1998); and Kobayashi et. al. Therapeutic Apheresis 2(4) 257 (1998).

Transcription may be increased by inserting a transcriptional activator sequence into the vector. These activators are cis-acting elements of DNA, usually about from 10 to 300 bp, which act on a promoter to increase its transcription. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter.

Expression vectors used in eukaryotic host cells may also contain sequences necessary for the termination of transcription and for stabilizing the mRNA. Such sequences are commonly available from 3' to the translation termination codon, in untranslated regions of eukaryotic or viral DNAs or cDNAs. These regions contain nucleotide segments transcribed as polyadenylated fragments in the untranslated portion of the mRNA.

Construction of suitable vectors containing one or more of the above-listed components employs standard ligation techniques or PCR/recombination methods. Isolated plasmids or DNA fragments are cleaved, tailored, and re-ligated in the form desired to generate the plasmids required or via recombination methods. For analysis to confirm correct sequences in plasmids constructed, the ligation mixtures are used to transform host cells, and successful transformants selected by antibiotic resistance (e.g. ampicillin or Zeocin™ (phleomycin)) where appropriate. Plasmids from the transformants are prepared, analyzed by restriction endonuclease digestion and/or sequenced.

As an alternative to restriction and ligation of fragments, recombination methods based on att sites and recombination enzymes may be used to insert DNA sequences into a vector. Such methods are described, for example, by Landy (1989) Ann.Rev.Biochem. 58:913-949; and are known to those of skill in the art. Such methods utilize intermolecular DNA recombination that is mediated by a mixture of lambda and *E.coli* -encoded recombination proteins. Recombination occurs between specific attachment (*att*) sites on the interacting DNA molecules. For a description of att sites see Weisberg and Landy (1983) Site-Specific Recombination in Phage Lambda, in Lambda II, Weisberg, ed.(Cold Spring Harbor, NY:Cold Spring Harbor Press), pp.211-250. The DNA segments flanking the recombination sites are switched, such that after recombination, the *att* sites are hybrid sequences comprised of sequences donated by each parental vector. The recombination can occur between DNAs of any topology.

*Att* sites may be introduced into a sequence of interest by ligating the sequence of interest into an appropriate vector; generating a PCR product containing *att* B sites through the use of specific primers; and generating a cDNA library cloned into an appropriate vector containing att sites.

*Folding,* as used herein, refers to the three-dimensional structure of polypeptides and proteins, where interactions between amino acid residues act to stabilize the structure. While non-covalent interactions are important in determining structure, usually the proteins of interest will have intra- and/or intermolecular covalent disulfide bonds formed by two cysteine residues. For naturally occurring proteins and polypeptides or derivatives and variants thereof, the proper folding is typically the arrangement that results in optimal biological activity, and can conveniently be monitored by assays for activity, *e.g.* ligand binding, enzymatic activity, *etc.*

In some instances, for example where the desired product is of synthetic origin, assays based on biological activity will be less meaningful. The proper folding of such molecules may be determined on the basis of physical properties, energetic considerations or modeling studies.

The expression host may be further modified by the introduction of sequences encoding one or more enzymes that enhance folding and disulfide bond formation, *i.e.* foldases, chaperonins, *etc.* Such sequences may be constitutively or inducibly expressed in the yeast host cell, using vectors, markers, *etc.* as known in the art. The sequences, including transcriptional regulatory elements sufficient for the desired pattern of expression, may be stably integrated in the yeast genome through a targeted methodology.

For example, the eukaryotic PDI is not only an efficient catalyst of protein cysteine oxidation and disulfide bond isomerization, but also exhibits chaperone activity. Co-expression of PDI can facilitate the production of active proteins having multiple disulfide bonds. Also of interest is the expression of BIP (immunoglobulin heavy chain binding protein) and cyclophilin. Each of the haploid parental strains may express a distinct folding enzyme, *e.g.* one strain may express BIP, and the other strain may express PDI or combinations thereof.

The terms "*desired protein*" or "*target protein*" are used interchangeably and refer generally to a humanized antibody or a binding portion thereof described herein. The term "antibody" is intended to include any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." Numerous antibody coding sequences have been described; and others may be raised by methods well-known in the art. Examples thereof include chimeric antibodies, human antibodies and other non-human mammalian antibodies, humanized antibodies, single chain antibodies such as scFvs, camelbodies, nanobodies, IgNAR (single-chain antibodies derived from sharks), small-modular immunopharmaceuticals (SMIPs), and antibody fragments such as Fabs, Fab', F(ab')₂. *See* Streltsov VA, et al., Structure of a shark IgNAR antibody variable domain and modeling of an early-developmental isotype, Protein Sci. 2005 Nov;14(11):2901-9. Epub 2005 Sep 30; Greenberg AS, et al., A new antigen receptor gene family that undergoes rearrangement and extensive somatic diversification in sharks, Nature. 1995 Mar 9;374(6518):168-73; Nuttall SD, et al., Isolation of the new antigen receptor from wobbegong sharks, and use as a scaffold for the display of protein loop libraries, Mol Immunol. 2001 Aug;38(4):313-26; Hamers-Casterman C, et al., Naturally occurring antibodies devoid of light chains, Nature. 1993 Jun 3;363(6428):446-8; Gill DS, et al., Biopharmaceutical drug discovery using novel protein scaffolds, Curr Opin Biotechnol. 2006 Dec;17(6):653-8. Epub 2006 Oct 19.

For example, antibodies or antigen binding fragments may be produced by genetic engineering. In this technique, as with other methods, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid (aa) substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (*e.g.,* a functional domain, catalytic amino acid residues, *etc*)*.* Variants also include fragments of the polypeptides disclosed herein, particularly biologically active fragments and/or fragments corresponding to functional domains. Techniques for in vitro mutagenesis of cloned genes are known. Also included in the subject invention are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (V_{L} and V_{H}), obtained from antibody producing cells of one species with the constant light and heavy chain regions from another. Typically chimeric antibodies utilize rodent or rabbit variable regions and human constant regions, in order to produce an antibody with predominantly human domains. The production of such chimeric antibodies is well known in the art, and may be achieved by standard means (as described, e.g., in U.S. Patent No. 5,624,659). It is further contemplated that the human constant regions of chimeric antibodies of the invention may be selected from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19 constant regions.

Humanized antibodies are engineered to contain even more human-like immunoglobulin domains, and incorporate only the complementarity-determining regions of the animal-derived antibody. This is accomplished by carefully examining the sequence of the hyper-variable loops of the variable regions of the monoclonal antibody, and fitting them to the structure of the human antibody chains. Although facially complex, the process is straightforward in practice. See, e.g., U.S. Patent No. 6,187,287.

In addition to entire immunoglobulins (or their recombinant counterparts), immunoglobulin fragments comprising the epitope binding site (e.g., Fab', F(ab')₂, or other fragments) may be synthesized. "Fragment," or minimal immunoglobulins may be designed utilizing recombinant immunoglobulin techniques. For instance "Fv" immunoglobulins for use in the present invention may be produced by synthesizing a fused variable light chain region and a variable heavy chain region. Combinations of antibodies are also of interest, *e.g.* diabodies, which comprise two distinct Fv specificities. SMIPs (small molecule immunopharmaceuticals), camelbodies, nanobodies, and IgNAR are encompassed by immunoglobulin fragments.

Immunoglobulins and fragments thereof may be modified post-translationally, *e.g.* to add effector moieties such as chemical linkers, detectable moieties, such as fluorescent dyes, enzymes, toxins, substrates, bioluminescent materials, radioactive materials, and chemiluminescent moieties, or specific binding moieties, such as streptavidin, avidin, or biotin may be utilized in the methods and compositions of the present invention. Examples of additional effector molecules are provided *infra.*

The term "polyploid yeast that stably expresses or expresses a desired secreted heterologous polypeptide for prolonged time" refers to a yeast culture that secretes said polypeptide for at least several days to a week, more preferably at least a month, still more preferably at least 1-6 months, and even more preferably for more than a year at threshold expression levels, typically at least 10-25 mg/liter and preferably substantially greater.

The term "polyploidal yeast culture that secretes desired amounts of recombinant polypeptide" refers to cultures that stably or for prolonged periods secrete at least 10-25 mg/liter of heterologous polypeptide, more preferably at least 50-500 mg/liter, and most preferably 500-1000 mg/liter or more.

A polynucleotide sequence "corresponds" to a polypeptide sequence if translation of the polynucleotide sequence in accordance with the genetic code yields the polypeptide sequence (i.e., the polynucleotide sequence "encodes" the polypeptide sequence), one polynucleotide sequence "corresponds" to another polynucleotide sequence if the two sequences encode the same polypeptide sequence.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "coding sequence" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide. A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism or host cell. Typical vectors include recombinant viruses (for polynucleotides) and liposomes or other lipid aggregates (for polypeptides and/or polynucleotides). A "DNA vector" is a replicon, such as plasmid, phage or cosmid, to which another polynucleotide segment may be attached so as to bring about the replication of the attached segment. An "expression vector" is a DNA vector which contains regulatory sequences which will direct polypeptide synthesis by an appropriate host cell. This usually means a promoter to bind RNA polymerase and initiate transcription of mRNA, as well as ribosome binding sites and initiation signals to direct translation of the mRNA into a polypeptide(s). Incorporation of a polynucleotide sequence into an expression vector at the proper site and in correct reading frame, followed by transformation of an appropriate host cell by the vector, enables the production of a polypepide encoded by said polynucleotide sequence. Exemplary expression vectors and techniques for their use are described in the following publications: Old et al., Principles of Gene Manipulation: An Introduction to Genetic Engineering, Blackwell Scientific Publications, 4th edition, 1989; Sambrook et al., Molecular Cloning: A Laboratory Manual, 2nd Edition, Cold Spring Harbor Laboratory Press, 1989; Sambrook et al., Molecular Cloning: A Laboratory Manual, 3rd Edition, Cold Spring Harbor Laboratory Press, 2001; Gorman, "High Efficiency Gene Transfer into Mammalian Cells," in DNA Cloning, Volume II, Glover, D. M., Ed., IRL Press, Washington, D.C., pp. 143 190 (1985).

For example, a liposomes or other lipid aggregate may comprise a lipid such as phosphatidylcholines (lecithins) (PC), phosphatidylethanolamines (PE), lysolecithins, lysophosphatidylethanolamines, phosphatidylserines (PS), phosphatidylglycerols (PG), phosphatidylinositol (PI), sphingomyelins, cardiolipin, phosphatidic acids (PA), fatty acids, gangliosides, glucolipids, glycolipids, mono-, di or triglycerides, ceramides, cerebrosides and combinations thereof; a cationic lipid (or other cationic amphiphile) such as 1,2-dioleyloxy-3-(trimethylamino) propane (DOTAP); N-cholesteryloxycarbaryl-3,7,12-triazapentadecane-1,15-diamine (CTAP); N-[1-(2,3,-ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3 beta [N-(N',N'-dimethylaminoethane)carbamoly] cholesterol (DC-Choi); and dimethyldioctadecylammonium (DDAB); dioleoylphosphatidyl ethanolamine (DOPE), cholesterol-containing DOPC; and combinations thereof; and/or a hydrophilic polymer such as polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide and combinations thereof. Other suitable cationic lipids are described in Miller, Angew. Chem. Int. Ed. 37:1768 1785 (1998), and Cooper et al., Chem. Eur. J. 4(1): 137 151 (1998). Liposomes can be crosslinked, partially crosslinked, or free from crosslinking. Crosslinked liposomes can include crosslinked as well as non-crosslinked components. Suitable cationic liposomes or cytofectins are commercially available and can also be prepared as described in Sipkins et al., Nature Medicine, 1998, 4(5):(1998), 623 626 or as described in Miller, supra. Exemplary liposomes includes a polymerizable zwitterionic or neutral lipid, a polymerizable integrin targeting lipid and a polymerizable cationic lipid suitable for binding a nucleic acid. Liposomes can optionally include peptides that provide increased efficiency, for example as described in U.S. Pat. No. 7,297,759. Additional exemplary liposomes and other lipid aggregates are described in U.S. Pat. No. 7,166,298.

"Amplification" of polynucleotide sequences is the *in vitro* production of multiple copies of a particular nucleic acid sequence. The amplified sequence is usually in the form of DNA. A variety of techniques for carrying out such amplification are described in a review article by Van Brunt (1990, Bio/Technol., 8(4):291-294). Polymerase chain reaction or PCR is a prototype of nucleic acid amplification, and use of PCR herein should be considered exemplary of other suitable amplification techniques.

The general structure of antibodies in vertebrates now is well understood (Edelman, G. M., Ann. N.Y. Acad. Sci., 190: 5 (1971)). Antibodies consist of two identical light polypeptide chains of molecular weight approximately 23,000 daltons (the "light chain"), and two identical heavy chains of molecular weight 53,000-70,000 (the "heavy chain"). The four chains are joined by disulfide bonds in a "Y" configuration wherein the light chains bracket the heavy chains starting at the mouth of the "Y" configuration. The "branch" portion of the "Y" configuration is designated the F_{ab} region; the stem portion of the "Y" configuration is designated the F_{C} region. The amino acid sequence orientation runs from the N-terminal end at the top of the "Y" configuration to the C-terminal end at the bottom of each chain. The N-terminal end possesses the variable region having specificity for the antigen that elicited it, and is approximately 100 amino acids in length, there being slight variations between light and heavy chain and from antibody to antibody.

The variable region is linked in each chain to a constant region that extends the remaining length of the chain and that within a particular class of antibody does not vary with the specificity of the antibody (i.e., the antigen eliciting it). There are five known major classes of constant regions that determine the class of the immunoglobulin molecule (IgG, IgM, IgA, IgD, and IgE corresponding to γ, µ, α, δ, and ε (gamma, mu, alpha, delta, or epsilon) heavy chain constant regions). The constant region or class determines subsequent effector function of the antibody, including activation of complement (Kabat, E. A., Structural Concepts in Immunology and Immunochemistry, 2nd Ed., p. 413-436, Holt, Rinehart, Winston (1976)), and other cellular responses (Andrews, D. W., et al., Clinical Immunobiology, pp 1-18, W. B. Sanders (1980); Kohl, S., et al., Immunology, 48: 187 (1983)); while the variable region determines the antigen with which it will react. Light chains are classified as either κ (kappa) or λ (lambda). Each heavy chain class can be paired with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

The expression "variable region" or "VR" refers to the domains within each pair of light and heavy chains in an antibody that are involved directly in binding the antibody to the antigen. Each heavy chain has at one end a variable domain (V_{H}) followed by a number of constant domains. Each light chain has a variable domain (V_{L}) at one end and a constant domain at its other end; the constant domain of the light chain is aligned with the first constant domain of the heavy chain, and the light chain variable domain is aligned with the variable domain of the heavy chain.

The expressions "complementarity determining region," "hypervariable region," or "CDR" refer to one or more of the hyper-variable or complementarity determining regions (CDRs) found in the variable regions of light or heavy chains of an antibody (*See* Kabat, E. A. et al., Sequences of Proteins of Immunological Interest, National Institutes of Health, Bethesda, Md., (1987)). These expressions include the hypervariable regions as defined by Kabat et al. ("Sequences of Proteins of Immunological Interest," Kabat E., et al., US Dept. of Health and Human Services, 1983) or the hypervariable loops in 3-dimensional structures of antibodies (Chothia and Lesk, J Mol. Biol. 196 901-917 (1987)). The CDRs in each chain are held in close proximity by framework regions and, with the CDRs from the other chain, contribute to the formation of the antigen binding site. Within the CDRs there are select amino acids that have been described as the selectivity determining regions (SDRs) which represent the critical contact residues used by the CDR in the antibody-antigen interaction (Kashmiri, S., Methods, 36:25-34 (2005)).

The expressions "framework region" or "FR" refer to one or more of the framework regions within the variable regions of the light and heavy chains of an antibody (*See* Kabat, E. A. et al., Sequences of Proteins of Immunological Interest, National Institutes of Health, Bethesda, Md., (1987)). These expressions include those amino acid sequence regions interposed between the CDRs within the variable regions of the light and heavy chains of an antibody.

### Anti-IL-6 Antibodies and Binding Fragments Thereof

The invention utilises antibodies having binding specificity to IL-6 and possessing a variable light chain sequence comprising the sequence set forth below:

The invention also utilises antibodies having binding specificity to IL-6 and possessing a variable heavy chain sequence comprising the sequence set forth below:

The invention further contemplates antibodies comprising the polypeptide sequences of SEQ ID NO: 4; SEQ ID NO: 5; and SEQ ID NO: 6 which correspond to the complementarity-determining regions (CDRs, or hypervariable regions) of the variable light chain sequence of SEQ ID NO: 2 or 709, and the polypeptide sequences of SEQ ID NO: 7; SEQ ID NO: 8 or SEQ ID NO: 120; and SEQ ID NO: 9 which correspond to the complementarity-determining regions (CDRs, or hypervariable regions) of the variable heavy chain sequence of SEQ ID NO: 3 or 657, or combinations of these polypeptide sequences.

The invention also contemplates fragments of the antibody having binding specificity to IL-6. In one embodiment of the invention, antibody fragments for use in the invention comprise, or alternatively consist of, the polypeptide sequence of SEQ ID NO: 2 or 657. In another embodiment of the invention, antibody fragments of the invention comprise, or alternatively consist of, the polypeptide sequence of SEQ ID NO: 3 or 709.

In a further embodiment of the invention, fragments of the antibody having binding specificity to IL-6 comprise the polypeptide sequences of SEQ ID NO: 4; SEQ ID NO: 5; and SEQ ID NO: 6 which correspond to the complementarity-determining regions (CDRs, or hypervariable regions) of the variable light chain sequence of SEQ ID NO: 2 or SEQ ID NO:709.

In a further embodiment of the invention, fragments of the antibody having binding specificity to IL-6 comprise the polypeptide sequences of SEQ ID NO: 7; SEQ ID NO: 8; and SEQ ID NO: 9 which correspond to the complementarity-determining regions (CDRs, or hypervariable regions) of the variable heavy chain sequence of SEQ ID NO: 3 and SEQ ID NO:657 .

The invention also contemplates variants wherein either of the heavy chain polypeptide sequences of SEQ ID NO: 18 or SEQ ID NO: 19 is substituted for the heavy chain polypeptide sequence of SEQ ID NO: 3 or SEQ ID NO:657; the light chain polypeptide sequence of SEQ ID NO: 20 is substituted for the light chain polypeptide sequence of SEQ ID NO: 2 or SEQ ID NO:709; and the heavy chain CDR sequence of SEQ ID NO: 120 is substituted for the heavy chain CDR sequence of SEQ ID NO: 8.

In a preferred embodiment of the invention, the anti-IL-6 antibody is Ab1, comprising SEQ ID NO: 2 and SEQ ID NO: 3, or an antibody comprising SEQ ID NO:657 and SEQ ID NO:709 (which are respectively encoded by the nucleic acid sequences in SEQ ID NO:700 and SEQ ID NO:723) or one comprised of the alternative SEQ ID NOs set forth in the preceding paragraph.

In a preferred embodiment of the invention, the anti-IL-6 antibody is Ab13, comprising SEQ ID NO: 202 and SEQ ID NO: 203, and having at least one of the biological activities set forth herein.

In another embodiment, the invention contemplates other antibodies, such as for example chimeric or humanized antibodies, comprising the polypeptide sequences of SEQ ID NO: 4; SEQ ID NO: 5; and SEQ ID NO: 6 which correspond to the complementarity-determining regions (CDRs, or hypervariable regions) of the variable light chain sequence of SEQ ID NO: 2, and/ the polypeptide sequences of SEQ ID NO: 7 (CDR1) ; SEQ ID NO: 8 (CDR2) or SEQ ID NO: 120 (CDR2); and SEQ ID NO: 9 (CDR3) which correspond to the complementarity-determining regions (CDRs, or hypervariable regions) of the variable heavy chain sequence of SEQ ID NO: 3 or SEQ ID NO: 19, or combinations of these polypeptide sequences.

In another embodiment the invention is directed to amino acid sequences corresponding to the CDRs of said anti-IL-6 antibody which are SEQ ID NO: 4 (CDR1), SEQ ID NO: 5 (CDR2), SEQ ID NO: 6 (CDR3), SEQ ID NO: 7, SEQ ID NO: 120 and SEQ ID NO: 9.

In another embodiment the anti-IL-6 antibody of the invention comprises a light chain nucleic acid sequence of SEQ ID NO: 723; and a heavy chain nucleic acid sequence of SEQ ID NO: 700. In addition the invention is directed to the corresponding polypeptides encoded by any of the foregoing nucleic acid sequences and combinations thereof.

There are described nucleic acid constructs containing nucleic acid sequences encoding antibodies for use in the invention as well as recombinant cells containing these nucleic acid sequences and constructs containing wherein these nucleic acid sequences or constructs may be extrachromosomal or integrated into the host cell genome

In another specific embodiment the invention covers polypeptides containing any of the CDRs or combinations thereof recited in SEQ ID NO: 4, SEQ ID NO: 5, SEQ ID NO: 6, SEQ ID NO: 7, SEQ ID NO: 8, SEQ ID NO: 120, SEQ ID NO: 9 or polypeptides comprising any of the variable light polypeptides comprised in SEQ ID NO: 2 and 20, and the variable heavy polypeptides comprised in SEQ ID NO: 3, 18 and 19,. These polypeptides optionally may be attached directly or indirectly to other immunoglobulin polypeptides or effector moieties such as therapeutic or detectable entities.

Antibodies for use in the invention may have a binding affinity (Kd) for IL-6 of less than about 50 picomolar, and/or a rate of dissociation (K_{off}) from IL-6 of less than or equal to 10⁻⁴ S⁻¹; an *in-vivo* half-life of at least about 22 days in a healthy human subject; ability to prevent or treat hypoalbunemia; ability to prevent or treat elevated CRP; ability to prevent or treat abnormal coagulation; and/or ability to decrease the risk of thrombosis in an individual having a disease or condition associated with increased risk of thrombosis. Additional non-limiting examples of anti-IL-6 activity are set forth herein, for example, under the heading "Anti-IL-6 Activity."

In another embodiment the anti-IL-6 antibody includes the Ab1 light-chain and heavy chain CDR sequences (see Table 1) which has one or more of the properties of Ab1 *in vitro* and/or upon *in vivo* administration (examples of such properties are discussed in the preceding paragraph).

Antibody fragments may be present in one or more of the following non-limiting forms: Fab, Fab', F(ab')₂, Fv and single chain Fv antibody forms. In a preferred embodiment, the anti-IL-6 antibodies described herein further comprises the kappa constant light chain sequence comprising the sequence set forth below:

In another preferred embodiment, the anti-IL-6 antibodies described herein further comprises the gamma-1 constant heavy chain polypeptide sequence comprising one of the sequences set forth below: and

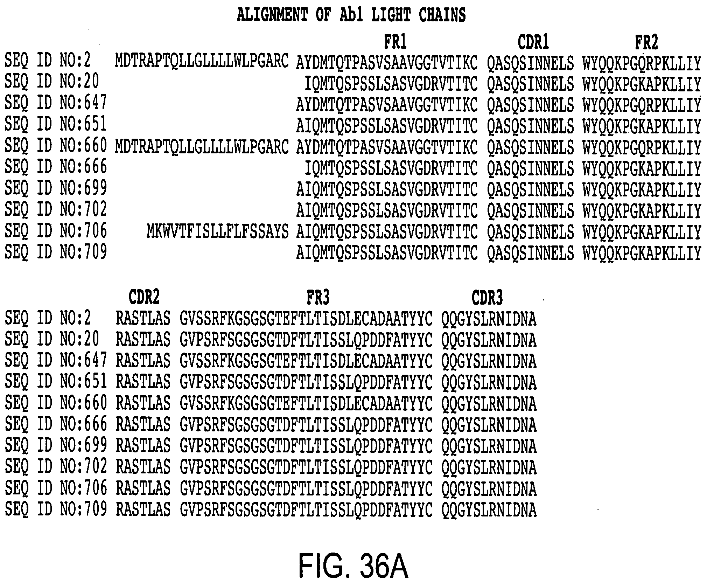

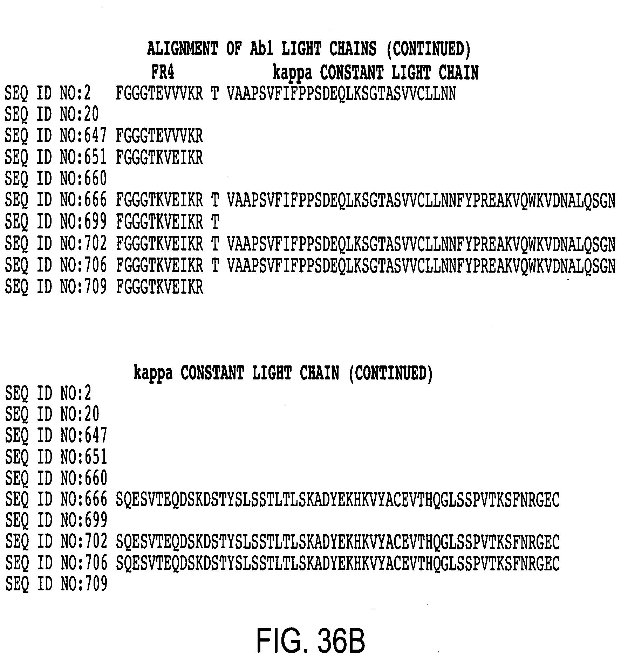

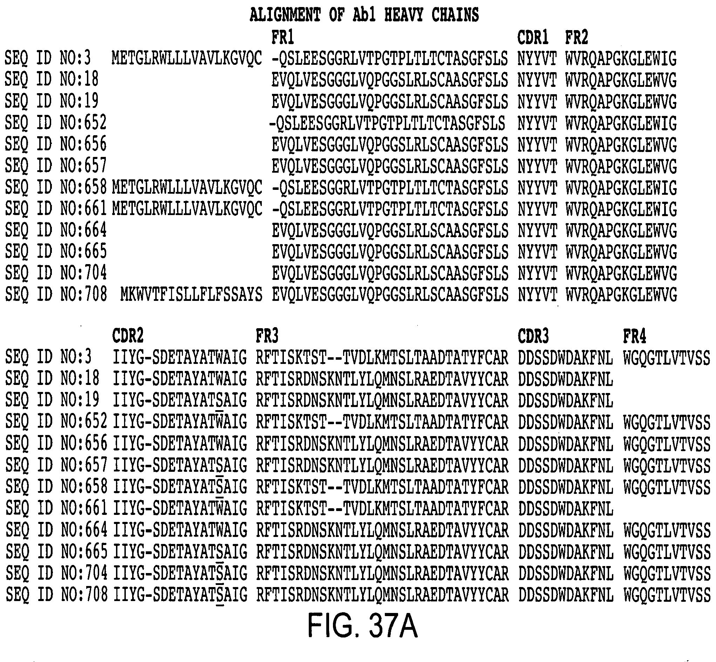

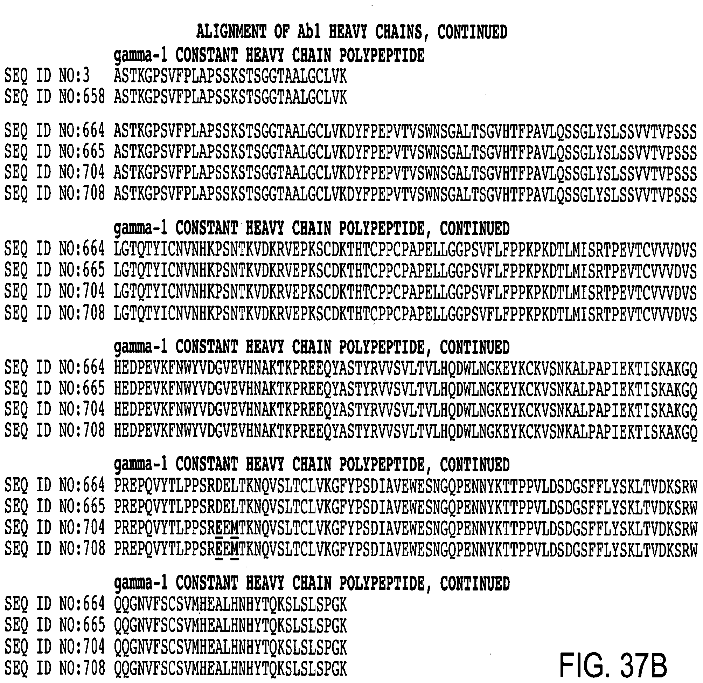

Embodiments of antibodies described herein may include a leader sequence, such as a rabbit Ig leader, albumin pre-peptide, a yeast mating factor pre pro secretion leader sequence (such as *P. pastoris* or *Saccharomyces cerevisiae* a or alpha factor), or human HAS leader. Exemplary leader sequences are shown offset from FR1 at the N-terminus of polypeptides shown in Figs. 36A and 37A as follows: rabbit Ig leader sequences in SEQ ID NOs: 2 (MD...) and SEQ ID NOs: 3 (ME...); and an albumin prepeptide in SEQ ID NOs: 706 and 708, which facilitates secretion. Other leader sequences known in the art to confer desired properties, such as secretion, improved stability or half-life, etc. may also be used, either alone or in combinations with one another, on the heavy and/or light chains, which may optionally be cleaved prior to administration to a subject. For example, a polypeptide may be expressed in a cell or cell-free expression system that also expresses or includes (or is modified to express or include) a protease, e.g., a membrane-bound signal peptidase, that cleaves a leader sequence.

In another embodiment, the invention contemplates an isolated anti-IL-6 antibody comprising a V_{H} polypeptide sequence comprising: SEQ ID NO: 3, 18, 19, or 657; and further comprising a V_{L} polypeptide sequence comprising: SEQ ID NO: 2, 20 or 709. The invention contemplates humanized and chimeric forms of these antibodies. The chimeric antibodies may include an Fc derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19 constant regions and in particular a variable heavy and light chain constant region as contained in SEQ ID NO:588 and SEQ ID NO:586.

In one embodiment of the invention, the antibodies or V_{H} or V_{L} polypeptides originate or are selected from one or more rabbit B cell populations prior to initiation of the humanization process referenced herein.

In another embodiment of the invention, the anti-IL-6 antibodies and fragments thereof have binding specificity for primate homologs of the human IL-6 protein. Non-limiting examples of primate homologs of the human IL-6 protein are IL-6 obtained from *Macaca fascicularis* (also known as the cynomolgus monkey) and the Rhesus monkey. In another embodiment of the invention, the anti-IL-6 antibodies and fragments thereof inhibits the association of IL-6 with IL-6R, and/or the production of IL-6/IL-6R/gp130 complexes and/or the production of IL-6/IL-6R/gp130 multimers and/or antagonizes the biological effects of one or more of the foregoing.

As stated above, antibodies and fragments thereof may be modified post-translationally to add effector moieties such as chemical linkers, detectable moieties such as for example fluorescent dyes, enzymes, substrates, bioluminescent materials, radioactive materials, and chemiluminescent moieties, or functional moieties such as for example streptavidin, avidin, biotin, a cytotoxin, a cytotoxic agent, and radioactive materials.

Regarding detectable moieties, further exemplary enzymes include, but are not limited to, horseradish peroxidase, acetylcholinesterase, alkaline phosphatase, *beta*-galactosidase and luciferase. Further exemplary fluorescent materials include, but are not limited to, rhodamine, fluorescein, fluorescein isothiocyanate, umbelliferone, dichlorotriazinylamine, phycoerythrin and dansyl chloride. Further exemplary chemiluminescent moieties include, but are not limited to, luminol. Further exemplary bioluminescent materials include, but are not limited to, luciferin and aequorin. Further exemplary radioactive materials include, but are not limited to, Iodine 125 (¹²⁵I), Carbon 14 (¹⁴C), Sulfur 35 (³⁵S), Tritium (³H) and Phosphorus 32 (³²P).

Regarding functional moieties, exemplary cytotoxic agents include, but are not limited to, methotrexate, aminopterin, 6-mercaptopurine, 6-thioguanine, cytarabine, 5-fluorouracil decarbazine; alkylating agents such as mechlorethamine, thioepa chlorambucil, melphalan, carmustine (BSNU), mitomycin C, lomustine (CCNU), 1-methylnitrosourea, cyclothosphamide, mechlorethamine, busulfan, dibromomannitol, streptozotocin, mitomycin C, cis-dichlorodiamine platinum (II) (DDP) cisplatin and carboplatin (paraplatin); anthracyclines include daunorubicin (formerly daunomycin), doxorubicin (adriamycin), detorubicin, carminomycin, idarubicin, epirubicin, mitoxantrone and bisantrene; antibiotics include dactinomycin (actinomycin D), bleomycin, calicheamicin, mithramycin, and anthramycin (AMC); and antimytotic agents such as the vinca alkaloids, vincristine and vinblastine. Other cytotoxic agents include paclitaxel (taxol), ricin, pseudomonas exotoxin, gemcitabine, cytochalasin B, gramicidin D, ethidium bromide, emetine, etoposide, tenoposide, colchicin, dihydroxy anthracin dione, 1-dehydrotestosterone, glucocorticoids, procaine, tetracaine, lidocaine, propranolol, puromycin, procarbazine, hydroxyurea, asparaginase, corticosteroids, mytotane (O,P'-(DDD)), interferons, and mixtures of these cytotoxic agents.

Further cytotoxic agents include, but are not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine, bleomycin, VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof. Toxic enzymes from plants and bacteria such as ricin, diphtheria toxin and *Pseudomonas* toxin may be conjugated to the humanized antibodies, or binding fragments thereof, to generate cell-type-specific-killing reagents (Youle, et al., Proc. Nat'l Acad. Sci. USA 77:5483 (1980); Gilliland, et al., Proc. Nat'l Acad. Sci. USA 77:4539 (1980); Krolick, et al., Proc. Nat'l Acad. Sci. USA 77:5419 (1980)).

Other cytotoxic agents include cytotoxic ribonucleases as described by Goldenberg in U.S. Pat. No. 6,653,104. Embodiments of the invention also relate to radioimmunoconjugates where a radionuclide that emits alpha or beta particles is stably coupled to the antibody, or binding fragments thereof, with or without the use of a complex-forming agent. Such radionuclides include beta-emitters such as Phosphorus-32 (³²P), Scandium-47 (⁴⁷Sc), Copper-67 (⁶⁷Cu), Gallium-67 (⁶⁷Ga), Yttrium-88 (⁸⁸Y), Yttrium-90 (⁹⁰Y), Iodine-125 (¹²⁵I), Iodine-131 (¹³¹I), Samarium-153 (¹⁵³Sm), Lutetium-177 (¹⁷⁷Lu), Rhenium-186 (¹⁸⁶Re) or Rhenium-188 (¹⁸⁸Re), and alpha-emitters such as Astatine-211 (²¹¹At), Lead-212 (²¹²Pb), Bismuth-212 (²¹²Bi) or -213 (²¹³Bi) or Actinium-225 (²²⁵Ac).

Methods are known in the art for conjugating an antibody or binding fragment thereof to a detectable moiety, such as for example those methods described by Hunter et al, Nature 144:945 (1962); David et al, Biochemistry 13:1014 (1974); Pain et al, J. Immunol. Meth. 40:219 (1981); and Nygren, J., Histochem. and Cytochem. 30:407 (1982).

The present invention also contemplates anti-IL-6 antibodies comprising any of the polypeptide or polynucleotide sequences described herein substituted for any of the other polynucleotide sequences described herein. For example, without limitation thereto, the present invention contemplates antibodies comprising the combination of any of the variable light chain and variable heavy chain sequences described herein, and further contemplates antibodies resulting from substitution of any of the CDR sequences described herein for any of the other CDR sequences described herein.

### Additional Exemplary Embodiments of the Invention

The invention further contemplates that the one or more anti-IL-6 antibodies discussed above are aglycosylated; that contain an Fc region that has been modified to alter effector function, half-life, proteolysis, and/or glycosylation; are human, humanized, single chain or chimeric; and are a humanized antibody derived from a rabbit (parent) anti-IL-6 antibody. Exemplary constant regions that provide for the production of aglycosylated antibodies in Pichia are comprised in SEQ ID NO:588 and SEQ ID NO:586 which respectively are encode by the nucleic acid sequences in SEQ ID NO:589 and SEQ ID NO:587.

In one embodiment of the invention, the anti-IL-6 antibody or fragment may specifically bind to IL-6 expressing human cells and/or to circulating soluble IL-6 molecules in vivo, including IL-6 expressed on or by human cells in a patient with a disease associated with cells that express IL-6.

The disease treated by the present invention is cachexia in a patient diagnosed with an IL-6 associated disorder selected from AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

The invention further contemplates anti-IL-6 antibodies or fragments directly or indirectly attached to a detectable label or therapeutic agent.

The invention also contemplates one or more nucleic acid sequences which result in the expression of an anti-IL-6 antibody or antibody fragment as set forth above, including those comprising, or alternatively consisting of, yeast or human preferred codons. The invention also contemplates vectors (including plasmids or recombinant viral vectors) comprising said nucleic acid sequence(s). The invention also contemplates host cells or recombinant host cells expressing at least one of the antibodies set forth above, including a mammalian, yeast, bacterial, and insect cells. In a preferred embodiment, the host cell is a yeast cell. In a further preferred embodiment, the yeast cell is a diploidal yeast cell. In a more preferred embodiment, the yeast cell is a Pichia yeast.

In another embodiment the treatment further includes the administration of another therapeutic agent or regimen selected from chemotherapy or radiotherapy.

There is described a method of in vivo imaging which detects the presence of cells which express IL-6 comprising administering a diagnostically effective amount of at least one anti-IL-6 antibody. In one embodiment, said administration further includes the administration of a radionuclide or fluorophore that facilitates detection of the antibody at IL-6 expressing disease sites. In another embodiment of the invention, the method of in vivo imaging is used to detect IL-6 expressing tumors or metastases or is used to detect the presence of sites of autoimmune disorders associated with IL-6 expressing cells. In a further embodiment, the results of said in vivo imaging method are used to facilitate design of an appropriate therapeutic regimen, including therapeutic regimens including radiotherapy, chemotherapy or a combination thereof.

### Polynucleotides Encoding Anti-IL-6 Antibody Polypeptides

The invention is further directed to polynucleotides encoding polypeptides of the antibodies having binding specificity to IL-6. In one embodiment of the invention, polynucleotides of the invention comprise, or alternatively consist of, the following polynucleotide sequence encoding the variable light chain polypeptide sequence of SEQ ID NO: 2:

In another embodiment of the invention, polynucleotides of the invention comprise, or alternatively consist of, the following polynucleotide sequence encoding the variable heavy chain polypeptide sequence of SEQ ID NO: 3:

In a further embodiment of the invention, polynucleotides encoding fragments of the antibody having binding specificity to IL-6 comprise, or alternatively consist of, one or more of the polynucleotide sequences of SEQ ID NO: 12; SEQ ID NO: 13; and SEQ ID NO: 14 which correspond to polynucleotides encoding the complementarity-determining regions (CDRs, or hypervariable regions) of the light chain variable sequence of SEQ ID NO: 2.

In a further embodiment of the invention, polynucleotides encoding fragments of the antibody having binding specificity to IL-6 comprise, or alternatively consist of, one or more of the polynucleotide sequences of SEQ ID NO: 15; SEQ ID NO: 16; and SEQ ID NO: 17 which correspond to polynucleotides encoding the complementarity-determining regions (CDRs, or hypervariable regions) of the heavy chain variable sequence of SEQ ID NO: 3.

The invention also contemplates polynucleotide sequences including one or more of the polynucleotide sequences encoding antibody fragments described herein. In one embodiment of the invention, polynucleotides encoding fragments of the antibody having binding specificity to IL-6 comprise, or alternatively consist of, one, two, three or more, including all of the following polynucleotides encoding antibody fragments: the polynucleotide SEQ ID NO: 10 encoding the light chain variable region of SEQ ID NO: 2; the polynucleotide SEQ ID NO: 11 encoding the heavy chain variable region of SEQ ID NO: 3; polynucleotides encoding the complementarity-determining regions (SEQ ID NO: 12; SEQ ID NO: 13; and SEQ ID NO: 14) of the light chain variable region of SEQ ID NO: 10; polynucleotides encoding the complementarity-determining regions (SEQ ID NO: 15; SEQ ID NO: 16; and SEQ ID NO: 17) of the heavy chain variable region of SEQ ID NO: 11 and polynucleotides encoding the variable heavy and light chain sequences in SEQ ID NO:657 and SEQ ID NO:709 respectively, e.g., the nucleic acid sequences in SEQ ID NO:700 and SEQ ID NO:723 and fragments or variants thereof , e.g., based on codon degeneracy. These nucleic acid sequences encoding variable heavy and light chain sequences may be expressed alone or in combination and these sequences preferably are fused to suitable variable constant sequences, e.g., those in SEQ ID NO:589 and SEQ ID NO:587.

In another embodiment of the invention, polynucleotides of the invention further comprise, the following polynucleotide sequence encoding the kappa constant light chain sequence of SEQ ID NO: 586:

In another embodiment of the invention, polynucleotides of the invention further comprise, the following polynucleotide sequence encoding the gamma-1 constant heavy chain polypeptide sequence of SEQ ID NO: 588:

In another embodiment of the invention, polynucleotides of the invention further comprise, the following polynucleotide sequence encoding the variable heavy chain polypeptide sequence of SEQ ID NO: 700:

In another embodiment of the invention, polynucleotides of the invention further comprise, the following polynucleotide sequence encoding the variable light chain polypeptide sequence of SEQ ID NO: 723:

In one embodiment, the invention is directed to an isolated polynucleotide comprising a polynucleotide encoding an anti-IL-6 V_{H} antibody amino acid sequence selected from SEQ ID NO: 3, 18 or 19.

In another embodiment, the invention is directed to an isolated polynucleotide comprising the polynucleotide sequence encoding an anti-IL-6 V_{L} antibody amino acid sequence of SEQ ID NO: 2 or 20.

In yet another embodiment, the invention is directed to one or more heterologous polynucleotides comprising a sequence encoding the polypeptides contained in SEQ ID NO:2 and SEQ ID NO:3; SEQ ID NO:2 and SEQ ID NO:18; SEQ ID NO:2 and SEQ ID NO:19; SEQ ID NO:20 and SEQ ID NO:3; SEQ ID NO:20 and SEQ ID NO:18; or SEQ ID NO:20 and SEQ ID NO:19.

In another embodiment, the invention is directed to an isolated polynucleotide that expresses a polypeptide containing the V_{L} or V_{H} polypeptides contained in SEQ ID NO: 3, 18, 19, 2 or 20. Exemplary nucleic acid sequence encoding the VH and VL polypeptides SEQ ID NO:657 and SEQ ID NO:709 are comprised in SEQ ID NO:700 and SEQ ID NO:723 respectively.

Host cells and vectors comprising said polynucleotides are also contemplated.

In another specific embodiment the invention covers nucleic acid constructs containing any of the foregoing nucleic acid sequences and combinations thereof as well as recombinant cells containing these nucleic acid sequences and constructs containing wherein these nucleic acid sequences or constructs may be extrachromosomal or integrated into the host cell genome.

The invention further contemplates vectors comprising the polynucleotide sequences encoding the variable heavy and light chain polypeptide sequences, as well as host cells comprising said sequences. In one embodiment of the invention, the host cell is a yeast cell. In another embodiment of the invention, the yeast host cell belongs to the genus *Pichia.*

In some instances, more than one exemplary polynucleotide encoding a given polypeptide sequence is provided, as summarized in Table 3.

**Table 3. Multiple exemplary polynucleotides encoding particular polypeptides.**

| **Polypeptide SEQ ID NO** | **Exemplary coding SEQ ID NOs** |
|---|---|
| 4 | 12, 111, 694 |
| 5 | 13, 112, 389, 501 |
| 6 | 14, 113, 695 |
| 9 | 17, 116, 697 |

The phrase "high stringency hybridization conditions" refers to conditions under which a probe will hybridize to its target subsequence, typically in a complex mixture of nucleic acid, but to no other sequences. High stringency conditions are sequence dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, high stringency conditions are selected to be about 5-10 °C lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). High stringency conditions will be those in which the salt concentration is less than about 1.0 M sodium ion, typically about 0.01 to 1.0 M sodium ion concentration (or other salts) at pH 7.0 to 8.3 and the temperature is at least about 30 °C for short probes (e.g., 10 to 50 nucleotides) and at least about 60 °C for long probes (e.g., greater than 50 nucleotides). High stringency conditions may also be achieved with the addition of destabilizing agents such as formamide. For selective or specific hybridization, a positive signal is at least two times background, optionally 10 times background hybridization. Exemplary high stringency hybridization conditions can be as following: 50% formamide, 5×SSC, and 1% SDS, incubating at 42 °C, or, 5×SSC, 1% SDS, incubating at 65 °C, with wash in 0.2×SSC, and 0.1% SDS at 65 °C. Such hybridizations and wash steps can be carried out for, e.g., 1, 2, 5, 10, 15, 30, 60; or more minutes.

Nucleic acids that do not hybridize to each other under high stringency conditions are still substantially related if the polypeptides that they encode are substantially related. This occurs, for example, when a copy of a nucleic acid is created using the maximum codon degeneracy permitted by the genetic code. In such cases, the nucleic acids typically hybridize under moderate stringency hybridization conditions. Exemplary "moderate stringency hybridization conditions" include a hybridization in a buffer of 40% formamide, 1 M NaCl, 1% SDS at 37 °C., and a wash in 1×SSC at 45 °C. Such hybridizations and wash steps can be carried out for, e.g., 1, 2, 5, 10, 15, 30, 60, or more minutes. A positive hybridization is at least twice background. Those of ordinary skill will readily recognize that alternative hybridization and wash conditions can be utilized to provide conditions of similar stringency.

### Exemplary Embodiments of Heavy and Light Chain Polypeptides and Polynucleotides

This section recites exemplary embodiments of heavy and light chain polypeptides, as well as exemplary polynucleotides encoding such polypeptides. These exemplary polynucleotides are suitable for expression in the disclosed Pichia expression system.

In another aspect, the invention encompasses a composition comprising such a polynucleotide and/or a polypeptide encoded by such a polynucleotide. The invention utilises the Ab1 antibody described herein in a method of treatment of cachexia in a patient diagnosed with an IL-6 associated disorder wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof, comprising administration of a composition comprising such a polynucleotide and/or polypeptide.

In any of the embodiments recited herein, certain of the sequences recited may be substituted for each other, unless the context indicates otherwise. The recitation that particular sequences may be substituted for one another, where such recitations are made, are understood to be illustrative rather than limiting, and it is also understood that such substitutions are encompassed even when no illustrative examples of substitutions are recited, For example, wherever one or more of the Ab1 light chain polypeptides is recited, e.g. any of SEQ ID NO: 2, 20, 647, 651, 660, 666, 699, 702, 706, or 709, another Ab1 light chain polypeptide may be substituted unless the context indicates otherwise. Similarly, wherever one of the Ab1 heavy chain polypeptides is recited, e.g. any of SEQ ID NO: 3, 18, 19, 652, 656, 657, 658, 661, 664, 665, 704, or 708, another Ab1 heavy chain polypeptide may be substituted unless the context indicates otherwise. Likewise, wherever one of the Ab1 light chain polynucleotides is recited, e.g. any of SEQ ID NO: 10, 662, 698, 701, or 705, another Ab1 light chain polynucleotide may be substituted unless the context indicates otherwise. Similarly, wherever one of the Ab1 heavy chain polynucleotides is recited, e.g. any of SEQ ID NO: 11, 663, 700, 703, or 707, another Ab1 heavy chain polynucleotide may be substituted unless the context indicates otherwise.

### Anti-IL-6 Activity

As stated previously, IL-6 is a member of a family of cytokines that promote cellular responses through a receptor complex consisting of at least one subunit of the signal-transducing glycoprotein gp130 and the IL-6 receptor (IL-6R). The IL-6R may also be present in a soluble form (sIL-6R). IL-6 binds to IL-6R, which then dimerizes the signal-transducing receptor gp130.

It is believed that the anti-IL-6 antibodies of the invention, or IL-6 binding fragments thereof, are useful by exhibiting anti-IL-6 activity. In one non-limiting embodiment of the invention, the anti-IL-6 antibodies of the invention, or IL-6 binding fragments thereof, exhibit anti-IL-6 activity by binding to IL-6 which may be soluble IL-6 or cell surface expressed IL-6 and/or may prevent or inhibit the binding of IL-6 to IL-6R and/or activation (dimerization) of the gp130 signal-transducing glycoprotein and the formation of IL-6/IL-6R/gp130 multimers and the biological effects of any of the foregoing. The subject anti-IL-6 antibodies may possess different antagonistic activities based on where (i.e., epitope) the particular antibody binds IL-6 and/or how it affects the formation of the foregoing IL-6 complexes and/or multimers and the biological effects thereof. Consequently, different anti-IL-6 antibodies according to the invention e.g., may be better suited for preventing or treating conditions involving the formation and accumulation of substantial soluble IL-6 such as rheumatoid arthritis whereas other antibodies may be favored in treatments wherein the prevention of IL-6/IL-6R/gp130 or IL-6/IL-6R/gp130 multimers is a desired therapeutic outcome. This can be determined in binding and other assays.

The anti-IL-6 activity of the anti-IL-6 antibody of the present invention, and fragments thereof having binding specificity to IL-6, may also be described by their strength of binding or their affinity for IL-6. This also may affect their therapeutic properties. In one embodiment of the invention, the anti-IL-6 antibodies of the present invention, and fragments thereof having binding specificity to IL-6, bind to IL-6 with a dissociation constant (K_{D}) of less than or equal to 5x10⁻⁷, 10⁻⁷, 5x10⁻⁸, 10⁻⁸, 5x10⁻⁹, 10⁻⁹, 5x10⁻¹⁰, 10⁻¹⁰, 5x10⁻¹¹, 10⁻¹¹, 5x10⁻¹², 10⁻¹², 5x10⁻¹³, 10⁻¹³, 5x10⁻¹⁴, 10⁻¹⁴, 5x10⁻¹⁵ or 10⁻¹⁵. Preferably, the anti-IL-6 antibodies and fragments thereof bind IL-6 with a dissociation constant of less than or equal to 5x10⁻¹⁰.

In another embodiment of the invention, the anti-IL-6 activity of the anti-IL-6 antibodies of the present invention, and fragments thereof having binding specificity to IL-6, bind to IL-6 with an off-rate of less than or equal to 10⁻⁴ S⁻¹, 5x10⁻⁵ S⁻¹, 10⁻⁵ S⁻¹, 5x10⁻⁶ S⁻¹, 10⁻⁶ S⁻¹, 5x10⁻⁷ S⁻¹, or 10⁻⁷ S⁻¹. In one embodiment of the invention, the anti-IL-6 antibodies of the invention, and fragments thereof having binding specificity to IL-6, bind to a linear or conformational IL-6 epitope.

In a further embodiment of the invention, the anti-IL-6 activity of the anti-IL-6 antibodies of the present invention, and fragments thereof having binding specificity to IL-6, exhibit anti-IL-6 activity by ameliorating or reducing the symptoms of, or alternatively treating, or preventing, cachexia associated with IL-6, wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

In another embodiment of the invention, the anti-IL-6 antibodies described herein, or IL-6 binding fragments thereof, do not have binding specificity for IL-6R or the gp-130 signal-transducing glycoprotein.

### B-cell Screening and Isolation

There are described methods of isolating a clonal population of antigen-specific B cells that may be used for isolating at least one antigen-specific cell. As described and exemplified infra, these methods contain a series of culture and selection steps that can be used separately, in combination, sequentially, repetitively, or periodically. Preferably, these methods are used for isolating at least one antigen-specific cell, which can be used to produce a monoclonal antibody, which is specific to a desired antigen, or a nucleic acid sequence corresponding to such an antibody.

There is described a method comprising the steps of:
a. preparing a cell population comprising at least one antigen-specific B cell;
b. enriching the cell population, e.g., by chromatography, to form an enriched cell population comprising at least one antigen-specific B cell;
c. isolating a single B cell from the enriched B cell population; and
d. determining whether the single B cell produces an antibody specific to the antigen.

There is described an improvement to a method of isolating a single, antibody-producing B cell, the improvement comprising enriching a B cell population obtained from a host that has been immunized or naturally exposed to an antigen, wherein the enriching step precedes any selection steps, comprises at least one culturing step, and results in a clonal population of B cells that produces a single monoclonal antibody specific to said antigen.

Throughout this application, a "clonal population of B cells" refers to a population of B cells that only secrete a single antibody specific to a desired antigen. That is to say that these cells produce only one type of monoclonal antibody specific to the desired antigen.

In the present application, "enriching" a cell population cells means increasing the frequency of desired cells, typically antigen-specific cells, contained in a mixed cell population, e.g., a B cell-containing isolate derived from a host that is immunized against a desired antigen. Thus, an enriched cell population encompasses a cell population having a higher frequency of antigen-specific cells as a result of an enrichment step, but this population of cells may contain and produce different antibodies.

The general term "cell population" encompasses pre- and a post-enrichment cell populations, keeping in mind that when multiple enrichment steps are performed, a cell population can be both pre- and post-enrichment. For example, there is described a method:
a. harvesting a cell population from an immunized host to obtain a harvested cell population;
b. creating at least one single cell suspension from the harvested cell population;
c. enriching at least one single cell suspension to form a first enriched cell population;
d. enriching the first enriched cell population to form a second enriched cell population;
e. enriching the second enriched cell population to form a third enriched cell population; and
f. selecting an antibody produced by an antigen-specific cell of the third enriched cell population.

Each cell population may be used directly in the next step, or it can be partially or wholly frozen for long- or short- term storage or for later steps. Also, cells from a cell population can be individually suspended to yield single cell suspensions. The single cell suspension can be enriched, such that a single cell suspension serves as the pre-enrichment cell population. Then, one or more antigen-specific single cell suspensions together form the enriched cell population; the antigen-specific single cell suspensions can be grouped together, e.g., re-plated for further analysis and/or antibody production.

There is described a method of enriching a cell population to yield an enriched cell population having an antigen-specific cell frequency that is about 50% to about 100%, or increments therein. Preferably, the enriched cell population has an antigen-specific cell frequency greater than or equal to about 50%, 60%, 70%, 75%, 80%, 90%, 95%, 99%, or 100%.

There is described a method of enriching a cell population whereby the frequency of antigen-specific cells is increased by at least about 2-fold, 5-fold, 10-fold, 20-fold, 50-fold, 100-fold, or increments therein.

Throughout this application, the term "increment" is used to define a numerical value in varying degrees of precision, e.g., to the nearest 10, 1, 0.1, 0.01, etc. The increment can be rounded to any measurable degree of precision, and the increment need not be rounded to the same degree of precision on both sides of a range. For example, the range 1 to 100 or increments therein includes ranges such as 20 to 80, 5 to 50, and 0.4 to 98. When a range is open-ended, e.g., a range of less than 100, increments therein means increments between 100 and the measurable limit. For example, less than 100 or increments therein means 0 to 100 or increments therein unless the feature, e.g., temperature, is not limited by 0.

Antigen-specificity can be measured with respect to any antigen. The antigen can be any substance to which an antibody can bind including, but not limited to, peptides, proteins or fragments thereof; carbohydrates; organic and inorganic molecules; receptors produced by animal cells, bacterial cells, and viruses; enzymes; agonists and antagonists of biological pathways; hormones; and cytokines. Exemplary antigens include, but are not limited to, IL-2, IL-4, IL-6, IL-10, IL-12, IL-13, IL-18, IFN-α, IFN-γ, BAFF, CXCL13, IP-10, VEGF, EPO, EGF, HRG, Hepatocyte Growth Factor (HGF) and Hepcidin. Preferred antigens include IL-6, IL-13, TNF-α, VEGF-α, Hepatocyte Growth Factor (HGF) and Hepcidin. In a method utilizing more than one enrichment step, the antigen used in each enrichment step can be the same as or different from one another. Multiple enrichment steps with the same antigen may yield a large and/or diverse population of antigen-specific cells; multiple enrichment steps with different antigens may yield an enriched cell population with cross-specificity to the different antigens.

Enriching a cell population can be performed by any cell-selection means known in the art for isolating antigen-specific cells. For example, a cell population can be enriched by chromatographic techniques, e.g., Miltenyi bead or magnetic bead technology. The beads can be directly or indirectly attached to the antigen of interest. In a preferred embodiment, the method of enriching a cell population includes at least one chromatographic enrichment step.

A cell population can also be enriched by performed by any antigen-specificity assay technique known in the art, e.g., an ELISA assay or a halo assay. ELISA assays include, but are not limited to, selective antigen immobilization (e.g., biotinylated antigen capture by streptavidin, avidin, or neutravidin coated plate), nonspecific antigen plate coating, and through an antigen build-up strategy (e.g., selective antigen capture followed by binding partner addition to generate a heteromeric protein-antigen complex). The antigen can be directly or indirectly attached to a solid matrix or support, e.g., a column. A halo assay comprises contacting the cells with antigen-loaded beads and labeled anti-host antibody specific to the host used to harvest the B cells. The label can be, e.g., a fluorophore. In one embodiment, at least one assay enrichment step is performed on at least one single cell suspension. In another embodiment, the method of enriching a cell population includes at least one chromatographic enrichment step and at least one assay enrichment step.

Methods of "enriching" a cell population by size or density are known in the art. See, e.g., U.S. Patent 5,627,052. These steps can be used in the present method in addition to enriching the cell population by antigen-specificity.

The cell populations described herein contain at least one cell capable of recognizing an antigen. Antigen-recognizing cells include, but are not limited to, B cells, plasma cells, and progeny thereof. There is described a clonal cell population containing a single type of antigen-specific B-cell, i.e., the cell population produces a single monoclonal antibody specific to a desired antigen.

It is believed that the clonal antigen-specific population of B cells consists predominantly of antigen-specific, antibody-secreting cells, which are obtained by the novel culture and selection protocol provided herein. Accordingly, there are described methods for obtaining an enriched cell population containing at least one antigen-specific, antibody-secreting cell. The enriched cell population may contain about 50% to about 100%, or increments therein, or greater than or equal to about 60%, 70%, 80%, 90%, or 100% of antigen-specific, antibody-secreting cells.

There is described a method of isolating a single B cell by enriching a cell population obtained from a host before any selection steps, e.g., selecting a particular B cell from a cell population and/or selecting an antibody produced by a particular cell. The enrichment step can be performed as one, two, three, or more steps. A single B cell may be isolated from an enriched cell population before confirming whether the single B cell secretes an antibody with antigen-specificity and/or a desired property.

A method of enriching a cell population may be used in a method for antibody production and/or selection. Thus, there is described a method comprising enriching a cell population before selecting an antibody. The method can include the steps of: preparing a cell population comprising at least one antigen-specific cell, enriching the cell population by isolating at least one antigen-specific cell to form an enriched cell population, and inducing antibody production from at least one antigen-specific cell. The enriched cell population may contain more than one antigen-specific cell. Each antigen-specific cell of the enriched population may be cultured under conditions that yield a clonal antigen-specific B cell population before isolating an antibody producing cell therefrom and/or producing an antibody using said B cell, or a nucleic acid sequence corresponding to such an antibody. In contrast to prior techniques where antibodies are produced from a cell population with a low frequency of antigen-specific cells, the described methods allow antibody selection from among a high frequency of antigen-specific cells. Because an enrichment step is used prior to antibody selection, the majority of the cells, preferably virtually all of the cells, used for antibody production are antigen-specific. By producing antibodies from a population of cells with an increased frequency of antigen specificity, the quantity and variety of antibodies are increased.

In the described antibody selection methods, an antibody may be selected after an enrichment step and a culture step that results in a clonal population of antigen-specific B cells. The methods can further comprise a step of sequencing a selected antibody or portions thereof from one or more isolated, antigen-specific cells. Any method known in the art for sequencing can be employed and can include sequencing the heavy chain, light chain, variable region(s), and/or complementarity determining region(s) (CDR).

In addition to the enrichment step, the method for antibody selection can also include one or more steps of screening a cell population for antigen recognition and/or antibody functionality. For example, the desired antibodies may have specific structural features, such as binding to a particular epitope or mimicry of a particular structure; antagonist or agonist activity; or neutralizing activity, e.g., inhibiting binding between the antigen and a ligand. The antibody functionality screen may be ligand-dependent. Screening for antibody functionality includes, but is not limited to, an in vitro protein-protein interaction assay that recreates the natural interaction of the antigen ligand with recombinant receptor protein; and a cell-based response that is ligand dependent and easily monitored (e.g., proliferation response). The method for antibody selection may a step of screening the cell population for antibody functionality by measuring the inhibitory concentration (IC50). At least one of the isolated, antigen-specific cells may produce an antibody having an IC50 of less than about 100, 50, 30, 25, 10 µg/mL, or increments therein.

In addition to the enrichment step, the method for antibody selection can also include one or more steps of screening a cell population for antibody binding strength. Antibody binding strength can be measured by any method known in the art (e.g., Biacore™). At least one of the isolated, antigen-specific cells may produce an antibody having a high antigen affinity, e.g., a dissociation constant (Kd) of less than about 5x10⁻¹⁰ M-1, preferably about 1x10⁻¹³ to 5x10⁻¹⁰, 1x10⁻¹² to 1x10⁻¹⁰, 1x10⁻¹² to 7.5x10⁻¹¹, 1x10⁻¹¹ to 2x10⁻¹¹, about 1.5x10⁻¹¹ or less, or increments therein. In this embodiment, the antibodies are said to be affinity mature. The affinity of the antibodies may be comparable to or higher than the affinity of any one of Panorex® (edrecolomab), Rituxan® (rituximab), Herceptin® (traztuzumab), Mylotarg® (gentuzumab), Campath® (alemtuzumab), Zevalin™ (ibritumomab), Erbitux™ (cetuximab), Avastin™ (bevicizumab), Raptiva™ (efalizumab), Remicade® (infliximab), Humira™ (adalimumab), and Xolair™ (omalizumab). The affinity of an antibody can also be increased by known affinity maturation techniques. At least one cell population may be screened for at least one of, preferably both, antibody functionality and antibody binding strength.

In addition to the enrichment step, the method for antibody selection can also include one or more steps of screening a cell population for antibody sequence homology, especially human homology. At least one of the isolated, antigen-specific cells may produce an antibody that has a homology to a human antibody of about 50% to about 100%, or increments therein, or greater than about 60%, 70%, 80%, 85%, 90%, or 95% homologous. The antibodies can be humanized to increase the homology to a human sequence by techniques known in the art such as CDR grafting or selectivity determining residue grafting (SDR).

The B cell selection protocol disclosed herein has a number of intrinsic advantages versus other methods for obtaining antibody-secreting B cells and monoclonal antibodies specific to desired target antigens. These advantages include, but are not restricted to, the following:

First, it has been found that when these selection procedures are utilized with a desired antigen such as IL-6 or TNF-α, the methods reproducibly result in antigen-specific B cells capable of generating what appears to be a substantially comprehensive complement of antibodies, i.e., antibodies that bind to the various different epitopes of the antigen. Without being bound by theory, it is hypothesized that the comprehensive complement is attributable to the antigen enrichment step that is performed prior to initial B cell recovery. Moreover, this advantage allows for the isolation and selection of antibodies with different properties as these properties may vary depending on the epitopic specificity of the particular antibody.

Second, it has been found that the B cell selection protocol reproducibly yields a clonal B cell culture containing a single B cell, or its progeny, secreting a single monoclonal antibody that generally binds to the desired antigen with a relatively high binding affinity, i.e. picomolar or better antigen binding affinities. By contrast, prior antibody selection methods tend to yield relatively few high affinity antibodies and therefore require extensive screening procedures to isolate an antibody with therapeutic potential. Without being bound by theory, it is hypothesized that the protocol results in both in vivo B cell immunization of the host (primary immunization) followed by a second in vitro B cell stimulation (secondary antigen priming step) that may enhance the ability and propensity of the recovered clonal B cells to secrete a single high affinity monoclonal antibody specific to the antigen target.

Third, it has been observed (as shown herein with IL-6 specific B cells) that the B cell selection protocol reproducibly yields enriched B cells producing IgG's that are, on average, highly selective (antigen specific) to the desired target. Antigen-enriched B cells recovered by these methods are believed to contain B cells capable of yielding the desired full complement of epitopic specificities as discussed above.

Fourth, it has been observed that the B cell selection protocols, even when used with small antigens, i.e., peptides of 100 amino acids or less, e.g., 5-50 amino acids long, reproducibly give rise to a clonal B cell culture that secretes a single high affinity antibody to the small antigen, e.g., a peptide. This is highly surprising as it is generally quite difficult, labor intensive, and sometimes not even feasible to produce high affinity antibodies to small peptides. Accordingly, the invention can be used to produce therapeutic antibodies to desired peptide targets, e.g., viral, bacterial or autoantigen peptides, thereby allowing for the production of monoclonal antibodies with very discrete binding properties or even the production of a cocktail of monoclonal antibodies to different peptide targets, e.g., different viral strains. This advantage may especially be useful in the context of the production of a therapeutic or prophylactic vaccine having a desired valency, such as an HPV vaccine that induces protective immunity to different HPV strains.

Fifth, the B cell selection protocol, particularly when used with B cells derived from rabbits, tends to reproducibly yield antigen-specific antibody sequences that are very similar to endogenous human immunoglobulins (around 90% similar at the amino acid level) and that contain CDRs that possess a length very analogous to human immunoglobulins and therefore require little or no sequence modification (typically at most only a few CDR residues may be modified in the parent antibody sequence and no framework exogenous residues introduced) in order to eliminate potential immunogenicity concerns. In particular, preferably the recombinant antibody will contain only the host (rabbit) CDR1 and CDR2 residues required for antigen recognition and the entire CDR3. Thereby, the high antigen binding affinity of the recovered antibody sequences produced according to the B cell and antibody selection protocol remains intact or substantially intact even with humanization.

In sum, these methods can be used to produce antibodies exhibiting higher binding affinities to more distinct epitopes by the use of a more efficient protocol than was previously known.

There is described a method for identifying a single B cell that secretes an antibody specific to a desired antigen and that optionally possesses at least one desired functional property such as affinity, avidity or cytolytic activity by a process including the following steps:
a. immunizing a host against an antigen;
b. harvesting B cells from the host;
c. enriching the harvested B cells to increase the frequency of antigen-specific cells;
d. creating at least one single cell suspension;
e. culturing a sub-population from the single cell suspension under conditions that favor the survival of a single antigen-specific B cell per culture well;
f. isolating B cells from the sub-population; and
g. determining whether the single B cell produces an antibody specific to the antigen.

Typically, these methods will further comprise an additional step of isolating and sequencing, in whole or in part, the polypeptide and nucleic acid sequences encoding the desired antibody. These sequences or modified versions or portions thereof can be expressed in desired host cells in order to produce recombinant antibodies to a desired antigen.

As noted previously, it is believed that the clonal population of B cells predominantly comprises antibody-secreting B cells producing antibody against the desired antigen. It is also believed based on experimental results obtained with several antigens and with different B cell populations that the clonally produced B cells and the isolated antigen-specific B cells derived therefrom produced according to the invention secrete a monoclonal antibody that is typically of relatively high affinity and moreover is capable of efficiently and reproducibly producing a selection of monoclonal antibodies of greater epitopic variability as compared to other methods of deriving monoclonal antibodies from cultured antigen-specific B cells. In an exemplary embodiment the population of immune cells used in such B cell selection methods will be derived from a rabbit. However, other hosts that produce antibodies, including non-human and human hosts, can alternatively be used as a source of immune B cells. It is believed that the use of rabbits as a source of B cells may enhance the diversity of monoclonal antibodies that may be derived by the methods. Also, the antibody sequences derived from rabbits according to the invention typically possess sequences having a high degree of sequence identity to human antibody sequences making them favored for use in humans since they should possess little antigenicity. In the course of humanization, the final humanized antibody contains a much lower foreign/host residue content, usually restricted to a subset of the host CDR residues that differ dramatically due to their nature versus the human target sequence used in the grafting. This enhances the probability of complete activity recovery in the humanized antibody protein.

The methods of antibody selection using an enrichment step disclosed herein include a step of obtaining an immune cell-containing cell population from an immunized host. Methods of obtaining an immune cell-containing cell population from an immunized host are known in the art and generally include inducing an immune response in a host and harvesting cells from the host to obtain one or more cell populations. The response can be elicited by immunizing the host against a desired antigen. Alternatively, the host used as a source of such immune cells can be naturally exposed to the desired antigen such as an individual who has been infected with a particular pathogen such as a bacterium or virus or alternatively has mounted a specific antibody response to a cancer that the individual is afflicted with.

Host animals are well-known in the art and include, but are not limited to, guinea pig, rabbit, mouse, rat, non-human primate, human, as well as other mammals and rodents, chicken, cow, pig, goat, and sheep. Preferably the host is a mammal, more preferably, rabbit, mouse, rat, or human. When exposed to an antigen, the host produces antibodies as part of the native immune response to the antigen. As mentioned, the immune response can occur naturally, as a result of disease, or it can be induced by immunization with the antigen. Immunization can be performed by any method known in the art, such as, by one or more injections of the antigen with or without an agent to enhance immune response, such as complete or incomplete Freund's adjuvant. In another embodiment, the invention also contemplates intrasplenic immunization. As an alternative to immunizing a host animal in vivo, the method can comprise immunizing a host cell culture in vitro.

After allowing time for the immune response (e.g., as measured by serum antibody detection), host animal cells are harvested to obtain one or more cell populations. In a preferred embodiment, a harvested cell population is screened for antibody binding strength and/or antibody functionality. A harvested cell population is preferably from at least one of the spleen, lymph nodes, bone marrow, and/or peripheral blood mononuclear cells (PBMCs). The cells can be harvested from more than one source and pooled. Certain sources may be preferred for certain antigens. For example, the spleen, lymph nodes, and PBMCs are preferred for IL-6; and the lymph nodes are preferred for TNF. The cell population is harvested about 20 to about 90 days or increments therein after immunization, preferably about 50 to about 60 days. A harvested cell population and/or a single cell suspension therefrom can be enriched, screened, and/or cultured for antibody selection. The frequency of antigen-specific cells within a harvested cell population is usually about 1% to about 5%, or increments therein.

In one embodiment, a single cell suspension from a harvested cell population is enriched, preferably by using Miltenyi beads. From the harvested cell population having a frequency of antigen-specific cells of about 1% to about 5%, an enriched cell population is thus derived having a frequency of antigen-specific cells approaching 100%.

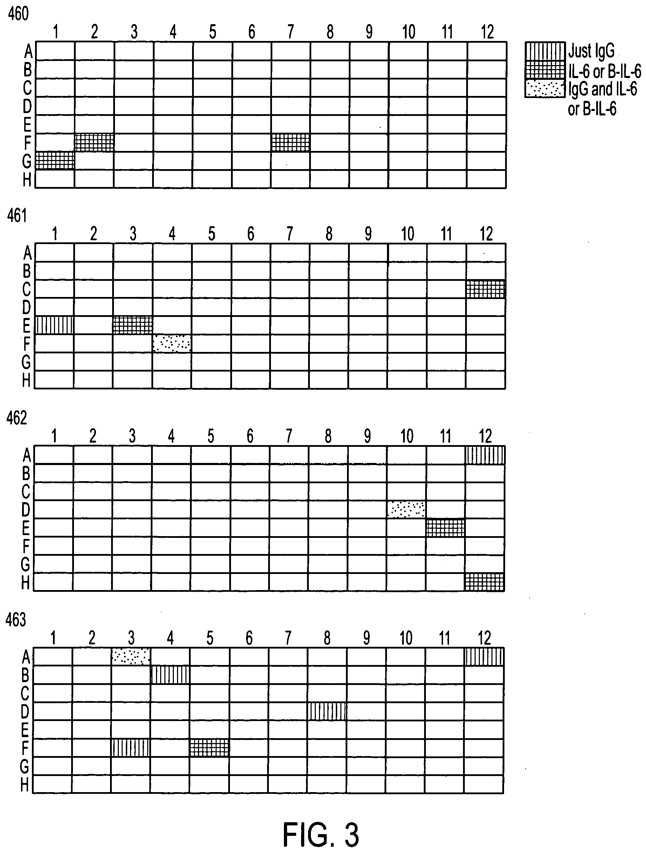

The method of antibody selection using an enrichment step includes a step of producing antibodies from at least one antigen-specific cell from an enriched cell population. Methods of producing antibodies in vitro are well known in the art, and any suitable method can be employed. In one embodiment, an enriched cell population, such as an antigen-specific single cell suspension from a harvested cell population, is plated at various cell densities, such as 50, 100, 250, 500, or other increments between 1 and 1000 cells per well. Preferably, the sub-population comprises no more than about 10,000 antigen-specific, antibody-secreting cells, more preferably about 50-10,000, about 50-5,000, about 50-1,000, about 50-500, about 50-250 antigen-specific, antibody-secreting cells, or increments therein. Then, these sub-populations are cultured with suitable medium (e.g., an activated T cell conditioned medium, particularly 1-5% activated rabbit T cell conditioned medium) on a feeder layer, preferably under conditions that favor the survival of a single proliferating antibody-secreting cell per culture well. The feeder layer, generally comprised of irradiated cell matter, e.g., EL4B cells, does not constitute part of the cell population. The cells are cultured in a suitable media for a time sufficient for antibody production, for example about 1 day to about 2 weeks, about 1 day to about 10 days, at least about 3 days, about 3 to about 5 days, about 5 days to about 7 days, at least about 7 days, or other increments therein. In one embodiment, more than one sub-population is cultured simultaneously. Preferably, a single antibody-producing cell and progeny thereof survives in each well, thereby providing a clonal population of antigen-specific B cells in each well. At this stage, the immunoglobulin G (IgG) produced by the clonal population is highly correlative with antigen specificity. In a preferred embodiment, the IgGs exhibit a correlation with antigen specificity that is greater than about 50%, more preferably greater than 70%, 85%, 90%, 95%, 99%, or increments therein. See Fig. 3, which demonstrates an exemplary correlation for IL-6. The correlations were demonstrated by setting up B cell cultures under limiting conditions to establish single antigen-specific antibody products per well. Antigen-specific versus general IgG synthesis was compared. Three populations were observed: IgG that recognized a single format of antigen (biotinylated and direct coating), detectable IgG and antigen recognition irrespective of immobilization, and IgG production alone. IgG production was highly correlated with antigen-specificity.

A supernatant containing the antibodies is optionally collected, which can be enriched, screened, and/or cultured for antibody selection according to the steps described above. The supernatant may be enriched (preferably by an antigen-specificity assay, especially an ELISA assay) and/or screened for antibody functionality.

The enriched, preferably clonal, antigen-specific B cell population from which a supernatant described above is optionally screened in order to detect the presence of the desired secreted monoclonal antibody is used for the isolation of a few B cells, preferably a single B cell, which is then tested in an appropriate assay in order to confirm the presence of a single antibody-producing B cell in the clonal B cell population. About 1 to about 20 cells may be isolated from the clonal B cell population, preferably less than about 15, 12, 10, 5, or 3 cells, or increments therein, most preferably a single cell. The screen is preferably effected by an antigen-specificity assay, especially a halo assay. The halo assay can be performed with the full length protein, or a fragment thereof. The antibody-containing supernatant can also be screened for at least one of: antigen binding affinity; agonism or antagonism of antigen-ligand binding, induction or inhibition of the proliferation of a specific target cell type; induction or inhibition of lysis of a target cell, and induction or inhibition of a biological pathway involving the antigen.

The identified antigen-specific cell can be used to derive the corresponding nucleic acid sequences encoding the desired monoclonal antibody. (An AluI digest can confirm that only a single monoclonal antibody type is produced per well.) As mentioned above, these sequences can be mutated, such as by humanization, in order to render them suitable for use in human medicaments.

As mentioned, the enriched B cell population used in the process can also be further enriched, screened, and/or cultured for antibody selection according to the steps described above which can be repeated or performed in a different order. At least one cell of an enriched, preferably clonal, antigen-specific cell population may be isolated, cultured, and used for antibody selection.

There is described a method comprising:
a. harvesting a cell population from an immunized host to obtain a harvested cell population;
b. creating at least one single cell suspension from a harvested cell population;
c. enriching at least one single cell suspension, preferably by chromatography, to form a first enriched cell population;
d. enriching the first enriched cell population, preferably by ELISA assay, to form a second enriched cell population which preferably is clonal, i.e., it contains only a single type of antigen-specific B cell;
e. enriching the second enriched cell population, preferably by halo assay, to form a third enriched cell population containing a single or a few number of B cells that produce an antibody specific to a desired antigen; and
f. selecting an antibody produced by an antigen-specific cell isolated from the third enriched cell population.

The method can further include one or more steps of screening the harvested cell population for antibody binding strength (affinity, avidity) and/or antibody functionality. Suitable screening steps include, but are not limited to, assay methods that detect: whether the antibody produced by the identified antigen-specific B cell produces an antibody possessing a minimal antigen binding affinity, whether the antibody agonizes or antagonizes the binding of a desired antigen to a ligand; whether the antibody induces or inhibits the proliferation of a specific cell type; whether the antibody induces or elicits a cytolytic reaction against target cells; whether the antibody binds to a specific epitope; and whether the antibody modulates (inhibits or agonizes) a specific biological pathway or pathways involving the antigen.

Similarly, the method can include one or more steps of screening the second enriched cell population for antibody binding strength and/or antibody functionality.

The method can further include a step of sequencing the polypeptide sequence or the corresponding nucleic acid sequence of the selected antibody. The method can also include a step of producing a recombinant antibody using the sequence, a fragment thereof, or a genetically modified version of the selected antibody. Methods for mutating antibody sequences in order to retain desired properties are well known to those skilled in the art and include humanization, chimerisation, production of single chain antibodies; these mutation methods can yield recombinant antibodies possessing desired effector function, immunogenicity, stability, or removal or addition of glycosylation. The recombinant antibody can be produced by any suitable recombinant cell, including, but not limited to mammalian cells such as CHO, COS, BHK, HEK-293, bacterial cells, yeast cells, plant cells, insect cells, and amphibian cells. The antibodies may be expressed in polyploidal yeast cells, i.e., diploid yeast cells, particularly Pichia.

Ther eis described a method comprising:
a. immunizing a host against an antigen to yield host antibodies;
b. screening the host antibodies for antigen specificity and neutralization;
c. harvesting B cells from the host;
d. enriching the harvested B cells to create an enriched cell population having an increased frequency of antigen-specific cells;
e. culturing one or more sub-populations from the enriched cell population under conditions that favor the survival of a single B cell to produce a clonal population in at least one culture well;
f. determining whether the clonal population produces an antibody specific to the antigen;
g. isolating a single B cell; and
h. sequencing the nucleic acid sequence of the antibody produced by the single B cell.

### Methods of Humanizing Antibodies

There is described a method for humanizing antibody heavy and light chains. The following method may be followed for the humanization of the heavy and light chains:

### Light Chain

1. Identify the amino acid that is the first one following the signal peptide sequence. This is the start of Framework 1. The signal peptide starts at the first initiation methionine and is typically, but not necessarily 22 amino acids in length for rabbit light chain protein sequences. The start of the mature polypeptide can also be determined experimentally by N-terminal protein sequencing, or can be predicted using a prediction algorithm. This is also the start of Framework 1 as classically defined by those in the field.
   Example: RbtVL Amino acid residue 1 in Fig. 2, starting 'AYDM...'
2. Identify the end of Framework 3. This is typically 86-90 amino acids following the start of Framework 1 and is typically a cysteine residue preceded by two tyrosine residues. This is the end of the Framework 3 as classically defined by those in the field.
   Example: RbtVL amino acid residue 88 in Fig. 2, ending as 'TYYC'
3. Use the rabbit light chain sequence of the polypeptide starting from the beginning of Framework 1 to the end of Framework 3 as defined above and perform a sequence homology search for the most similar human antibody protein sequences. This will typically be a search against human germline sequences prior to antibody maturation in order to reduce the possibility of immunogenicity, however any human sequences can be used. Typically a program like BLAST can be used to search a database of sequences for the most homologous. Databases of human antibody sequences can be found from various sources such as NCBI (National Center for Biotechnology Information).
   Example: RbtVL amino acid sequence from residues numbered 1 through 88 in Fig. 2 is BLASTed against a human antibody germline database. The top three unique returned sequences are shown in Fig. 2 as L12A, VI and Vx02.
4. Generally the most homologous human germline variable light chain sequence is then used as the basis for humanization. However those skilled in the art may decide to use another sequence that wasn't the highest homology as determined by the homology algorithm, based on other factors including sequence gaps and framework similarities.
   Example: In Fig. 2, L12A was the most homologous human germline variable light chain sequence and is used as the basis for the humanization of RbtVL.
5. Determine the framework and CDR arrangement (FR1, FR2, FR3, CDR1 & CDR2) for the human homolog being used for the light chain humanization. This is using the traditional layout as described in the field. Align the rabbit variable light chain sequence with the human homolog, while maintaining the layout of the framework and CDR regions.
   Example: In Fig. 2, the RbtVL sequence is aligned with the human homologous sequence L12A, and the framework and CDR domains are indicated.
6. Replace the human homologous light chain sequence CDR1 and CDR2 regions with the CDR1 and CDR2 sequences from the rabbit sequence. If there are differences in length between the rabbit and human CDR sequences then use the entire rabbit CDR sequences and their lengths. It is possible that the specificity, affinity and/or immunogenicity of the resulting humanized antibody may be unaltered if smaller or larger sequence exchanges are performed, or if specific residue(s) are altered, however the exchanges as described have been used successfully, but do not exclude the possibility that other changes may be permitted.
   Example: In Fig. 2, the CDR1 and CDR2 amino acid residues of the human homologous variable light chain L12A are replaced with the CDR1 and CDR2 amino acid sequences from the RbtVL rabbit antibody light chain sequence. The human L12A frameworks 1, 2 and 3 are unaltered. The resulting humanized sequence is shown below as VLh from residues numbered 1 through 88. Note that the only residues that are different from the L12A human sequence are underlined, and are thus rabbit-derived amino acid residues. In this example only 8 of the 88 residues are different than the human sequence.
7. After framework 3 of the new hybrid sequence created in Step 6, attach the entire CDR3 of the rabbit light chain antibody sequence. The CDR3 sequence can be of various lengths, but is typically 9 to 15 amino acid residues in length. The CDR3 region and the beginning of the following framework 4 region are defined classically and identifiable by those skilled in the art. Typically the beginning of Framework 4, and thus after the end of CDR3 consists of the sequence 'FGGG...', however some variation may exist in these residues.
   Example: In Fig. 2, the CDR3 of RbtVL (amino acid residues numbered 89-100) is added after the end of framework 3 in the humanized sequence indicated as VLh.
8. The rabbit light chain framework 4, which is typically the final 11 amino acid residues of the variable light chain and begins as indicated in Step 7 above and typically ends with the amino acid sequence '...VVKR' is replaced with the nearest human light chain framework 4 homolog, usually from germline sequence. Frequently this human light chain framework 4 is of the sequence 'FGGGTKVEIKR'. It is possible that other human light chain framework 4 sequences that are not the most homologous or otherwise different may be used without affecting the specificity, affinity and/or immunogenicity of the resulting humanized antibody. This human light chain framework 4 sequence is added to the end of the variable light chain humanized sequence immediately following the CDR3 sequence from Step 7 above. This is now the end of the variable light chain humanized amino acid sequence.
   Example: In Fig. 2, Framework 4 (FR4) of the RbtVL rabbit light chain sequence is shown above a homologous human FR4 sequence. The human FR4 sequence is added to the humanized variable light chain sequence (VLh) right after the end of the CD3 region added in Step 7 above.

### Heavy Chain

1. Identify the amino acid that is the first one following the signal peptide sequence. This is the start of Framework 1. The signal peptide starts at the first initiation methionine and is typically 19 amino acids in length for rabbit heavy chain protein sequences. Typically, but not necessarily always, the final 3 amino acid residues of a rabbit heavy chain signal peptide are '...VQC', followed by the start of Framework 1. The start of the mature polypeptide can also be determined experimentally by N-terminal protein sequencing, or can be predicted using a prediction algorithm. This is also the start of Framework 1 as classically defined by those in the field.
   Example: RbtVH Amino acid residue 1 in Fig. 2, starting 'QEQL...'
2. Identify the end of Framework 3. This is typically 95-100 amino acids following the start of Framework 1 and typically has the final sequence of '...CAR' (although the alanine can also be a valine). This is the end of the Framework 3 as classically defined by those in the field.
   Example: RbtVH amino acid residue 98 in Fig. 2, ending as '...FCVR'.
3. Use the rabbit heavy chain sequence of the polypeptide starting from the beginning of Framework 1 to the end of Framework 3 as defined above and perform a sequence homology search for the most similar human antibody protein sequences. This will typically be against a database of human germline sequences prior to antibody maturation in order to reduce the possibility of immunogenicity, however any human sequences can be used. Typically a program like BLAST can be used to search a database of sequences for the most homologous. Databases of human antibody sequences can be found from various sources such as NCBI (National Center for Biotechnology Information).
   Example: RbtVH amino acid sequence from residues numbered 1 through 98 in Fig. 2 is BLASTed against a human antibody germline database. The top three unique returned sequences are shown in Fig. 2 as 3-64-04, 3-66-04, and 3-53-02.
4. Generally the most homologous human germline variable heavy chain sequence is then used as the basis for humanization. However those skilled in the art may decide to use another sequence that wasn't the most homologous as determined by the homology algorithm, based on other factors including sequence gaps and framework similarities.
   Example: 3-64-04 in Fig. 2 was the most homologous human germline variable heavy chain sequence and is used as the basis for the humanization of RbtVH.
5. Determine the framework and CDR arrangement (FR1, FR2, FR3, CDR1 & CDR2) for the human homolog being used for the heavy chain humanization. This is using the traditional layout as described in the field. Align the rabbit variable heavy chain sequence with the human homolog, while maintaining the layout of the framework and CDR regions.
   Example: In Fig. 2, the RbtVH sequence is aligned with the human homologous sequence 3-64-04, and the framework and CDR domains are indicated.
6. Replace the human homologous heavy chain sequence CDR1 and CDR2 regions with the CDR1 and CDR2 sequences from the rabbit sequence. If there are differences in length between the rabbit and human CDR sequences then use the entire rabbit CDR sequences and their lengths. In addition, it may be necessary to replace the final three amino acids of the human heavy chain Framework 1 region with the final three amino acids of the rabbit heavy chain Framework 1. Typically but not always, in rabbit heavy chain Framework 1 these three residues follow a Glycine residue preceded by a Serine residue. In addition, it may be necessary replace the final amino acid of the human heavy chain Framework 2 region with the final amino acid of the rabbit heavy chain Framework 2. Typically, but not necessarily always, this is a Glycine residue preceded by an Isoleucine residue in the rabbit heavy chain Framework 2. It is possible that the specificity, affinity and/or immunogenicity of the resulting humanized antibody may be unaltered if smaller or larger sequence exchanges are performed, or if specific residue(s) are altered, however the exchanges as described have been used successfully, but do not exclude the possibility that other changes may be permitted. For example, a tryptophan amino acid residue typically occurs four residues prior to the end of the rabbit heavy chain CDR2 region, whereas in human heavy chain CDR2 this residue is typically a Serine residue. Changing this rabbit tryptophan residue to a the human Serine residue at this position has been demonstrated to have minimal to no effect on the humanized antibody's specificity or affinity, and thus further minimizes the content of rabbit sequence-derived amino acid residues in the humanized sequence.
   Example: In Fig. 2, The CDR1 and CDR2 amino acid residues of the human homologous variable heavy chain are replaced with the CDR1 and CDR2 amino acid sequences from the RbtVH rabbit antibody light chain sequence, except for the boxed residue, which is tryptophan in the rabbit sequence (position number 63) and Serine at the same position in the human sequence, and is kept as the human Serine residue. In addition to the CDR1 and CDR2 changes, the final three amino acids of Framework 1 (positions 28-30) as well as the final residue of Framework 2 (position 49) are retained as rabbit amino acid residues instead of human. The resulting humanized sequence is shown below as VHh from residues numbered 1 through 98. Note that the only residues that are different from the 3-64-04 human sequence are underlined, and are thus rabbit-derived amino acid residues. In this example only 15 of the 98 residues are different than the human sequence.
7. After framework 3 of the new hybrid sequence created in Step 6, attach the entire CDR3 of the rabbit heavy chain antibody sequence. The CDR3 sequence can be of various lengths, but is typically 5 to 19 amino acid residues in length. The CDR3 region and the beginning of the following framework 4 region are defined classically and are identifiable by those skilled in the art. Typically the beginning of framework 4, and thus after the end of CDR3 consists of the sequence WGXG...(where X is usually Q or P), however some variation may exist in these residues.
   Example: The CDR3 of RbtVH (amino acid residues numbered 99-110) is added after the end of framework 3 in the humanized sequence indicated as VHh.
8. The rabbit heavy chain framework 4, which is typically the final 11 amino acid residues of the variable heavy chain and begins as indicated in Step 7 above and typically ends with the amino acid sequence '...TVSS' is replaced with the nearest human heavy chain framework 4 homolog, usually from germline sequence. Frequently this human heavy chain framework 4 is of the sequence 'WGQGTLVTVSS'. It is possible that other human heavy chain framework 4 sequences that are not the most homologous or otherwise different may be used without affecting the specificity, affinity and/or immunogenicity of the resulting humanized antibody. This human heavy chain framework 4 sequence is added to the end of the variable heavy chain humanized sequence immediately following the CDR3 sequence from Step 7 above. This is now the end of the variable heavy chain humanized amino acid sequence.
   Example: In Fig. 2, framework 4 (FR4) of the RbtVH rabbit heavy chain sequence is shown above a homologous human heavy FR4 sequence. The human FR4 sequence is added to the humanized variable heavy chain sequence (VHh) right after the end of the CD3 region added in Step 7 above.

### Methods of Producing Antibodies and Fragments thereof

Recombinant polypeptides corresponding to the antibodies described herein or fragments thereof are secreted from polyploidal, preferably diploid or tetraploid strains of mating competent yeast. There are described methods for producing these recombinant polypeptides in secreted form for prolonged periods using cultures comprising polyploid yeast, i.e., at least several days to a week, more preferably at least a month or several months, and even more preferably at least 6 months to a year or longer. These polyploid yeast cultures will express at least 10-25 mg/liter of the polypeptide, more preferably at least 50-250 mg/liter, still more preferably at least 500-1000 mg/liter, and most preferably a gram per liter or more of the recombinant polypeptide(s).

A pair of genetically marked yeast haploid cells may be transformed with expression vectors comprising subunits of a desired heteromultimeric protein. One haploid cell comprises a first expression vector, and a second haploid cell comprises a second expression vector. Diploid yeast cells may be be transformed with one or more expression vectors that provide for the expression and secretion of one or more of the recombinant polypeptides. In still another embodiment a single haploid cell may be transformed with one or more vectors and used to produce a polyploidal yeast by fusion or mating strategies. A diploid yeast culture may be transformed with one or more vectors providing for the expression and secretion of a desired polypeptide or polypeptides. These vectors may comprise vectors e.g., linearized plasmids or other linear DNA products that integrate into the yeast cell's genome randomly, through homologous recombination, or using a recombinase such as Cre/Lox or Flp/Frt. Optionally, additional expression vectors may be introduced into the haploid or diploid cells; or the first or second expression vectors may comprise additional coding sequences; for the synthesis of heterotrimers; heterotetramers; *etc.* The expression levels of the non-identical polypeptides may be individually calibrated, and adjusted through appropriate selection, vector copy number, promoter strength and/or induction. The transformed haploid cells are genetically crossed or fused. The resulting diploid or tetraploid strains are utilized to produce and secrete fully assembled and biologically functional proteins, humanized antibodies described herein or fragments thereof.

The use of diploid or tetraploid cells for protein production provides for unexpected benefits. The cells can be grown for production purposes, *i.e.* scaled up, and for extended periods of time, in conditions that can be deleterious to the growth of haploid cells, which conditions may include high cell density; growth in minimal media; growth at low temperatures; stable growth in the absence of selective pressure; and which may provide for maintenance of heterologous gene sequence integrity and maintenance of high level expression over time. Without wishing to be bound thereby, the inventors theorize that these benefits may arise, at least in part, from the creation of diploid strains from two distinct parental haploid strains. Such haploid strains can comprise numerous minor autotrophic mutations, which mutations are complemented in the diploid or tetraploid, enabling growth and enhanced production under highly selective conditions.

Transformed mating competent haploid yeast cells provide a genetic method that enables subunit pairing of a desired protein. Haploid yeast strains are transformed with each of two expression vectors, a first vector to direct the synthesis of one polypeptide chain and a second vector to direct the synthesis of a second, non-identical polypeptide chain. The two haploid strains are mated to provide a diploid host where optimized target protein production can be obtained.

Optionally, additional non-identical coding sequence(s) are provided. Such sequences may be present on additional expression vectors or in the first or the second expression vectors. As is known in the art, multiple coding sequences may be independently expressed from individual promoters; or may be coordinately expressed through the inclusion of an "internal ribosome entry site" or "IRES", which is an element that promotes direct internal ribosome entry to the initiation codon, such as ATG, of a cistron (a protein encoding region), thereby leading to the cap-independent translation of the gene. IRES elements functional in yeast are described by Thompson et al. (2001) P.N.A.S. 98:12866-12868.

Antibody sequences may be produced in combination with a secretory J chain, which provides for enhanced stability of IgA (see U.S. Patent Nos. 5,959,177; and 5,202,422).

The two haploid yeast strains may each be auxotrophic, and require supplementation of media for growth of the haploid cells. The pair of auxotrophs are complementary, such that the diploid product will grow in the absence of the supplements required for the haploid cells. Many such genetic markers are known in yeast, including requirements for amino acids (*e.g. met, lys, his, arg, etc*.), and nucleosides (*e.g. ura3, ade1, etc*.). Amino acid markers may be preferred. Alternatively diploid cells which contain the desired vectors can be selected by other means, e.g., by use of other markers, such as green fluorescent protein, antibiotic resistance genes, and various dominant selectable markers.

Two transformed haploid cells may be genetically crossed and diploid strains arising from this mating event selected by their hybrid nutritional requirements and/or antibiotic resistance spectra. Alternatively, populations of the two transformed haploid strains are spheroplasted and fused, and diploid progeny regenerated and selected. By either method, diploid strains can be identified and selectively grown based on their ability to grow in different media than their parents. For example, the diploid cells may be grown in minimal medium that may include antibiotics. The diploid synthesis strategy has certain advantages. Diploid strains have the potential to produce enhanced levels of heterologous protein through broader complementation to underlying mutations, which may impact the production and/or secretion of recombinant protein. Furthermore, once stable strains have been obtained, any antibiotics used to select those strains do not necessarily need to be continuously present in the growth media.

As noted above, a haploid yeast may be transformed with a single or multiple vectors and mated or fused with a non-transformed cell to produce a diploid cell containing the vector or vectors. Alternatively, a diploid yeast cell may be transformed with one or more vectors that provide for the expression and secretion of a desired heterologous polypeptide by the diploid yeast cell.

Two haploid strains may be transformed with a library of polypeptides, *e.g.* a library of antibody heavy or light chains. Transformed haploid cells that synthesize the polypeptides are mated with the complementary haploid cells. The resulting diploid cells are screened for functional protein. The diploid cells provide a means of rapidly, conveniently and inexpensively bringing together a large number of combinations of polypeptides for functional testing. This technology is especially applicable for the generation of heterodimeric protein products, where optimized subunit synthesis levels are critical for functional protein expression and secretion.

The expression level ratio of the two subunits may be regulated in order to maximize product generation. Heterodimer subunit protein levels have been shown previously to impact the final product generation (Simmons LC, J Immunol Methods. 2002 May 1;263(1-2):133-47). Regulation can be achieved prior to the mating step by selection for a marker present on the expression vector. By stably increasing the copy number of the vector, the expression level can be increased. In some cases, it may be desirable to increase the level of one chain relative to the other, so as to reach a balanced proportion between the subunits of the polypeptide. Antibiotic resistance markers are useful for this purpose, *e.g.* Zeocin™ (phleomycin) resistance marker, G418 resistance, *etc.* and provide a means of enrichment for strains that contain multiple integrated copies of an expression vector in a strain by selecting for transformants that are resistant to higher levels of Zeocin™ (phleomycin) or G418. The proper ratio, *e.g.* 1:1; 1:2; *etc.* of the subunit genes may be important for efficient protein production. Even when the same promoter is used to transcribe both subunits, many other factors contribute to the final level of protein expressed and therefore, it can be useful to increase the number of copies of one encoded gene relative to the other. Alternatively, diploid strains that produce higher levels of a polypeptide, relative to single copy vector strains, are created by mating two haploid strains, both of which have multiple copies of the expression vectors.

Host cells are transformed with the above-described expression vectors, mated to form diploid strains, and cultured in conventional nutrient media modified as appropriate for inducing promoters, selecting transformants or amplifying the genes encoding the desired sequences. A number of minimal media suitable for the growth of yeast are known in the art. Any of these media may be supplemented as necessary with salts (such as sodium chloride, calcium, magnesium, and phosphate), buffers (such as phosphate, HEPES), nucleosides (such as adenosine and thymidine), antibiotics, trace elements, and glucose or an equivalent energy source. Any other necessary supplements may also be included at appropriate concentrations that would be known to those skilled in the art. The culture conditions, such as temperature and pH, are those previously used with the host cell selected for expression, and will be apparent to the ordinarily skilled artisan.

Secreted proteins are recovered from the culture medium. A protease inhibitor, such as phenyl methyl sulfonyl fluoride (PMSF) may be useful to inhibit proteolytic degradation during purification, and antibiotics may be included to prevent the growth of adventitious contaminants. The composition may be concentrated, filtered, dialyzed, etc., using methods known in the art.

The diploid cells of the invention are grown for production purposes. Such production purposes desirably include growth in minimal media, which media lacks pre-formed amino acids and other complex biomolecules, e.g., media comprising ammonia as a nitrogen source, and glucose as an energy and carbon source, and salts as a source of phosphate or calcium. Preferably such production media lacks selective agents such as antibiotics, amino acids, purines, pyrimidines, *etc.* The diploid cells can be grown to high cell density, for example at least about 50 g/L; more usually at least about 100 g/L; and may be at least about 300, about 400, about 500 g/L or more.

The growth of the subject cells for production purposes may be performed at low temperatures, which temperatures may be lowered during log phase, during stationary phase, or both. The term "low temperature" refers to temperatures of at least about 15 °C, more usually at least about 17 °C, and may be about 20 °C, and is usually not more than about 25 °C, more usually not more than about 22 °C. The low temperature is usually not more than about 28 °C. Growth temperature can impact the production of full-length secreted proteins in production cultures, and decreasing the culture growth temperature can strongly enhance the intact product yield. The decreased temperature appears to assist intracellular trafficking through the folding and post-translational processing pathways used by the host to generate the target product, along with reduction of cellular protease degradation.

The described methods provide for expression of secreted, active protein, preferably a mammalian protein. Secreted, "active antibodies", as used herein, refers to a correctly folded multimer of at least two properly paired chains, which accurately binds to its cognate antigen. Expression levels of active protein are usually at least about 10-50 mg/liter culture, more usually at least about 100 mg/liter, preferably at least about 500 mg/liter, and may be 1000 mg/liter or more.

The described methods can provide for increased stability of the host and heterologous coding sequences during production. The stability is evidenced, for example, by maintenance of high levels of expression of time, where the starting level of expression is decreased by not more than about 20%, usually not more than 10%, and may be decreased by not more than about 5% over about 20 doublings, 50 doublings, 100 doublings, or more.

The strain stability also provides for maintenance of heterologous gene sequence integrity over time, where the sequence of the active coding sequence and requisite transcriptional regulatory elements are maintained in at least about 99% of the diploid cells, usually in at least about 99.9% of the diploid cells, and preferably in at least about 99.99% of the diploid cells over about 20 doublings, 50 doublings, 100 doublings, or more. Preferably, substantially all of the diploid cells maintain the sequence of the active coding sequence and requisite transcriptional regulatory elements.

Other methods of producing antibodies are well known to those of ordinary skill in the art. For example, methods of producing chimeric antibodies are now well known in the art (*See,* for example, U.S. Patent No. 4,816,567 to Cabilly et al.*;* Morrison et al., P.N.A.S. USA, 81:8651-55 (1984); Neuberger, M.S. et al., Nature, 314:268-270 (1985); Boulianne, G.L. et al., Nature, 312:643-46 (1984)).

Likewise, other methods of producing humanized antibodies are now well known in the art (*See,* for example, U.S. Patent Nos. 5,530,101, 5,585,089, 5,693,762, and 6,180,370 to Queen et al*;* U.S. Patent Nos. 5,225,539 and 6,548,640 to Winter; U.S. Patent Nos. 6,054,297, 6,407,213 and 6,639,055 to Carter et al*;* U.S. Patent No. 6,632,927 to Adair; Jones, P.T. et al, Nature, 321:522-525 (1986); Reichmann, L., et al, Nature, 332:323-327 (1988); Verhoeyen, M, et al, Science, 239:1534-36 (1988)).

Antibody polypeptides having IL-6 binding specificity may also be produced by constructing, using conventional techniques well known to those of ordinary skill in the art, an expression vector containing an operon and a DNA sequence encoding an antibody heavy chain in which the DNA sequence encoding the CDRs required for antibody specificity is derived from a non-human cell source, preferably a rabbit B-cell source, while the DNA sequence encoding the remaining parts of the antibody chain is derived from a human cell source.

A second expression vector is produced using the same conventional means well known to those of ordinary skill in the art, said expression vector containing an operon and a DNA sequence encoding an antibody light chain in which the DNA sequence encoding the CDRs required for antibody specificity is derived from a non-human cell source, preferably a rabbit B-cell source, while the DNA sequence encoding the remaining parts of the antibody chain is derived from a human cell source.

The expression vectors are transfected into a host cell by convention techniques well known to those of ordinary skill in the art to produce a transfected host cell, said transfected host cell cultured by conventional techniques well known to those of ordinary skill in the art to produce said antibody polypeptides.

The host cell may be co-transfected with the two expression vectors described above, the first expression vector containing DNA encoding an operon and a light chain-derived polypeptide and the second vector containing DNA encoding an operon and a heavy chain-derived polypeptide. The two vectors contain different selectable markers, but preferably achieve substantially equal expression of the heavy and light chain polypeptides. Alternatively, a single vector may be used, the vector including DNA encoding both the heavy and light chain polypeptides. The coding sequences for the heavy and light chains may comprise cDNA.

The host cells used to express the antibody polypeptides may be either a bacterial cell such as *E. coli,* or a eukaryotic cell. A mammalian cell of a well-defined type for this purpose, such as a myeloma cell or a Chinese hamster ovary (CHO) cell line may be used.

The general methods by which the vectors may be constructed, transfection methods required to produce the host cell and culturing methods required to produce the antibody polypeptides from said host cells all include conventional techniques. Although preferably the cell line used to produce the antibody is a mammalian cell line, any other suitable cell line, such as a bacterial cell line such as an *E. coli*-derived bacterial strain, or a yeast cell line, may alternatively be used.

Similarly, once produced the antibody polypeptides may be purified according to standard procedures in the art, such as for example cross-flow filtration, ammonium sulphate precipitation, or affinity column chromatography.

The antibody polypeptides described herein may also be used for the design and synthesis of either peptide or non-peptide mimetics that would be useful for the same therapeutic applications as the antibody polypeptides of the invention. *See,* for example, Saragobi et al, Science, 253:792-795 (1991).

### Methods of Ameliorating or Reducing Symptoms of, or Treating, or Preventing, Diseases and Disorders Associated with, IL-6

The invention relates to use of anti-IL-6 antibodies described herein, or fragments thereof, for ameliorating or reducing the symptoms of, or treating, or preventing, cachexia in a patient diagnosed with and IL-6 associated disorder wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof. Anti-IL-6 antibodies described herein, or fragments thereof, can also be administered in a therapeutically effective amount to patients in need of treatment of diseases and disorders associated with IL-6 in the form of a pharmaceutical composition as described in greater detail below.

In another embodiment of the invention, anti-IL-6 antibodies described herein, or fragments thereof, are administered to a patient in combination with another active agent. For example, an IL-6 antibody or antibody fragment may be co-administered with one or more chemotherapy agents, such as VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof.

The anti-IL-6 antibodies described herein, or fragments thereof, are useful for ameliorating or reducing the symptoms of, or treating, or preventing, cachexia. Diseases and disorders associated with cachexia include, but are not limited to, cancer-related cachexia, cardiac-related cachexia, respiratory-related cachexia, renal-related cachexia and age-related cachexia. *See,* for example, Barton, BE., Interleukin-6 and new strategies for the treatment of cancer, hyperproliferative diseases and paraneoplastic syndromes, Expert Opin Ther Targets, 2005 Aug;9(4):737-52; Zaki MH, et al, CNTO 328, a monoclonal antibody to IL-6, inhibits human tumor-induced cachexia in nude mice, Int J Cancer, 2004 Sep 10;111(4):592-5; Trikha M, et al, Targeted anti-interleukin-6 monoclonal antibody therapy for cancer: a review of the rationale and clinical evidence, Clin Cancer Res., 2003 Oct 15;9(13):4653-65; Lelli G, et al, Treatment of the cancer anorexia-cachexia syndrome: a critical reappraisal, J Chemother., 2003 Jun;15(3):220-5; Argiles JM, et al, Cytokines in the pathogenesis of cancer cachexia, Curr Opin Clin Nutr Metab Care, 2003 Jul;6(4):401-6; Barton BE., IL-6-like cytokines and cancer cachexia: consequences of chronic inflammation, Immunol Res., 2001;23(1):41-58; Yamashita JI, et al, Medroxyprogesterone acetate and cancer cachexia: interleukin-6 involvement, Breast Cancer, 2000;7(2):130-5; Yeh SS, et al, Geriatric cachexia: the role of cytokines, Am J Clin Nutr., 1999 Aug;70(2): 183-97; Strassmann G, et al, Inhibition of experimental cancer cachexia by anti-cytokine and anti-cytokine-receptor therapy, Cytokines Mol Ther., 1995 Jun;1(2):107-13; Fujita J, et al, Anti-interleukin-6 receptor antibody prevents muscle atrophy in colon-26 adenocarcinoma-bearing mice with modulation of lysosomal and ATP-ubiquitin-dependent proteolytic pathways, Int J Cancer, 1996 Nov 27;68(5):637-43; Tsujinaka T, et al, Interleukin 6 receptor antibody inhibits muscle atrophy and modulates proteolytic systems in interleukin 6 transgenic mice, J Clin Invest., 1996 Jan 1;97(1):244-9; Emilie D, et al, Administration of an anti-interleukin-6 monoclonal antibody to patients with acquired immunodeficiency syndrome and lymphoma: effect on lymphoma growth and on B clinical Symptoms, Blood, 1994 Oct 15;84 (8):2472-9; and Strassmann G, et al, Evidence for the involvement of interleukin 6 in experimental cancer cachexia, J Clin Invest., 1992 May;89(5):1681-4.

### Administration

In one embodiment of the invention, the anti-IL-6 antibodies described herein, or IL-6 binding fragments thereof, as well as combinations of said antibody fragments, are administered to a subject at a concentration of between about 0.1 and 20 mg/kg, such as about 0.4 mg/kg, about 0.8 mg/kg, about 1.6 mg/kg, or about 4 mg/kg, of body weight of recipient subject. In a preferred embodiment of the invention, the anti-IL-6 antibodies described herein, or IL-6 binding fragments thereof, as well as combinations of said antibody fragments, are administered to a subject at a concentration of about 0.4 mg/kg of body weight of recipient subject. In a preferred embodiment of the invention, the anti-IL-6 antibodies described herein, or IL-6 binding fragments thereof, as well as combinations of said antibody fragments, are administered to a recipient subject with a frequency of once every twenty-six weeks or less, such as once every sixteen weeks or less, once every eight weeks or less, or once every four weeks, or less. In another preferred embodiment of the invention, the anti-IL-6 antibodies described herein, or IL-6 binding fragments thereof, as well as combinations thereof, are administered to a recipient subject with a frequency at most once per period of approximately one week, such as at most once per period of approximately two weeks, such as at most once per period of approximately four weeks, such as at most once per period of approximately eight weeks, such as at most once per period of approximately twelve weeks, such as at most once per period of approximately sixteen weeks, such as at most once per period of approximately twenty-four weeks.

It is understood that the effective dosage may depend on recipient subject attributes, such as, for example, age, gender, pregnancy status, body mass index, lean body mass, condition or conditions for which the composition is given, other health conditions of the recipient subject that may affect metabolism or tolerance of the composition, levels of IL-6 in the recipient subject, and resistance to the composition (for example, arising from the patient developing antibodies against the composition). A person of skill in the art would be able to determine an effective dosage and frequency of administration through routine experimentation, for example guided by the disclosure herein and the teachings in Goodman, L. S., Gilman, A., Brunton, L. L., Lazo, J. S., & Parker, K. L. (2006). Goodman & Gilman's the pharmacological basis of therapeutics. New York: McGraw-Hill; Howland, R. D., Mycek, M. J., Harvey, R. A., Champe, P. C., & Mycek, M. J. (2006). Pharmacology. Lippincott's illustrated reviews. Philadelphia: Lippincott Williams & Wilkins; and Golan, D. E. (2008). Principles of pharmacology: the pathophysiologic basis of drug therapy. Philadelphia, Pa., [etc.]: Lippincott Williams & Wilkins.

In another embodiment of the invention, the anti-IL-6 antibodies described herein, or IL-6 binding fragments thereof, as well as combinations of said antibody fragments, are administered to a subject in a pharmaceutical formulation.

A "pharmaceutical composition" refers to a chemical or biological composition suitable for administration to a mammal. Such compositions may be specifically formulated for administration *via* one or more of a number of routes, including but not limited to buccal, epicutaneous, epidural, inhalation, intraarterial, intracardial, intracerebroventricular, intradermal, intramuscular, intranasal, intraocular, intraperitoneal, intraspinal, intrathecal, intravenous, oral, parenteral, rectally *via* an enema or suppository, subcutaneous, subdermal, sublingual, transdermal, and transmucosal. In addition, administration can occur by means of injection, powder, liquid, gel, drops, or other means of administration.

In one embodiment of the invention, the anti-IL-6 antibodies described herein, or IL-6 binding fragments thereof, as well as combinations of said antibody fragments, may be optionally administered in combination with one or more active agents. Such active agents include analgesic, antipyretic, anti-inflammatory, antibiotic, antiviral, and anti-cytokine agents. Active agents include agonists, antagonists, and modulators of TNF-α, IL-2, IL-4, IL-6, IL-10, IL-12, IL-13, IL-18, IFN-α, IFN-γ, BAFF, CXCL13, IP-10, VEGF, EPO, EGF, HRG, Hepatocyte Growth Factor (HGF), Hepcidin, including antibodies reactive against any of the foregoing, and antibodies reactive against any of their receptors. Active agents also include 2-Arylpropionic acids, Aceclofenac, Acemetacin, Acetylsalicylic acid (Aspirin), Alclofenac, Alminoprofen, Amoxiprin, Ampyrone, Arylalkanoic acids, Azapropazone, Benorylate/Benorilate, Benoxaprofen, Bromfenac, Carprofen, Celecoxib, Choline magnesium salicylate, Clofezone, COX-2 inhibitors, Dexibuprofen, Dexketoprofen, Diclofenac, Diflunisal, Droxicam, Ethenzamide, Etodolac, Etoricoxib, Faislamine, fenamic acids, Fenbufen, Fenoprofen, Flufenamic acid, Flunoxaprofen, Flurbiprofen, Ibuprofen, Ibuproxam, Indometacin, Indoprofen, Kebuzone, Ketoprofen, Ketorolac, Lornoxicam, Loxoprofen, Lumiracoxib, Magnesium salicylate, Meclofenamic acid, Mefenamic acid, Meloxicam, Metamizole, Methyl salicylate, Mofebutazone, Nabumetone, Naproxen, N-Arylanthranilic acids, Oxametacin, Oxaprozin, Oxicams, Oxyphenbutazone, Parecoxib, Phenazone, Phenylbutazone, Phenylbutazone, Piroxicam, Pirprofen, profens, Proglumetacin, Pyrazolidine derivatives, Rofecoxib, Salicyl salicylate, Salicylamide, Salicylates, Sulfinpyrazone, Sulindac, Suprofen, Tenoxicam, Tiaprofenic acid, Tolfenamic acid, Tolmetin, and Valdecoxib. Antibiotics include Amikacin, Aminoglycosides, Amoxicillin, Ampicillin, Ansamycins, Arsphenamine, Azithromycin, Azlocillin, Aztreonam, Bacitracin, Carbacephem, Carbapenems, Carbenicillin, Cefaclor, Cefadroxil, Cefalexin, Cefalothin, Cefalotin, Cefamandole, Cefazolin, Cefdinir, Cefditoren, Cefepime, Cefixime, Cefoperazone, Cefotaxime, Cefoxitin, Cefpodoxime, Cefprozil, Ceftazidime, Ceftibuten, Ceftizoxime, Ceftobiprole, Ceftriaxone, Cefuroxime, Cephalosporins, Chloramphenicol, Cilastatin, Ciprofloxacin, Clarithromycin, Clindamycin, Cloxacillin, Colistin, Co-trimoxazole, Dalfopristin, Demeclocycline, Dicloxacillin, Dirithromycin, Doripenem, Doxycycline, Enoxacin, Ertapenem, Erythromycin, Ethambutol, Flucloxacillin, Fosfomycin, Furazolidone, Fusidic acid, Gatifloxacin, Geldanamycin, Gentamicin, Glycopeptides, Herbimycin, Imipenem, Isoniazid, Kanamycin, Levofloxacin, Lincomycin, Linezolid, Lomefloxacin, Loracarbef, Macrolides, Mafenide, Meropenem, Meticillin, Metronidazole, Mezlocillin, Minocycline, Monobactams, Moxifloxacin, Mupirocin, Nafcillin, Neomycin, Netilmicin, Nitrofurantoin, Norfloxacin, Ofloxacin, Oxacillin, Oxytetracycline, Paromomycin, Penicillin, Penicillins, Piperacillin, Platensimycin, Polymyxin B, Polypeptides, Prontosil, Pyrazinamide, Quinolones, Quinupristin, Rifampicin, Rifampin, Roxithromycin, Spectinomycin, Streptomycin, Sulfacetamide, Sulfamethizole, Sulfanilimide, Sulfasalazine, Sulfisoxazole, Sulfonamides, Teicoplanin, Telithromycin, Tetracycline, Tetracyclines, Ticarcillin, Tinidazole, Tobramycin, Trimethoprim, Trimethoprim-Sulfamethoxazole, Troleandomycin, Trovafloxacin, and Vancomycin. Active agents also include Aldosterone, Beclometasone, Betamethasone, Corticosteroids, Cortisol, Cortisone acetate, Deoxycorticosterone acetate, Dexamethasone, Fludrocortisone acetate, Glucocorticoids, Hydrocortisone, Methylprednisolone, Prednisolone, Prednisone, Steroids, and Triamcinolone. Antiviral agents include abacavir, aciclovir, acyclovir, adefovir, amantadine, amprenavir, an antiretroviral fixed dose combination, an antiretroviral synergistic enhancer, arbidol, atazanavir, atripla, brivudine, cidofovir, combivir, darunavir, delavirdine, didanosine, docosanol, edoxudine, efavirenz, emtricitabine, enfuvirtide, entecavir, entry inhibitors, famciclovir, fomivirsen, fosamprenavir, foscarnet, fosfonet, fusion inhibitor, ganciclovir, gardasil, ibacitabine, idoxuridine, imiquimod, imunovir, indinavir, inosine, integrase inhibitor, interferon, interferon type I, interferon type II, interferon type III, lamivudine, lopinavir, loviride, maraviroc, MK-0518, moroxydine, nelfinavir, nevirapine, nexavir, nucleoside analogues, oseltamivir, penciclovir, peramivir, pleconaril, podophyllotoxin, protease inhibitor, reverse transcriptase inhibitor, ribavirin, rimantadine, ritonavir, saquinavir, stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine. Any suitable combination of these active agents is also contemplated.

A "pharmaceutical excipient" or a "pharmaceutically acceptable excipient" is a carrier, usually a liquid, in which an active therapeutic agent is formulated. In one embodiment of the invention, the active therapeutic agent is a humanized antibody described herein, or one or more fragments thereof. The excipient generally does not provide any pharmacological activity to the formulation, though it may provide chemical and/or biological stability, and release characteristics. Exemplary formulations can be found, for example, in Remington's Pharmaceutical Sciences, 19th Ed., Grennaro, A., Ed., 1995.

As used herein "pharmaceutically acceptable carrier" or "excipient" includes any and all solvents, dispersion media, coatings, antibacterial and antifungal agents, isotonic and absorption delaying agents that are physiologically compatible. In one embodiment, the carrier is suitable for parenteral administration. Alternatively, the carrier can be suitable for intravenous, intraperitoneal, intramuscular, or sublingual administration. Pharmaceutically acceptable carriers include sterile aqueous solutions or dispersions and sterile powders for the extemporaneous preparation of sterile injectable solutions or dispersions. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

In one embodiment of the invention that may be used to intraveneously administer antibodies of the invention, including Ab1, for cancer indications, the administration formulation comprises, or alternatively consists of, about 10.5 mg/mL of antibody, 25 mM Histidine base, Phosphoric acid q.s. to pH 6, and 250 mM sorbitol.

In another embodiment of the invention that may be used to intraveneously administer antibodies of the invention, including Ab1, for cancer indications, the administration formulation comprises, or alternatively consists of, about 10.5 mg/mL of antibody, 12.5 mM Histidine base, 12.5 mM Histidine HCl (or 25 mM Histidine base and Hydrochloric acid q.s. to pH 6), 250 mM sorbitol, and 0.015% (w/w) Polysorbate 80.

In one embodiment of the invention that may be used to subcutaneously administer antibodies of the invention, including Ab1, for rheumatoid arthritis indications, the administration formulation comprises, or alternatively consists of, about 50 or 100 mg/mL of antibody, about 5 mM Histidine base, about 5 mM Histidine HCl to make final pH 6, 250 mM sorbitol, and 0.015% (w/w) Polysorbate 80.

In another embodiment of the invention that may be used to subcutaneously administer antibodies of the invention, including Ab1, for rheumatoid arthritis indications, the administration formulation comprises, or alternatively consists of, about 20 or 100 mg/mL of antibody, about 5 mM Histidine base, about 5 mM Histidine HCl to make final pH 6, 250 to 280 mM sorbitol (or sorbitol in combination with sucrose), and 0.015% (w/w) Polysorbate 80, said formulation having a nitrogen headspace in the shipping vials.

Pharmaceutical compositions typically must be sterile and stable under the conditions of manufacture and storage. The invention contemplates that the pharmaceutical composition is present in lyophilized form. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol), and suitable mixtures thereof. The invention further contemplates the inclusion of a stabilizer in the pharmaceutical composition.

In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the alkaline polypeptide can be formulated in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are known to those skilled in the art.

For each of the recited embodiments, the compounds can be administered by a variety of dosage forms. Any biologically-acceptable dosage form known to persons of ordinary skill in the art, and combinations thereof, are contemplated. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal), infusions, and combinations thereof.

Certain teachings related to methods for obtaining a clonal population of antigen-specific B cells were disclosed in U.S. Provisional patent application no. 60/801,412, filed May 19, 2006.

Certain teachings related to humanization of rabbit-derived monoclonal antibodies and preferred sequence modifications to maintain antigen binding affinity were disclosed in International Application No. 12/124,723, corresponding to Attorney Docket No. 67858.704001, entitled "Novel Rabbit Antibody Humanization Method and Humanized Rabbit Antibodies", filed May 21, 2008.

Certain teachings related to producing antibodies or fragments thereof using mating competent yeast and corresponding methods were disclosed in U.S. Patent application no. 11/429,053, filed May 8, 2006, (U.S. Patent Application Publication No. US2006/0270045).

Certain teachings related to anti-IL-6 antibodies, methods of producing antibodies or fragments thereof using mating competent yeast and corresponding methods were disclosed in U.S. provisional patent application no. 60/924,550, filed May 21, 2007

Certain teachings related to anti-IL-6 antibodies and methods of using those antibodies or fragments thereof to treat cachexia, weakness, fatigue and/or fever were disclosed in U.S. provisional patent application no. 61/117,839, filed November 25, 2008.

Certain anti-IL-6 antibody polynucleotides and polypeptides are disclosed in the sequence listing accompanying this patent application filing.

The following examples are put forth so as to provide those of ordinary skill in the art with a complete disclosure and description of how to make and use the subject invention,. Efforts have been made to ensure accuracy with respect to the numbers used (*e.g.* amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

### EXAMPLES

### Example 1 Production of Enriched Antigen-Specific B Cell Antibody Culture

Panels of antibodies are derived by immunizing traditional antibody host animals to exploit the native immune response to a target antigen of interest. Typically, the host used for immunization is a rabbit or other host that produces antibodies using a similar maturation process and provides for a population of antigen-specific B cells producing antibodies of comparable diversity, e.g., epitopic diversity. The initial antigen immunization can be conducted using complete Freund's adjuvant (CFA), and the subsequent boosts effected with incomplete adjuvant. At about 50-60 days after immunization, preferably at day 55, antibody titers are tested, and the Antibody Selection (ABS) process is initiated if appropriate titers are established. The two key criteria for ABS initiation are potent antigen recognition and function-modifying activity in the polyclonal sera.

At the time positive antibody titers are established, animals are sacrificed and B cell sources isolated. These sources include: the spleen, lymph nodes, bone marrow, and peripheral blood mononuclear cells (PBMCs). Single cell suspensions are generated, and the cell suspensions are washed to make them compatible for low temperature long term storage. The cells are then typically frozen.

To initiate the antibody identification process, a small fraction of the frozen cell suspensions are thawed, washed, and placed in tissue culture media. These suspensions are then mixed with a biotinylated form of the antigen that was used to generate the animal immune response, and antigen-specific cells are recovered using the Miltenyi magnetic bead cell selection methodology. Specific enrichment is conducted using streptavidin beads. The enriched population is recovered and progressed in the next phase of specific B cell isolation.

### Example 2 Production of Clonal, Antigen-Specific B Cell-Containing Culture

Enriched B cells produced according to Example 1 are then plated at varying cell densities per well in a 96 well microtiter plate. Generally, this is at 50, 100, 250, or 500 cells per well with 10 plates per group. The media is supplemented with 4% activated rabbit T cell conditioned media along with 50K frozen irradiated EL4B feeder cells. These cultures are left undisturbed for 5-7 days at which time supernatant-containing secreted antibody is collected and evaluated for target properties in a separate assay setting. The remaining supernatant is left intact, and the plate is frozen at -70 °C. Under these conditions, the culture process typically results in wells containing a mixed cell population that comprises a clonal population of antigen-specific B cells, i.e., a single well will only contain a single monoclonal antibody specific to the desired antigen.

### Example 3 Screening of Antibody Supernatants for Monoclonal Antibody of Desired Specificity and/or Functional Properties

Antibody-containing supernatants derived from the well containing a clonal antigen-specific B cell population produced according to Example 2 are initially screened for antigen recognition using ELISA methods. This includes selective antigen immobilization (e.g., biotinylated antigen capture by streptavidin coated plate), nonspecific antigen plate coating, or alternatively, through an antigen build-up strategy (e.g., selective antigen capture followed by binding partner addition to generate a heteromeric protein-antigen complex). Antigen-positive well supernatants are then optionally tested in a function-modifying assay that is strictly dependant on the ligand. One such example is an in vitro protein-protein interaction assay that recreates the natural interaction of the antigen ligand with recombinant receptor protein. Alternatively, a cell-based response that is ligand dependent and easily monitored (e.g., proliferation response) is utilized. Supernatant that displays significant antigen recognition and potency is deemed a positive well. Cells derived from the original positive well are then transitioned to the antibody recovery phase.

### Example 4 Recovery of Single, Antibody-Producing B Cell of Desired Antigen Specificity

Cells are isolated from a well that contains a clonal population of antigen-specific B cells (produced according to Example 2 or 3), which secrete a single antibody sequence. The isolated cells are then assayed to isolate a single, antibody-secreting cell. Dynal streptavidin beads are coated with biotinylated target antigen under buffered medium to prepare antigen-containing microbeads compatible with cell viability. Next antigen-loaded beads, antibody-producing cells from the positive well, and a fluorescein isothiocyanate (FITC)-labeled anti-host H&L IgG antibody (as noted, the host can be any mammalian host, e.g., rabbit, mouse, rat, etc.) are incubated together at 37 °C. This mixture is then re-pipetted in aliquots onto a glass slide such that each aliquot has on average a single, antibody-producing B-cell. The antigen-specific, antibody-secreting cells are then detected through fluorescence microscopy. Secreted antibody is locally concentrated onto the adjacent beads due to the bound antigen and provides localization information based on the strong fluorescent signal. Antibody-secreting cells are identified via FITC detection of antibody-antigen complexes formed adjacent to the secreting cell. The single cell found in the center of this complex is then recovered using a micromanipulator. The cell is snap-frozen in an eppendorf PCR tube for storage at -80 °C until antibody sequence recovery is initiated.

### Example 5 Isolation of Antibody Sequences From Antigen-Specific B Cell

Antibody sequences are recovered using a combined RT-PCR based method from a single isolated B-cell produced according to Example 4 or an antigenic specific B cell isolated from the clonal B cell population obtained according to Example 2. Primers are designed to anneal in conserved and constant regions of the target immunoglobulin genes (heavy and light), such as rabbit immunoglobulin sequences, and a two-step nested PCR recovery step is used to obtain the antibody sequence. Amplicons from each well are analyzed for recovery and size integrity. The resulting fragments are then digested with AluI to fingerprint the sequence clonality. Identical sequences display a common fragmentation pattern in their electrophoretic analysis. Significantly, this common fragmentation pattern which proves cell clonality is generally observed even in the wells originally plated up to 1000 cells/well. The original heavy and light chain amplicon fragments are then restriction enzyme digested with HindIII and XhoI or HindIII and BsiWI to prepare the respective pieces of DNA for cloning. The resulting digestions are then ligated into an expression vector and transformed into bacteria for plasmid propagation and production. Colonies are selected for sequence characterization.

### Example 6 Recombinant Production of Monoclonal Antibody of Desired Antigen Specificity and/or Functional Properties

Correct full-length antibody sequences for each well containing a single monoclonal antibody is established and miniprep DNA is prepared using Qiagen solid-phase methodology. This DNA is then used to transfect mammalian cells to produce recombinant full-length antibody. Crude antibody product is tested for antigen recognition and functional properties to confirm the original characteristics are found in the recombinant antibody protein. Where appropriate, large-scale transient mammalian transfections are completed, and antibody is purified through Protein A affinity chromatography. Kd is assessed using standard methods (e.g., Biacore™) as well as IC50 in a potency assay.

### Example 7 Preparation of Antibodies that Bind Human IL-6

By using the antibody selection protocol described herein, one can generate an extensive panel of antibodies. The antibodies have high affinity towards IL-6 (single to double digit pM Kd) and demonstrate potent antagonism of IL-6 in multiple cell-based screening systems (T1165 and HepG2). Furthermore, the collection of antibodies displays distinct modes of antagonism toward IL-6-driven processes.

### Immunization Strategy

Rabbits were immunized with huIL-6 (R&R). Immunization consisted of a first subcutaneous (sc) injection of 100 µg in complete Freund's adjuvant (CFA) (Sigma) followed by two boosts, two weeks apart, of 50 µg each in incomplete Freund's adjuvant (IFA) (Sigma). Animals were bled on day 55, and serum titers were determined by ELISA (antigen recognition) and by non-radioactive proliferation assay (Promega) using the T1165 cell line.

### Antibody Selection Titer Assessment

Antigen recognition was determined by coating Immulon 4 plates (Thermo) with 1 µg/ml of huIL-6 (50 µL/well) in phosphate buffered saline (PBS, Hyclone) overnight at 4 °C. On the day of the assay, plates were washed 3 times with PBS /Tween 20 (PBST tablets, Calbiochem). Plates were then blocked with 200 µL/well of 0.5% fish skin gelatin (FSG, Sigma) in PBS for 30 minutes at 37 °C. Blocking solution was removed, and plates were blotted. Serum samples were made (bleeds and pre-bleeds) at a starting dilution of 1:100 (all dilutions were made in FSG 50 µL/well) followed by 1:10 dilutions across the plate (column 12 was left blank for background control). Plates were incubated for 30 minutes at 37 °C. Plates were washed 3 times with PBS/Tween 20. Goat anti-rabbit FC-HRP (Pierce) diluted 1:5000 was added to all wells (50 µL/well), and plates were incubated for 30 minutes at 37 °C. Plates were washed as described above. 50 µL/well of TMB-Stable stop (Fitzgerald Industries) was added to plates, and color was allowed to develop, generally for 3 to 5 minutes. The development reaction was stopped with 50 µL/well 0.5 M HCl. Plates were read at 450 nm. Optical density (OD) versus dilution was plotted using Graph Pad Prizm software, and titers were determined.

### Functional Titer Assessment

The functional activity of the samples was determined by a T1165 proliferation assay. T1165 cells were routinely maintained in modified RPMI medium (Hyclone) supplemented with HEPES, sodium pyruvate, sodium bicarbonate, L-glutamine, high glucose, penicillin/streptomycin, 10% heat inactivated fetal bovine serum (FBS) (all supplements from Hyclone), 2-mercaptoethanol (Sigma), and 10 ng/ml of huIL-6 (R&D). On the day of the assay, cell viability was determined by trypan blue (Invitrogen), and cells were seeded at a fixed density of 20,000 cells/well. Prior to seeding, cells were washed twice in the medium described above without human-IL-6 (by centrifuging at 13000 rpm for 5 minutes and discarding the supernatant). After the last wash, cells were resuspended in the same medium used for washing in a volume equivalent to 50 µL/well. Cells were set aside at room temperature.

In a round-bottom, 96-well plate (Costar), serum samples were added starting at 1:100, followed by a 1:10 dilution across the plate (columns 2 to 10) at 30 µL/well in replicates of 5 (rows B to F: dilution made in the medium described above with no huIL-6). Column 11 was medium only for IL-6 control. 30 µL/well of huIL-6 at 4x concentration of the final EC50 (concentration previously determined) were added to all wells (huIL-6 was diluted in the medium described above). Wells were incubated for 1 hour at 37 °C to allow antibody binding to occur. After 1 hour, 50 µL/well of antibody-antigen (Ab-Ag) complex were transferred to a flat-bottom, 96-well plate (Costar) following the plate map format laid out in the round-bottom plate. On Row G, 50 µL/well of medium were added to all wells (columns 2 to 11) for background control. 50 µL/well of the cell suspension set aside were added to all wells (columns 2 to 11, rows B to G). On Columns 1 and 12 and on rows A and H, 200 µL/well of medium was added to prevent evaporation of test wells and to minimize edge effect. Plates were incubated for 72 h at 37 °C in 4% CO₂. At 72 h, 20 µL/well of CellTiter96 (Promega) reagents was added to all test wells per manufacturer protocol, and plates were incubated for 2 h at 37 °C. At 2 h, plates were gently mixed on an orbital shaker to disperse cells and to allow homogeneity in the test wells. Plates were read at 490 nm wavelength. Optical density (OD) versus dilution was plotted using Graph Pad Prizm software, and functional titer was determined. A positive assay control plate was conducted as described above using MAB2061 (R&D Systems) at a starting concentration of 1 µg/ml (final concentration) followed by 1:3 dilutions across the plate.

### Tissue Harvesting

Once acceptable titers were established, the rabbit(s) were sacrificed. Spleen, lymph nodes, and whole blood were harvested and processed as follows:

Spleen and lymph nodes were processed into a single cell suspension by disassociating the tissue and pushing through sterile wire mesh at 70 µm (Fisher) with a plunger of a 20 cc syringe. Cells were collected in the modified RPMI medium described above without huIL-6, but with low glucose. Cells were washed twice by centrifugation. After the last wash, cell density was determined by trypan blue. Cells were centrifuged at 1500 rpm for 10 minutes; the supernatant was discarded. Cells were resuspended in the appropriate volume of 10% dimethyl sulfoxide (DMSO, Sigma) in FBS (Hyclone) and dispensed at 1 ml/vial. Vials were then stored at -70 °C for 24 h prior to being placed in a liquid nitrogen (LN2) tank for long-term storage.

Peripheral blood mononuclear cells (PBMCs) were isolated by mixing whole blood with equal parts of the low glucose medium described above without FBS. 35 ml of the whole blood mixture was carefully layered onto 8 ml of Lympholyte Rabbit (Cedarlane) into a 45 ml conical tube (Corning) and centrifuged 30 minutes at 2500 rpm at room temperature without brakes. After centrifugation, the PBMC layers were carefully removed using a glass Pasteur pipette (VWR), combined, and placed into a clean 50 ml vial. Cells were washed twice with the modified medium described above by centrifugation at 1500 rpm for 10 minutes at room temperature, and cell density was determined by trypan blue staining. After the last wash, cells were resuspended in an appropriate volume of 10% DMSO/FBS medium and frozen as described above.

### B cell culture

On the day of setting up B cell culture, PBMC, splenocyte, or lymph node vials were thawed for use. Vials were removed from LN2 tank and placed in a 37 °C water bath until thawed. Contents of vials were transferred into 15 ml conical centrifuge tube (Corning) and 10 ml of modified RPMI described above was slowly added to the tube. Cells were centrifuged for 5 minutes at 1.5K rpm, and the supernatant was discarded. Cells were resuspended in 10 ml of fresh media. Cell density and viability was determined by trypan blue. Cells were washed again and resuspended at 1E07 cells/80 µL medium. Biotinylated huIL-6 (B huIL-6) was added to the cell suspension at the final concentration of 3 µg/mL and incubated for 30 minutes at 4 °C. Unbound B huIL-6 was removed with two 10 ml washes of phosphate-buffered (PBF):Ca/Mg free PBS (Hyclone), 2 mM ethylenediamine tetraacetic acid (EDTA), 0.5% bovine serum albumin (BSA) (Sigma-biotin free). After the second wash, cells were resuspended at 1E07 cells/80 µL PBF. 20 µL of MACS® streptavidin beads (Milteni)/10E7 cells were added to the cell suspension. Cells were incubated at 4 °C for 15 minutes. Cells were washed once with 2 ml of PBF/10E7 cells. After washing, the cells were resuspended at 1E08 cells/500 µL of PBF and set aside. A MACS® MS column (Milteni) was pre-rinsed with 500 ml of PBF on a magnetic stand (Milteni). Cell suspension was applied to the column through a pre-filter, and unbound fraction was collected. The column was washed with 1.5 ml of PBF buffer. The column was removed from the magnet stand and placed onto a clean, sterile 5 ml Polypropylene Falcon tube. 1 ml of PBF buffer was added to the top of the column, and positive selected cells were collected. The yield and viability of positive and negative cell fraction was determined by trypan blue staining. Positive selection yielded an average of 1 % of the starting cell concentration.

A pilot cell screen was established to provide information on seeding levels for the culture. Three 10-plate groups (a total of 30 plates) were seeded at 50, 100, and 200 enriched B cells/well. In addition, each well contained 50K cells/well of irradiated EL-4.B5 cells (5,000 Rads) and an appropriate level of T cell supernatant (ranging from 1-5% depending on preparation) in high glucose modified RPMI medium at a final volume of 250 µL/well. Cultures were incubated for 5 to 7 days at 37 °C in 4% CO₂ .

### Identification of Selective Antibody Secreting B Cells

Cultures were tested for antigen recognition and functional activity between days 5 and 7.

### Antigen Recognition Screening

The ELISA format used is as described above except 50 µL of supernatant from the B cell cultures (BCC) wells (all 30 plates) was used as the source of the antibody. The conditioned medium was transferred to antigen-coated plates. After positive wells were identified, the supernatant was removed and transferred to a 96-well master plate(s). The original culture plates were then frozen by removing all the supernatant except 40 µL/well and adding 60 µL/well of 16% DMSO in FBS. Plates were wrapped in paper towels to slow freezing and placed at -70 °C.

### Functional Activity Screening

Master plates were then screened for functional activity in the T1165 proliferation assay as described before, except row B was media only for background control, row C was media + IL-6 for positive proliferation control, and rows D-G and columns 2-11 were the wells from the BCC (50 µL/well, single points). 40 µL of IL-6 was added to all wells except the media row at 2.5 times the EC50 concentration determined for the assay. After 1 h incubation, the Ab/Ag complex was transferred to a tissue culture (TC) treated, 96-well, flat-bottom plate. 20 µL of cell suspension in modified RPMI medium without huIL-6 (T1165 at 20,000 cells/well) was added to all wells (100 µL final volume per well). Background was subtracted, and observed OD values were transformed into % of inhibition.

### B cell recovery

Plates containing wells of interest were removed from -70 °C, and the cells from each well were recovered with 5-200 µL washes of medium/well. The washes were pooled in a 1.5 ml sterile centrifuge tube, and cells were pelleted for 2 minutes at 1500 rpm.

The tube was inverted, the spin repeated, and the supernatant carefully removed. Cells were resuspended in 100 µL/tube of medium. 100 µL biotinylated IL-6 coated streptavidin M280 dynabeads (Invitrogen) and 16 µL of goat anti-rabbit H&L IgG-FITC diluted 1:100 in medium was added to the cell suspension.

20 µL of cell/beads/FITC suspension was removed, and 5 µL droplets were prepared on a glass slide (Corning) previously treated with Sigmacote (Sigma), 35 to 40 droplets/slide. An impermeable barrier of paraffin oil (JT Baker) was added to submerge the droplets, and the slide was incubated for 90 minutes at 37 °C, 4% CO₂ in the dark.

Specific B cells that produce antibody can be identified by the fluorescent ring around them due to antibody secretion, recognition of the bead-associated biotinylated antigen, and subsequent detection by the fluorescent-IgG detection reagent. Once a cell of interest was identified, the cell in the center of the fluorescent ring was recovered via a micromanipulator (Eppendorf). The single cell synthesizing and exporting the antibody was transferred into a 250 µL microcentrifuge tube and placed in dry ice. After recovering all cells of interest, these were transferred to -70 °C for long-term storage.

### Example 8 Yeast Cell Expression

*Antibody genes:* Genes were cloned and constructed that directed the synthesis of a chimeric humanized rabbit monoclonal antibody.

*Expression vector:* The vector contains the following functional components: 1) a mutant ColE1 origin of replication, which facilitates the replication of the plasmid vector in cells of the bacterium *Escherichia coli*; 2) a bacterial *Sh ble* gene, which confers resistance to the antibiotic Zeocin™ (phleomycin) and serves as the selectable marker for transformations of both *E. coli* and *P. pastoris*; 3) an expression cassette composed of the glyceraldehyde dehydrogenase gene (*GAP* gene) promoter, fused to sequences encoding the *Saccharomyces cerevisiae* alpha mating factor pre pro secretion leader sequence, followed by sequences encoding a *P. pastoris* transcriptional termination signal from the *P. pastoris* alcohol oxidase I gene (*AOX1*). The Zeocin™ (phleomycin) resistance marker gene provides a means of enrichment for strains that contain multiple integrated copies of an expression vector in a strain by selecting for transformants that are resistant to higher levels of Zeocin™ (phleomycin).

*P. pastoris* strains: *P. pastoris* strains *met1, lys3, ura3* and *adel* may be used. Although any two complementing sets of auxotrophic strains could be used for the construction and maintenance of diploid strains, these two strains are especially suited for this method for two reasons. First, they grow more slowly than diploid strains that are the result of their mating or fusion. Thus, if a small number of haploid *adel* or *ura3* cells remain present in a culture or arise through meiosis or other mechanism, the diploid strain should outgrow them in culture.

The second is that it is easy to monitor the sexual state of these strains since diploid Ade+ colonies arising from their mating are a normal white or cream color, whereas cells of any strains that are haploid *ade1* mutants will form a colony with a distinct pink color. In addition, any strains that are haploid *ura3* mutants are resistant to the drug 5-fluoro-orotic acid (FOA) and can be sensitively identified by plating samples of a culture on minimal medium + uracil plates with FOA. On these plates, only uracil-requiring *ura3* mutant (presumably haploid) strains can grow and form colonies. Thus, with haploid parent strains marked with *ade1* and *ura3,* one can readily monitor the sexual state of the resulting antibody-producing diploid strains (haploid versus diploid).

### Methods

*Construction of pGAPZ-alpha expression vectors for transcription of light and heavy chain antibody genes.* The humanized light and heavy chain fragments were cloned into the pGAPZ expression vectors through a PCR directed process. The recovered humanized constructs were subjected to amplification under standard KOD polymerase (Novagen) kit conditions ((1) 94 °C, 2 minutes; (2) 94 °C, 30 seconds (3) 55 °C, 30 seconds; (4) 72 °C, 30 seconds-cycling through steps 2-4 for 35 times; (5) 72 °C 2 minutes) employing the following primers (1) light chain forward AGCGCTTATTCCGCTATCCAGATGACCCAGTC-the AfeI site is single underlined. The end of the HSA signal sequence is double underlined, followed by the sequence for the mature variable light chain (not underlined); the reverse CGTACGTTTGATTTCCACCTTG.

Variable light chain reverse primer. BsiWI site is underlined, followed by the reverse complement for the 3' end of the variable light chain. Upon restriction enzyme digest with AfeI and BsiWI this enable insertion in-frame with the pGAPZ vector using the human HAS leader sequence in frame with the human kapp light chain constant region for export. (2) A similar strategy is performed for the heavy chain. The forward primer employed is AGCGCTTATTCCGAGGTGCAGCTGGTGGAGTC. The AfeI site is single underlined. The end of the HSA signal sequence is double underlined, followed by the sequence for the mature variable heavy chain (not underlined). The reverse heavy chain primer is CTCGAGACGGTGACGAGGGT. The XhoI site is underlined, followed by the reverse complement for the 3' end of the variable heavy chain. This enables cloning of the heavy chain in-frame with IgG-γ1 CH1-CH2-CH3 region previous inserted within pGAPZ using a comparable directional cloning strategy.

*Transformation of expression vectors into haploid adel ura3, met1 and lys3 host strains of P. pastoris.* All methods used for transformation of haploid *P. pastoris* strains and genetic manipulation of the *P. pastoris* sexual cycle are as described in Higgins, D. R., and Cregg, J. M., Eds. 1998. Pichia Protocols. Methods in Molecular Biology. Humana Press, Totowa, NJ.

Prior to transformation, each expression vector is linearized within the *GAP* promoter sequences with AvrII to direct the integration of the vectors into the *GAP* promoter locus of the *P. pastoris* genome. Samples of each vector are then individually transformed into electrocompetent cultures of the *ade1*, *ura3, met1* and *lys3* strains by electroporation and successful transformants are selected on YPD Zeocin™ (phleomycin) plates by their resistance to this antibiotic. Resulting colonies are selected, streaked for single colonies on YPD Zeocin™ (phleomycin) plates and then examined for the presence of the antibody gene insert by a PCR assay on genomic DNA extracted from each strain for the proper antibody gene insert and/or by the ability of each strain to synthesize an antibody chain by a colony lift/immunoblot method (Wung et al. Biotechniques 21 808-812 (1996). Haploid *ade1, met1* and *lys3* strains expressing one of the three heavy chain constructs are collected for diploid constructions along with haploid *ura3* strain expressing light chain gene. The haploid expressing heavy chain genes are mated with the appropriate light chain haploid *ura3* to generate diploid secreting protein.

Mating of haploid strains synthesizing a single antibody chain and selection of diploid derivatives synthesizing tetrameric functional antibodies. To mate P. pastoris haploid strains, each ade1 (or met1 or lys3) heavy chain producing strain to be crossed is streaked across a rich YPD plate and the ura3 light chain producing strain is streaked across a second YPD plate (-10 streaks per plate). After one or two days incubation at 30 °C, cells from one plate containing heavy chain strains and one plate containing ura3 light chain strains are transferred to a sterile velvet cloth on a replica-plating block in a cross hatched pattern so that each heavy chain strain contain a patch of cells mixed with each light chain strain. The cross-streaked replica plated cells are then transferred to a mating plate and incubated at 25 °C to stimulate the initiation of mating between strains. After two days, the cells on the mating plates are transferred again to a sterile velvet on a replica-plating block and then transferred to minimal medium plates. These plates are incubated at 30 °C for three days to allow for the selective growth of colonies of prototrophic diploid strains. Colonies that arose are picked and streaked onto a second minimal medium plate to single colony isolate and purify each diploid strain. The resulting diploid cell lines are then examined for antibody production.

Putative diploid strains are tested to demonstrate that they are diploid and contain both expression vectors for antibody production. For diploidy, samples of a strain are spread on mating plates to stimulate them to go through meiosis and form spores. Haploid spore products are collected and tested for phenotype. If a significant percentage of the resulting spore products are single or double auxotrophs it may be concluded that the original strain must have been diploid. Diploid strains are examined for the presence of both antibody genes by extracting genomic DNA from each and utilizing this DNA in PCR reactions specific for each gene.

Fusion of haploid strains synthesizing a single antibody chain and selection of diploid derivatives synthesizing tetrameric functional antibodies. As an alternative to the mating procedure described above, individual cultures of single-chain antibody producing haploid ade1 and ura3 strains are spheroplasted and their resulting spheroplasts fused using polyethylene glycol/CaCl₂. The fused haploid strains are then embedded in agar containing 1 M sorbitol and minimal medium to allow diploid strains to regenerate their cell wall and grow into visible colonies. Resulting colonies are picked from the agar, streaked onto a minimal medium plate, and the plates are incubated for two days at 30 °C to generate colonies from single cells of diploid cell lines. The resulting putative diploid cell lines are then examined for diploidy and antibody production as described above.

Purification and analysis of antibodies. A diploid strain for the production of full length antibody is derived through the mating of met1 light chain and lys3 heavy chain using the methods described above. Culture media from shake-flask or fermenter cultures of diploid P. pastoris expression strains are collected and examined for the presence of antibody protein via SDS-PAGE and immunoblotting using antibodies directed against heavy and light chains of human IgG, or specifically against the heavy chain of IgG.

To purify the yeast secreted antibodies, clarified media from antibody producing cultures are passed through a protein A column and after washing with 20 mM sodium phosphate, pH 7.0, binding buffer, protein A bound protein is eluted using 0.1 M glycine HCl buffer, pH 3.0. Fractions containing the most total protein are examined by Coomasie blue strained SDS-PAGE and immunoblotting for antibody protein. Antibody is characterized using the ELISA described above for IL-6 recognition.

*Assay for antibody activity.* The recombinant yeast-derived humanized antibody is evaluated for functional activity through the IL-6 driven T1165 cell proliferation assay and IL-6 stimulated HepG2 haptoglobin assay described above.

### Example 9 Acute Phase Response Neutralization by Intravenous Administration of Anti-IL-6 Antibody Ab1.

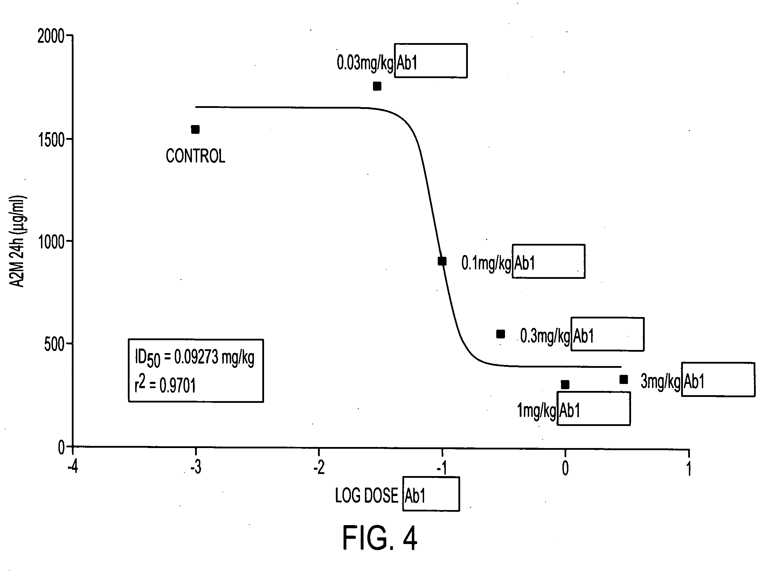

Human IL-6 can provoke an acute phase response in rats, and one of the major acute phase proteins that is stimulated in the rat is α-2 macroglobulin (A2M). A study was designed to assess the dose of antibody Ab1 required to ablate the A2M response to a single s.c. injection of 100 µg of human IL-6 given one hour after different doses (0.03, 0.1, 0.3, 1, and 3 mg/kg) of antibody Ab1 administered intravenously (n=10 rats/dose level) or polyclonal human IgG1 as the control (n=10 rats). Plasma was recovered and the A2M was quantitated via a commercial sandwich ELISA kit (ICL Inc., Newberg OR; cat. no.- E-25A2M). The endpoint was the difference in the plasma concentration of A2M at the 24 hour time point (post-Abl). The results are presented in Fig. 4.

The ID50 for antibody Ab1 was 0.1 mg/kg with complete suppression of the A2M response at the 0.3 mg/kg. This firmly establishes *in vivo* neutralization of human IL-6 can be accomplished by antibody Ab1.

### Example 10 RXF393 Cachexia Model Study 1.

### Introduction

The human renal cell cancer cell line, RXF393 produces profound weight loss when transplanted into athymic nude mice. Weight loss begins around day 15 after transplantation with 80% of all animals losing at least 30% of their total body weight by day 18 - 20 after transplantation. RXF393 secretes human IL-6 and the plasma concentration of human IL-6 in these animals is very high at around 10 ng/ml. Human IL-6 can bind murine soluble IL-6 receptor and activate IL-6 responses in the mouse. Human IL-6 is approximately 10 times less potent than murine IL-6 at activating IL-6 responses in the mouse. The objectives of this study were to determine the effect of antibody Ab1, on survival, body weight, serum amyloid A protein, hematology parameters, and tumor growth in athymic nude mice transplanted with the human renal cell cancer cell line, RXF393.

### Methods

Eighty, 6 week old, male athymic nude mice were implanted with RXF393 tumor fragments (30-40 mg) subcutaneously in the right flank. Animals were then divided into eight groups of ten mice. Three groups were given either antibody Ab1 at 3 mg/kg, 10 mg/kg, or 30 mg/kg intravenously weekly on day 1, day 8, day 15 and day 22 after transplantation (progression groups). Another three groups were given either antibody Ab1 at 3 mg/kg, or 10 mg/kg, or 30 mg/kg intravenously weekly on day 8, day 15 and day 22 after transplantation (regression groups). Finally, one control group was given polyclonal human IgG 30 mg/kg and a second control group was given phosphate buffered saline intravenously weekly on day 1, day 8, day 15 and day 22 after transplantation.

Animals were euthanized at either day 28, when the tumor reached 4,000 mm³ or if they became debilitated (>30% loss of body weight). Animals were weighed on days 1, 6 and then daily from days 9 to 28 after transplantation. Mean Percent Body Weight (MPBW) was used as the primary parameter to monitor weight loss during the study. It was calculated as follows: (Body Weight - Tumor Weight)/Baseline Body Weight x 100. Tumor weight was measured on days 1, 6, 9, 12, 15, 18, 22, 25 and 28 after transplantation. Blood was taken under anesthesia from five mice in each group on days 5 and 13 and all ten mice in each group when euthanized (day 28 in most cases). Blood was analyzed for hematology and serum amyloid A protein (SAA) concentration. An additional group of 10 non-tumor bearing 6 week old, athymic nude male mice had blood samples taken for hematology and SAA concentration estimation to act as a baseline set of values.

### Results - Survival

No animals were euthanized or died in any of the antibody Ab1 groups prior to the study termination date of day 28. In the two control groups, 15 animals (7/9 in the polyclonal human IgG group and 8/10 in the phosphate buffered saline group) were found dead or were euthanized because they were very debilitated (>30% loss of body weight). Median survival time in both control groups was 20 days.

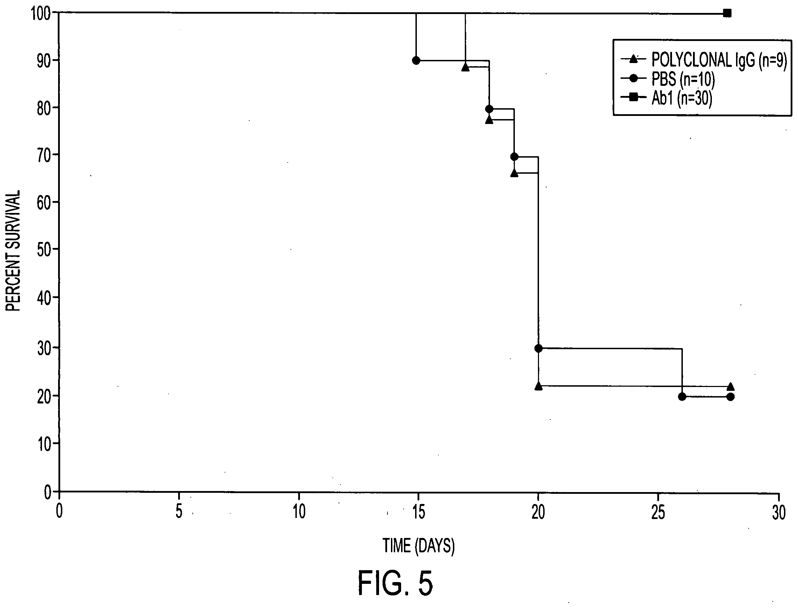

The survival curves for the two control groups and the antibody Ab1 progression (dosed from day 1 of the study) groups are presented in Fig. 5.

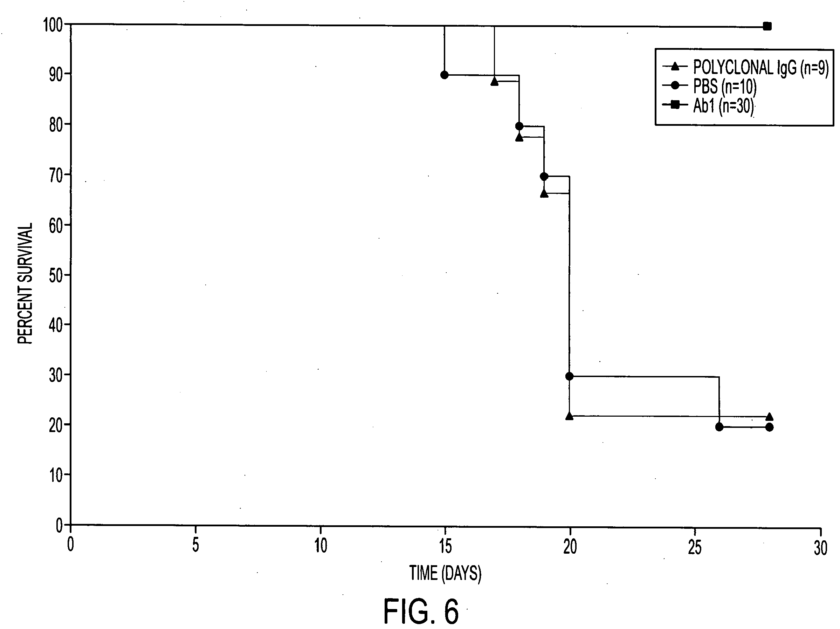

The survival curves for the two control groups and the antibody Ab1 regression (dosed from day 8 of the study) groups are presented in Fig. 6.

There was a statistically significant difference between the survival curves for the polyclonal human IgG (p=0.0038) and phosphate buffered saline (p=0.0003) control groups and the survival curve for the six antibody Ab1 groups. There was no statistically significant difference between the two control groups (p=0.97).

### Results - Tumor Size

Tumor size in surviving mice was estimated by palpation. For the first 15 days of the study, none of the mice in any group were found dead or were euthanized, and so comparison of tumor sizes between groups on these days was free from sampling bias. No difference in tumor size was observed between the antibody Ab1 progression or regression groups and the control groups through day 15. Comparison of the tumor size between surviving mice in the control and treatment groups subsequent to the onset of mortality in the controls (on day 15) was not undertaken because tumor size the surviving control mice was presumed to be biased and accordingly the results of such comparison would not be meaningful.

As administration of antibody Ab1 promoted survival without any apparent reduction in tumor size, elevated serum IL-6 may contribute to mortality through mechanisms independent of tumor growth. These observations supports the hypothesis that antibody Ab1 can promote cancer patient survivability without directly affecting tumor growth, possibly by enhancing general patient well-being.

### Results - Weight Loss

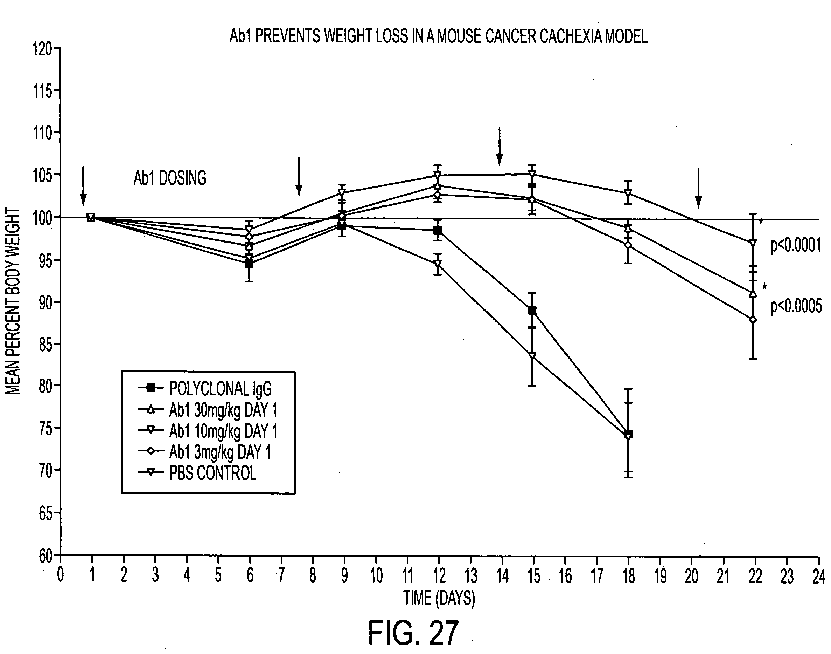

Mean Percent Body Weight (MPBW) (± SEM) versus time is shown in Fig. 27. Compared to controls, mice dosed with Ab1 were protected from weight loss. On day 18, MPBW in control mice was 75%, corresponding to an average weight loss of 25%. In contrast, on the same day, MPBW in Ab-1 treatment groups was minimally changed (between 97% and 103%). There was a statistically significant difference between the MPBW curves for the controls (receiving polyclonal human IgG or PBS) and the 10 mg/kg dosage group (p<0.0001) or 3 mg/kg and 30 mg/kg dosage groups (p<0.0005). There was no statistically significant difference between the two control groups.

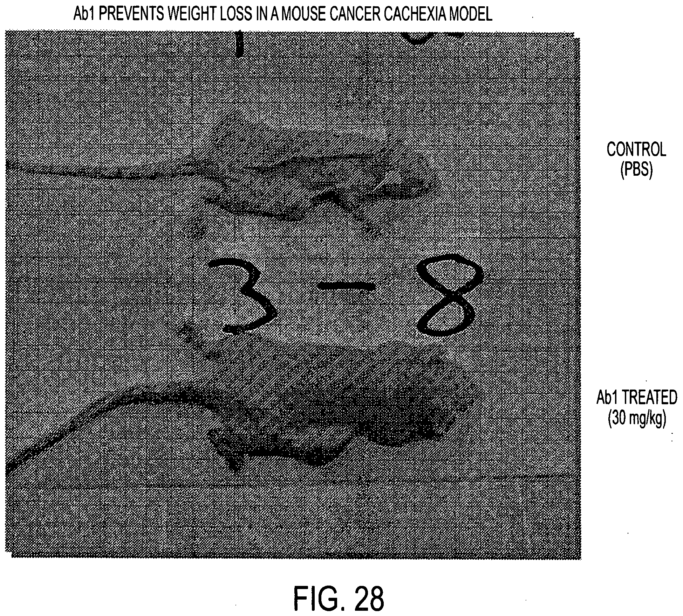

Representative photographs of control and Ab1-treated mice (Fig. 28) illustrate the emaciated condition of the control mice, compared to the normal appearance of the Ab1-treated mouse, at the end of the study (note externally visible tumor sites in right flank).

These results suggest that Ab1 may be useful to prevent or treat cachexia caused by elevated IL-6 in humans.

### Results - Plasma Serum Amyloid A

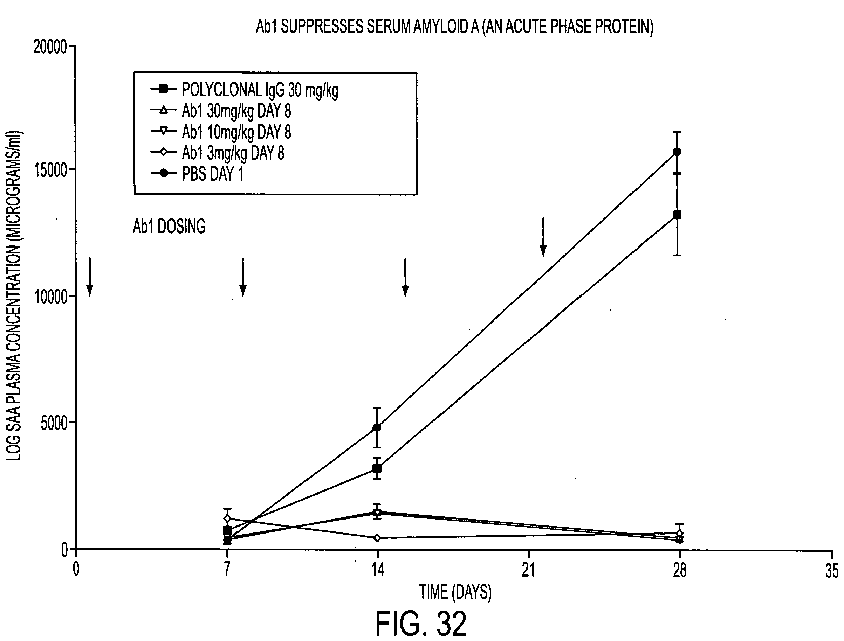

The mean (± SEM) plasma serum amyloid A concentration versus time for the two control groups and the antibody Ab1 progression (dosed from day 1 of the study) and regression (dosed from day 8 of the study) groups are presented in Table 5 and graphically in Fig. 32.

**Table 5: Mean Plasma SAA - antibody Ab1, all groups versus control groups**

| | Mean Plasma SAA±SEM Day 5 (µg/ml) | Mean Plasma SAA±SEM Day 13 (µg/ml) | Mean Plasma SAA±SEM Terminal Bleed (µg/ml) |
|---|---|---|---|
| Polyclonal IgG iv weekly from day 1 | 675 ± 240 (n=5) | 3198 ± 628 (n=4) | 13371 ± 2413 (n=4) |
| PBS iv weekly from day 1 | 355 ± 207 (n=5) | 4844 ± 1126 (n=5) | 15826 ± 802 (n=3) |
| Ab 1 30 mg/kg iv weekly from day 1 | 246 ± 100 (n=5) | 2979 ± 170 (n=5) | 841 ± 469 (n=10) |
| Ab1 10 mg/kg iv weekly from day 1 | 3629 ± 624 (n=5) | 3096 ± 690 (n=5) | 996 ± 348 (n=10) |
| Ab1 3 mg/kg iv weekly from day 1 | 106 ± 9 (n=5) | 1623 ± 595 (n=4) | 435 ± 70 (n=9) |
| Ab 1 30 mg/kg iv weekly from day 8 | 375 ± 177 (n=5) | 1492 ± 418 (n=4) | 498 ± 83 (n=9) |
| Ab1 10 mg/kg iv weekly from day 8 | 487 ± 170 (n=5) | 1403 ± 187 (n=5) | 396 ± 58 (n=10) |
| Ab1 3 mg/kg iv weekly from day 8 | 1255 ± 516 (n=5) | 466 ± 157 (n=5) | 685 ± 350 (n=5) |

SAA is up-regulated via the stimulation of hIL-6 and this response is directly correlated with circulating levels of hIL-6 derived from the implanted tumor. The surrogate marker provides an indirect readout for active hIL-6. Thus in the two treatment groups described above there are significantly decreased levels of SAA due to the neutralization of tumor-derived hIL-6. This further supports the contention that antibody Ab1 displays *in vivo* efficacy.

### Example 11 RXF393 Cachexia Model Study 2.

### Introduction

A second study was performed in the RXF-393 cachexia model where treatment with antibody Ab1 was started at a later stage (days 10 and 13 post-transplantation) and with a more prolonged treatment phase (out to 49 days post transplantation). The dosing interval with antibody Ab1 was shortened to 3 days from 7 and also daily food consumption was measured. There was also an attempt to standardize the tumor sizes at the time of initiating dosing with antibody Ab1.

### Methods

Eighty, 6 week old, male athymic nude mice were implanted with RXF393 tumor fragments (30-40 mg) subcutaneously in the right flank. 20 mice were selected whose tumors had reached between 270 - 320 mg in size and divided into two groups. One group received antibody Ab1 at 10 mg/kg i.v. every three days and the other group received polyclonal human IgG 10 mg/kg every 3 days from that time-point (day 10 after transplantation). Another 20 mice were selected when their tumor size had reached 400 - 527 mg in size and divided into two groups. One group received antibody Ab1 at 10 mg/kg i.v. every three days and the other group received polyclonal human IgG 10 mg/kg every 3 days from that time-point (day 13 after transplantation). The remaining 40 mice took no further part in the study and were euthanized at either day 49, when the tumor reached 4,000 mm³ or if they became very debilitated (>30% loss of body weight).

Animals were weighed every 3-4 days from day 1 to day 49 after transplantation. Mean Percent Body Weight (MPBW) was used as the primary parameter to monitor weight loss during the study. It was calculated as follows: ((Body Weight - Tumor Weight)/Baseline Body Weight) x 100. Tumor weight was measured every 3-4 days from day 5 to day 49 after transplantation. Food consumption was measured (amount consumed in 24 hours by weight (g) by each treatment group) every day from day 10 for the 270-320 mg tumor groups and day 13 for the 400-527 mg tumor groups.

### Results -survival

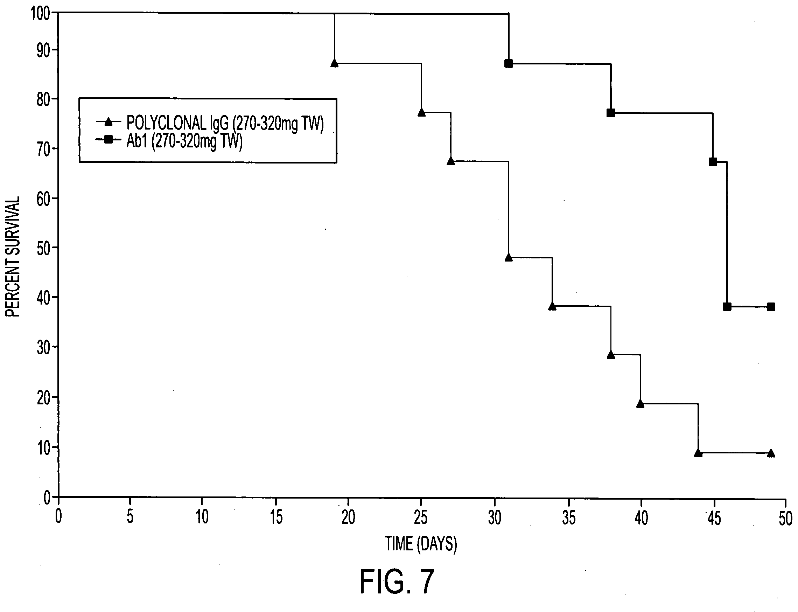

The survival curves for antibody Ab1 at 10 mg/kg i.v. every three days (270-320 mg tumor size) and for the polyclonal human IgG 10 mg/kg i.v. every three days (270-320 mg tumor size) are presented in Fig. 7.

Median survival for the antibody Ab1 at 10 mg/kg i.v. every three days (270-320 mg tumor size) was 46 days and for the polyclonal human IgG at 10 mg/kg i.v. every three days (270-320 mg tumor size) was 32.5 days (p=0.0071).

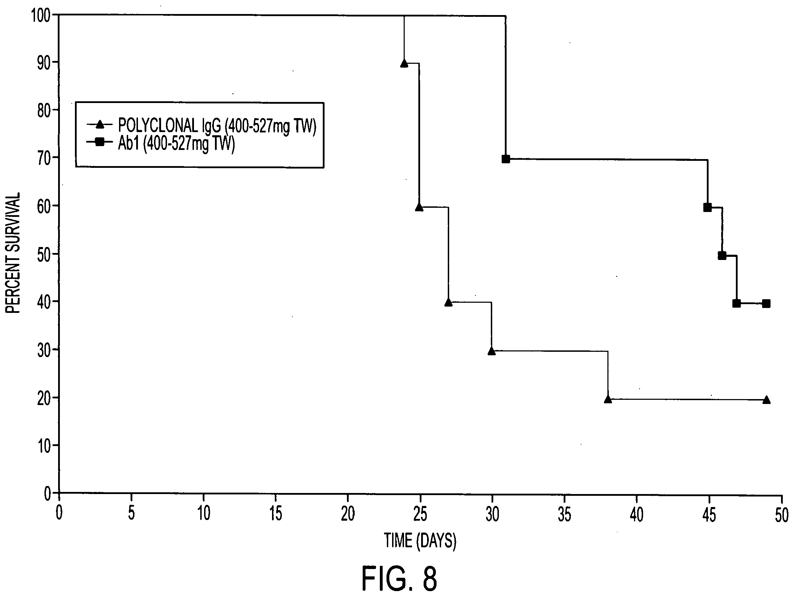

The survival curves for the antibody Ab1 at 10 mg/kg i.v. every three days (400-527 mg tumor size) and for the polyclonal human IgG at 10 mg/kg i.v. every three days (400-527 mg tumor size) are presented in Fig. 8. Median survival for the antibody Ab1 at 10 mg/kg i.v. every three days (400-527 mg tumor size) was 46.5 days and for the polyclonal human IgG at 10 mg/kg i.v. every three days (400-527 mg tumor size) was 27 days (p=0.0481).

### Example 12 Multi-dose Pharmacokinetic Evaluation of Antibody Ab1 in Non-human Primates.

Antibody Ab1 was dosed in a single bolus infusion to a single male and single female cynomologus monkey in phosphate buffered saline. Plasma samples were removed at fixed time intervals and the level of antibody Ab1 was quantitated through of the use of an antigen capture ELISA assay. Biotinylated IL-6 (50 µl of 3 µg/mL) was captured on Streptavidin coated 96 well microtiter plates. The plates were washed and blocked with 0.5% Fish skin gelatin. Appropriately diluted plasma samples were added and incubated for 1 hour at room temperature. The supernatants removed and an anti-hFc-HRP conjugated secondary antibody applied and left at room temperature.

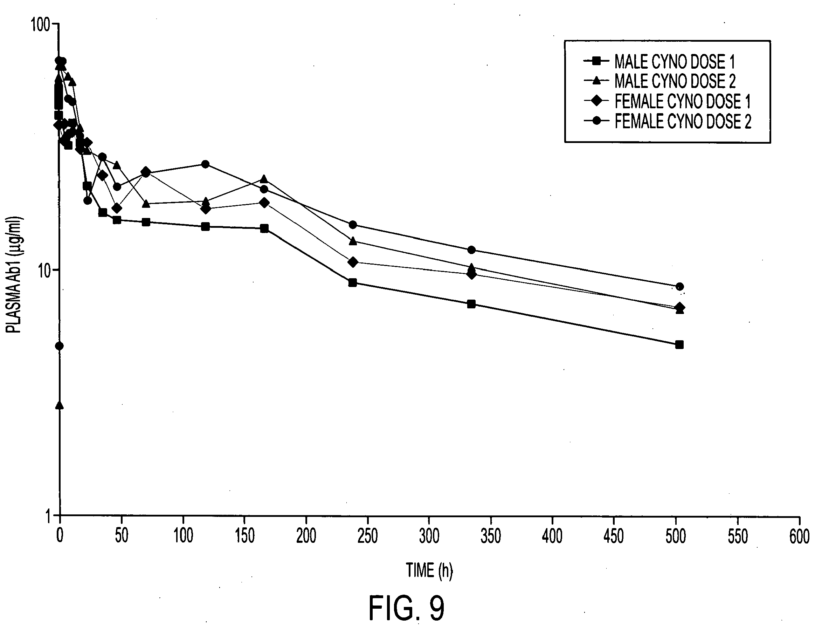

The plates were then aspirated and TMB added to visualize the amount of antibody. The specific levels were then determined through the use of a standard curve. A second dose of antibody Ab1 was administered at day 35 to the same two cynomologus monkeys and the experiment replicated using an identical sampling plan. The resulting concentrations are then plot vs. time as show in Fig. 9.

This humanized full length aglycosylated antibody expressed and purified *Pichia pastoris* displays comparable characteristics to mammalian expressed protein. In addition, multiple doses of this product display reproducible half-lives inferring that this production platform does not generate products that display enhanced immunogenicity.

### Example 13 Octet Mechanistic Characterization of Antibody Proteins.

IL-6 signaling is dependent upon interactions between IL-6 and two receptors, IL-6R1 (CD126) and gp130 (IL-6 signal transducer). To determine the antibody mechanism of action, mechanistic studies were performed using bio-layer interferometry with an Octet QK instrument (ForteBio; Menlo Park, CA). Studies were performed in two different configurations. In the first orientation, biotinylated IL-6 (R&D systems part number 206-IL-001MG/CF, biotinylated using Pierce EZ-link sulfo-NHS-LC-LC-biotin product number 21338 according to manufacturer's protocols) was initially bound to a streptavidin coated biosensor (ForteBio part number 18-5006). Binding is monitored as an increase in signal.

The IL-6 bound to the sensor was then incubated either with the antibody in question or diluent solution alone. The sensor was then incubated with soluble IL-6R1 (R&D systems product number 227-SR-025/CF) molecule. If the IL-6R1 molecule failed to bind, the antibody was deemed to block IL-6/IL-6R1 interactions. These complexes were incubated with gp130 (R&D systems 228-GP-010/CF) in the presence of IL-6R1 for stability purposes. If gp130 did not bind, it was concluded that the antibody blocked gp130 interactions with IL-6.

In the second orientation, the antibody was bound to a biosensor coated with an anti-human IgG1 Fc-specific reagent (ForteBio part number 18-5001). The IL-6 was bound to the immobilized antibody and the sensor was incubated with IL-6R1. If the IL-6R1 did not interact with the IL-6, then it was concluded that the IL-6 binding antibody blocked IL-6/IL-6R1 interactions. In those situations where antibody/IL-6/IL-6R1 was observed, the complex was incubated with gp130 in the presence of IL-6R1. If gp130 did not interact, then it was concluded that the antibody blocked IL-6/gp130 interactions. All studies were performed in a 200 µL final volume, at 30C and 1000 rpm. For these studies, all proteins were diluted using ForteBio's sample diluent buffer (part number 18-5028).

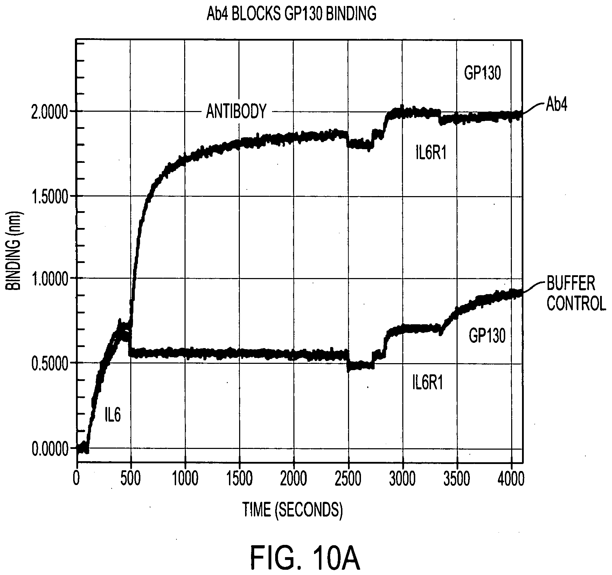

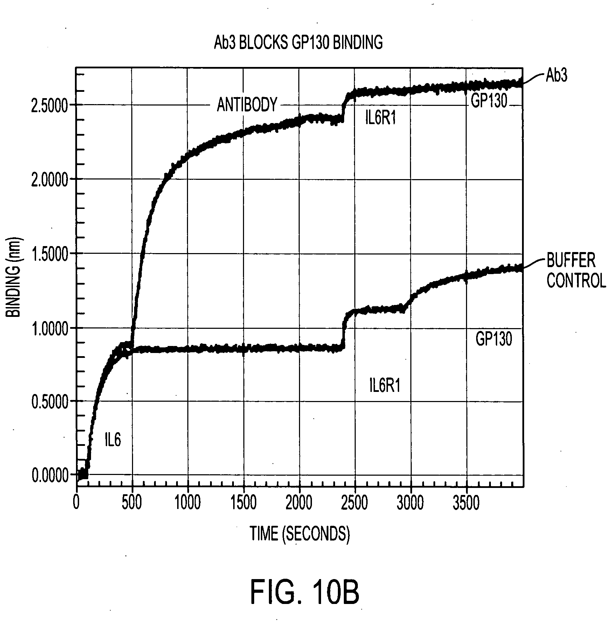

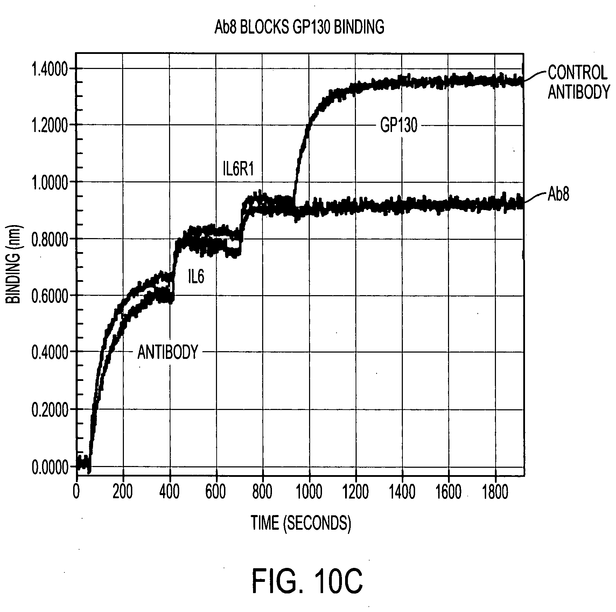

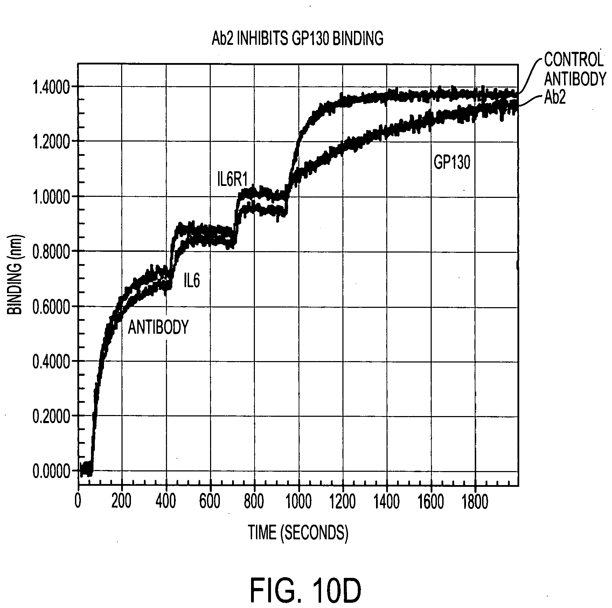

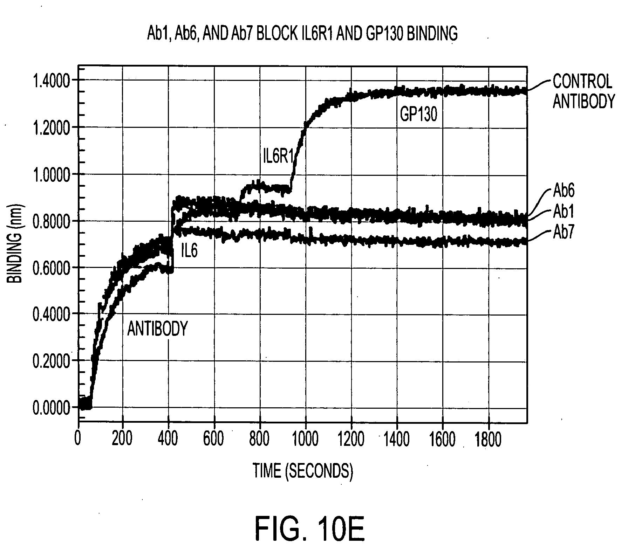

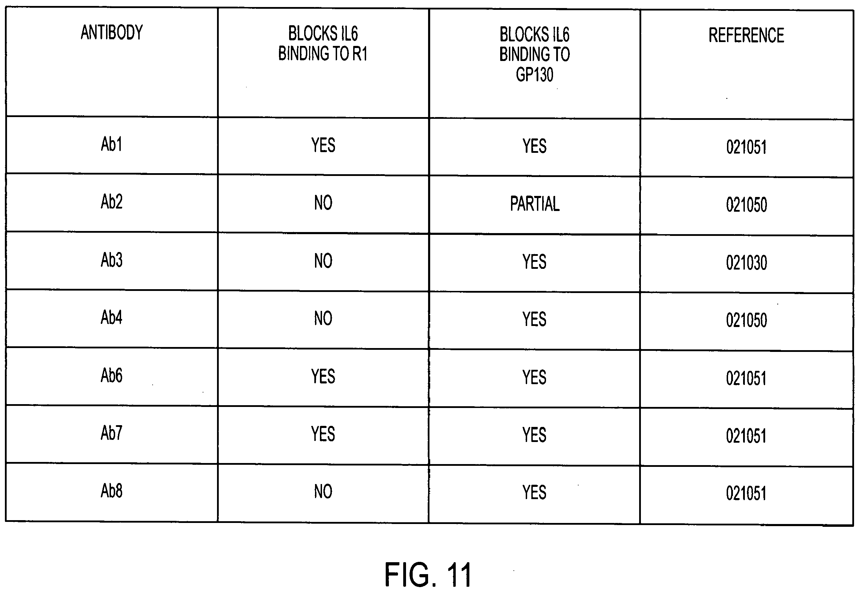

Results are presented in Fig. 10(A-E) and Fig. 11.

### Example 14 Peptide Mapping.

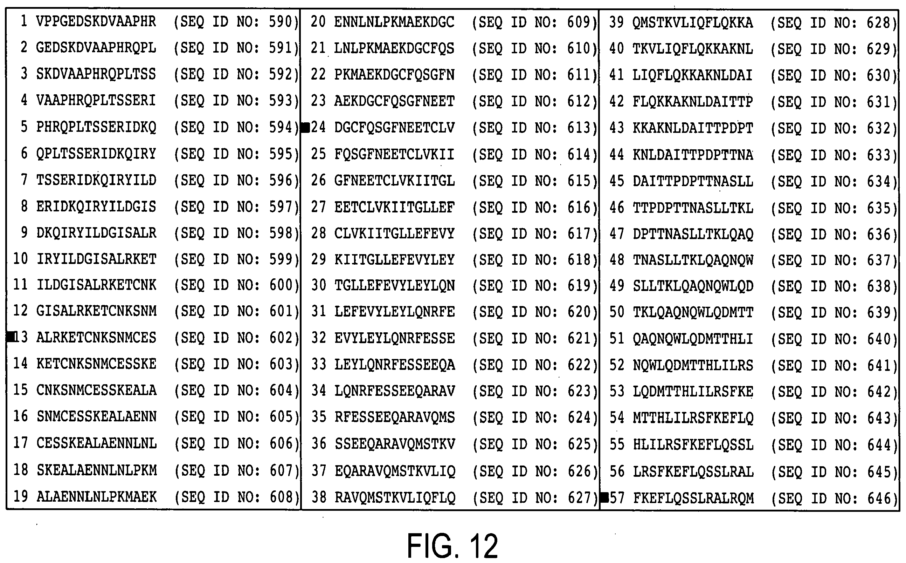

In order to determine the epitope recognized by Ab1 on human IL-6, the antibody was employed in a western-blot based assay. The form of human IL-6 utilized in this example had a sequence of 183 amino acids in length (shown below). A 57-member library of overlapping 15 amino acid peptides encompassing this sequence was commercially synthesized and covalently bound to a PepSpots nitrocellulose membrane (JPT Peptide technologies, Berlin, Germany). The sequences of the overlapping 15 amino acid peptides is shown in Fig. 12. Blots were prepared and probed according to the manufacturer's recommendations.

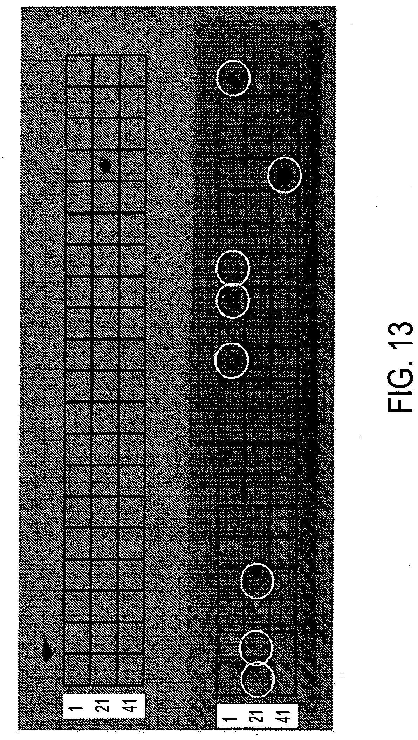

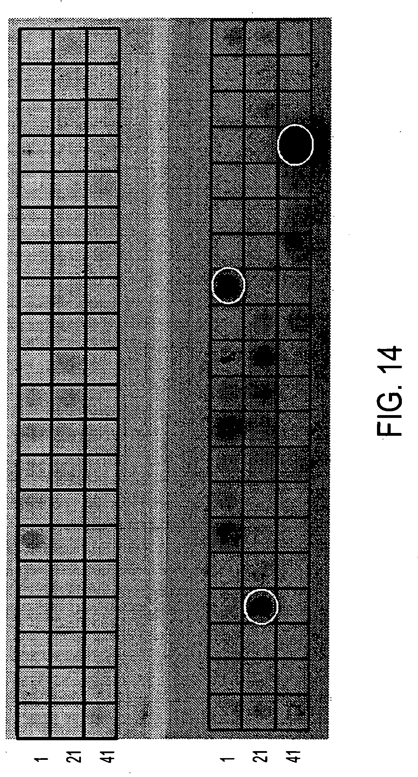

Briefly, blots were pre-wet in methanol, rinsed in PBS, and blocked for over 2 hours in 10% non-fat milk in PBS/0.05% Tween (Blocking Solution). The Ab1 antibody was used at 1 mg/ml final dilution, and the HRP-conjugated Mouse Anti-Human-Kappa secondary antibody (Southern BioTech #9220-05) was used at a 1:5000 dilution. Antibody dilutions/incubations were performed in blocking solution. Blots were developed using Amersham ECL advance reagents (GE# RPN2135) and chemiluminescent signal documented using a CCD camera (AlphaInnotec). The results of the blots is shown in Fig. 13 and Fig. 14.

The sequence of the form of human IL-6 utilized to generate peptide library is set forth:

### Example 15 Ab1 has high affinity for IL-6.

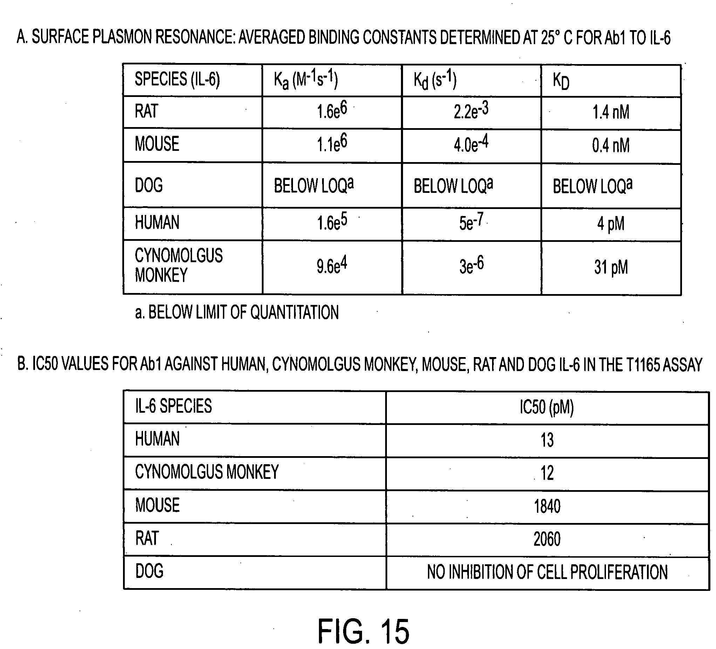

Surface plasmon resonance was used to measure association rate (Kₐ), dissociation rate (K_{d}) and dissociation constant (K_{D}) for Ab1 to IL-6 from rat, mouse, dog, human, and cynomolgus monkey at 25 °C (Fig. 15A). The dissociation constant for human IL-6 was 4 pM, indicating very high affinity. As expected, affinity generally decreased with phylogenetic distance from human. The dissociation constants of Ab1 for IL-6 of cynomolgus monkey, rat, and mouse were 31 pM, 1.4 nM, and 0.4 nM, respectively. Ab1 affinity for dog IL-6 below the limit of quantitation of the experiment.

The high affinity of Ab1 for mouse, rat, and cynomolgus monkey IL-6 suggest that Ab1 may be used to inhibit IL-6 of these species. This hypothesis was tested using a cell proliferation assay. In brief, each species's IL-6 was used to stimulate proliferation of T1165 cells, and the concentration at which Ab1 could inhibit 50% of proliferation (IC50) was measured. Inhibition was consistent with the measured dissociation constants (Fig. 15B). These results demonstrate that Ab1 can inhibit the native IL-6 of these species, and suggest the use of these organisms for *in vitro* or *in vivo* modeling of IL-6 inhibition by Ab1.

### Example 16 Multi-dose Pharmacokinetic Evaluation of Antibody Ab1 in Healthy Human Volunteers.

Antibody Ab1 was dosed in a single bolus infusion in histidine and sorbitol to healthy human volunteers. Dosages of 1 mg, 3 mg, 10 mg, 30 mg or 100 mg were administered to each individual in dosage groups containing five to six individuals. Plasma samples were removed at fixed time intervals for up to twelve weeks. Human plasma was collected via venipuncture into a vacuum collection tube containing EDTA. Plasma was separated and used to assess the circulating levels of Ab1 using a monoclonal antibody specific for Ab1, as follows. A 96 well microtiter plate was coated overnight with the monoclonal antibody specific for Ab1 in IX PBS overnight at 4 °C. The remaining steps were conducted at room temperature. The wells were aspirated and subsequently blocked using 0.5% Fish Skin Gelatin (FSG) (Sigma) in IX PBS for 60 minutes. Human plasma samples were then added and incubated for 60 minutes, then aspirated, then 50 µL of 1 µg/mL biotinylated IL-6 was then added to each well and incubated for 60 minutes. The wells were aspirated, and 50 µL streptavidin-HRP (Pharmingen), diluted 1:5,000 in 0.5% FSG/PBS, was added and incubated for 45 minutes. Development was conducted using standard methods employing TMB for detection. Levels were then determined via comparison to a standard curve prepared in a comparable format.

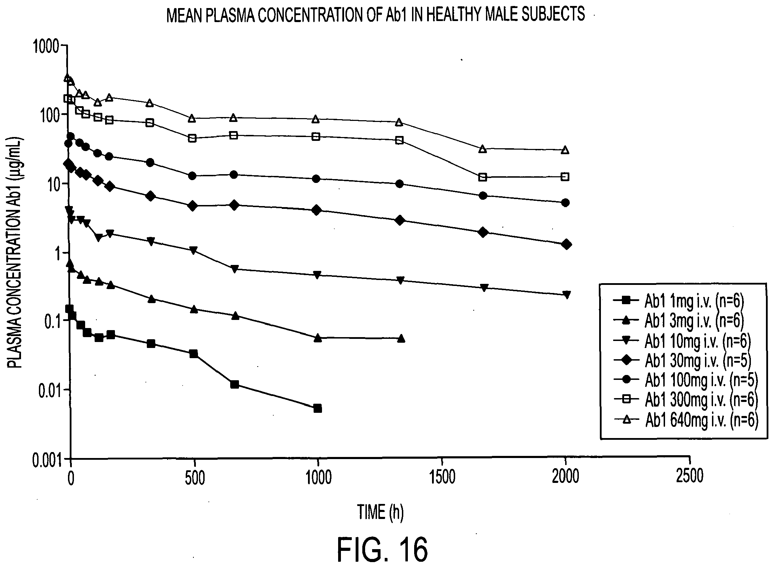

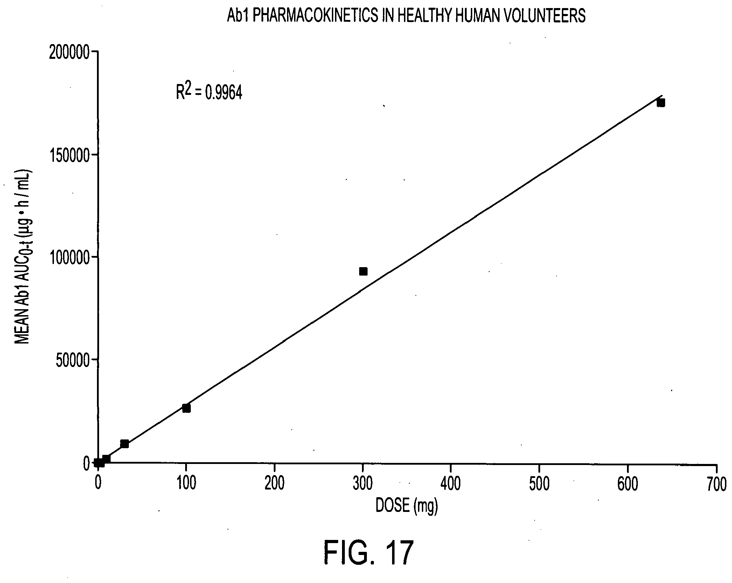

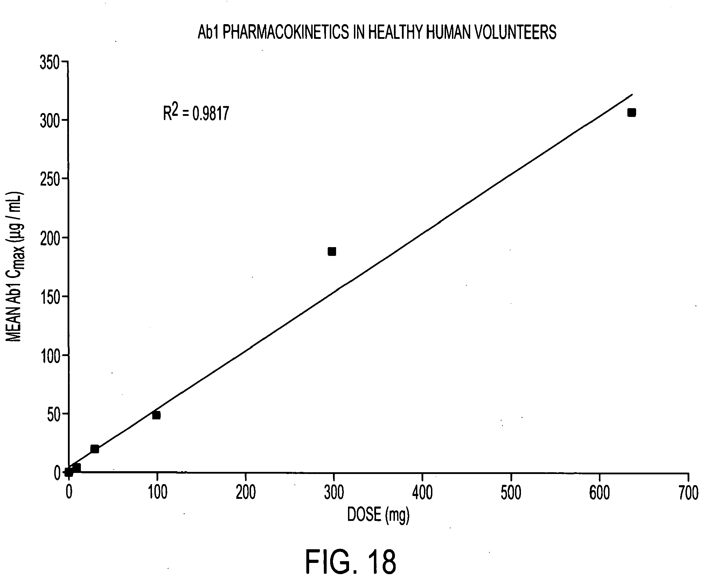

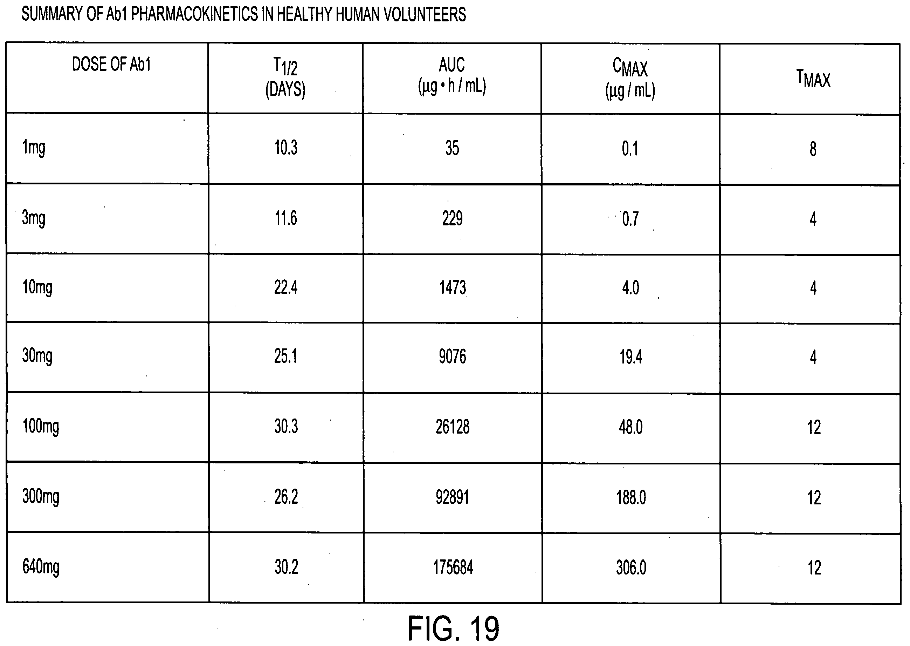

Average plasma concentration of Ab1 for each dosage group versus time is shown in Fig. 16. Mean AUC and Cₘₐₓ increased linearly with dosage (Fig. 17 and Fig. 18, respectively). For dosages of 30 mg and above, the average Ab1 half-life in each dosage group was between approximately 25 and 30 days (Fig. 19).

### Example 17 Pharmacokinetics of Ab1 in patients with advanced cancer.

Antibody Ab1 was dosed in a single bolus infusion in phosphate buffered saline to five individuals with advanced cancer. Each individual received a dosage of 80 mg (n=2) or 160 mg (n=3) of Ab1. Plasma samples were drawn weekly, and the level of antibody Ab1 was quantitated as in Example 16.

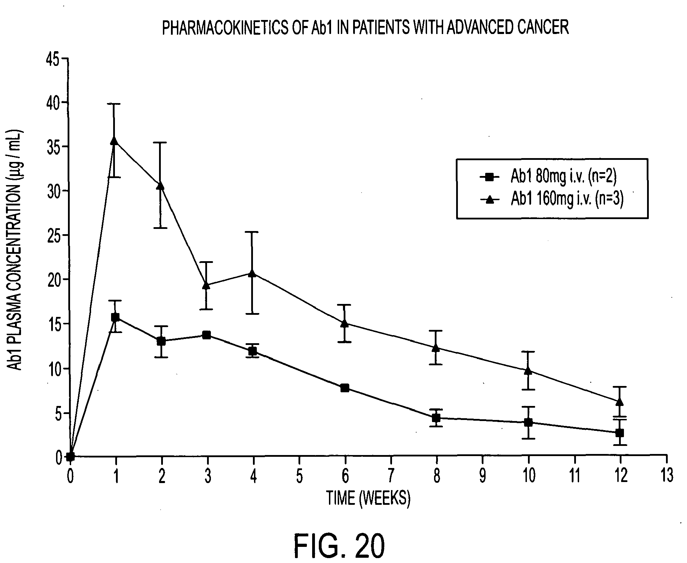

Average plasma concentration of Ab1 in these individuals as a function of time is shown in Fig. 20. The average Ab1 half-life was approximately 31 days.

### Example 18 Unprecedented half-life of Ab1.

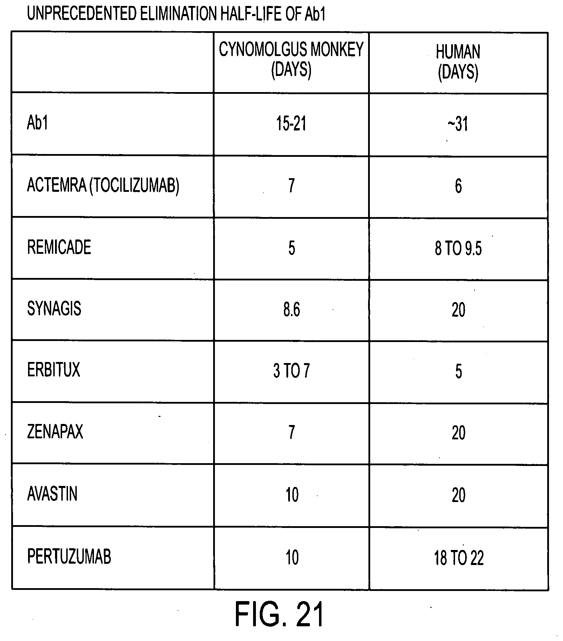

Overall, the average half-life of Ab1 was approximately 31 days in humans (for dosages of 10 mg and above), and approximately 15-21 days in cynomolgus monkey. The Ab1 half-life in humans and cynomolgus monkeys are unprecedented when compared with the half-lives of other anti-IL-6 antibodies (Fig. 21). As described above, Ab1 was derived from humanization of a rabbit antibody, and is produced from Pichia pastoris in an aglycosylated form. These characteristics results in an antibody with very low immunogenicity in humans. Moreover, the lack of glycosylation prevents Ab1 from interacting with the Fc receptor or complement. Without intent to be limited by theory, it is believed that the unprecedented half-life of Ab1 is at least partially attributable to the humanization and lack of glycosylation. The particular sequence and/or structure of the antigen binding surfaces may also contribute to Ab1's half-life.

### Example 19 Ab1 Effect on Hemoglobin Concentration, Plasma Lipid Concentration, and Neutrophil Counts in Patients with Advanced Cancer.

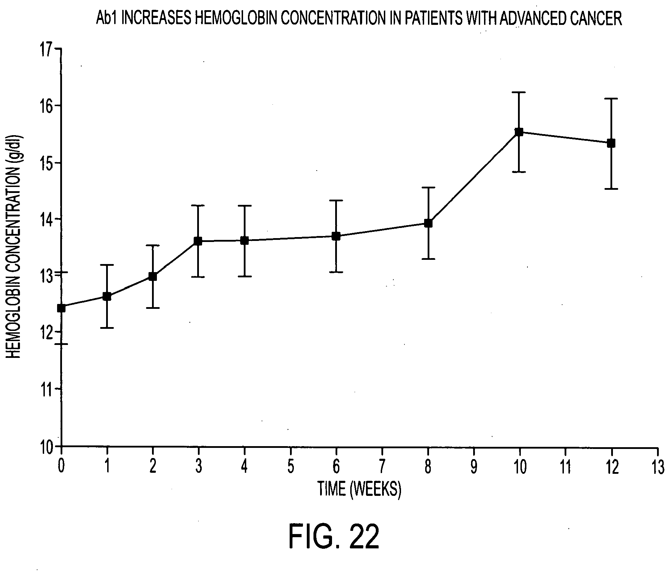

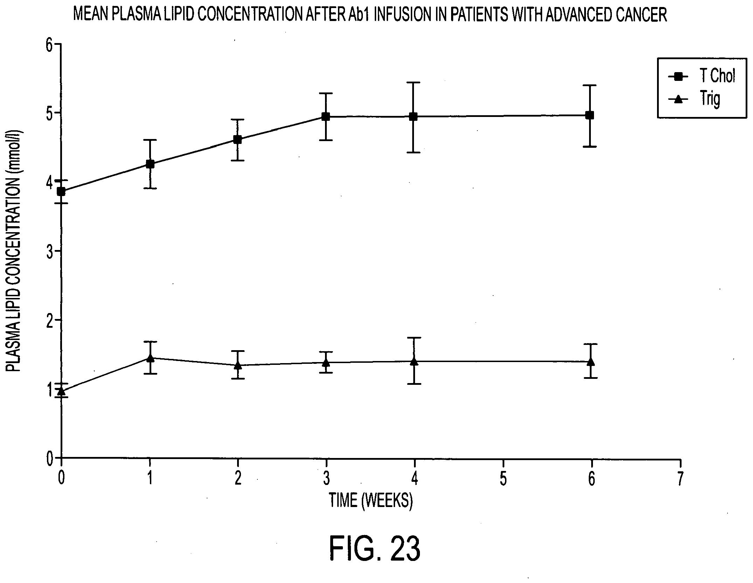

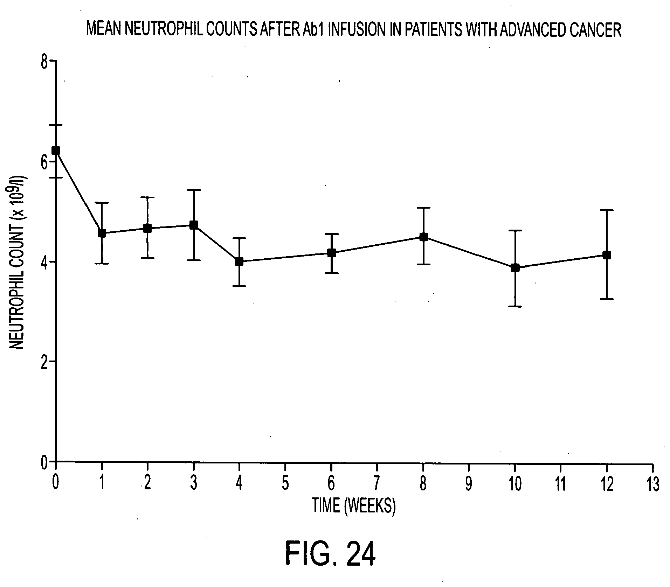

Antibody Ab1 was dosed in a single bolus infusion in phosphate buffered saline to eight individuals with advanced cancer (NSCLC, colorectal cancer, cholangiocarcinoma, or mesothelioma). Each individual received a dosage of 80 mg, 160 mg, or 320 mg of Ab1. Blood samples were removed just prior to infusion and at fixed time intervals for six weeks, and the hemoglobin concentration, plasma lipid concentration, and neutrophil counts were determined. Average hemoglobin concentration rose slightly (Fig. 22), as did total cholesterol and triglycerides (Fig. 23), while mean neutrophil counts fell slightly (Fig. 24).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration in these individuals. Similarly, as IL-6 is centrally important in increasing neutrophil counts in inflammation, the observed slight reduction in neutrophil counts further confirms that Ab1 inhibits IL-6. Finally, IL-6 causes anorexia as well as cachexia in these patients; neutralization of IL-6 by Ab1 results in the return of appetite and reversal of cachexia. The increase in plasma lipid concentrations reflect the improved nutritional status of the patients. Taken together, these results further demonstrate that Ab1 effectively reverses these adverse consequences of IL-6 in these patients.

### Example 20 Ab1 Suppresses Serum CRP in Healthy Volunteers and in Patients with Advanced Cancer.

### Introduction

Serum CRP concentrations have been identified as a strong prognostic indicator in patients with certain forms of cancer. For example, Hashimoto *et al.* performed univariate and multivariate analysis of preoperative serum CRP concentrations in patients with hepatocellular carcinoma in order to identify factors affecting survival and disease recurrence (Hashimoto, K., et al., Cancer, 103(9):1856-1864 (2005)). Patients were classified into two groups, those with serum CRP levels > 1.0 mg/dL ("the CRP positive group") and those with serum CRP levels < 1.0 mg/dL ("the CRP negative group"). The authors identified "a significant correlation between preoperative serum CRP level and tumor size." *Id.* Furthermore, the authors found that "[t]he overall survival and recurrence-free survival rates in the CRP-positive group were significantly lower compared with the rates in the CRP-negative group." *Id.* The authors concluded that the preoperative CRP level of patients is an independent and significant predictive indicator of poor prognosis and early recurrence in patients with hepatocellular carcinoma.

Similar correlations have been identified by other investigators. For example, Karakiewicz *et al.* determined that serum CRP was an independent and informative predictor of renal cell carcinoma-specific mortality (Karakiewicz, P.I., et al., Cancer, 110(6):1241-1247 (2007)). Accordingly, there remains a need in the art for methods and/or treatments that reduce serum C-Reactive Protein (CRP) concentrations in cancer patients, and particularly those with advanced cancers.

### Methods

Healthy volunteers received a single 1-hour intravenous (IV) infusion of either 100 mg (5 patients), 30 mg (5 patients), 10 mg (6 patients), 3 mg (6 patients) or 1 mg (6 patients) of the Ab1 monoclonal antibody, while another 14 healthy volunteers received intravenous placebo. Comparatively, 2 patients with advanced forms of colorectal cancer received a single 1-hour intravenous (IV) infusion of 80 mg of the Ab1 monoclonal antibody. No further dosages of the Ab1 monoclonal antibody were administered to the test population.

Patients were evaluated prior to administration of the dosage, and thereafter on a weekly basis for at least 5 weeks post dose. At the time of each evaluation, patients were screened for serum CRP concentration.

### Results

### Healthy Volunteers

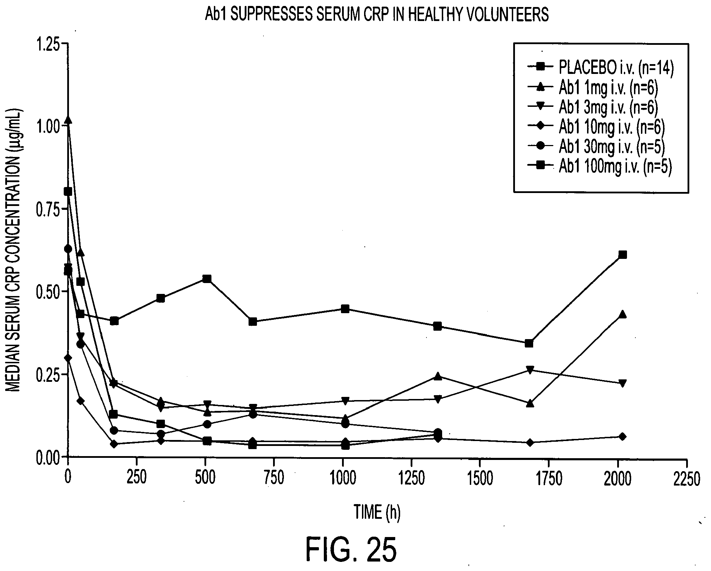

As noted above, serum CRP levels are a marker of inflammation; accordingly, baseline CRP levels are typically low in healthy individuals. The low baseline CRP levels can make a further reduction in CRP levels difficult to detect. Nonetheless, a substantial reduction in serum CRP concentrations was detectable in healthy volunteers receiving all concentrations of the Ab1 monoclonal antibody, compared to controls (Fig. 25). The reduction in serum CRP levels was rapid, occurring within one week of antibody administration, and prolonged, continuing at least through the final measurement was taken (8 or 12 weeks from antibody administration).

### Cancer Patients

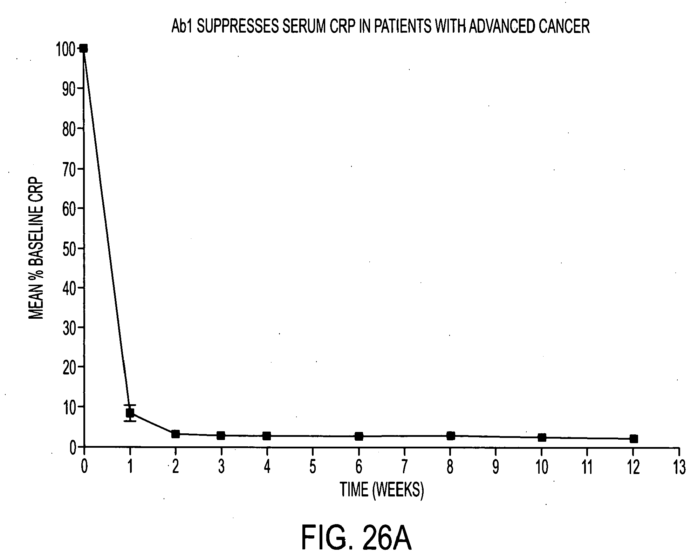

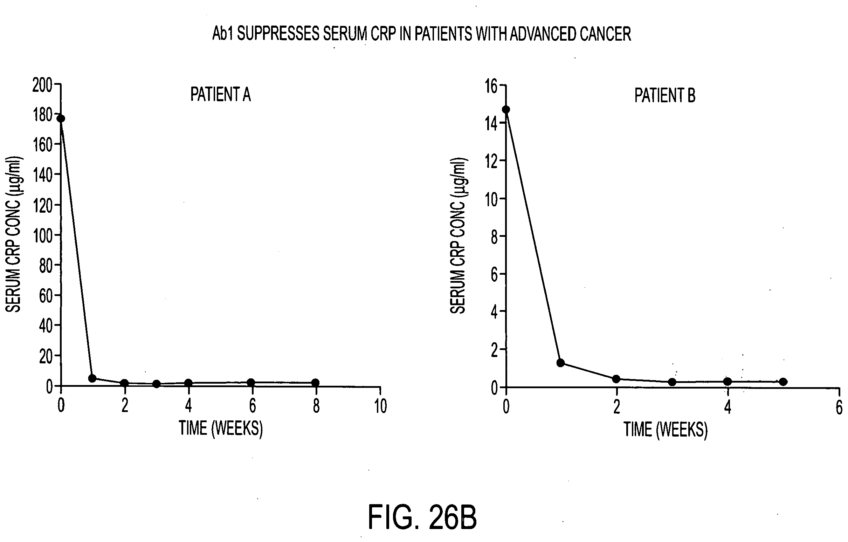

Five advanced cancer patients (colorectal cancer, cholangiocarcinoma, or NSCLC) having elevated serum CRP levels were dosed with 80 mg or 160 mg of Ab1. Serum CRP levels were greatly reduced in these patients (Fig. 26A). The reduction in serum CRP levels was rapid, with 90% of the decrease occurring within one week of Ab1 administration, and prolonged, continuing at least until the final measurement was taken (up to twelve weeks). The CRP levels of two representative individuals are shown in Fig. 26B. In those individuals, the CRP levels were lowered to below the normal reference range (less than 5 - 6 mg/1) within one week. Thus, administration of Ab1 to advanced cancer patients can cause a rapid and sustained suppression of serum CRP levels.

### Example 21 Ab1 Improved Muscular Strength, Improved Weight, and Reduced Fatigue in Patients with Advanced Cancer

### Introduction

Weight loss and fatigue (and accompanying muscular weakness) are very common symptoms of patients with advanced forms of cancer, and these symptoms can worsen as the cancer continues to progress. Fatigue, weight loss and muscular weakness can have significant negative effects on the recovery of patients with advanced forms of cancer, for example by disrupting lifestyles and relationships and affecting the willingness or ability of patients to continue cancer treatments. Known methods of addressing fatigue, weight loss and muscular weakness include regular routines of fitness and exercise, methods of conserving the patient's energy, and treatments that address anemia-induced fatigue and muscular weakness. Nevertheless, there remains a need in the art for methods and/or treatments that improve fatigue, weight loss and muscular weakness in cancer patients.

### Methods

Four patients with advanced forms of cancer (colorectal cancer (2), NSCLC (1), cholangiocarcinoma (1) received a single 1-hour intravenous (IV) infusion of either 80 mg or 160 mg of the Ab1 monoclonal antibody. No further dosages of the Ab1 monoclonal antibody were administered to the test population.

Patients were evaluated prior to administration of the dosage, and thereafter for at least 6 weeks post dose. At the time of each evaluation, patients were screened for the following: a.) any change in weight; b.) fatigue as measured using the Facit-F Fatigue Subscale questionnaire a medically recognized test for evaluating fatigue (See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.); and hand-grip strength (a medically recognized test for evaluating muscle strength, typically employing a handgrip dynamometer).

### Results

### Weight Change

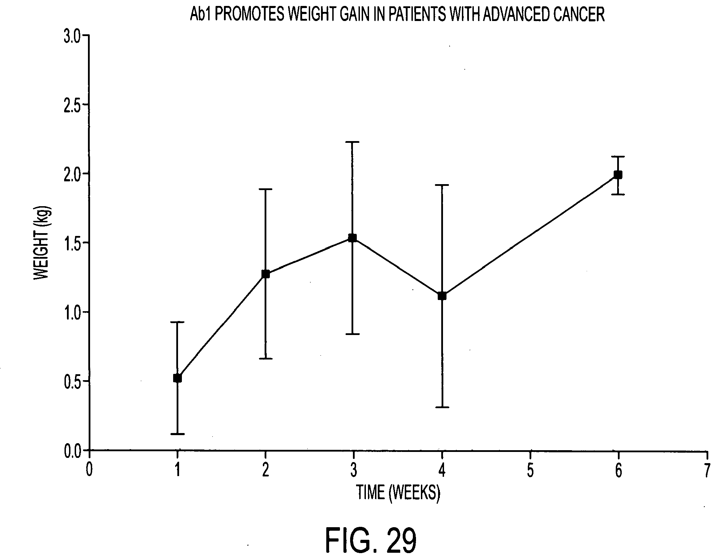

The averaged data for both dosage concentrations (80 mg and 160 mg) of the Ab1 monoclonal antibody demonstrated an increase of about 2 kilograms of weight per patient over the period of 6 weeks (Fig. 29).

### Fatigue

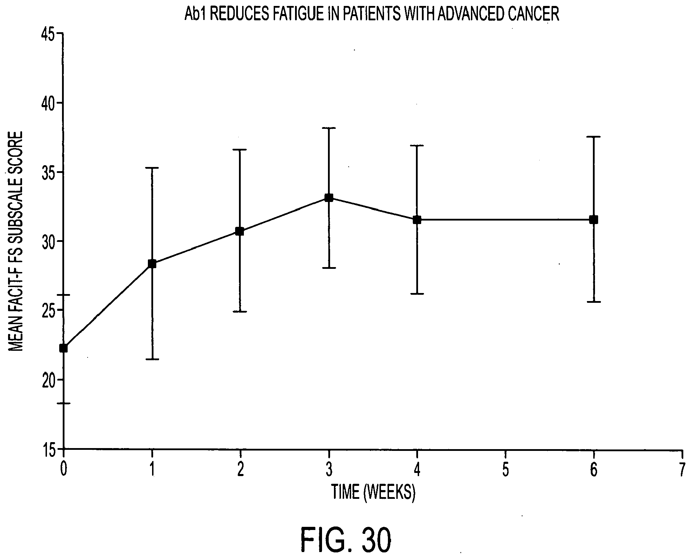

The averaged data for both dosage concentrations (80 mg and 160 mg) of the Ab1 monoclonal antibody demonstrated an increase in the mean Facit-F FS subscale score of at least about 10 points in the patient population over the period of 6 weeks (Fig. 30).

### Hand-Grip Strength

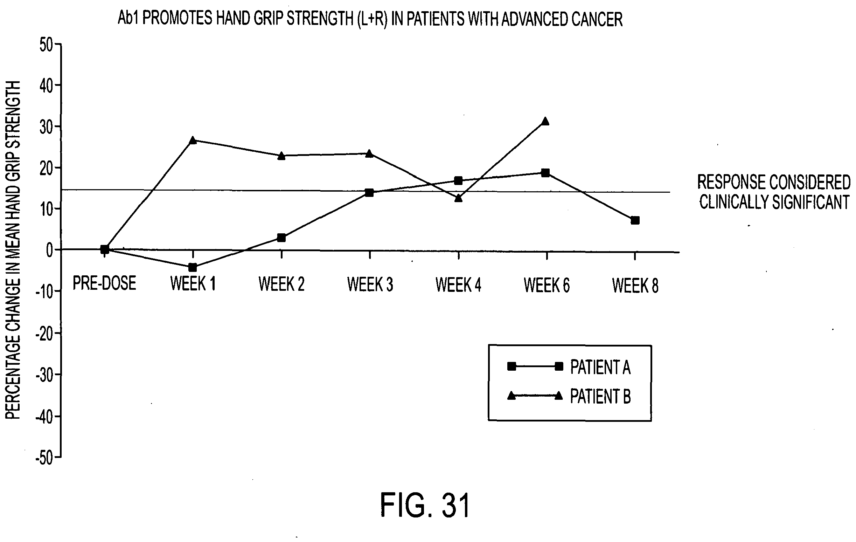

The averaged data for both dosage concentrations (80 mg and 160 mg) of the Ab1 monoclonal antibody demonstrated an increase in the mean hand-grip strength of at least about 10 percent in the patient population over the period of 6 weeks (Fig. 31).

### Example 22 Ab1 Increases Plasma Albumin Concentration in Patients with Advanced Cancer

### Introduction

Serum albumin concentrations are recognized as predictive indicators of survival and/or recovery success of cancer patients. Hypoalbumenia correlates strongly with poor patient performance in numerous forms of cancer. For example, in one study no patients undergoing systemic chemotherapy for metastatic pancreatic adenocarcinoma and having serum albumin levels less than 3.5 g/dL successfully responded to systemic chemotherapy (Fujishiro, M., et al., Hepatogastroenterology, 47(36):1744-46 (2000)). The authors conclude that "[p]atients with ... hypoalbuminemia ... might be inappropriate candidates for systemic chemotherapy and might be treated with other experimental approaches or supportive care." *Id.*

Similarly, Senior and Maroni state that "[t]he recent appreciation that hypoalbuminemia is the most powerful predictor of mortality in end-stage renal disease highlights the critical importance of ensuring adequate protein intake in this patient population." (J.R. Senior and B.J. Maroni, Am. Soc. Nutr. Sci., 129:313S-314S (1999)).

In at least one study, attempts to rectify hypoalbuminemia in 27 patients with metastatic cancer by daily intravenous albumin infusion of 20 g until normal serum albumin levels (>3.5 g/dL) were achieved had little success. The authors note that "[a]lbumin infusion for the advanced stage cancer patients has limited value in clinical practice. Patients with PS 4 and hypoalbuminemia have poorer prognosis." (Demirkazik, A., et al., Proc. Am. Soc. Clin. Oncol., 21:Abstr 2892 (2002)).

Accordingly, there remains a need in the art for methods and/or treatments that improve serum albumin concentrations in cancer patients and address hypoalbuminemic states in cancer patients, particularly those with advanced cancers.

### Methods

Four patients with advanced forms of cancer (colorectal cancer (2), NSCLC (1), cholangiocarcinoma (1) received a single 1-hour intravenous (IV) infusion of either 80 mg or 160 mg of the Ab1 monoclonal antibody. No further dosages of the Ab1 monoclonal antibody were administered to the test population.

Patients were evaluated prior to administration of the dosage, and thereafter for at least 6 weeks post dose. At the time of each evaluation, patients were screened for plasma albumin concentration.

### Results

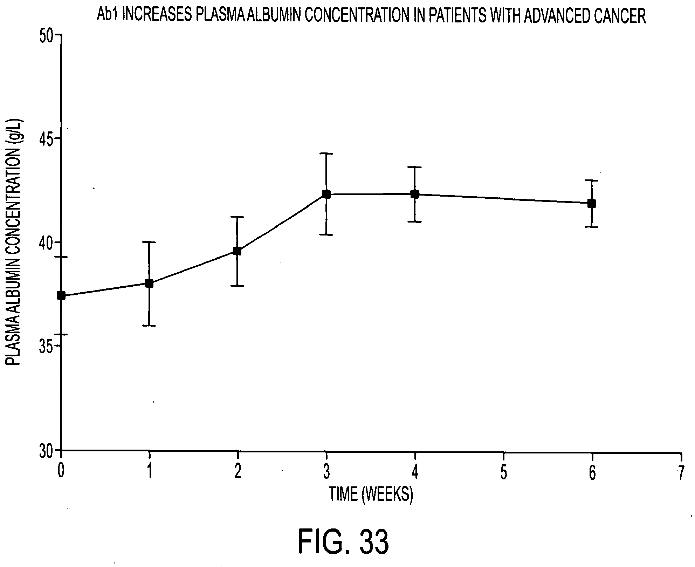

The averaged data for both dosage concentrations (80 mg and 160 mg) of the Ab1 monoclonal antibody demonstrated an increase of about 5 g/L of plasma albumin concentration per patient over the period of 6 weeks (Fig. 33).

### Example 23 Ab1 Increases Hemoglobin in Patients with Advanced Cancer

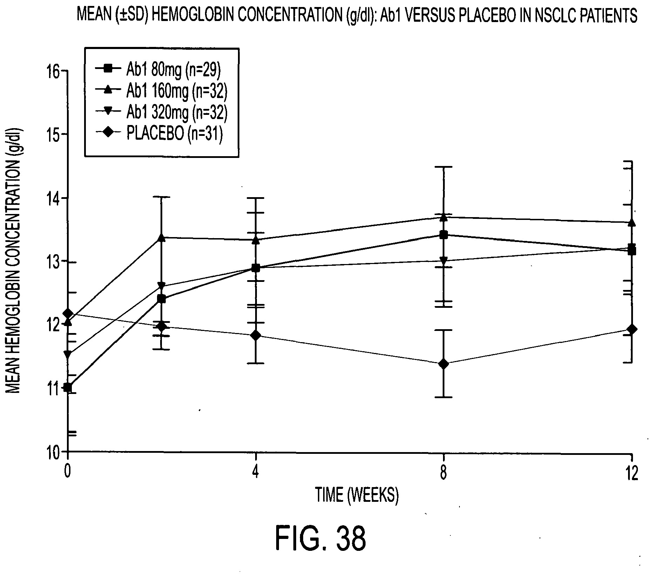

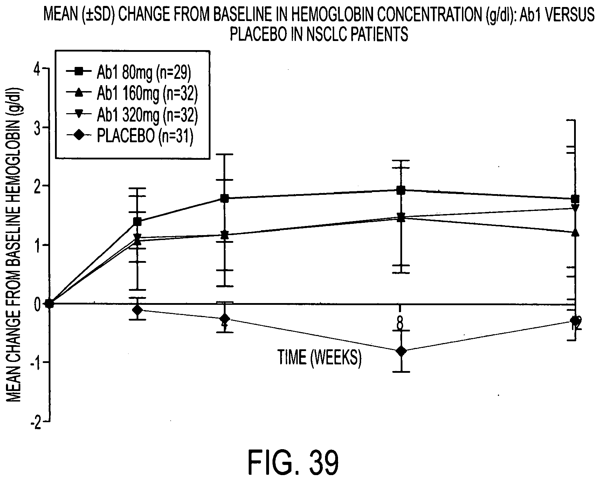

Antibody Ab1 was dosed at 80 mg, 160 mg, or 320 mg of Ab1 in phosphate buffered saline to 93 individuals with non-small cell lung carcinoma. The placebo group of 31 individuals with non-small cell lung carcinoma was dosed with with phosphate buffered saline only. Blood samples were removed just prior to dosing (zero week), and at two, four, eight and twelve weeks, and the hemoglobin concentration was determined. Mean hemoglobin concentration rose for those receiving antibody Ab1, while mean hemoglobin concentration of those receiving placebo did not rise after twelve weeks when compared to the concentration just prior to dosing (zero week) (Figs. 38 and 39).

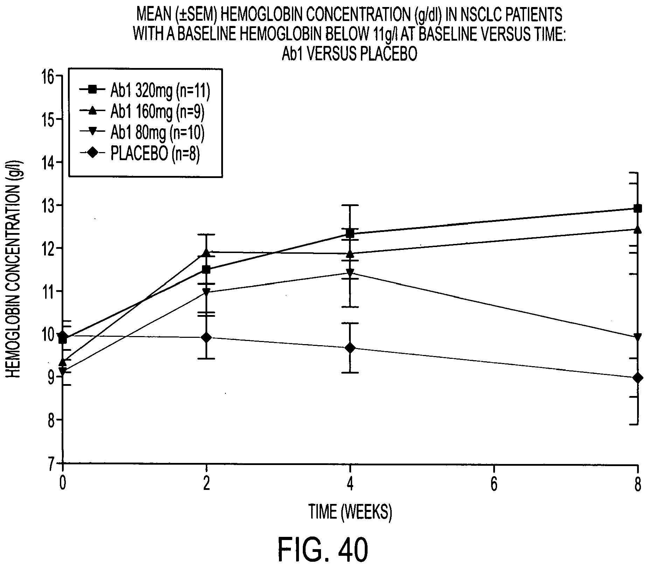

A subset of the study population began the study with low levels of hemoglobin, defined as a baseline hemoglobin concentration below 11 g/l. Mean hemoglobin concentration rose above 11 g/l after eight weeks for those receiving antibody Ab1 at dosages of 160 mg and 320 mg, while mean hemoglobin concentration of those receiving antibody Ab1 at dosages of 80 mg or placebo did not rise above 11 g/l after eight weeks (Fig. 40).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration in these individuals.

### Example 24 Ab1 Increases Hemoglobin in Patients with Rheumatoid Arthritis.

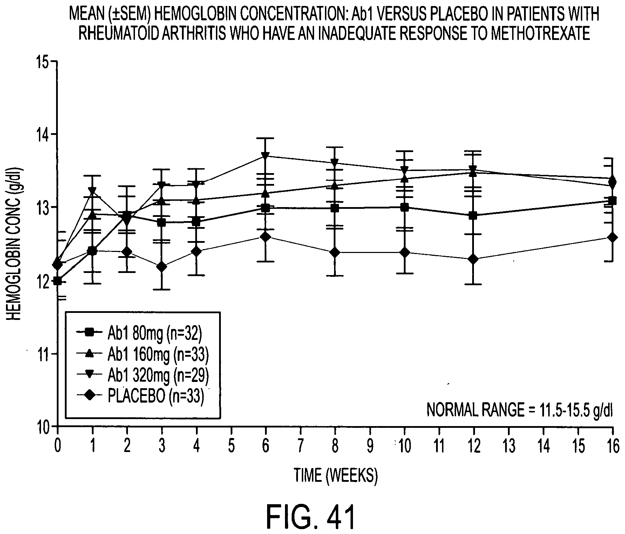

Hemoglobin levels were analyzed in patients with rheumatoid arthritis during treatment with Ab1 antibody. Ab1 antibody was dosed at 80 mg, 160 mg, or 320 mg in phosphate buffered saline to 94 individuals with rheumatoid arthritis. The placebo group of 33 individuals with rheumatoid arthritis was dosed with phosphate buffered saline only. Blood samples were removed just prior to dosing (zero week), and at one, two, three, four, six, eight, ten, twelve, and sixteen weeks, and the hemoglobin concentration was determined. Mean hemoglobin concentration rose for those receiving antibody Ab1, while mean hemoglobin concentration of those receiving placebo did not appreciably rise after sixteen weeks when compared to the concentration just prior to dosing (zero week) (Fig. 41).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration.

### Example 25 Ab1 Improved Weight and Reduced Fatigue in Patients with Advanced Cancer

### Introduction

Weight loss and fatigue are very common symptoms of patients with advanced forms of cancer, and these symptoms can worsen as the cancer continues to progress. Fatigue and weight loss can have significant negative effects on the recovery of patients with advanced forms of cancer, for example by disrupting lifestyles and relationships and affecting the willingness or ability of patients to continue cancer treatments. Known methods of addressing fatigue and weight loss include regular routines of fitness and exercise, methods of conserving the patient's energy, and treatments that address anemia-induced fatigue. Nevertheless, there remains a need in the art for methods and/or treatments that improve fatigue and weight loss in cancer patients.

### Methods

One-hundred twenty-four patients with non-small cell lung cancer (NSCLC) were divided into 4 treatment groups. Patients in one group received one 1-hour intravenous (IV) infusion of either placebo (n=31), 80 mg (n=29), 160 mg (n=32), or 320 mg (n=32) of the Ab1 monoclonal antibody every 8 weeks over a 24 week duration for a total of 3 doses.

Patients were evaluated prior to administration of the dosage, and thereafter for at least 12 weeks post dose. At the time of each evaluation, patients were screened for the following: a.) any change in weight; and b.) fatigue as measured using the Facit-F Fatigue Subscale questionnaire a medically recognized test for evaluating fatigue (See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.).

### Results

### Weight Change

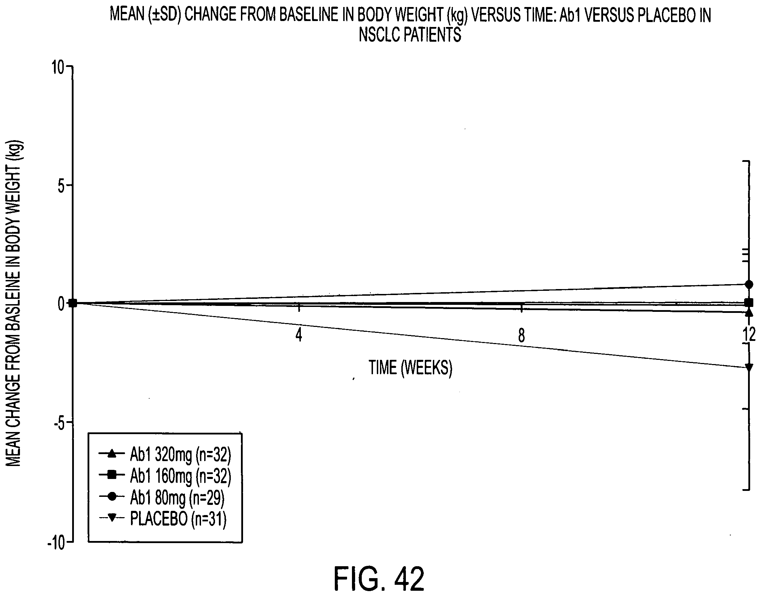

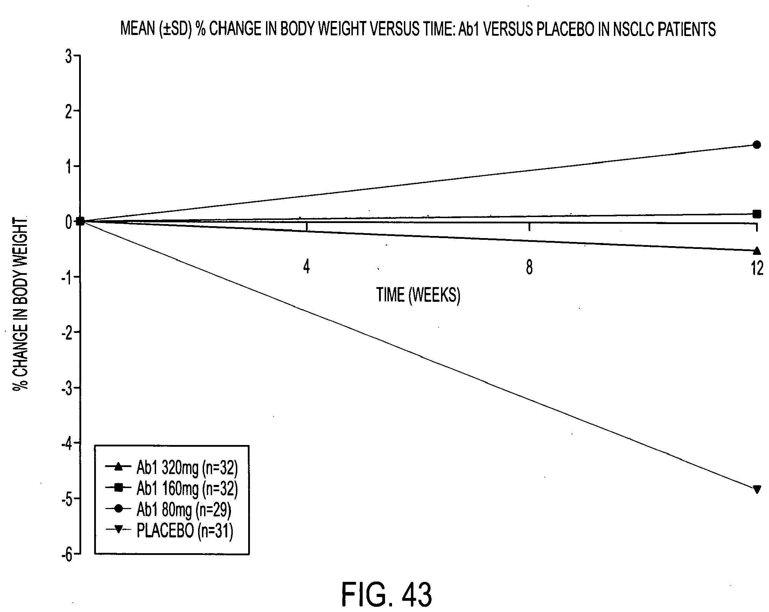

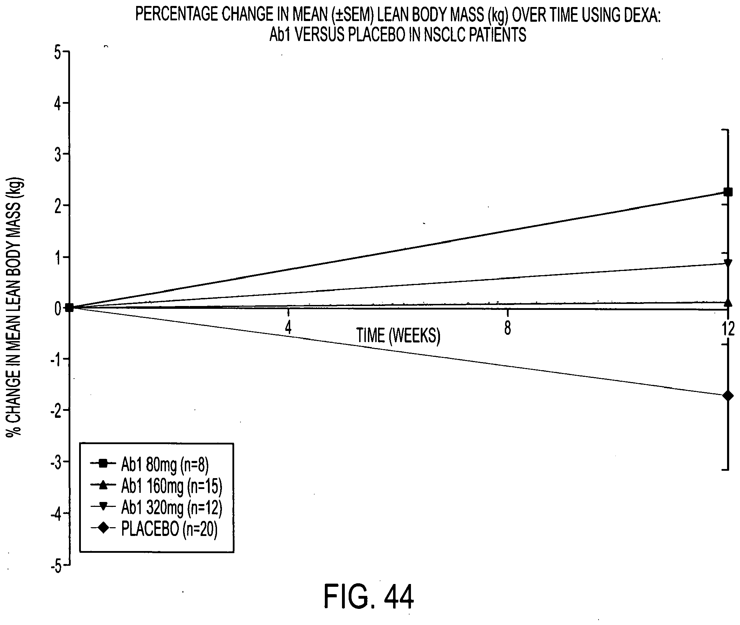

The averaged weight change data from each dosage concentration group (placebo, 80 mg, 160 mg, and 320 mg) of the Ab1 monoclonal antibody over 12 weeks is plotted in Figure 42. The average percent change in body weight from each dosage concentration is plotted in Fig. 43. The averaged lean body mass data for the dosage concentration groups is plotted in Figure 44.

### Fatigue

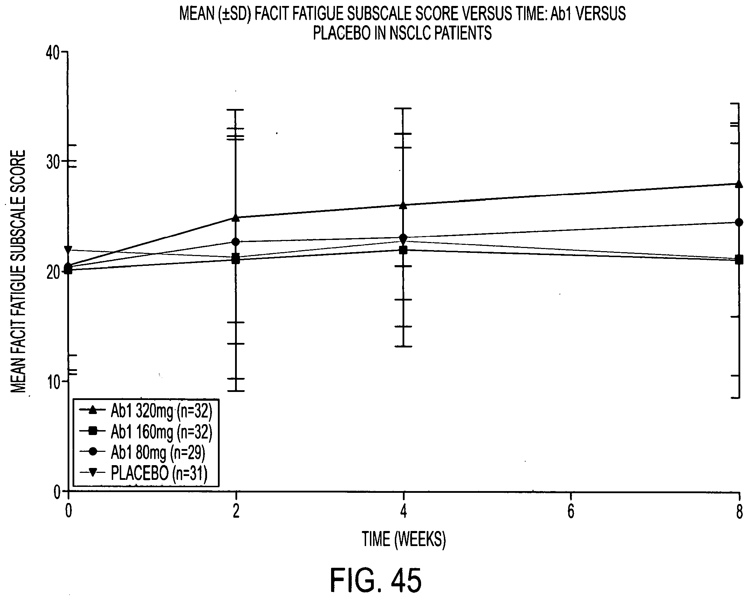

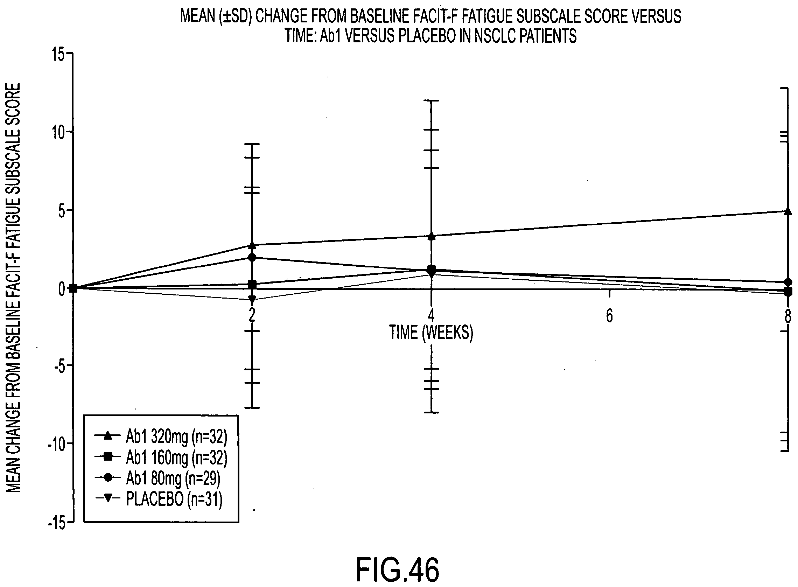

The averaged fatigue from each dosage concentration group (placebo, 80 mg, 160 mg, and 320 mg) of the Ab1 monoclonal antibody demonstrated increases in the mean Facit-F FS subscale score for some of the dosage concentration groups in the patient population over the period of 8 weeks (Fig. 45). The change from baseline Facit-F subscale score is plotted in Figure 46.

### SEQUENCE LISTING

The biological sequences referenced herein are provided below:
**SEQ ID NO: 1**
**SEQ ID NO: 2**
**SEQ ID NO: 3**
**SEQ ID NO: 4**
   QASQSINNELS
**SEQ ID NO: 5**
   RASTLAS
**SEQ ID NO: 6**
   QQGYSLRNIDNA
**SEQ ID NO: 7**
   NYYVT
**SEQ ID NO: 8**
   IIYGSDETAYATWAIG
**SEQ ID NO: 9**
   DDSSDWDAKFNL
**SEQ ID NO: 10**
**SEQ ID NO: 11**
**SEQ ID NO: 12**
   CAGGCCAGTCAGAGCATTAACAATGAATTATCC
**SEQ ID NO: 13**
   AGGGCATCCACTCTGGCATCT
**SEQ ID NO: 14**
   CAACAGGGTTATAGTCTGAGGAATATTGATAATGCT
**SEQ ID NO: 15**
   AACTACTACGTGACC
**SEQ ID NO: 16**
   ATCATTTATGGTAGTGATGAAACGGCCTACGCGACCTGGGCGATAGGC
**SEQ ID NO: 17**
   GATGATAGTAGTGACTGGGATGCAAAATTTAACTTG
**SEQ ID NO: 18**
**SEQ ID NO: 19**
**SEQ ID NO: 20**
**SEQ ID NO: 120**
   IIYGSDETAYATSAIG
**SEQ ID NO: 586**
**SEQ ID NO: 587**
**SEQ ID NO: 588**
**SEQ ID NO: 589**
**SEQ ID NO: 657**
**SEQ ID NO: 700**
**SEQ ID NO: 706**
**SEQ ID NO: 708**
**SEQ ID NO: 709**
**SEQ ID NO: 719**
**SEQ ID NO: 723**
**SEQ ID NO: 726**
**SEQ ID NO: 727**
**SEQ ID NO: 728**

## Claims

1. Use of an anti-IL-6 antibody or antibody fragment in the manufacture of a medicament for use in a method of preventing or treating cachexia in a patient diagnosed with an IL-6 associated disorder,
wherein the administered antibody or fragment comprises:
(a) the V_{L} CDRs in SEQ ID NO:4, 5 and 6 and the V_{H} CDRs in SEQ ID NO: 7, 8 or 120 and 9, or
(b) a V_{H} polypeptide comprising the polypeptide depicted in SEQ ID NO: 3, 18, or 19, and further comprises a V_{L} polypeptide comprising the polypeptide depicted in SEQ ID NO: 2, or 20, and
wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

2. An antibody or antibody fragment for use in a method of preventing or treating cachexia in a patient diagnosed with an IL-6 associated disorder, wherein the antibody or fragment comprises:
(a) the V_{L} CDRs in SEQ ID NO:4, 5 and 6 and the V_{H} CDRs in SEQ ID NO: 7, 8 or 120 and 9, or
(b) a V_{H} polypeptide comprising the polypeptide depicted in SEQ ID NO: 3, 18, or 19, and further comprises a V_{L} polypeptide comprising the polypeptide depicted in SEQ ID NO: 2, or 20, and
wherein the IL-6 associated disorder is AIDS, malnutrition, lead exposure, malaria, hypothyroidism, tuberculosis, hypopituitarism, neurasthenia, hypernatremia, hyponatremia, splenica, failure to thrive (faltering growth), or combinations thereof.

3. The use of the antibody according to claim 1 or the antibody for use according to claim 2, wherein the anti-IL-6 antibody or antibody fragment in the medicament is aglycosylated or contains an Fc region that has been modified to alter effector function, half-life, proteolysis, and/or glycosylation.

4. The use of the antibody according to claim 1 or the antibody for use according to claim 2, wherein the anti-IL-6 antibody or antibody fragment in the medicament is a human, humanized, single chain or chimeric antibody.

5. The use of the antibody of claim 1 or the antibody for use according to claim 2, wherein the anti-IL-6 antibody or antibody fragment in the medicament has an elimination half-life of at least about 22 days, 25 days, or 30 days.

6. The use of the antibody of claim 1 or the antibody for use according to claim 2, wherein the medicament is for administration to the patient with a frequency at most once per period of approximately four weeks, eight weeks, twelve weeks, or sixteen weeks.

7. The use of the antibody of claim 1 or the antibody for use according to claim 2, wherein the anti-IL-6 antibody or antibody fragment is co administered with an antagonist of a cachexia-associated factor, weakness-associated factor, fatigue-associated factor, and/or fever-associated factor, wherein the cachexia-associated factor, weakness-associated factor, fatigue-associated factor, and/or fever-associated factor is selected from tumor necrosis factor-alpha, Interferon gamma, Interleukin 1 alpha, Interleukin 1 beta, Interleukin 6, proteolysis inducing factor, leukemia-inhibitory factor, or any combination thereof, or an anti-cachexia agent selected from cannabis, dronabinol (Marinol™), nabilone (Cesamet), cannabidiol, cannabichromene, tetrahydrocannabinol, Sativex, megestrol acetate, or any combination thereof or an anti-nausea or antiemetic agent selected from 5-HT3 receptor antagonists, ajwain, alizapride, anticholinergics, antihistamines, aprepitant, benzodiazepines, cannabichromene, cannabidiol, cannabinoids, cannabis, casopitant, chlorpromazine, cyclizine, dexamethasone, dexamethasone, dimenhydrinate (Gravol™), diphenhydramine, dolasetron, domperidone, dopamine antagonists, doxylamine, dronabinol (Marinol™), droperidol, emetrol, ginger, granisetron, haloperidol, hydroxyzine, hyoscine, lorazepam, meclizine, metoclopramide, midazolam, muscimol, nabilone (Cesamet), nkl receptor antagonists, ondansetron, palonosetron, peppermint, Phenergan, prochlorperazine, Promacot, promethazine, Pentazine, pro pofol, sativex, tetrahydrocannabinol, trimethobenzamide, tropisetron, nandrolone, stilbestrol, thalidomide, lenalidomide, ghrelin agonists, myostatin antagonists, anti-myostatin antibodies, selective androgen receptor modulators, selective estrogen receptor modulators, angiotensin All antagonists, beta two adenergic receptor agonists, beta three adenergic receptor agonists, or any combination thereof.

8. The use of the antibody according to claim 1 or the antibody for use according to claim 2, wherein the anti-IL-6 antibody or antibody fragment containing medicament is used in combination with a chemotherapy agent selected from VEGF antagonists, EGFR antagonists, platins, taxols, irinotecan, 5-fluorouracil, gemcytabine, leucovorine, steroids, cyclophosphamide, melphalan, vinca alkaloids, vinblastine, mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins, IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof or an inhibitor of JAK1, JAK2, JAK3, or SYK or IL- 12 and IL-2 or vinblastine, vincristine, vindesine or vinorelbine.

9. The use of the antibody according to claim 1 or the antibody for use according to claim 2, wherein the anti-IL-6 antibody further comprises the heavy chain and light chain constant regions comprised in SEQ ID NO: 588 and SEQ ID NO: 586 respectively.

10. The use of the antibody according to claim 1 or the antibody for use according to claim 2, wherein the anti-IL-6 antibody or fragment comprises the variable heavy chain polypeptide of SEQ ID NO: 18 or 19 and the variable light chain polypeptide of SEQ ID NO: 20.

11. The use of the antibody or the antibody for use according to any one of the preceding claims, wherein said anti-IL-6 antibody or antibody fragment comprises a human F_{c}, preferably derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19.

## Patentansprüche

1. Verwendung eines anti-IL-6 Antikörpers oder Antikörperfragments bei der Herstellung eines Medikaments zur Verwendung in einem Verfahren zur Vorbeugung oder Behandlung von Kachexie in einem Patienten, bei dem eine mit IL-6 assoziierte Störung diagnostiziert wurde,
wobei der verabreichte Antikörper oder das verabreichte Fragment umfasst:
(a) die V_{L} CDRs in SEQ ID NR:4, 5 und 6 und die V_{H} CDRs in SEQ ID NR: 7, 8 oder 120 und 9 oder
(b) ein V_{H} Polypeptid umfassend das in SEQ ID NR: 3, 18 oder 19 dargestellte Polypeptid und ferner ein V_{L} Polypeptid umfassend das in SEQ ID NR: 2 oder 20 dargestellte Polypeptid umfasst, und
wobei die mit IL-6 assoziierte Störung AIDS, Mangelernährung, Bleiexposition, Malaria, Hypothyreose, Tuberkulose, Hypopituitarismus, Neurasthenie, Hypernatriämie, Hyponatriämie, Splenica, Gedeihstörung (stagnierendes Wachstum) oder Kombinationen davon ist.

2. Antikörper oder Antikörperfragment zur Verwendung in einem Verfahren zur Vorbeugung oder Behandlung von Kachexie in einem Patienten, bei dem eine mit IL-6 assoziierte Störung diagnostiziert wurde, wobei der Antikörper oder das Fragment umfasst:
(a) die V_{L} CDRs in SEQ ID NR:4, 5 und 6 und die V_{H} CDRs in SEQ ID NR: 7, 8 oder 120 und 9 oder
(b) ein V_{H} Polypeptid umfassend das in SEQ ID NR: 3, 18 oder 19 dargestellte Polypeptid und ferner ein V_{L} Polypeptid umfassend das in SEQ ID NR: 2 oder 20 dargestellte Polypeptid umfasst, und
wobei die mit IL-6 assoziierte Störung AIDS, Mangelernährung, Bleiexposition, Malaria, Hypothyreose, Tuberkulose, Hypopituitarismus, Neurasthenie, Hypernatriämie, Hyponatriämie, Splenica, Gedeihstörung (stagnierendes Wachstum) oder Kombinationen davon ist.

3. Verwendung des Antikörpers gemäß Anspruch 1 oder Antikörper zur Verwendung gemäß Anspruch 2, wobei der anti-IL-6 Antikörper oder das anti-IL-6 Antikörperfragment in dem Medikament aglykosiliert ist oder eine Fc Region enthält, die modifiziert wurde, um Effektorfunktion, Halbwertszeit, Proteolyse und/oder Glykosylierung zu verändern.

4. Verwendung des Antikörpers gemäß Anspruch 1 oder Antikörper zur Verwendung gemäß Anspruch 2, wobei der anti-IL-6 Antikörper oder das anti-IL-6 Antikörperfragment in dem Medikament ein humaner, humanisierter, einkettiger oder chimärer Antikörper ist.

5. Verwendung des Antikörpers nach Anspruch 1 oder Antikörper zur Verwendung gemäß Anspruch 2, wobei der anti-IL-6 Antikörper oder das anti-IL-6 Antikörperfragment in dem Medikament eine Eliminationshalbwertszeit von mindestens etwa 22 Tagen, 25 Tagen oder 30 Tagen aufweist.

6. Verwendung des Antikörpers nach Anspruch 1 oder Antikörper zur Verwendung gemäß Anspruch 2, wobei das Medikament für die Verabreichung an den Patienten mit einer Häufigkeit von höchstens einmal pro Zeitraum von ungefähr vier Wochen, acht Wochen, zwölf Wochen oder sechzehn Wochen ist.

7. Verwendung des Antikörpers nach Anspruch 1 oder Antikörper zur Verwendung gemäß Anspruch 2, wobei der anti-IL-6 Antikörper oder das anti-IL-6 Antikörperfragment zusammen verabreicht wird mit einem Antagonisten eines mit Kachexie assoziierten Faktors, mit Schwäche assoziierten Faktors, mit Ermüdung assoziierten Faktors und/oder mit Fieber assoziierten Faktors, wobei der mit Kachexie assoziierte Faktor, mit Schwäche assoziierte Faktor, mit Ermüdung assoziierte Faktor und/oder mit Fieber assoziierte Faktor ausgewählt ist aus Tumor-Nekrose-Faktor-alpha, Interferon gamma, Interleukin 1 alpha, Interleukin 1 beta, Interleukin 6, Proteolyse-induzierendem Faktor, Leukämie-hemmendem Faktor oder jeder Kombination davon oder einem anti-Kachexie Mittel ausgewählt aus Cannabis, Dronabinol (Marinol™), Nabilon (Cesamet), Cannabidiol, Cannabichromen, Tetrahydrocannabinol, Sativex, Megestrolacetat oder jeder Kombination davon oder einem Mittel gegen Übelkeit oder Erbrechen ausgewählt aus 5-HT3 Rezeptorantagonisten, Ajowan, Alizaprid, Anticholinergika, Antihistaminen, Aprepitant, Benzodiazepinen, Cannabichromen, Cannabidiol, Cannabinoiden, Cannabis, Casopitant, Chlorpromazin, Cyclizin, Dexamethason, Dexamethason, Dimenhydrinat (Gravol™), Diphenhydramin, Dolasetron, Domperidon, Dopaminantagonisten, Doxylamin, Dronabinol (Marinol™), Droperidol, Emetrol, Ingwer, Granisetron, Haloperidol, Hydroxyzin, Hyoscin, Lorazepam, Meclozin, Metoclopramid, Midazolam, Muscimol, Nabilon (Cesamet), nkl Rezeptorantagonisten, Ondansetron, Palonosetron, Pfefferminze, Phenergan, Prochlorperazin, Promacot, Promethazin, Pentazin, Propofol, Sativex, Tetrahydrocannabinol, Trimethobenzamid, Tropisetron, Nandrolon, Stilbestrol, Thalidomid, Lenalidomid, Ghrelinagonisten, Myostatinantagonisten, anti-Myostatin Antikörpern, selektiven Androgen-Rezeptormodulatoren, selektiven Östrogen-Rezeptormodulatoren, Angiotensin A11 Antagonisten, beta zwei Adrenozeptoragonisten, beta drei Adrenozeptoragonisten oder jeder Kombination davon.

8. Verwendung des Antikörpers gemäß Anspruch 1 oder Antikörper zur Verwendung gemäß Anspruch 2, wobei das anti-IL-6 Antikörper oder Antikörperfragment enthaltende Medikament in Kombination mit einem chemotherapeutischen Mittel ausgewählt aus VEGF Antagonisten, EGFR Antagonisten, Platinmitteln, Taxolen, Irinotecan, 5-Fluoruracil, Gemcytabin, Leucovorin, Steroiden, Cyclophosphamid, Melphalan, Vincaalkaloiden, Vinblastin, Mustinen, Tyrosinkinaseinhibitoren, Strahlentherapie, Sexualhormonantagonisten, selektiven Androgen-Rezeptormodulatoren, selektiven Östrogen-Rezeptormodulatoren, PDGF Antagonisten, TNF Antagonisten, IL-1 Antagonisten, Interleukinen, IL-12R Antagonisten, Toxinkonjugierten monoklonalen Antikörpern, Tumorantigen-spezifischen monoklonalen Antikörpern, Erbitux™, Avastin™, Pertuzumab, anti-CD20 Antikörpern, Rituxan®, Ocrelizumab, Ofatumumab, DXL625, Herceptin® oder jeder Kombination davon oder einem Inhibitor von JAK1, JAK2, JAK3 oder SYK oder IL- 12 und IL-2 oder Vinblastin, Vincristin, Vindesin oder Vinorelbin verwendet wird.

9. Verwendung des Antikörpers gemäß Anspruch 1 oder Antikörper zur Verwendung gemäß Anspruch 2, wobei der anti-IL-6 Antikörper ferner die von SEQ ID NR: 588 bzw. SEQ ID NR: 586 umfassten konstanten Regionen der schweren Kette und der leichten Kette umfasst.

10. Verwendung des Antikörpers gemäß Anspruch 1 oder Antikörper zur Verwendung gemäß Anspruch 2, wobei der anti-IL-6 Antikörper oder das anti-IL-6 Fragment das variable Schwerkettenpolypeptid von SEQ ID NR: 18 oder 19 und das variable Leichtkettenpolypeptid von SEQ ID NR: 20 umfasst.

11. Verwendung des Antikörpers oder Antikörper zur Verwendung gemäß einem der vorhergehenden Ansprüche, wobei der anti-IL-6 Antikörper oder das anti-IL-6 Antikörperfragment ein humanes F_{c}, bevorzugt abgeleitet aus IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 oder IgG19, umfasst.

## Revendications

1. Utilisation d'un anticorps ou d'un fragment d'anticorps anti-IL-6 dans la fabrication d'un médicament pour utilisation dans un procédé de prévention ou de traitement de la cachexie chez un patient chez qui un trouble associé à IL-6 a été diagnostiqué,
dans laquelle l'anticorps ou le fragment administré comprend :
(a) des CDR de V_{L} dans les SEQ ID NO: 4, 5 et 6 et des CDR de V_{H} dans les SEQ ID NO: 7, 8 ou 120 et 9, ou
(b) un polypeptide V_{H} comprenant le polypeptide représenté dans la SEQ ID NO: 3, 18 ou 19, et comprend en outre un polypeptide V_{L} comprenant le polypeptide représenté dans la SEQ ID NO: 2 ou 20, et
où le trouble associé à IL-6 est le SIDA, la malnutrition, l'exposition au plomb, le paludisme, l'hypothyroïdie, la tuberculose, l'hypopituitarisme, la neurasthénie, l'hypernatrémie, l'hyponatrémie, un trouble splénique, le retard de développement (un retard de croissance), ou des combinaisons de ceux-ci.

2. Anticorps ou fragment d'anticorps pour utilisation dans un procédé de prévention ou de traitement de la cachexie chez un patient chez qui un trouble associé à IL-6 a été diagnostiqué, l'anticorps ou le fragment comprenant :
(a) des CDR de V_{L} dans les SEQ ID NO: 4, 5 et 6 et des CDR de V_{H} dans les SEQ ID NO: 7, 8 ou 120 et 9, ou
(b) un polypeptide V_{H} comprenant le polypeptide représenté dans la SEQ ID NO: 3, 18 ou 19, et comprend en outre un polypeptide V_{L} comprenant le polypeptide représenté dans la SEQ ID NO: 2 ou 20, et
où le trouble associé à IL-6 est le SIDA, la malnutrition, l'exposition au plomb, le paludisme, l'hypothyroïdie, la tuberculose, l'hypopituitarisme, la neurasthénie, l'hypernatrémie, l'hyponatrémie, un trouble splénique, le retard de développement (un retard de croissance), ou des combinaisons de ceux-ci.

3. Utilisation de l'anticorps selon la revendication 1 ou anticorps pour utilisation selon la revendication 2, où l'anticorps ou le fragment d'anticorps anti-IL-6 dans le médicament est aglycosylé ou contient une région Fc qui a été modifiée pour altérer la fonction effectrice, la demi-vie, la protéolyse et/ou la glycosylation.

4. Utilisation de l'anticorps selon la revendication 1 ou anticorps pour utilisation selon la revendication 2, où l'anticorps ou le fragment d'anticorps anti-IL-6 dans le médicament est un anticorps humain, humanisé, à chaîne unique ou chimère.

5. Utilisation de l'anticorps de la revendication 1 ou anticorps pour utilisation selon la revendication 2, où l'anticorps ou le fragment d'anticorps anti-IL-6 dans le médicament a une demi-vie d'élimination d'au moins environ 22 jours, 25 jours, ou 30 jours.

6. Utilisation de l'anticorps de la revendication 1 ou anticorps pour utilisation selon la revendication 2, où le médicament est destiné à être administré au patient avec une fréquence d'au plus une fois par période d'environ quatre semaines, huit semaines, douze semaines ou seize semaines.

7. Utilisation de l'anticorps de la revendication 1 ou anticorps pour utilisation selon la revendication 2, où l'anticorps ou le fragment d'anticorps anti-IL-6 est coadministré avec un antagoniste d'un facteur associé à la cachexie, d'un facteur associé à la faiblesse, d'un facteur associé à la fatigue et/ou d'un facteur associé à la fièvre, où le facteur associé à la cachexie, le facteur associé à la faiblesse, le facteur associé à la fatigue et/ou le facteur associé à la fièvre sont choisis entre le facteur de nécrose tumorale alpha, l'interféron gamma, l'interleukine 1 alpha, l'interleukine 1 bêta, l'interleukine 6, un facteur induisant la protéolyse, un facteur inhibiteur de la leucémie ou toute combinaison de ceux-ci, ou un agent anti-cachexie choisi entre le cannabis, le dronabinol (Marinol™), le nabilone (Cesamet), le cannabidiol, le cannabichromène, le tétrahydrocannabinol, le Sativex, l'acétate de mégestrol ou toute combinaison de ceux-ci, ou un agent anti-nauséeux ou antiémétique choisi entre des antagonistes de récepteur 5-HT3, l'ajowan, l'alizapride, des anticholinergiques, des antihistaminiques, l'aprépitant, des benzodiazépines, le cannabichromène, le cannabidiol, des cannabinoïdes, le cannabis, le casopitant, la chlorpromazine, la cyclizine, la dexaméthasone, la dexaméthasone, le dimenhydrinate (GravolTM), la diphénhydramine, le dolasétron, la dompéridone, des antagonistes de la dopamine, la doxylamine, le dronabinol (Marinol™), le dropéridol, l'Emetrol, le gingembre, le granisétron, l'halopéridol, l'hydroxyzine, l'hyoscine, le lorazépam, la méclizine, le métoclopramide, le midazolam, le muscimol, le nabilone (Cesamet), des antagonistes de récepteur nk1, l'ondansétron, le palonosétron, la menthe poivrée, le Phénergan, la prochlorpérazine, le Promacot, la prométhazine, la Pentazine, le propofol, le sativex, le tétra-hydrocannabinol, le triméthobenzamide, le tropisétron, la nandrolone, le stilbestrol, le thalidomide, le lénalidomide, des agonistes de la ghréline, des antagonistes de la myostatine, des anticorps anti-myostatine, des modulateurs de récepteurs d'androgènes sélectifs, des modulateurs de récepteurs d'oestrogènes sélectifs, des antagonistes de l'angiotensine Al1, des agonistes des récepteurs adrénergiques bêta-2, des agonistes des récepteurs adrénergiques bêta-3 ou toute combinaison de ceux-ci.

8. Utilisation de l'anticorps selon la revendication 1 ou anticorps pour utilisation selon la revendication 2, où le médicament contenant l'anticorps ou le fragment d'anticorps anti-IL-6 est utilisé en combinaison avec un agent de chimiothérapie choisi entre des antagonistes de VEGF, des antagonistes d'EGFR, des platines, des taxols, l'irinotécan, le 5-fluorouracile, la gemcitabine, la leucovorine, des stéroïdes, le cyclophosphamide, le melphalan, les vinca-alcaloïdes, la vinblastine, les mustines, des inhibiteurs de la tyrosine-kinase, la radiothérapie, des antagonistes des hormones sexuelles, des modulateurs de récepteurs d'androgènes sélectifs, des modulateurs de récepteurs d'oestrogènes sélectifs, des antagonistes de PDGF, des antagonistes de TNF, des antagonistes de l'IL-1, des interleukines, des antagonistes de l'IL-12R, des anticorps monoclonaux conjugués à des toxines, des anticorps monoclonaux spécifiques d'un antigène tumoral, l'ErbituxTM, l'AvastinTM, le Pertuzumab, des anticorps anti-CD20, le Rituxan®, l'ocrelizumab, l'ofatumumab, DXL625, l'Herceptin®, ou toute combinaison de ceux-ci, ou un inhibiteur de JAK1, JAK2, JAK3 ou SYK ou IL-12 et IL-2 ou la vinblastine, la vincristine, la vindésine ou la vinorelbine.

9. Utilisation de l'anticorps selon la revendication 1 ou anticorps pour utilisation selon la revendication 2, où l'anticorps anti-IL-6 comprend en outre les régions constantes de chaîne lourde et de chaîne légère contenues dans les SEQ ID NO: 588 et SEQ ID NO: 586 respectivement.

10. Utilisation de l'anticorps selon la revendication 1 ou anticorps pour utilisation selon la revendication 2, où l'anticorps ou le fragment anti-IL-6 comprend le polypeptide de chaîne lourde variable de la SEQ ID NO: 18 ou 19 et le polypeptide de chaîne légère variable de la SEQ ID NO: 20.

11. Utilisation de l'anticorps ou anticorps pour utilisation selon l'une quelconque des revendications précédentes, où ledit anticorps ou fragment d'anticorps anti-IL-6 comprend un F_{c} humain, de préférence dérivé de IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 ou IgG19.


### SEQUENCE LISTING

<110> ALDER BIOPHARMACEUTICALS, INC. Smith, Jeffrey T.L. Schatzman, Randall C. Litton, Mark J. Latham, John
<120> ANTAGONISTS OF IL-6 TO PREVENT OR TREAT CACHEXIA, WEAKNESS, FATIGUE,
   AND/OR FEVER
<130> 67858.707002
<140> PCTUS096273
   <141> 2009-11-24
<150> 61/117,861
   <151> 2008-11-25
<150> 61/117,839
   <151> 2008-11-25
<150> 61/117,811
   <151> 2008-11-25
<150> 12/502,581
   <151> 2009-07-14
<150> 12/399,156
   <151> 2009-03-06
<150> 12/391,717
   <151> 2009-02-24
<150> 12/366,567
   <151> 2009-02-05
<160> 728
<170> PatentIn version 3.5
<210> 1
   <211> 183
   <212> PRT
   <213> Homo sapiens
<400> 1
   Val Pro Pro Gly Glu Asp Ser Lys Asp Val Ala Ala Pro His Arg Gln
   1               5                   10                  15
   Pro Leu Thr Ser Ser Glu Arg Ile Asp Lys Gln Ile Arg Tyr Ile Leu
               20                  25                  30
   Asp Gly Ile Ser Ala Leu Arg Lys Glu Thr Cys Asn Lys Ser Asn Met
           35                  40                  45
   Cys Glu Ser Ser Lys Glu Ala Leu Ala Glu Asn Asn Leu Asn Leu Pro
       50                  55                  60
   Lys Met Ala Glu Lys Asp Gly Cys Phe Gln Ser Gly Phe Asn Glu Glu
   65                  70                  75                  80
   Thr Cys Leu Val Lys Ile Ile Thr Gly Leu Leu Glu Phe Glu Val Tyr
                   85                  90                  95
   Leu Glu Tyr Leu Gln Asn Arg Phe Glu Ser Ser Glu Glu Gln Ala Arg
               100                 105                 110
   Ala Val Gln Met Ser Thr Lys Val Leu Ile Gln Phe Leu Gln Lys Lys
           115                 120                 125
   Ala Lys Asn Leu Asp Ala Ile Thr Thr Pro Asp Pro Thr Thr Asn Ala
       130                 135                 140
   Ser Leu Leu Thr Lys Leu Gln Ala Gln Asn Gln Trp Leu Gln Asp Met
   145                 150                 155                 160
   Thr Thr His Leu Ile Leu Arg Ser Phe Lys Glu Phe Leu Gln Ser Ser
                   165                 170                 175
   Leu Arg Ala Leu Arg Gln Met
               180
<210> 2
   <211> 163
   <212> PRT
   <213> oryctolagus cuniculus
<400> 2
   Met Asp Thr Arg Ala Pro Thr Gln Leu Leu Gly Leu Leu Leu Leu Trp
   1 5 10 15
   Leu Pro Gly Ala Arg Cys Ala Tyr Asp Met Thr Gln Thr Pro Ala Ser
   20 25 30
   Val Ser Ala Ala Val Gly Gly Thr Val Thr Ile Lys Cys Gln Ala Ser
   35 40 45
   Gln Ser Ile Asn Asn Glu Leu Ser Trp Tyr Gln Gln Lys Pro Gly Gln
   50 55 60
   Arg Pro Lys Leu Leu Ile Tyr Arg Ala Ser Thr Leu Ala Ser Gly Val
   65 70 75 80
   Ser Ser Arg Phe Lys Gly Ser Gly Ser Gly Thr Glu Phe Thr Leu Thr
   85 90 95
   Ile Ser Asp Leu Glu Cys Ala Asp Ala Ala Thr Tyr Tyr Cys Gln Gln
   100 105 110
   Gly Tyr Ser Leu Arg Asn Ile Asp Asn Ala Phe Gly Gly Gly Thr Glu
   115 120 125
   Val Val Val Lys Arg Thr Val Ala Ala Pro Ser Val Phe Ile Phe Pro
   130 135 140
   Pro Ser Asp Glu Gln Leu Lys Ser Gly Thr Ala Ser Val Val Cys Leu
   145 150 155 160
   Leu Asn Asn
<210> 3
   <211> 166
   <212> PRT
   <213> oryctolagus cuniculus
<400> 3
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Asn Tyr Tyr Val Thr Trp Val Arg Gln Ala Pro Gly Lys Gly Leu Glu
   50 55 60
   Trp Ile Gly Ile Ile Tyr Gly Ser Asp Glu Thr Ala Tyr Ala Thr Trp
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu Trp Gly Gln
   115 120 125
   Gly Thr Leu Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys
   165
<210> 4
   <211> 11
   <212> PRT
   <213> Oryctolagus cuniculus
<400> 4
   Gln Ala Ser Gln Ser Ile Asn Asn Glu Leu Ser
   1 5 10
<210> 5
   <211> 7
   <212> PRT
   <213> Oryctolagus cuniculus
<400> 5
   Arg Ala Ser Thr Leu Ala Ser
   1               5
<210> 6
   <211> 12
   <212> PRT
   <213> Oryctolagus cuniculus
<400> 6
   Gln Gln Gly Tyr Ser Leu Arg Asn Ile Asp Asn Ala
   1               5                   10
<210> 7
   <211> 5
   <212> PRT
   <213> Oryctolagus cuniculus
<400> 7
   Asn Tyr Tyr Val Thr
   1               5
<210> 8
   <211> 16
   <212> PRT
   <213> Oryctolagus cuniculus
<400> 8
   Ile Ile Tyr Gly Ser Asp Glu Thr Ala Tyr Ala Thr Trp Ala Ile Gly
   1               5                   10                  15
<210> 9
   <211> 12
   <212> PRT
   <213> Oryctolagus cuniculus
<400> 9
   Asp Asp Ser Ser Asp Trp Asp Ala Lys Phe Asn Leu
   1               5                   10
<210> 10
   <211> 491
   <212> DNA
   <213> Oryctolagus cuniculus
<400> 10
   atggacacga gggcccccac tcagctgctg gggctcctgc tgctctggct cccaggtgcc    60
   agatgtgcct atgatatgac ccagactcca gcctcggtgt ctgcagctgt gggaggcaca   120
   gtcaccatca agtgccaggc cagtcagagc attaacaatg aattatcctg gtatcagcag   180
   aaaccagggc agcgtcccaa gctcctgatc tatagggcat ccactctggc atctggggtc   240
   tcatcgcggt tcaaaggcag tggatctggg acagagttca ctctcaccat cagcgacctg   300
   gagtgtgccg atgctgccac ttactactgt caacagggtt atagtctgag gaatattgat   360
   aatgctttcg gcggagggac cgaggtggtg gtcaaacgta cggtagcggc cccatctgtc   420
   ttcatcttcc cgccatctga tgagcagttg aaatctggaa ctgcctctgt tgtgtgcctg   480
   ctgaataact t                                                        491
<210> 11
   <211> 499
   <212> DNA
   <213> Oryctolagus cuniculus
<400> 11
   atggagactg ggctgcgctg gcttctcctg gtcgctgtgc tcaaaggtgt ccagtgtcag    60
   tcgctggagg agtccggggg tcgcctggtc acgcctggga cacccctgac actcacctgc   120
   acagcctctg gattctccct cagtaactac tacgtgacct gggtccgcca ggctccaggg   180
   aaggggctgg aatggatcgg aatcatttat ggtagtgatg aaacggccta cgcgacctgg   240
   gcgataggcc gattcaccat ctccaaaacc tcgaccacgg tggatctgaa aatgaccagt   300
   ctgacagccg cggacacggc cacctatttc tgtgccagag atgatagtag tgactgggat   360
   gcaaaattta acttgtgggg ccaaggcacc ctggtcaccg tctcgagcgc ctccaccaag   420
   ggcccatcgg tcttccccct ggcaccctcc tccaagagca cctctggggg cacagcggcc   480
   ctgggctgcc tggtcaagg                                                499
<210> 12
   <211> 33
   <212> DNA
   <213> Oryctolagus cuniculus
<400> 12
   caggccagtc agagcattaa caatgaatta tcc 33
<210> 13
   <211> 21
   <212> DNA
   <213> Oryctolagus cuniculus
<400> 13
   agggcatcca ctctggcatc t 21
<210> 14
   <211> 36
   <212> DNA
   <213> Oryctolagus cuniculus
<400> 14
   caacagggtt atagtctgag gaatattgat aatgct 36
<210> 15
   <211> 15
   <212> DNA
   <213> Oryctolagus cuniculus
<400> 15
   aactactacg tgacc 15
<210> 16
   <211> 48
   <212> DNA
   <213> oryctolagus cuniculus
<400> 16
   atcatttatg gtagtgatga aacggcctac gcgacctggg cgataggc 48
<210> 17
   <211> 36
   <212> DNA
   <213> Oryctolagus cuniculus
<400> 17
   gatgatagta gtgactggga tgcaaaattt aacttg 36
<210> 18
   <211> 109
   <212> PRT
   <213> Oryctolagus cuniculus
<400> 18
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Trp Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 19
   <211> 109
   <212> PRT
   <213> Oryctolagus cuniculus
<400> 19
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 20
   <211> 99
   <212> PRT
   <213> Oryctolagus cuniculus
<400> 20
   Ile Gln Met Thr Gln Ser Pro Ser Ser Leu Ser Ala Ser Val Gly Asp
   1               5                   10                  15
   Arg Val Thr Ile Thr Cys Gln Ala Ser Gln Ser Ile Asn Asn Glu Leu
               20                  25                  30
   Ser Trp Tyr Gln Gln Lys Pro Gly Lys Ala Pro Lys Leu Leu Ile Tyr
           35                  40                  45
   Arg Ala Ser Thr Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Asp
   65                  70                  75                  80
   Asp Phe Ala Thr Tyr Tyr Cys Gln Gln Gly Tyr Ser Leu Arg Asn Ile
                   85                  90                  95
   Asp Asn Ala
<210> 21
   <211> 170
   <212> PRT
   <213> Oryctolagus cuniculus
<400> 21
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Asn Cys Gln Ala Ser
           35                  40                  45
   Glu Thr Ile Tyr Ser Trp Leu Ser Trp Tyr Gln Gln Lys Pro Gly Gln
       50                  55                  60
   Pro Pro Lys Leu Leu Ile Tyr Gln Ala Ser Asp Leu Ala Ser Gly Val
   65                  70                  75                  80
   Pro Ser Arg Phe Ser Gly Ser Gly Ala Gly Thr Glu Tyr Thr Leu Thr
                   85                  90                  95
   Ile Ser Gly Val Gln Cys Asp Asp Ala Ala Thr Tyr Tyr Cys Gln Gln
               100                 105                 110
   Gly Tyr Ser Gly Ser Asn Val Asp Asn Val Phe Gly Gly Gly Thr Glu
           115                 120                 125
   Val Val Val Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys
                   165                 170
<210> 22
   <211> 167
   <212> PRT
   <213> Oryctolagus cuniculus
<400> 22
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Arg Leu Val Thr
   20 25 30
   Pro Gly Thr Pro Leu Thr Leu Thr Cys Thr Ala Ser Gly Phe Ser Leu
   35 40 45
   Asn Asp His Ala Met Gly Trp Val Arg Gln Ala Pro Gly Lys Gly Leu
   50 55 60
   Glu Tyr Ile Gly Phe Ile Asn Ser Gly Gly Ser Ala Arg Tyr Ala Ser
   65 70 75 80
   Trp Ala Glu Gly Arg Phe Thr Ile Ser Arg Thr Ser Thr Thr Val Asp
   85 90 95
   Leu Lys Met Thr Ser Leu Thr Thr Glu Asp Thr Ala Thr Tyr Phe Cys
   100 105 110
   Val Arg Gly Gly Ala Val Trp Ser Ile His Ser Phe Asp Pro Trp Gly
   115 120 125
   Pro Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys
   165
<210> 23
   <211> 11
   <212> PRT
   <213> Oryctolagus cuniculus
<400> 23
   Gln Ala Ser Glu Thr Ile Tyr Ser Trp Leu Ser
   1 5 10
<210> 24
   <211> 7
   <212> PRT
   <213> Oryctolagus cuniculus
<400> 24
   Gln Ala Ser Asp Leu Ala Ser
   1 5
<210> 25
   <211> 12
   <212> PRT
   <213> Oryctolagus cuniculus
<400> 25
   Gln Gln Gly Tyr Ser Gly Ser Asn Val Asp Asn Val
   1               5                   10
<210> 26
   <211> 5
   <212> PRT
   <213> Oryctolagus cuniculus
<400> 26
   Asp His Ala Met Gly
   1               5
<210> 27
   <211> 16
   <212> PRT
   <213> Oryctolagus cuniculus
<400> 27
   Phe Ile Asn Ser Gly Gly Ser Ala Arg Tyr Ala Ser Trp Ala Glu Gly
   1               5                   10                  15
<210> 28
   <211> 12
   <212> PRT
   <213> Oryctolagus cuniculus
<400> 28
   Gly Gly Ala Val Trp Ser Ile His Ser Phe Asp Pro
   1               5                   10
<210> 29
   <211> 511
   <212> DNA
   <213> Oryctolagus cuniculus
<400> 29
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca attgccaggc cagtgagacc atttacagtt ggttatcctg gtatcagcag 180
   aagccagggc agcctcccaa gctcctgatc taccaggcat ccgatctggc atctggggtc 240
   ccatcgcgat tcagcggcag tggggctggg acagagtaca ctctcaccat cagcggcgtg 300
   cagtgtgacg atgctgccac ttactactgt caacagggtt atagtggtag taatgttgat 360
   aatgttttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tctatcccag agaggccaaa g 511
<210> 30
   <211> 501
   <212> DNA
   <213> Oryctolagus cuniculus
<400> 30
   atggagactg ggctgcgctg gcttctcctg gtcgctgtgc tcaaaggtgt ccagtgtcag 60
   gagcagctga aggagtccgg gggtcgcctg gtcacgcctg ggacacccct gacacttacc 120
   tgcacagcct ctggattctc cctcaatgac catgcaatgg gctgggtccg ccaggctcca 180
   gggaaggggc tggaatacat cggattcatt aatagtggtg gtagcgcacg ctacgcgagc 240
   tgggcagaag gccgattcac catctccaga acctcgacca cggtggatct gaaaatgacc 300
   agtctgacaa ccgaggacac ggccacctat ttctgtgtca gagggggtgc tgtttggagt 360
   attcatagtt ttgatccctg gggcccaggg accctggtca ccgtctcgag cgcctccacc 420
   aagggcccat cggtcttccc cctggcaccc tcctccaaga gcacctctgg gggcacagcg 480
   gccctgggct gcctggtcaa g 501
<210> 31
   <211> 33
   <212> DNA
   <213> oryctolagus cuniculus
<400> 31
   caggccagtg agaccattta cagttggtta tcc 33
<210> 32
   <211> 21
   <212> DNA
   <213> Oryctolagus cuniculus
<400> 32
   caggcatccg atctggcatc t 21
<210> 33
   <211> 36
   <212> DNA
   <213> Oryctolagus cuniculus
<400> 33
   caacagggtt atagtggtag taatgttgat aatgtt 36
<210> 34
   <211> 15
   <212> DNA
   <213> Oryctolagus cuniculus
<400> 34
   gaccatgcaa tgggc 15
<210> 35
   <211> 48
   <212> DNA
   <213> Oryctolagus cuniculus
<400> 35
   ttcattaata gtggtggtag cgcacgctac gcgagctggg cagaaggc 48
<210> 36
   <211> 36
   <212> DNA
   <213> Oryctolagus cuniculus
<400> 36
   gggggtgctg tttggagtat tcatagtttt gatccc 36
<210> 37
   <211> 165
   <212> PRT
   <213> Oryctolagus cuniculus
<400> 37
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Pro Ser Pro
   20 25 30
   Val Ser Ala Ala Val Gly Gly Thr Val Ser Ile Ser Cys Gln Ala Ser
   35 40 45
   Gln Ser Val Tyr Asp Asn Asn Tyr Leu Ser Trp Phe Gln Gln Lys Pro
   50 55 60
   Gly Gln Pro Pro Lys Leu Leu Ile Tyr Gly Ala Ser Thr Leu Ala Ser
   65 70 75 80
   Gly Val Pro Ser Arg Phe Val Gly Ser Gly Ser Gly Thr Gln Phe Thr
   85 90 95
   Leu Thr Ile Thr Asp Val Gln Cys Asp Asp Ala Ala Thr Tyr Tyr Cys
   100 105 110
   Ala Gly Val Tyr Asp Asp Asp Ser Asp Asn Ala Phe Gly Gly Gly Thr
   115 120 125
   Glu Val Val Val Lys Arg Thr Val Ala Ala Pro Ser Val Phe Ile Phe
   130 135 140
   Pro Pro Ser Asp Glu Gln Leu Lys Ser Gly Thr Ala Ser Val Val Cys
   145 150 155 160
   Leu Leu Asn Asn Phe
   165
<210> 38
   <211> 166
   <212> PRT
   <213> Oryctolagus cuniculus
<400> 38
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Val Tyr Tyr Met Asn Trp Val Arg Gln Ala Pro Gly Lys Gly Leu Glu
   50 55 60
   Trp Ile Gly Phe Ile Thr Met Ser Asp Asn Ile Asn Tyr Ala Ser Trp
   65 70 75 80
   Ala Lys Gly Arg Phe Thr Ile Ser Lys Thr Ser Thr Thr Val Asp Leu
   85 90 95
   Lys Met Thr Ser Pro Thr Thr Glu Asp Thr Ala Thr Tyr Phe Cys Ala
   100 105 110
   Arg Ser Arg Gly Trp Gly Thr Met Gly Arg Leu Asp Leu Trp Gly Pro
   115 120 125
   Gly Thr Leu Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys
   165
<210> 39
   <211> 13
   <212> PRT
   <213> oryctolagus cuniculus
<400> 39
   Gln Ala Ser Gln Ser Val Tyr Asp Asn Asn Tyr Leu Ser
   1 5 10
<210> 40
   <211> 7
   <212> PRT
   <213> Oryctolagus cuniculus
<400> 40
   Gly Ala Ser Thr Leu Ala Ser
   1 5
<210> 41
   <211> 11
   <212> PRT
   <213> Oryctolagus cuniculus
<400> 41
   Ala Gly Val Tyr Asp Asp Asp Ser Asp Asn Ala
   1 5 10
<210> 42
   <211> 5
   <212> PRT
   <213> Oryctolagus cuniculus
<400> 42
   Val Tyr Tyr Met Asn
   1 5
<210> 43
   <211> 16
   <212> PRT
   <213> Oryctolagus cuniculus
<400> 43
   Phe Ile Thr Met Ser Asp Asn Ile Asn Tyr Ala Ser Trp Ala Lys Gly
   1 5 10 15
<210> 44
   <211> 12
   <212> PRT
   <213> Oryctolagus cuniculus
<400> 44
   Ser Arg Gly Trp Gly Thr Met Gly Arg Leu Asp Leu
   1 5 10
<210> 45
   <211> 496
   <212> DNA
   <213> oryctolagus cuniculus
<400> 45
   atggacacga gggcccccac tcagctgctg gggctcctgc tgctctggct cccaggtgcc 60
   acatttgccg ccgtgctgac ccagactcca tctcccgtgt ctgcagctgt gggaggcaca 120
   gtcagcatca gttgccaggc cagtcagagt gtttatgaca acaactactt atcctggttt 180
   cagcagaaac cagggcagcc tcccaagctc ctgatctatg gtgcatccac tctggcatct 240
   ggggtcccat cgcggttcgt gggcagtgga tctgggacac agttcactct caccatcaca 300
   gacgtgcagt gtgacgatgc tgccacttac tattgtgcag gcgtttatga tgatgatagt 360
   gataatgcct tcggcggagg gaccgaggtg gtggtcaaac gtacggtagc ggccccatct 420
   gtcttcatct tcccgccatc tgatgagcag ttgaaatctg gaactgcctc tgttgtgtgc 480
   ctgctgaata acttct 496
<210> 46
   <211> 499
   <212> DNA
   <213> Oryctolagus cuniculus
<400> 46
   atggagactg ggctgcgctg gcttctcctg gtggctgtgc tcaaaggtgt ccagtgtcag 60
   tcgctggagg agtccggggg tcgcctggtc acccctggga cacccctgac actcacctgc 120
   acagcctctg gattctccct cagtgtctac tacatgaact gggtccgcca ggctccaggg 180
   aaggggctgg aatggatcgg attcattaca atgagtgata atataaatta cgcgagctgg 240
   gcgaaaggcc gattcaccat ctccaaaacc tcgaccacgg tggatctgaa aatgaccagt 300
   ccgacaaccg aggacacggc cacctatttc tgtgccagga gtcgtggctg gggtacaatg 360
   ggtcggttgg atctctgggg cccaggcacc ctcgtcaccg tctcgagcgc ctccaccaag 420
   ggcccatcgg tcttccccct ggcaccctcc tccaagagca cctctggggg cacagcggcc 480
   ctgggctgcc tggtcaagg 499
<210> 47
   <211> 39
   <212> DNA
   <213> Oryctolagus cuniculus
<400> 47
   caggccagtc agagtgttta tgacaacaac tacttatcc 39
<210> 48
   <211> 21
   <212> DNA
   <213> Oryctolagus cuniculus
<400> 48
   ggtgcatcca ctctggcatc t 21
<210> 49
   <211> 33
   <212> DNA
   <213> Oryctolagus cuniculus
<400> 49
   gcaggcgttt atgatgatga tagtgataat gcc 33
<210> 50
   <211> 15
   <212> DNA
   <213> Oryctolagus cuniculus
<400> 50
   gtctactaca tgaac 15
<210> 51
   <211> 48
   <212> DNA
   <213> Oryctolagus cuniculus
<400> 51
   ttcattacaa tgagtgataa tataaattac gcgagctggg cgaaaggc 48
<210> 52
   <211> 36
   <212> DNA
   <213> Oryctolagus cuniculus
<400> 52
   agtcgtggct ggggtacaat gggtcggttg gatctc 36
<210> 53
   <211> 164
   <212> PRT
   <213> Oryctolagus cuniculus
<400> 53
   Met Asp Thr Arg Ala Pro Thr Gln Leu Leu Gly Leu Leu Leu Leu Trp
   1               5                   10                  15
   Leu Pro Gly Ala Ile Cys Asp Pro Val Leu Thr Gln Thr Pro Ser Pro
               20                  25                  30
   Val Ser Ala Pro Val Gly Gly Thr Val Ser Ile Ser Cys Gln Ala Ser
           35                  40                  45
   Gln Ser Val Tyr Glu Asn Asn Tyr Leu Ser Trp Phe Gln Gln Lys Pro
       50                  55                  60
   Gly Gln Pro Pro Lys Leu Leu Ile Tyr Gly Ala Ser Thr Leu Asp Ser
   65                  70                  75                  80
   Gly Val Pro Ser Arg Phe Lys Gly Ser Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Tyr Asp Asp Asp Ser Asp Asp Ala Phe Gly Gly Gly Thr
           115                 120                 125
   Glu Val Val Val Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn
<210> 54
   <211> 167
   <212> PRT
   <213> Oryctolagus cuniculus
<400> 54
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Gly Leu Val Thr
               20                  25                  30
   Pro Gly Gly Thr Leu Thr Leu Thr Cys Thr Ala Ser Gly Phe Ser Leu
           35                  40                  45
   Asn Ala Tyr Tyr Met Asn Trp Val Arg Gln Ala Pro Gly Lys Gly Leu
       50                  55                  60
   Glu Trp Ile Gly Phe Ile Thr Leu Asn Asn Asn Val Ala Tyr Ala Asn
   65                  70                  75                  80
   Trp Ala Lys Gly Arg Phe Thr Phe Ser Lys Thr Ser Thr Thr Val Asp
                   85                  90                  95
   Leu Lys Met Thr Ser Pro Thr Pro Glu Asp Thr Ala Thr Tyr Phe Cys
               100                 105                 110
   Ala Arg Ser Arg Gly Trp Gly Ala Met Gly Arg Leu Asp Leu Trp Gly
           115                 120                 125
   His Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys
                   165
<210> 55
   <211> 13
   <212> PRT
   <213> Oryctolagus cuniculus
<400> 55
   Gln Ala Ser Gln Ser Val Tyr Glu Asn Asn Tyr Leu Ser
   1               5                   10
<210> 56
   <211> 7
   <212> PRT
   <213> Oryctolagus cuniculus
<400> 56
   Gly Ala Ser Thr Leu Asp Ser
   1 5
<210> 57
   <211> 11
   <212> PRT
   <213> Oryctolagus cuniculus
<400> 57
   Ala Gly Val Tyr Asp Asp Asp Ser Asp Asp Ala
   1 5 10
<210> 58
   <211> 5
   <212> PRT
   <213> Oryctolagus cuniculus
<400> 58
   Ala Tyr Tyr Met Asn
   1 5
<210> 59
   <211> 16
   <212> PRT
   <213> Oryctolagus cuniculus
<400> 59
   Phe Ile Thr Leu Asn Asn Asn Val Ala Tyr Ala Asn Trp Ala Lys Gly
   1 5 10 15
<210> 60
   <211> 12
   <212> PRT
   <213> Oryctolagus cuniculus
<400> 60
   Ser Arg Gly Trp Gly Ala Met Gly Arg Leu Asp Leu
   1 5 10
<210> 61
   <211> 494
   <212> DNA
   <213> Oryctolagus cuniculus
<400> 61
   atggacacga gggcccccac tcagctgctg gggctcctgc tgctctggct cccaggtgcc 60
   atatgtgacc ctgtgctgac ccagactcca tctcccgtat ctgcacctgt gggaggcaca 120
   gtcagcatca gttgccaggc cagtcagagt gtttatgaga acaactattt atcctggttt 180
   cagcagaaac cagggcagcc tcccaagctc ctgatctatg gtgcatccac tctggattct 240
   ggggtcccat cgcggttcaa aggcagtgga tctgggacac agttcactct caccattaca 300
   gacgtgcagt gtgacgatgc tgccacttac tattgtgcag gcgtttatga tgatgatagt 360
   gatgatgcct tcggcggagg gaccgaggtg gtggtcaaac gtacggtagc ggccccatct 420
   gtcttcatct tcccgccatc tgatgagcag ttgaaatctg gaactgcctc tgttgtgtgc 480
   ctgctgaata actt 494
<210> 62
   <211> 502
   <212> DNA
   <213> Oryctolagus cuniculus
<400> 62
   atggagactg ggctgcgctg gcttctcctg gtggctgtgc tcaaaggtgt ccagtgtcag 60
   gagcagctga aggagtccgg aggaggcctg gtaacgcctg gaggaaccct gacactcacc 120
   tgcacagcct ctggattctc cctcaatgcc tactacatga actgggtccg ccaggctcca 180
   gggaaggggc tggaatggat cggattcatt actctgaata ataatgtagc ttacgcgaac 240
   tgggcgaaag gccgattcac cttctccaaa acctcgacca cggtggatct gaaaatgacc 300
   agtccgacac ccgaggacac ggccacctat ttctgtgcca ggagtcgtgg ctggggtgca 360
   atgggtcggt tggatctctg gggccatggc accctggtca ccgtctcgag cgcctccacc 420
   aagggcccat cggtcttccc cctggcaccc tcctccaaga gcacctctgg gggcacagcg 480
   gccctgggct gcctggtcaa gg 502
<210> 63
   <211> 39
   <212> DNA
   <213> Oryctolagus cuniculus
<400> 63
   caggccagtc agagtgttta tgagaacaac tatttatcc 39
<210> 64
   <211> 21
   <212> DNA
   <213> Oryctolagus cuniculus
<400> 64
   ggtgcatcca ctctggattc t 21
<210> 65
   <211> 33
   <212> DNA
   <213> Oryctolagus cuniculus
<400> 65
   gcaggcgttt atgatgatga tagtgatgat gcc 33
<210> 66
   <211> 15
   <212> DNA
   <213> Oryctolagus cuniculus
<400> 66
   gcctactaca tgaac 15
<210> 67
   <211> 48
   <212> DNA
   <213> Oryctolagus cuniculus
<400> 67
   ttcattactc tgaataataa tgtagcttac gcgaactggg cgaaaggc 48
<210> 68
   <211> 36
   <212> DNA
   <213> Oryctolagus cuniculus
<400> 68
   agtcgtggct ggggtgcaat gggtcggttg gatctc 36
<210> 69
   <211> 164
   <212> PRT
   <213> Oryctolagus cuniculus
<400> 69
   Met Asp Thr Arg Ala Pro Thr Gln Leu Leu Gly Leu Leu Leu Leu Trp
   1 5 10 15
   Leu Pro Gly Ala Thr Phe Ala Gln Val Leu Thr Gln Thr Pro Ser Pro
   20 25 30
   Val Ser Ala Ala Val Gly Gly Thr Val Thr Ile Asn Cys Gln Ala Ser
   35 40 45
   Gln Ser Val Asp Asp Asn Asn Trp Leu Gly Trp Tyr Gln Gln Lys Arg
   50 55 60
   Gly Gln Pro Pro Lys Tyr Leu Ile Tyr Ser Ala Ser Thr Leu Ala Ser
   65 70 75 80
   Gly Val Pro Ser Arg Phe Lys Gly Ser Gly Ser Gly Thr Gln Phe Thr
   85 90 95
   Leu Thr Ile Ser Asp Leu Glu Cys Asp Asp Ala Ala Thr Tyr Tyr Cys
   100 105 110
   Ala Gly Gly Phe Ser Gly Asn Ile Phe Ala Phe Gly Gly Gly Thr Glu
   115 120 125
   Val Val Val Lys Arg Thr Val Ala Ala Pro Ser Val Phe Ile Phe Pro
   130 135 140
   Pro Ser Asp Glu Gln Leu Lys Ser Gly Thr Ala Ser Val Val Cys Leu
   145 150 155 160
   Leu Asn Asn Phe
<210> 70
   <211> 164
   <212> PRT
   <213> oryctolagus cuniculus
<400> 70
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Ser Tyr Ala Met Ser Trp Val Arg Gln Ala Pro Gly Lys Gly Leu Glu
   50 55 60
   Trp Ile Gly Ile Ile Gly Gly Phe Gly Thr Thr Tyr Tyr Ala Thr Trp
   65 70 75 80
   Ala Lys Gly Arg Phe Thr Ile Ser Lys Thr Ser Thr Thr Val Asp Leu
   85 90 95
   Arg Ile Thr Ser Pro Thr Thr Glu Asp Thr Ala Thr Tyr Phe Cys Ala
   100 105 110
   Arg Gly Gly Pro Gly Asn Gly Gly Asp Ile Trp Gly Gln Gly Thr Leu
   115 120 125
   Val Thr Val Ser Ser Ala Ser Thr Lys Gly Pro Ser Val Phe Pro Leu
   130 135 140
   Ala Pro Ser Ser Lys Ser Thr Ser Gly Gly Thr Ala Ala Leu Gly Cys
   145 150 155 160
   Leu Val Lys Asp
<210> 71
   <211> 13
   <212> PRT
   <213> Oryctolagus cuniculus
<400> 71
   Gln Ala Ser Gln Ser Val Asp Asp Asn Asn Trp Leu Gly
   1 5 10
<210> 72
   <211> 7
   <212> PRT
   <213> oryctolagus cuniculus
<400> 72
   Ser Ala Ser Thr Leu Ala Ser
   1 5
<210> 73
   <211> 10
   <212> PRT
   <213> Oryctolagus cuniculus
<400> 73
   Ala Gly Gly Phe Ser Gly Asn Ile Phe Ala
   1 5 10
<210> 74
   <211> 5
   <212> PRT
   <213> Oryctolagus cuniculus
<400> 74
   Ser Tyr Ala Met Ser
   1 5
<210> 75
   <211> 16
   <212> PRT
   <213> Oryctolagus cuniculus
<400> 75
   Ile Ile Gly Gly Phe Gly Thr Thr Tyr Tyr Ala Thr Trp Ala Lys Gly
   1 5 10 15
<210> 76
   <211> 9
   <212> PRT
   <213> Oryctolagus cuniculus
<400> 76
   Gly Gly Pro Gly Asn Gly Gly Asp Ile
   1 5
<210> 77
   <211> 493
   <212> DNA
   <213> oryctolagus cuniculus
<400> 77
   atggacacga gggcccccac tcagctgctg gggctcctgc tgctctggct cccaggtgcc 60
   acatttgccc aagtgctgac ccagactcca tcgcctgtgt ctgcagctgt gggaggcaca 120
   gtcaccatca actgccaggc cagtcagagt gttgatgata acaactggtt aggctggtat 180
   cagcagaaac gagggcagcc tcccaagtac ctgatctatt ctgcatccac tctggcatct 240
   ggggtcccat cgcggttcaa aggcagtgga tctgggacac agttcactct caccatcagc 300
   gacctggagt gtgacgatgc tgccacttac tactgtgcag gcggttttag tggtaatatc 360
   tttgctttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tct 493
<210> 78
   <211> 493
   <212> DNA
   <213> oryctolagus cuniculus
<400> 78
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtctctg gcttctccct cagtagctat gcaatgagct gggtccgcca ggctccagga 180
   aaggggctgg agtggatcgg aatcattggt ggttttggta ccacatacta cgcgacctgg 240
   gcgaaaggcc gattcaccat ctccaaaacc tcgaccacgg tggatctgag aatcaccagt 300
   ccgacaaccg aggacacggc cacctatttc tgtgccagag gtggtcctgg taatggtggt 360
   gacatctggg gccaagggac cctggtcacc gtctcgagcg cctccaccaa gggcccatcg 420
   gtcttccccc tggcaccctc ctccaagagc acctctgggg gcacagcggc cctgggctgc 480
   ctggtcaagg act 493
<210> 79
   <211> 39
   <212> DNA
   <213> Oryctolagus cuniculus
<400> 79
   caggccagtc agagtgttga tgataacaac tggttaggc 39
<210> 80
   <211> 21
   <212> DNA
   <213> Oryctolagus cuniculus
<400> 80
   tctgcatcca ctctggcatc t 21
<210> 81
   <211> 30
   <212> DNA
   <213> Oryctolagus cuniculus
<400> 81
   gcaggcggtt ttagtggtaa tatctttgct 30
<210> 82
   <211> 15
   <212> DNA
   <213> Oryctolagus cuniculus
<400> 82
   agctatgcaa tgagc 15
<210> 83
   <211> 48
   <212> DNA
   <213> Oryctolagus cuniculus
<400> 83
   atcattggtg gttttggtac cacatactac gcgacctggg cgaaaggc 48
<210> 84
   <211> 27
   <212> DNA
   <213> Oryctolagus cuniculus
<400> 84
   ggtggtcctg gtaatggtgg tgacatc 27
<210> 85
   <211> 164
   <212> PRT
   <213> Oryctolagus cuniculus
<400> 85
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Pro Ser Pro
   20 25 30
   Val Ser Val Pro Val Gly Gly Thr Val Thr Ile Lys Cys Gln Ser Ser
   35 40 45
   Gln Ser Val Tyr Asn Asn Phe Leu Ser Trp Tyr Gln Gln Lys Pro Gly
   50 55 60
   Gln Pro Pro Lys Leu Leu Ile Tyr Gln Ala Ser Lys Leu Ala Ser Gly
   65 70 75 80
   Val Pro Asp Arg Phe Ser Gly Ser Gly Ser Gly Thr Gln Phe Thr Leu
   85 90 95
   Thr Ile Ser Gly Val Gln Cys Asp Asp Ala Ala Thr Tyr Tyr Cys Leu
   100 105 110
   Gly Gly Tyr Asp Asp Asp Ala Asp Asn Ala Phe Gly Gly Gly Thr Glu
   115 120 125
   Val Val Val Lys Arg Thr Val Ala Ala Pro Ser Val Phe Ile Phe Pro
   130 135 140
   Pro Ser Asp Glu Gln Leu Lys Ser Gly Thr Ala Ser Val Val Cys Leu
   145 150 155 160
   Leu Asn Asn Phe
<210> 86
   <211> 170
   <212> PRT
   <213> Oryctolagus cuniculus
<400> 86
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Ile Asp Leu Ser
   35 40 45
   Asp Tyr Ala Met Ser Trp Val Arg Gln Ala Pro Gly Lys Gly Leu Glu
   50 55 60
   Trp Ile Gly Ile Ile Tyr Ala Gly Ser Gly Ser Thr Trp Tyr Ala Ser
   65 70 75 80
   Trp Ala Lys Gly Arg Phe Thr Ile Ser Lys Thr Ser Thr Thr Val Asp
   85 90 95
   Leu Lys Ile Thr Ser Pro Thr Thr Glu Asp Thr Ala Thr Tyr Phe Cys
   100 105 110
   Ala Arg Asp Gly Tyr Asp Asp Tyr Gly Asp Phe Asp Arg Leu Asp Leu
   115 120 125
   Trp Gly Pro Gly Thr Leu Val Thr Val Ser Ser Ala Ser Thr Lys Gly
   130 135 140
   Pro Ser Val Phe Pro Leu Ala Pro Ser Ser Lys Ser Thr Ser Gly Gly
   145 150 155 160
   Thr Ala Ala Leu Gly Cys Leu Val Lys Asp
   165 170
<210> 87
   <211> 12
   <212> PRT
   <213> Oryctolagus cuniculus
<400> 87
   Gln Ser Ser Gln Ser Val Tyr Asn Asn Phe Leu Ser
   1 5 10
<210> 88
   <211> 7
   <212> PRT
   <213> oryctolagus cuniculus
<400> 88
   Gln Ala Ser Lys Leu Ala Ser
   1 5
<210> 89
   <211> 11
   <212> PRT
   <213> Oryctolagus cuniculus
<400> 89
   Leu Gly Gly Tyr Asp Asp Asp Ala Asp Asn Ala
   1 5 10
<210> 90
   <211> 5
   <212> PRT
   <213> Oryctolagus cuniculus
<400> 90
   Asp Tyr Ala Met Ser
   1 5
<210> 91
   <211> 17
   <212> PRT
   <213> Oryctolagus cuniculus
<400> 91
   Ile Ile Tyr Ala Gly Ser Gly Ser Thr Trp Tyr Ala Ser Trp Ala Lys
   1 5 10 15
   Gly
<210> 92
   <211> 14
   <212> PRT
   <213> Oryctolagus cuniculus
<400> 92
   Asp Gly Tyr Asp Asp Tyr Gly Asp Phe Asp Arg Leu Asp Leu
   1 5 10
<210> 93
   <211> 492
   <212> DNA
   <213> Oryctolagus cuniculus
<400> 93
   atggacacga gggcccccac tcagctgctg gggctcctgc tgctctggct cccaggtgcc 60
   acatttgcag ccgtgctgac ccagacacca tcgcccgtgt ctgtacctgt gggaggcaca 120
   gtcaccatca agtgccagtc cagtcagagt gtttataata atttcttatc gtggtatcag 180
   cagaaaccag ggcagcctcc caagctcctg atctaccagg catccaaact ggcatctggg 240
   gtcccagata ggttcagcgg cagtggatct gggacacagt tcactctcac catcagcggc 300
   gtgcagtgtg acgatgctgc cacttactac tgtctaggcg gttatgatga tgatgctgat 360
   aatgctttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tc 492
<210> 94
   <211> 511
   <212> DNA
   <213> Oryctolagus cuniculus
<400> 94
   atggagactg ggctgcgctg gcttctcctg gtcgctgtgc tcaaaggtgt ccagtgtcag 60
   tcggtggagg agtccggggg tcgcctggtc acgcctggga cacccctgac gctcacctgc 120
   acagtctctg gaatcgacct cagtgactat gcaatgagct gggtccgcca ggctccaggg 180
   aaggggctgg aatggatcgg aatcatttat gctggtagtg gtagcacatg gtacgcgagc 240
   tgggcgaaag gccgattcac catctccaaa acctcgacca cggtggatct gaaaatcacc 300
   agtccgacaa ccgaggacac ggccacctat ttctgtgcca gagatggata cgatgactat 360
   ggtgatttcg atcgattgga tctctggggc ccaggcaccc tcgtcaccgt ctcgagcgcc 420
   tccaccaagg gcccatcggt cttccccctg gcaccctcct ccaagagcac ctctgggggc 480
   acagcggccc tgggctgcct ggtcaaggac t 511
<210> 95
   <211> 36
   <212> DNA
   <213> Oryctolagus cuniculus
<400> 95
   cagtccagtc agagtgttta taataatttc ttatcg 36
<210> 96
   <211> 21
   <212> DNA
   <213> Oryctolagus cuniculus
<400> 96
   caggcatcca aactggcatc t 21
<210> 97
   <211> 33
   <212> DNA
   <213> Oryctolagus cuniculus
<400> 97
   ctaggcggtt atgatgatga tgctgataat gct 33
<210> 98
   <211> 15
   <212> DNA
   <213> Oryctolagus cuniculus
<400> 98
   gactatgcaa tgagc 15
<210> 99
   <211> 51
   <212> DNA
   <213> Oryctolagus cuniculus
<400> 99
   atcatttatg ctggtagtgg tagcacatgg tacgcgagct gggcgaaagg c 51
<210> 100
   <211> 42
   <212> DNA
   <213> Oryctolagus cuniculus
<400> 100
   gatggatacg atgactatgg tgatttcgat cgattggatc tc 42
<210> 101
   <211> 164
   <212> PRT
   <213> Oryctolagus cuniculus
<400> 101
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Ser Ala Ala Val Gly Gly Thr Val Thr Ile Lys Cys Gln Ala Ser
           35                  40                  45
   Gln Ser Ile Asn Asn Glu Leu Ser Trp Tyr Gln Gln Lys Ser Gly Gln
       50                  55                  60
   Arg Pro Lys Leu Leu Ile Tyr Arg Ala Ser Thr Leu Ala Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Gln
               100                 105                 110
   Gly Tyr Ser Leu Arg Asn Ile Asp Asn Ala Phe Gly Gly Gly Thr Glu
           115                 120                 125
   Val Val Val Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe
<210> 102
   <211> 166
   <212> PRT
   <213> Oryctolagus cuniculus
<400> 102
   Met Glu Thr Gly Leu Arg Trp Leu Leu Leu Val Ala Val Leu Ser Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Asn Tyr Tyr Met Thr Trp Val Arg Gln Ala Pro Gly Lys Gly Leu Glu
   50 55 60
   Trp Ile Gly Met Ile Tyr Gly Ser Asp Glu Thr Ala Tyr Ala Asn Trp
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu Trp Gly Gln
   115 120 125
   Gly Thr Leu Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys
   165
<210> 103
   <211> 11
   <212> PRT
   <213> Oryctolagus cuniculus
<400> 103
   Gln Ala Ser Gln Ser Ile Asn Asn Glu Leu Ser
   1 5 10
<210> 104
   <211> 7
   <212> PRT
   <213> Oryctolagus cuniculus
<400> 104
   Arg Ala Ser Thr Leu Ala Ser
   1 5
<210> 105
   <211> 12
   <212> PRT
   <213> Oryctolagus cuniculus
<400> 105
   Gln Gln Gly Tyr Ser Leu Arg Asn Ile Asp Asn Ala
   1 5 10
<210> 106
   <211> 5
   <212> PRT
   <213> Oryctolagus cuniculus
<400> 106
   Asn Tyr Tyr Met Thr
   1 5
<210> 107
   <211> 16
   <212> PRT
   <213> Oryctolagus cuniculus
<400> 107
   Met Ile Tyr Gly Ser Asp Glu Thr Ala Tyr Ala Asn Trp Ala Ile Gly
   1 5 10 15
<210> 108
   <211> 12
   <212> PRT
   <213> Oryctolagus cuniculus
<400> 108
   Asp Asp Ser Ser Asp Trp Asp Ala Lys Phe Asn Leu
   1 5 10
<210> 109
   <211> 492
   <212> DNA
   <213> oryctolagus cuniculus
<400> 109
   atggacacga gggcccccac tcagctgctg gggctcctgc tgctctggct cccaggtgcc 60
   agatgtgcct atgatatgac ccagactcca gcctcggtgt ctgcagctgt gggaggcaca 120
   gtcaccatca aatgccaggc cagtcagagc attaacaatg aattatcctg gtatcagcag 180
   aaatcagggc agcgtcccaa gctcctgatc tatagggcat ccactctggc atctggggtc 240
   tcatcgcggt tcaaaggcag tggatctggg acagagttca ctctcaccat cagcgacctg 300
   gagtgtgccg atgctgccac ttactactgt caacagggtt atagtctgag gaatattgat 360
   aatgctttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tc 492
<210> 110
   <211> 499
   <212> DNA
   <213> Oryctolagus cuniculus
<400> 110
   atggagactg ggctgcgctg gcttctcctg gtcgctgtgc tctcaggtgt ccagtgtcag 60
   tcgctggagg agtccggggg tcgcctggtc acgcctggga cacccctgac actcacctgc 120
   acagcctctg gattctccct cagtaactac tacatgacct gggtccgcca ggctccaggg 180
   aaggggctgg aatggatcgg aatgatttat ggtagtgatg aaacagccta cgcgaactgg 240
   gcgataggcc gattcaccat ctccaaaacc tcgaccacgg tggatctgaa aatgaccagt 300
   ctgacagccg cggacacggc cacctatttc tgtgccagag atgatagtag tgactgggat 360
   gcaaaattta acttgtgggg ccaagggacc ctcgtcaccg tctcgagcgc ctccaccaag 420
   ggcccatcgg tcttccccct ggcaccctcc tccaagagca cctctggggg cacagcggcc 480
   ctgggctgcc tggtcaagg 499
<210> 111
   <211> 33
   <212> DNA
   <213> oryctolagus cuniculus
<400> 111
   caggccagtc agagcattaa caatgaatta tcc 33
<210> 112
   <211> 21
   <212> DNA
   <213> Oryctolagus cuniculus
<400> 112
   agggcatcca ctctggcatc t 21
<210> 113
   <211> 36
   <212> DNA
   <213> Oryctolagus cuniculus
<400> 113
   caacagggtt atagtctgag gaatattgat aatgct 36
<210> 114
   <211> 15
   <212> DNA
   <213> Oryctolagus cuniculus
<400> 114
   aactactaca tgacc 15
<210> 115
   <211> 48
   <212> DNA
   <213> Oryctolagus cuniculus
<400> 115
   atgatttatg gtagtgatga aacagcctac gcgaactggg cgataggc 48
<210> 116
   <211> 36
   <212> DNA
   <213> Oryctolagus cuniculus
<400> 116
   gatgatagta gtgactggga tgcaaaattt aacttg 36
<210> 117
   <211> 109
   <212> PRT
   <213> Oryctolagus cuniculus
<400> 117
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Met Thr Trp Val Arg Gln Ala Pro Gly Lys Gly Leu Glu Trp Val
   35 40 45
   Gly Met Ile Tyr Gly Ser Asp Glu Thr Ala Tyr Ala Asn Trp Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 118
   <211> 109
   <212> PRT
   <213> Oryctolagus cuniculus
<400> 118
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Met Thr Trp Val Arg Gln Ala Pro Gly Lys Gly Leu Glu Trp Val
   35 40 45
   Gly Met Ile Tyr Gly Ser Asp Glu Thr Ala Tyr Ala Asn Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 119
   <211> 100
   <212> PRT
   <213> Oryctolagus cuniculus
<400> 119 Ile Asp Asn Ala 100
<210> 120
   <211> 16
   <212> PRT
   <213> oryctolagus cuniculus
<400> 120
   Ile Ile Tyr Gly Ser Asp Glu Thr Ala Tyr Ala Thr Ser Ala Ile Gly
   1 5 10 15
<210> 121
   <211> 16
   <212> PRT
   <213> oryctolagus cuniculus
<400> 121
   Met Ile Tyr Gly Ser Asp Glu Thr Ala Tyr Ala Asn Ser Ala Ile Gly
   1 5 10 15
<210> 122
   <211> 123
   <212> PRT
   <213> oryctolagus cuniculus
<400> 122
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Thr Ile Ser Cys Gln Ser Ser
           35                  40                  45
   Gln Ser Val Gly Asn Asn Gln Asp Leu Ser Trp Phe Gln Gln Arg Pro
       50                  55                  60
   Gly Gln Pro Pro Lys Leu Leu Ile Tyr Glu Ile Ser Lys Leu Glu Ser
   65                  70                  75                  80
   Gly Val Pro Ser Arg Phe Ser Gly Ser Gly Ser Gly Thr His Phe Thr
                   85                  90                  95
   Leu Thr Ile Ser Gly Val Gln Cys Asp Asp Ala Ala Thr Tyr Tyr Cys
               100                 105                 110
   Leu Gly Gly Tyr Asp Asp Asp Ala Asp Asn Ala
           115                 120
<210> 123
   <211> 128
   <212> PRT
   <213> Oryctolagus cuniculus
<400> 123
   Met Glu Thr Gly Leu Arg Trp Leu Leu Leu Val Ala Val Leu Lys Gly
   1               5                   10                  15
   Val Gln Cys His Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Arg Thr Met Ser Trp Val Arg Gln Ala Pro Gly Lys Gly Leu Glu
       50                  55                  60
   Trp Ile Gly Tyr Ile Trp Ser Gly Gly Ser Thr Tyr Tyr Ala Thr Trp
   65                  70                  75                  80
   Ala Lys Gly Arg Phe Thr Ile Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Leu Gly Asp Thr Gly Gly His Ala Tyr Ala Thr Arg Leu Asn Leu
           115                 120                 125
<210> 124
   <211> 13
   <212> PRT
   <213> oryctolagus cuniculus
<400> 124
   Gln Ser Ser Gln Ser Val Gly Asn Asn Gln Asp Leu Ser
   1               5                   10
<210> 125
   <211> 7
   <212> PRT
   <213> Oryctolagus cuniculus
<400> 125
   Glu Ile Ser Lys Leu Glu Ser
   1               5
<210> 126
   <211> 11
   <212> PRT
   <213> Oryctolagus cuniculus
<400> 126
   Leu Gly Gly Tyr Asp Asp Asp Ala Asp Asn Ala
   1               5                   10
<210> 127
   <211> 5
   <212> PRT
   <213> Oryctolagus cuniculus
<400> 127
   Ser Arg Thr Met Ser
   1 5
<210> 128
   <211> 16
   <212> PRT
   <213> Oryctolagus cuniculus
<400> 128
   Tyr Ile Trp Ser Gly Gly Ser Thr Tyr Tyr Ala Thr Trp Ala Lys Gly
   1 5 10 15
<210> 129
   <211> 15
   <212> PRT
   <213> Oryctolagus cuniculus
<400> 129
   Leu Gly Asp Thr Gly Gly His Ala Tyr Ala Thr Arg Leu Asn Leu
   1 5 10 15
<210> 130
   <211> 369
   <212> DNA
   <213> Oryctolagus cuniculus
<400> 130
   atggacacga gggcccccac tcagctgctg gggctcctgc tgctctggct cccaggtgcc 60
   acatttgcag ccgtgctgac ccagacacca tcacccgtgt ctgcagctgt gggaggcaca 120
   gtcaccatca gttgccagtc cagtcagagt gttggtaata accaggactt atcctggttt 180
   cagcagagac cagggcagcc tcccaagctc ctgatctacg aaatatccaa actggaatct 240
   ggggtcccat cgcggttcag cggcagtgga tctgggacac acttcactct caccatcagc 300
   ggcgtacagt gtgacgatgc tgccacttac tactgtctag gcggttatga tgatgatgct 360
   gataatgct 369
<210> 131
   <211> 384
   <212> DNA
   <213> Oryctolagus cuniculus
<400> 131
   atggagactg ggctgcgctg gcttctcctg gtcgctgtgc tcaaaggtgt ccagtgtcac 60
   tcggtggagg agtccggggg tcgcctggtc acgcctggga cacccctgac actcacctgc 120
   acagtctctg gattctccct cagtagtcgt acaatgtcct gggtccgcca ggctccaggg 180
   aaggggctgg agtggatcgg atacatttgg agtggtggta gcacatacta cgcgacctgg 240
   gcgaaaggcc gattcaccat ctccaaaacc tcgaccacgg tggatctgaa aatcaccagt 300
   ccgacaaccg aggacacggc cacctatttc tgtgccagat tgggcgatac tggtggtcac 360
   gcttatgcta ctcgcttaaa tctc 384
<210> 132
   <211> 39
   <212> DNA
   <213> oryctolagus cuniculus
<400> 132
   cagtccagtc agagtgttgg taataaccag gacttatcc 39
<210> 133
   <211> 21
   <212> DNA
   <213> Oryctolagus cuniculus
<400> 133
   gaaatatcca aactggaatc t 21
<210> 134
   <211> 33
   <212> DNA
   <213> Oryctolagus cuniculus
<400> 134
   ctaggcggtt atgatgatga tgctgataat gct 33
<210> 135
   <211> 15
   <212> DNA
   <213> Oryctolagus cuniculus
<400> 135
   agtcgtacaa tgtcc 15
<210> 136
   <211> 48
   <212> DNA
   <213> Oryctolagus cuniculus
<400> 136
   tacatttgga gtggtggtag cacatactac gcgacctggg cgaaaggc 48
<210> 137
   <211> 45
   <212> DNA
   <213> Oryctolagus cuniculus
<400> 137
   ttgggcgata ctggtggtca cgcttatgct actcgcttaa atctc 45
<210> 138
   <211> 123
   <212> PRT
   <213> Oryctolagus cuniculus
<400> 138
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Ser
               20                  25                  30
   Val Ser Ala Ala Val Gly Gly Thr Val Ser Ile Ser Cys Gln Ser Ser
           35                  40                  45
   Gln Ser Val Tyr Ser Asn Lys Tyr Leu Ala Trp Tyr Gln Gln Lys Pro
       50                  55                  60
   Gly Gln Pro Pro Lys Leu Leu Ile Tyr Trp Thr Ser Lys Leu Ala Ser
   65                  70                  75                  80
   Gly Ala Pro Ser Arg Phe Ser Gly Ser Gly Ser Gly Thr Gln Phe Thr
                   85                  90                  95
   Leu Thr Ile Ser Gly Val Gln Cys Asp Asp Ala Ala Thr Tyr Tyr Cys
               100                 105                 110
   Leu Gly Ala Tyr Asp Asp Asp Ala Asp Asn Ala
           115                 120
<210> 139
   <211> 126
   <212> PRT
   <213> Oryctolagus cuniculus
<400> 139
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Lys Pro
   20 25 30
   Asp Glu Thr Leu Thr Leu Thr Cys Thr Ala Ser Gly Phe Ser Leu Glu
   35 40 45
   Gly Gly Tyr Met Thr Trp Val Arg Gln Ala Pro Gly Lys Gly Leu Glu
   50 55 60
   Trp Ile Gly Ile Ser Tyr Asp Ser Gly Ser Thr Tyr Tyr Ala Ser Trp
   65 70 75 80
   Ala Lys Gly Arg Phe Thr Ile Ser Lys Thr Ser Ser Thr Thr Val Asp
   85 90 95
   Leu Lys Met Thr Ser Leu Thr Thr Glu Asp Thr Ala Thr Tyr Phe Cys
   100 105 110
   Val Arg Ser Leu Lys Tyr Pro Thr Val Thr Ser Asp Asp Leu
   115 120 125
<210> 140
   <211> 13
   <212> PRT
   <213> Oryctolagus cuniculus
<400> 140
   Gln Ser Ser Gln Ser Val Tyr Ser Asn Lys Tyr Leu Ala
   1 5 10
<210> 141
   <211> 7
   <212> PRT
   <213> Oryctolagus cuniculus
<400> 141
   Trp Thr Ser Lys Leu Ala Ser
   1 5
<210> 142
   <211> 11
   <212> PRT
   <213> oryctolagus cuniculus
<400> 142
   Leu Gly Ala Tyr Asp Asp Asp Ala Asp Asn Ala
   1 5 10
<210> 143
   <211> 5
   <212> PRT
   <213> oryctolagus cuniculus
<400> 143
   Gly Gly Tyr Met Thr
   1 5
<210> 144
   <211> 16
   <212> PRT
   <213> Oryctolagus cuniculus
<400> 144
   Ile Ser Tyr Asp Ser Gly Ser Thr Tyr Tyr Ala Ser Trp Ala Lys Gly
   1 5 10 15
<210> 145
   <211> 12
   <212> PRT
   <213> Oryctolagus cuniculus
<400> 145
   Ser Leu Lys Tyr Pro Thr Val Thr Ser Asp Asp Leu
   1 5 10
<210> 146
   <211> 369
   <212> DNA
   <213> Oryctolagus cuniculus
<400> 146
   atggacacga gggcccccac tcagctgctg gggctcctgc tgctctggct cccaggtgcc 60
   acatttgcag ccgtgctgac ccagacacca tcgtccgtgt ctgcagctgt gggaggcaca 120
   gtcagcatca gttgccagtc cagtcagagt gtttatagta ataagtacct agcctggtat 180
   cagcagaaac cagggcagcc tcccaagctc ctgatctact ggacatccaa actggcatct 240
   ggggccccat cacggttcag cggcagtgga tctgggacac aattcactct caccatcagc 300
   ggcgtgcagt gtgacgatgc tgccacttac tactgtctag gcgcttatga tgatgatgct 360
   gataatgct 369
<210> 147
   <211> 378
   <212> DNA
   <213> Oryctolagus cuniculus
<400> 147
   atggagactg ggctgcgctg gcttctcctg gtcgctgtgc tcaaaggtgt ccagtgtcag 60
   tcggtggaag agtccggggg tcgcctggtc aagcctgacg aaaccctgac actcacctgc 120
   acagcctctg gattctccct ggagggcggc tacatgacct gggtccgcca ggctccaggg 180
   aaggggctgg aatggatcgg aatcagttat gatagtggta gcacatacta cgcgagctgg 240
   gcgaaaggcc gattcaccat ctccaagacc tcgtcgacca cggtggatct gaaaatgacc 300
   agtctgacaa ccgaggacac ggccacctat ttctgcgtca gatcactaaa atatcctact 360
   gttacttctg atgacttg 378
<210> 148
   <211> 39
   <212> DNA
   <213> Oryctolagus cuniculus
<400> 148
   cagtccagtc agagtgttta tagtaataag tacctagcc 39
<210> 149
   <211> 21
   <212> DNA
   <213> Oryctolagus cuniculus
<400> 149
   tggacatcca aactggcatc t 21
<210> 150
   <211> 33
   <212> DNA
   <213> Oryctolagus cuniculus
<400> 150
   ctaggcgctt atgatgatga tgctgataat gct 33
<210> 151
   <211> 15
   <212> DNA
   <213> Oryctolagus cuniculus
<400> 151
   ggcggctaca tgacc 15
<210> 152
   <211> 48
   <212> DNA
   <213> Oryctolagus cuniculus
<400> 152
   atcagttatg atagtggtag cacatactac gcgagctggg cgaaaggc 48
<210> 153
   <211> 36
   <212> DNA
   <213> Oryctolagus cuniculus
<400> 153
   tcactaaaat atcctactgt tacttctgat gacttg 36
<210> 154
   <211> 123
   <212> PRT
   <213> Oryctolagus cuniculus
<400> 154
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Pro Ser Pro
   20 25 30
   Val Ser Ala Ala Val Gly Gly Thr Val Thr Ile Ser Cys Gln Ser Ser
   35 40 45
   Gln Ser Val Tyr Asn Asn Asn Asp Leu Ala Trp Tyr Gln Gln Lys Pro
   50 55 60
   Gly Gln Pro Pro Lys Leu Leu Ile Tyr Tyr Ala Ser Thr Leu Ala Ser
   65 70 75 80
   Gly Val Pro Ser Arg Phe Lys Gly Ser Gly Ser Gly Thr Gln Phe Thr
   85 90 95
   Leu Thr Ile Ser Gly Val Gln Cys Asp Asp Ala Ala Ala Tyr Tyr Cys
   100 105 110
   Leu Gly Gly Tyr Asp Asp Asp Ala Asp Asn Ala
   115 120
<210> 155
   <211> 129
   <212> PRT
   <213> Oryctolagus cuniculus
<400> 155
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Leu Ser Leu Ser
   35 40 45
   Ser Asn Thr Ile Asn Trp Val Arg Gln Ala Pro Gly Lys Gly Leu Glu
   50 55 60
   Trp Ile Gly Tyr Ile Trp Ser Gly Gly Ser Thr Tyr Tyr Ala Ser Trp
   65 70 75 80
   Val Asn Gly Arg Phe Thr Ile Ser Lys Thr Ser Thr Thr Val Asp Leu
   85 90 95
   Lys Ile Thr Ser Pro Thr Thr Glu Asp Thr Ala Thr Tyr Phe Cys Ala
   100 105 110
   Arg Gly Gly Tyr Ala Ser Gly Gly Tyr Pro Tyr Ala Thr Arg Leu Asp
   115 120 125
   Leu
<210> 156
   <211> 13
   <212> PRT
   <213> Oryctolagus cuniculus
<400> 156
   Gln Ser Ser Gln Ser Val Tyr Asn Asn Asn Asp Leu Ala
   1 5 10
<210> 157
   <211> 7
   <212> PRT
   <213> Oryctolagus cuniculus
<400> 157
   Tyr Ala Ser Thr Leu Ala Ser
   1 5
<210> 158
   <211> 11
   <212> PRT
   <213> oryctolagus cuniculus
<400> 158
   Leu Gly Gly Tyr Asp Asp Asp Ala Asp Asn Ala
   1 5 10
<210> 159
   <211> 5
   <212> PRT
   <213> Oryctolagus cuniculus
<400> 159
   Ser Asn Thr Ile Asn
   1 5
<210> 160
   <211> 16
   <212> PRT
   <213> Oryctolagus cuniculus
<400> 160
   Tyr Ile Trp Ser Gly Gly Ser Thr Tyr Tyr Ala Ser Trp Val Asn Gly
   1 5 10 15
<210> 161
   <211> 16
   <212> PRT
   <213> Oryctolagus cuniculus
<400> 161
   Gly Gly Tyr Ala Ser Gly Gly Tyr Pro Tyr Ala Thr Arg Leu Asp Leu
   1 5 10 15
<210> 162
   <211> 369
   <212> DNA
   <213> Oryctolagus cuniculus
<400> 162
   atggacacga gggcccccac tcagctgctg gggctcctgc tgctctggct cccaggtgcc 60
   acatttgcag ccgtgctgac ccagacacca tcacccgtgt ctgcagctgt gggaggcaca 120
   gtcaccatca gttgccagtc cagtcagagt gtttataata ataacgactt agcctggtat 180
   cagcagaaac cagggcagcc tcctaaactc ctgatctatt atgcatccac tctggcatct 240
   ggggtcccat cgcggttcaa aggcagtgga tctgggacac agttcactct caccatcagc 300
   ggcgtgcagt gtgacgatgc tgccgcttac tactgtctag gcggttatga tgatgatgct 360
   gataatgct 369
<210> 163
   <211> 387
   <212> DNA
   <213> Oryctolagus cuniculus
<400> 163
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtatctg gattatccct cagtagcaat acaataaact gggtccgcca ggctccaggg 180
   aaggggctgg agtggatcgg atacatttgg agtggtggta gtacatacta cgcgagctgg 240
   gtgaatggtc gattcaccat ctccaaaacc tcgaccacgg tggatctgaa aatcaccagt 300
   ccgacaaccg aggacacggc cacctatttc tgtgccagag ggggttacgc tagtggtggt 360
   tatccttatg ccactcggtt ggatctc 387
<210> 164
   <211> 39
   <212> DNA
   <213> Oryctolagus cuniculus
<400> 164
   cagtccagtc agagtgttta taataataac gacttagcc 39
<210> 165
   <211> 21
   <212> DNA
   <213> Oryctolagus cuniculus
<400> 165
   tatgcatcca ctctggcatc t 21
<210> 166
   <211> 33
   <212> DNA
   <213> Oryctolagus cuniculus
<400> 166
   ctaggcggtt atgatgatga tgctgataat gct 33
<210> 167
   <211> 15
   <212> DNA
   <213> Oryctolagus cuniculus
<400> 167
   agcaatacaa taaac 15
<210> 168
   <211> 48
   <212> DNA
   <213> Oryctolagus cuniculus
<400> 168
   tacatttgga gtggtggtag tacatactac gcgagctggg tgaatggt 48
<210> 169
   <211> 48
   <212> DNA
   <213> oryctolagus cuniculus
<400> 169
   gggggttacg ctagtggtgg ttatccttat gccactcggt tggatctc 48
<210> 170
   <211> 123
   <212> PRT
   <213> Oryctolagus cuniculus
<400> 170
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Ser
               20                  25                  30
   Val Ser Ala Ala Val Gly Gly Thr Val Thr Ile Asn Cys Gln Ser Ser
           35                  40                  45
   Gln Ser Val Tyr Asn Asn Asp Tyr Leu Ser Trp Tyr Gln Gln Arg Pro
       50                  55                  60
   Gly Gln Arg Pro Lys Leu Leu Ile Tyr Gly Ala Ser Lys Leu Ala Ser
   65                  70                  75                  80
   Gly Val Pro Ser Arg Phe Lys Gly Ser Gly Ser Gly Lys Gln Phe Thr
                   85                  90                  95
   Leu Thr Ile Ser Gly Val Gln Cys Asp Asp Ala Ala Thr Tyr Tyr Cys
               100                 105                 110
   Leu Gly Asp Tyr Asp Asp Asp Ala Asp Asn Thr
           115                 120
<210> 171
   <211> 123
   <212> PRT
   <213> Oryctolagus cuniculus
<400> 171
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Phe Thr Leu Ser
           35                  40                  45
   Thr Asn Tyr Tyr Leu Ser Trp Val Arg Gln Ala Pro Gly Lys Gly Leu
       50                  55                  60
   Glu Trp Ile Gly Ile Ile Tyr Pro Ser Gly Asn Thr Tyr Cys Ala Lys
   65                  70                  75                  80
   Trp Ala Lys Gly Arg Phe Thr Ile Ser Lys Thr Ser Ser Thr Thr Val
                   85                  90                  95
   Asp Leu Lys Met Thr Ser Pro Thr Thr Glu Asp Thr Ala Thr Tyr Phe
               100                 105                 110
   Cys Ala Arg Asn Tyr Gly Gly Asp Glu Ser Leu
           115                 120
<210> 172
   <211> 13
   <212> PRT
   <213> Oryctolagus cuniculus
<400> 172
   Gln Ser Ser Gln Ser Val Tyr Asn Asn Asp Tyr Leu Ser
   1               5                   10
<210> 173
   <211> 7
   <212> PRT
   <213> Oryctolagus cuniculus
<400> 173
   Gly Ala Ser Lys Leu Ala Ser
   1               5
<210> 174
   <211> 11
   <212> PRT
   <213> Oryctolagus cuniculus
<400> 174
   Leu Gly Asp Tyr Asp Asp Asp Ala Asp Asn Thr
   1 5 10
<210> 175
   <211> 6
   <212> PRT
   <213> Oryctolagus cuniculus
<400> 175
   Thr Asn Tyr Tyr Leu Ser
   1 5
<210> 176
   <211> 16
   <212> PRT
   <213> Oryctolagus cuniculus
<400> 176
   Ile Ile Tyr Pro Ser Gly Asn Thr Tyr Cys Ala Lys Trp Ala Lys Gly
   1 5 10 15
<210> 177
   <211> 8
   <212> PRT
   <213> Oryctolagus cuniculus
<400> 177
   Asn Tyr Gly Gly Asp Glu Ser Leu
   1 5
<210> 178
   <211> 369
   <212> DNA
   <213> Oryctolagus cuniculus
<400> 178
   atggacacga gggcccccac tcagctgctg gggctcctgc tgctctggct cccaggtgcc 60
   acatttgcag ccgtgctgac ccagacacca tcctccgtgt ctgcagctgt gggaggcaca 120
   gtcaccatca attgccagtc cagtcagagt gtttataata acgactactt atcctggtat 180
   caacagaggc cagggcaacg tcccaagctc ctaatctatg gtgcttccaa actggcatct 240
   ggggtcccgt cacggttcaa aggcagtgga tctgggaaac agtttactct caccatcagc 300
   ggcgtgcagt gtgacgatgc tgccacttac tactgtctgg gcgattatga tgatgatgct 360
   gataatact 369
<210> 179
   <211> 369
   <212> DNA
   <213> oryctolagus cuniculus
<400> 179
   atggagactg ggctgcgctg gcttctcctg gtcgctgtgc tcaaaggtgt ccagtgtcag 60
   tcgctggagg agtccggggg tcgcctggtc acgcctggga cacccctgac actcacttgc 120
   acagtctctg gattcaccct cagtaccaac tactacctga gctgggtccg ccaggctcca 180
   gggaaggggc tagaatggat cggaatcatt tatcctagtg gtaacacata ttgcgcgaag 240
   tgggcgaaag gccgattcac catctccaaa acctcgtcga ccacggtgga tctgaaaatg 300
   accagtccga caaccgagga cacagccacg tatttctgtg ccagaaatta tggtggtgat 360
   gaaagtttg 369
<210> 180
   <211> 39
   <212> DNA
   <213> oryctolagus cuniculus
<400> 180
   cagtccagtc agagtgttta taataacgac tacttatcc 39
<210> 181
   <211> 21
   <212> DNA
   <213> Oryctolagus cuniculus
<400> 181
   ggtgcttcca aactggcatc t 21
<210> 182
   <211> 33
   <212> DNA
   <213> Oryctolagus cuniculus
<400> 182
   ctgggcgatt atgatgatga tgctgataat act 33
<210> 183
   <211> 18
   <212> DNA
   <213> Oryctolagus cuniculus
<400> 183
   accaactact acctgagc 18
<210> 184
   <211> 48
   <212> DNA
   <213> Oryctolagus cuniculus
<400> 184
   atcatttatc ctagtggtaa cacatattgc gcgaagtggg cgaaaggc 48
<210> 185
   <211> 24
   <212> DNA
   <213> Oryctolagus cuniculus
<400> 185
   aattatggtg gtgatgaaag tttg 24
<210> 186
   <211> 119
   <212> PRT
   <213> Oryctolagus cuniculus
<400> 186
   Met Asp Thr Arg Ala Pro Thr Gln Leu Leu Gly Leu Leu Leu Leu Trp
   1 5 10 15
   Leu Pro Gly Ala Arg Cys Asp Val Val Met Thr Gln Thr Pro Ala Ser
   20 25 30
   Val Glu Ala Ala Val Gly Gly Thr Val Thr Ile Lys Cys Gln Ala Ser
   35 40 45
   Glu Thr Ile Gly Asn Ala Leu Ala Trp Tyr Gln Gln Lys Ser Gly Gln
   50 55 60
   Pro Pro Lys Leu Leu Ile Tyr Lys Ala Ser Lys Leu Ala Ser Gly Val
   65 70 75 80
   Pro Ser Arg Phe Lys Gly Ser Gly Ser Gly Thr Glu Tyr Thr Leu Thr
   85 90 95
   Ile Ser Asp Leu Glu Cys Ala Asp Ala Ala Thr Tyr Tyr Cys Gln Trp
   100 105 110
   Cys Tyr Phe Gly Asp Ser Val
   115
<210> 187
   <211> 128
   <212> PRT
   <213> Oryctolagus cuniculus
<400> 187
   Met Glu Thr Gly Leu Arg Trp Leu Leu Leu Val Thr Val Leu Lys Gly
   1 5 10 15
   Val Gln Cys Gln Glu Gln Leu Val Glu Ser Gly Gly Gly Leu Val Gln
   20 25 30
   Pro Glu Gly Ser Leu Thr Leu Thr Cys Thr Ala Ser Gly Phe Asp Phe
   35 40 45
   Ser Ser Gly Tyr Tyr Met Cys Trp Val Arg Gln Ala Pro Gly Lys Gly
   50 55 60
   Leu Glu Trp Ile Ala Cys Ile Phe Thr Ile Thr Thr Asn Thr Tyr Tyr
   65 70 75 80
   Ala Ser Trp Ala Lys Gly Arg Phe Thr Ile Ser Lys Thr Ser Ser Thr
   85 90 95
   Thr Val Thr Leu Gln Met Thr Ser Leu Thr Ala Ala Asp Thr Ala Thr
   100 105 110
   Tyr Leu Cys Ala Arg Gly Ile Tyr Ser Asp Asn Asn Tyr Tyr Ala Leu
   115 120 125
<210> 188
   <211> 11
   <212> PRT
   <213> Oryctolagus cuniculus
<400> 188
   Gln Ala Ser Glu Thr Ile Gly Asn Ala Leu Ala
   1 5 10
<210> 189
   <211> 7
   <212> PRT
   <213> Oryctolagus cuniculus
<400> 189
   Lys Ala Ser Lys Leu Ala Ser
   1 5
<210> 190
   <211> 9
   <212> PRT
   <213> Oryctolagus cuniculus
<400> 190
   Gln Trp Cys Tyr Phe Gly Asp Ser Val
   1 5
<210> 191
   <211> 6
   <212> PRT
   <213> Oryctolagus cuniculus
<400> 191
   Ser Gly Tyr Tyr Met Cys
   1 5
<210> 192
   <211> 17
   <212> PRT
   <213> Oryctolagus cuniculus
<400> 192
   Cys Ile Phe Thr Ile Thr Thr Asn Thr Tyr Tyr Ala Ser Trp Ala Lys
   1 5 10 15
   Gly
<210> 193
   <211> 11
   <212> PRT
   <213> Oryctolagus cuniculus
<400> 193
   Gly Ile Tyr Ser Asp Asn Asn Tyr Tyr Ala Leu
   1 5 10
<210> 194
   <211> 357
   <212> DNA
   <213> Oryctolagus cuniculus
<400> 194
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtgagacc attggcaatg cattagcctg gtatcagcag 180
   aaatcagggc agcctcccaa gctcctgatc tacaaggcat ccaaactggc atctggggtc 240
   ccatcgcggt tcaaaggcag tggatctggg acagagtaca ctctcaccat cagcgacctg 300
   gagtgtgccg atgctgccac ttactactgt caatggtgtt attttggtga tagtgtt 357
<210> 195
   <211> 384
   <212> DNA
   <213> Oryctolagus cuniculus
<400> 195
   atggagactg ggctgcgctg gcttctcctg gtcactgtgc tcaaaggtgt ccagtgtcag 60
   gagcagctgg tggagtccgg gggaggcctg gtccagcctg agggatccct gacactcacc 120
   tgcacagcct ctggattcga cttcagtagc ggctactaca tgtgctgggt ccgccaggct 180
   ccagggaagg ggctggagtg gatcgcgtgt attttcacta ttactactaa cacttactac 240
   gcgagctggg cgaaaggccg attcaccatc tccaagacct cgtcgaccac ggtgactctg 300
   caaatgacca gtctgacagc cgcggacacg gccacctatc tctgtgcgag agggatttat 360
   tctgataata attattatgc cttg 384
<210> 196
   <211> 33
   <212> DNA
   <213> Oryctolagus cuniculus
<400> 196
   caggccagtg agaccattgg caatgcatta gcc 33
<210> 197
   <211> 21
   <212> DNA
   <213> Oryctolagus cuniculus
<400> 197
   aaggcatcca aactggcatc t 21
<210> 198
   <211> 27
   <212> DNA
   <213> Oryctolagus cuniculus
<400> 198
   caatggtgtt attttggtga tagtgtt 27
<210> 199
   <211> 18
   <212> DNA
   <213> Oryctolagus cuniculus
<400> 199
   agcggctact acatgtgc 18
<210> 200
   <211> 51
   <212> DNA
   <213> Oryctolagus cuniculus
<400> 200
   tgtattttca ctattactac taacacttac tacgcgagct gggcgaaagg c 51
<210> 201
   <211> 33
   <212> DNA
   <213> Oryctolagus cuniculus
<400> 201
   gggatttatt ctgataataa ttattatgcc ttg 33
<210> 202
   <211> 119
   <212> PRT
   <213> Oryctolagus cuniculus
<400> 202
   Met Asp Thr Arg Ala Pro Thr Gln Leu Leu Gly Leu Leu Leu Leu Trp
   1 5 10 15
   Leu Pro Gly Ala Arg Cys Asp Val Val Met Thr Gln Thr Pro Ala Ser
   20 25 30
   Val Glu Ala Ala Val Gly Gly Thr Val Thr Ile Lys Cys Gln Ala Ser
   35 40 45
   Glu Ser Ile Gly Asn Ala Leu Ala Trp Tyr Gln Gln Lys Pro Gly Gln
   50 55 60
   Pro Pro Lys Leu Leu Ile Tyr Lys Ala Ser Thr Leu Ala Ser Gly Val
   65 70 75 80
   Pro Ser Arg Phe Ser Gly Ser Gly Ser Gly Thr Glu Phe Thr Leu Thr
   85 90 95
   Ile Ser Gly Val Gln Cys Ala Asp Ala Ala Ala Tyr Tyr Cys Gln Trp
   100 105 110
   Cys Tyr Phe Gly Asp Ser Val
   115
<210> 203
   <211> 128
   <212> PRT
   <213> Oryctolagus cuniculus
<400> 203
   Met Glu Thr Gly Leu Arg Trp Leu Leu Leu Val Ala Val Leu Lys Gly
   1               5                   10                  15
   Val Gln Cys Gln Gln Gln Leu Val Glu Ser Gly Gly Gly Leu Val Lys
               20                  25                  30
   Pro Gly Ala Ser Leu Thr Leu Thr Cys Lys Ala Ser Gly Phe Ser Phe
           35                  40                  45
   Ser Ser Gly Tyr Tyr Met Cys Trp Val Arg Gln Ala Pro Gly Lys Gly
       50                  55                  60
   Leu Glu Ser Ile Ala Cys Ile Phe Thr Ile Thr Asp Asn Thr Tyr Tyr
   65                  70                  75                  80
   Ala Asn Trp Ala Lys Gly Arg Phe Thr Ile Ser Lys Pro Ser Ser Pro
                   85                  90                  95
   Thr Val Thr Leu Gln Met Thr Ser Leu Thr Ala Ala Asp Thr Ala Thr
               100                 105                 110
   Tyr Phe Cys Ala Arg Gly Ile Tyr Ser Thr Asp Asn Tyr Tyr Ala Leu
           115                 120                 125
<210> 204
   <211> 11
   <212> PRT
   <213> Oryctolagus cuniculus
<400> 204
   Gln Ala Ser Glu Ser Ile Gly Asn Ala Leu Ala
   1               5                   10
<210> 205
   <211> 7
   <212> PRT
   <213> Oryctolagus cuniculus
<400> 205
   Lys Ala Ser Thr Leu Ala Ser
   1               5
<210> 206
   <211> 9
   <212> PRT
   <213> Oryctolagus cuniculus
<400> 206
   Gln Trp Cys Tyr Phe Gly Asp Ser Val
   1 5
<210> 207
   <211> 6
   <212> PRT
   <213> Oryctolagus cuniculus
<400> 207
   Ser Gly Tyr Tyr Met Cys
   1 5
<210> 208
   <211> 17
   <212> PRT
   <213> Oryctolagus cuniculus
<400> 208
   Cys Ile Phe Thr Ile Thr Asp Asn Thr Tyr Tyr Ala Asn Trp Ala Lys
   1 5 10 15
   Gly
<210> 209
   <211> 11
   <212> PRT
   <213> Oryctolagus cuniculus
<400> 209
   Gly Ile Tyr Ser Thr Asp Asn Tyr Tyr Ala Leu
   1 5 10
<210> 210
   <211> 357
   <212> DNA
   <213> Oryctolagus cuniculus
<400> 210
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtgagagc attggcaatg cattagcctg gtatcagcag 180
   aaaccagggc agcctcccaa gctcctgatc tacaaggcat ccactctggc atctggggtc 240
   ccatcgcggt tcagcggcag tggatctggg acagagttca ctctcaccat cagcggcgtg 300
   cagtgtgccg atgctgccgc ttactactgt caatggtgtt attttggtga tagtgtt 357
<210> 211
   <211> 384
   <212> DNA
   <213> Oryctolagus cuniculus
<400> 211
   atggagactg ggctgcgctg gcttctcctg gtcgctgtgc tcaaaggtgt ccagtgtcag 60
   cagcagctgg tggagtccgg gggaggcctg gtcaagccgg gggcatccct gacactcacc 120
   tgcaaagcct ctggattctc cttcagtagc ggctactaca tgtgctgggt ccgccaggct 180
   ccagggaagg ggctggagtc gatcgcatgc atttttacta ttactgataa cacttactac 240
   gcgaactggg cgaaaggccg attcaccatc tccaagccct cgtcgcccac ggtgactctg 300
   caaatgacca gtctgacagc cgcggacacg gccacctatt tctgtgcgag ggggatttat 360
   tctactgata attattatgc cttg 384
<210> 212
   <211> 33
   <212> DNA
   <213> Oryctolagus cuniculus
<400> 212
   caggccagtg agagcattgg caatgcatta gcc 33
<210> 213
   <211> 21
   <212> DNA
   <213> Oryctolagus cuniculus
<400> 213
   aaggcatcca ctctggcatc t 21
<210> 214
   <211> 27
   <212> DNA
   <213> Oryctolagus cuniculus
<400> 214
   caatggtgtt attttggtga tagtgtt 27
<210> 215
   <211> 18
   <212> DNA
   <213> Oryctolagus cuniculus
<400> 215
   agcggctact acatgtgc 18
<210> 216
   <211> 51
   <212> DNA
   <213> Oryctolagus cuniculus
<400> 216
   tgcattttta ctattactga taacacttac tacgcgaact gggcgaaagg c 51
<210> 217
   <211> 33
   <212> DNA
   <213> Oryctolagus cuniculus
<400> 217
   gggatttatt ctactgataa ttattatgcc ttg 33
<210> 218
   <211> 123
   <212> PRT
   <213> Oryctolagus cuniculus
<400> 218
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Gln Ser Val Ser Ser Tyr Leu Asn Trp Tyr Gln Gln Lys Pro Gly Gln
       50                  55                  60
   Pro Pro Lys Leu Leu Ile Tyr Arg Ala Ser Thr Leu Glu Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Thr Ser Ser Ser Tyr Gly Ala Ala
           115                 120
<210> 219
   <211> 133
   <212> PRT
   <213> Oryctolagus cuniculus
<400> 219
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Ile Ser Leu Ser
           35                  40                  45
   Ser Asn Ala Ile Ser Trp Val Arg Gln Ala Pro Gly Lys Gly Leu Glu
       50                  55                  60
   Trp Ile Gly Ile Ile Ser Tyr Ser Gly Thr Thr Tyr Tyr Ala Ser Trp
   65                  70                  75                  80
   Ala Lys Gly Arg Phe Thr Ile Ser Lys Thr Ser Ser Thr Thr Val Asp
                   85                  90                  95
   Leu Lys Ile Thr Ser Pro Thr Thr Glu Asp Thr Ala Thr Tyr Phe Cys
               100                 105                 110
   Ala Arg Asp Asp Pro Thr Thr Val Met Val Met Leu Ile Pro Phe Gly
           115                 120                 125
   Ala Gly Met Asp Leu
       130
<210> 220
   <211> 11
   <212> PRT
   <213> Oryctolagus cuniculus
<400> 220
   Gln Ala Ser Gln Ser Val Ser Ser Tyr Leu Asn
   1               5                   10
<210> 221
   <211> 7
   <212> PRT
   <213> Oryctolagus cuniculus
<400> 221
   Arg Ala Ser Thr Leu Glu Ser
   1               5
<210> 222
   <211> 13
   <212> PRT
   <213> Oryctolagus cuniculus
<400> 222
   Gln Cys Thr Tyr Gly Thr Ser Ser Ser Tyr Gly Ala Ala
   1 5 10
<210> 223
   <211> 5
   <212> PRT
   <213> Oryctolagus cuniculus
<400> 223
   Ser Asn Ala Ile Ser
   1 5
<210> 224
   <211> 16
   <212> PRT
   <213> Oryctolagus cuniculus
<400> 224
   Ile Ile Ser Tyr Ser Gly Thr Thr Tyr Tyr Ala Ser Trp Ala Lys Gly
   1 5 10 15
<210> 225
   <211> 19
   <212> PRT
   <213> oryctolagus cuniculus
<400> 225 Met Asp Leu
<210> 226
   <211> 369
   <212> DNA
   <213> Oryctolagus cuniculus
<400> 226
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtcagagc gttagtagct acttaaactg gtatcagcag 180
   aaaccagggc agcctcccaa gctcctgatc tacagggcat ccactctgga atctggggtc 240
   ccatcgcggt tcaaaggcag tggatctggg acagagttca ctctcaccat cagcgacctg 300
   gagtgtgccg atgctgccac ttactactgt caatgtactt atggtactag tagtagttat 360
   ggtgctgct 369
<210> 227
   <211> 399
   <212> DNA
   <213> Oryctolagus cuniculus
<400> 227
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   accgtctctg gtatctccct cagtagcaat gcaataagct gggtccgcca ggctccaggg 180
   aaggggctgg aatggatcgg aatcattagt tatagtggta ccacatacta cgcgagctgg 240
   gcgaaaggcc gattcaccat ctccaaaacc tcgtcgacca cggtggatct gaaaatcact 300
   agtccgacaa ccgaggacac ggccacctac ttctgtgcca gagatgaccc tacgacagtt 360
   atggttatgt tgataccttt tggagccggc atggacctc 399
<210> 228
   <211> 33
   <212> DNA
   <213> Oryctolagus cuniculus
<400> 228
   caggccagtc agagcgttag tagctactta aac 33
<210> 229
   <211> 21
   <212> DNA
   <213> Oryctolagus cuniculus
<400> 229
   agggcatcca ctctggaatc t 21
<210> 230
   <211> 39
   <212> DNA
   <213> Oryctolagus cuniculus
<400> 230
   caatgtactt atggtactag tagtagttat ggtgctgct 39
<210> 231
   <211> 15
   <212> DNA
   <213> Oryctolagus cuniculus
<400> 231
   agcaatgcaa taagc 15
<210> 232
   <211> 48
   <212> DNA
   <213> Oryctolagus cuniculus
<400> 232
   atcattagtt atagtggtac cacatactac gcgagctggg cgaaaggc 48
<210> 233
   <211> 57
   <212> DNA
   <213> Oryctolagus cuniculus
<400> 233
   gatgacccta cgacagttat ggttatgttg ataccttttg gagccggcat ggacctc 57
<210> 234
   <211> 125
   <212> PRT
   <213> Oryctolagus cuniculus
<400> 234
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Ala Ser Pro
               20                  25                  30
   Val Ser Ala Ala Val Gly Gly Thr Val Thr Ile Asn Cys Gln Ala Ser
           35                  40                  45
   Gln Ser Val Tyr Lys Asn Asn Tyr Leu Ser Trp Tyr Gln Gln Lys Pro
       50                  55                  60
   Gly Gln Pro Pro Lys Gly Leu Ile Tyr Ser Ala Ser Thr Leu Asp Ser
   65                  70                  75                  80
   Gly Val Pro Leu Arg Phe Ser Gly Ser Gly Ser Gly Thr Gln Phe Thr
                   85                  90                  95
   Leu Thr Ile Ser Asp Val Gln Cys Asp Asp Ala Ala Thr Tyr Tyr Cys
               100                 105                 110
   Leu Gly Ser Tyr Asp Cys Ser Ser Gly Asp Cys Tyr Ala
           115                 120                 125
<210> 235
   <211> 119
   <212> PRT
   <213> oryctolagus cuniculus
<400> 235
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Glu Gly Ser Leu Thr Leu Thr Cys Thr Ala Ser Gly Phe Ser Phe Ser
           35                  40                  45
   Ser Tyr Trp Met Cys Trp Val Arg Gln Ala Pro Gly Lys Gly Leu Glu
       50                  55                  60
   Trp Ile Ala Cys Ile Val Thr Gly Asn Gly Asn Thr Tyr Tyr Ala Asn
   65                  70                  75                  80
   Trp Ala Lys Gly Arg Phe Thr Ile Ser Lys Thr Ser Ser Thr Thr Val
                   85                  90                  95
   Thr Leu Gln Met Thr Ser Leu Thr Ala Ala Asp Thr Ala Thr Tyr Phe
               100                 105                 110
   Cys Ala Lys Ala Tyr Asp Leu
           115
<210> 236
   <211> 13
   <212> PRT
   <213> Oryctolagus cuniculus
<400> 236
   Gln Ala Ser Gln Ser Val Tyr Lys Asn Asn Tyr Leu Ser
   1               5                   10
<210> 237
   <211> 7
   <212> PRT
   <213> oryctolagus cuniculus
<400> 237
   Ser Ala Ser Thr Leu Asp Ser
   1               5
<210> 238
   <211> 13
   <212> PRT
   <213> Oryctolagus cuniculus
<400> 238
   Leu Gly Ser Tyr Asp Cys Ser Ser Gly Asp Cys Tyr Ala
   1               5                   10
<210> 239
   <211> 5
   <212> PRT
   <213> Oryctolagus cuniculus
<400> 239
   Ser Tyr Trp Met Cys
   1               5
<210> 240
   <211> 17
   <212> PRT
   <213> Oryctolagus cuniculus
<400> 240
   Cys Ile Val Thr Gly Asn Gly Asn Thr Tyr Tyr Ala Asn Trp Ala Lys
   1               5                   10                  15
   Gly
<210> 241
   <211> 4
   <212> PRT
   <213> Oryctolagus cuniculus
<400> 241
   Ala Tyr Asp Leu
   1
<210> 242
   <211> 375
   <212> DNA
   <213> Oryctolagus cuniculus
<400> 242
   atggacacga gggcccccac tcagctgctg gggctcctgc tgctctggct cccaggtgcc 60
   acatttgccc aagtgctgac ccagactgca tcgcccgtgt ctgcagctgt gggaggcaca 120
   gtcaccatca actgccaggc cagtcagagt gtttataaga acaactactt atcctggtat 180
   cagcagaaac cagggcagcc tcccaaaggc ctgatctatt ctgcatcgac tctagattct 240
   ggggtcccat tgcggttcag cggcagtgga tctgggacac agttcactct caccatcagc 300
   gacgtgcagt gtgacgatgc tgccacttac tactgtctag gcagttatga ttgtagtagt 360
   ggtgattgtt atgct 375
<210> 243
   <211> 357
   <212> DNA
   <213> Oryctolagus cuniculus
<400> 243
   atggagactg ggctgcgctg gcttctcctg gtcgctgtgc tcaaaggtgt ccagtgtcag 60
   tcgttggagg agtccggggg agacctggtc aagcctgagg gatccctgac actcacctgc 120
   acagcctctg gattctcctt cagtagctac tggatgtgct gggtccgcca ggctccaggg 180
   aaggggctgg agtggatcgc atgcattgtt actggtaatg gtaacactta ctacgcgaac 240
   tgggcgaaag gccgattcac catctccaaa acctcgtcga ccacggtgac tctgcaaatg 300
   accagtctga cagccgcgga cacggccacc tatttttgtg cgaaagccta tgacttg 357
<210> 244
   <211> 39
   <212> DNA
   <213> Oryctolagus cuniculus
<400> 244
   caggccagtc agagtgttta taagaacaac tacttatcc 39
<210> 245
   <211> 21
   <212> DNA
   <213> Oryctolagus cuniculus
<400> 245
   tctgcatcga ctctagattc t 21
<210> 246
   <211> 39
   <212> DNA
   <213> Oryctolagus cuniculus
<400> 246
   ctaggcagtt atgattgtag tagtggtgat tgttatgct 39
<210> 247
   <211> 15
   <212> DNA
   <213> Oryctolagus cuniculus
<400> 247
   agctactgga tgtgc 15
<210> 248
   <211> 51
   <212> DNA
   <213> Oryctolagus cuniculus
<400> 248
   tgcattgtta ctggtaatgg taacacttac tacgcgaact gggcgaaagg c 51
<210> 249
   <211> 12
   <212> DNA
   <213> Oryctolagus cuniculus
<400> 249
   gcctatgact tg 12
<210> 250
   <211> 123
   <212> PRT
   <213> Oryctolagus cuniculus
<400> 250
   Met Asp Thr Arg Ala Pro Thr Gln Leu Leu Gly Leu Leu Leu Leu Trp
   1               5                   10                  15
   Leu Pro Gly Ser Thr Phe Ala Ala Val Leu Thr Gln Thr Pro Ser Pro
               20                  25                  30
   Val Ser Ala Ala Val Gly Gly Thr Val Ser Ile Ser Cys Gln Ala Ser
           35                  40                  45
   Gln Ser Val Tyr Asp Asn Asn Tyr Leu Ser Trp Tyr Gln Gln Lys Pro
       50                  55                  60
   Gly Gln Pro Pro Lys Leu Leu Ile Tyr Gly Ala Ser Thr Leu Ala Ser
   65                  70                  75                  80
   Gly Val Pro Ser Arg Phe Lys Gly Thr Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Phe Asn Asp Asp Ser Asp Asp Ala
           115                 120
<210> 251
   <211> 125
   <212> PRT
   <213> oryctolagus cuniculus
<400> 251
   Met Glu Thr Gly Leu Arg Trp Leu Leu Leu Val Ala Val Pro Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Leu Ser Gly Phe Ser Leu Ser
   35 40 45
   Ala Tyr Tyr Met Ser Trp Val Arg Gln Ala Pro Gly Lys Gly Leu Glu
   50 55 60
   Trp Ile Gly Phe Ile Thr Leu Ser Asp His Ile Ser Tyr Ala Arg Trp
   65 70 75 80
   Ala Lys Gly Arg Phe Thr Ile Ser Lys Thr Ser Thr Thr Val Asp Leu
   85 90 95
   Lys Met Thr Ser Pro Thr Thr Glu Asp Thr Ala Thr Tyr Phe Cys Ala
   100 105 110
   Arg Ser Arg Gly Trp Gly Ala Met Gly Arg Leu Asp Leu
   115 120 125
<210> 252
   <211> 13
   <212> PRT
   <213> oryctolagus cuniculus
<400> 252
   Gln Ala Ser Gln Ser Val Tyr Asp Asn Asn Tyr Leu Ser
   1 5 10
<210> 253
   <211> 7
   <212> PRT
   <213> oryctolagus cuniculus
<400> 253
   Gly Ala Ser Thr Leu Ala Ser
   1 5
<210> 254
   <211> 11
   <212> PRT
   <213> oryctolagus cuniculus
<400> 254
   Ala Gly Val Phe Asn Asp Asp Ser Asp Asp Ala
   1               5                   10
<210> 255
   <211> 5
   <212> PRT
   <213> Oryctolagus cuniculus
<400> 255
   Ala Tyr Tyr Met Ser
   1               5
<210> 256
   <211> 16
   <212> PRT
   <213> Oryctolagus cuniculus
<400> 256
   Phe Ile Thr Leu Ser Asp His Ile Ser Tyr Ala Arg Trp Ala Lys Gly
   1               5                   10                  15
<210> 257
   <211> 12
   <212> PRT
   <213> Oryctolagus cuniculus
<400> 257
   Ser Arg Gly Trp Gly Ala Met Gly Arg Leu Asp Leu
   1               5                   10
<210> 258
   <211> 369
   <212> DNA
   <213> Oryctolagus cuniculus
<400> 258
   atggacacga gggcccccac tcagctgctg gggctcctgc tgctctggct cccaggttcc 60
   acatttgccg ccgtgctgac ccagactcca tctcccgtgt ctgcagctgt gggaggcaca 120
   gtcagcatca gttgccaggc cagtcagagt gtttatgaca acaactattt atcctggtat 180
   cagcagaaac caggacagcc tcccaagctc ctgatctatg gtgcatccac tctggcatct 240
   ggggtcccat cgcggttcaa aggcacggga tctgggacac agttcactct caccatcaca 300
   gacgtgcagt gtgacgatgc tgccacttac tattgtgcag gcgtttttaa tgatgatagt 360
   gatgatgcc 369
<210> 259
   <211> 375
   <212> DNA
   <213> Oryctolagus cuniculus
<400> 259
   atggagactg ggctgcgctg gcttctcctg gtcgctgtgc ccaaaggtgt ccagtgtcag 60
   tcgctggagg agtccggggg tcgcctggtc acgcctggga cacccctgac actcacctgc 120
   acactctctg gattctccct cagtgcatac tatatgagct gggtccgcca ggctccaggg 180
   aaggggctgg aatggatcgg attcattact ctgagtgatc atatatctta cgcgaggtgg 240
   gcgaaaggcc gattcaccat ctccaaaacc tcgaccacgg tggatctgaa aatgaccagt 300
   ccgacaaccg aggacacggc cacctatttc tgtgccagga gtcgtggctg gggtgcaatg 360
   ggtcggttgg atctc 375
<210> 260
   <211> 39
   <212> DNA
   <213> Oryctolagus cuniculus
<400> 260
   caggccagtc agagtgttta tgacaacaac tatttatcc 39
<210> 261
   <211> 21
   <212> DNA
   <213> oryctolagus cuniculus
<400> 261
   ggtgcatcca ctctggcatc t 21
<210> 262
   <211> 33
   <212> DNA
   <213> Oryctolagus cuniculus
<400> 262
   gcaggcgttt ttaatgatga tagtgatgat gcc 33
<210> 263
   <211> 15
   <212> DNA
   <213> Oryctolagus cuniculus
<400> 263
   gcatactata tgagc 15
<210> 264
   <211> 48
   <212> DNA
   <213> oryctolagus cuniculus
<400> 264
   ttcattactc tgagtgatca tatatcttac gcgaggtggg cgaaaggc 48
<210> 265
   <211> 36
   <212> DNA
   <213> Oryctolagus cuniculus
<400> 265
   agtcgtggct ggggtgcaat gggtcggttg gatctc 36
<210> 266
   <211> 123
   <212> PRT
   <213> Oryctolagus cuniculus
<400> 266
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Pro Ser Pro
   20 25 30
   Val Ser Ala Ala Val Gly Gly Thr Val Thr Ile Ser Cys Gln Ala Ser
   35 40 45
   Gln Ser Val Tyr Asn Asn Lys Asn Leu Ala Trp Tyr Gln Gln Lys Ser
   50 55 60
   Gly Gln Pro Pro Lys Leu Leu Ile Tyr Trp Ala Ser Thr Leu Ala Ser
   65 70 75 80
   Gly Val Ser Ser Arg Phe Ser Gly Ser Gly Ser Gly Thr Gln Phe Thr
   85 90 95
   Leu Thr Val Ser Gly Val Gln Cys Asp Asp Ala Ala Thr Tyr Tyr Cys
   100 105 110
   Leu Gly Val Phe Asp Asp Asp Ala Asp Asn Ala
   115 120
<210> 267
   <211> 121
   <212> PRT
   <213> Oryctolagus cuniculus
<400> 267
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Ser Tyr Ser Met Thr Trp Val Arg Gln Ala Pro Gly Lys Gly Leu Glu
       50                  55                  60
   Tyr Ile Gly Val Ile Gly Thr Ser Gly Ser Thr Tyr Tyr Ala Thr Trp
   65                  70                  75                  80
   Ala Lys Gly Arg Phe Thr Ile Ser Arg Thr Ser Thr Thr Val Ala Leu
                   85                  90                  95
   Lys Ile Thr Ser Pro Thr Thr Glu Asp Thr Ala Thr Tyr Phe Cys Val
               100                 105                 110
   Arg Ser Leu Ser Ser Ile Thr Phe Leu
           115                 120
<210> 268
   <211> 13
   <212> PRT
   <213> Oryctolagus cuniculus
<400> 268
   Gln Ala Ser Gln Ser Val Tyr Asn Asn Lys Asn Leu Ala
   1               5                   10
<210> 269
   <211> 7
   <212> PRT
   <213> Oryctolagus cuniculus
<400> 269
   Trp Ala Ser Thr Leu Ala Ser
   1               5
<210> 270
   <211> 11
   <212> PRT
   <213> Oryctolagus cuniculus
<400> 270
   Leu Gly Val Phe Asp Asp Asp Ala Asp Asn Ala
   1 5 10
<210> 271
   <211> 5
   <212> PRT
   <213> oryctolagus cuniculus
<400> 271
   Ser Tyr Ser Met Thr
   1 5
<210> 272
   <211> 16
   <212> PRT
   <213> Oryctolagus cuniculus
<400> 272
   Val Ile Gly Thr Ser Gly Ser Thr Tyr Tyr Ala Thr Trp Ala Lys Gly
   1 5 10 15
<210> 273
   <211> 8
   <212> PRT
   <213> oryctolagus cuniculus
<400> 273
   Ser Leu Ser Ser Ile Thr Phe Leu
   1 5
<210> 274
   <211> 369
   <212> DNA
   <213> Oryctolagus cuniculus
<400> 274
   atggacacga gggcccccac tcagctgctg gggctcctgc tgctctggct cccaggtgcc 60
   acattcgcag ccgtgctgac ccagacacca tcgcccgtgt ctgcggctgt gggaggcaca 120
   gtcaccatca gttgccaggc cagtcagagt gtttataaca acaaaaattt agcctggtat 180
   cagcagaaat cagggcagcc tcccaagctc ctgatctact gggcatccac tctggcatct 240
   ggggtctcat cgcggttcag cggcagtgga tctgggacac agttcactct caccgtcagc 300
   ggcgtgcagt gtgacgatgc tgccacttac tactgtctag gcgtttttga tgatgatgct 360
   gataatgct 369
<210> 275
   <211> 363
   <212> DNA
   <213> oryctolagus cuniculus
<400> 275
   atggagactg ggctgcgctg gcttctcctg gtcgctgtgc tcaaaggtgt ccaatgtcag      60
   tcggtggagg agtccggggg tcgcctggtc acgcctggga cacccctgac actcacctgc     120
   acagcctctg gattctccct cagtagctac tccatgacct gggtccgcca ggctccaggg     180
   aaggggctgg aatatatcgg agtcattggt actagtggta gcacatacta cgcgacctgg     240
   gcgaaaggcc gattcaccat ctccagaacc tcgaccacgg tggctctgaa aatcaccagt     300
   ccgacaaccg aggacacggc cacctatttc tgtgtcagga gtctttcttc tattactttc     360
   ttg                                                                   363
<210> 276
   <211> 39
   <212> DNA
   <213> oryctolagus cuniculus
<400> 276
   caggccagtc agagtgttta taacaacaaa aatttagcc 39
<210> 277
   <211> 21
   <212> DNA
   <213> Oryctolagus cuniculus
<400> 277
   tgggcatcca ctctggcatc t 21
<210> 278
   <211> 33
   <212> DNA
   <213> oryctolagus cuniculus
<400> 278
   ctaggcgttt ttgatgatga tgctgataat gct 33
<210> 279
   <211> 15
   <212> DNA
   <213> oryctolagus cuniculus
<400> 279
   agctactcca tgacc 15
<210> 280
   <211> 48
   <212> DNA
   <213> Oryctolagus cuniculus
<400> 280
   gtcattggta ctagtggtag cacatactac gcgacctggg cgaaaggc 48
<210> 281
   <211> 24
   <212> DNA
   <213> Oryctolagus cuniculus
<400> 281
   agtctttctt ctattacttt cttg 24
<210> 282
   <211> 120
   <212> PRT
   <213> Oryctolagus cuniculus
<400> 282
   Met Asp Thr Arg Ala Pro Thr Gln Leu Leu Gly Leu Leu Leu Leu Trp
   1               5                   10                  15
   Leu Pro Gly Ala Arg Cys Ala Phe Glu Leu Thr Gln Thr Pro Ala Ser
               20                  25                  30
   Val Glu Ala Ala Val Gly Gly Thr Val Thr Ile Asn Cys Gln Ala Ser
           35                  40                  45
   Gln Asn Ile Tyr Arg Tyr Leu Ala Trp Tyr Gln Gln Lys Pro Gly Gln
       50                  55                  60
   Pro Pro Lys Phe Leu Ile Tyr Leu Ala Ser Thr Leu Ala Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Ser
               100                 105                 110
   Tyr Tyr Ser Ser Asn Ser Val Ala
           115                 120
<210> 283
   <211> 128
   <212> PRT
   <213> Oryctolagus cuniculus
<400> 283
   Met Glu Thr Gly Leu Arg Trp Leu Leu Leu Val Ala Val Leu Lys Gly
   1 5 10 15
   Val Gln Cys Gln Glu Gln Leu Val Glu Ser Gly Gly Asp Leu Val Gln
   20 25 30
   Pro Glu Gly Ser Leu Thr Leu Thr Cys Thr Ala Ser Glu Leu Asp Phe
   35 40 45
   Ser Ser Gly Tyr Trp Ile Cys Trp Val Arg Gln Val Pro Gly Lys Gly
   50 55 60
   Leu Glu Trp Ile Gly Cys Ile Tyr Thr Gly Ser Ser Gly Ser Thr Phe
   65 70 75 80
   Tyr Ala Ser Trp Ala Lys Gly Arg Phe Thr Ile Ser Lys Thr Ser Ser
   85 90 95
   Thr Thr Val Thr Leu Gln Met Thr Ser Leu Thr Ala Ala Asp Thr Ala
   100 105 110
   Thr Tyr Phe Cys Ala Arg Gly Tyr Ser Gly Phe Gly Tyr Phe Lys Leu
   115 120 125
<210> 284
   <211> 11
   <212> PRT
   <213> Oryctolagus cuniculus
<400> 284
   Gln Ala Ser Gln Asn Ile Tyr Arg Tyr Leu Ala
   1 5 10
<210> 285
   <211> 7
   <212> PRT
   <213> Oryctolagus cuniculus
<400> 285
   Leu Ala Ser Thr Leu Ala Ser
   1 5
<210> 286
   <211> 10
   <212> PRT
   <213> Oryctolagus cuniculus
<400> 286
   Gln Ser Tyr Tyr Ser Ser Asn Ser Val Ala
   1 5 10
<210> 287
   <211> 6
   <212> PRT
   <213> Oryctolagus cuniculus
<400> 287
   Ser Gly Tyr Trp Ile Cys
   1 5
<210> 288
   <211> 18
   <212> PRT
   <213> oryctolagus cuniculus
<400> 288
   Cys Ile Tyr Thr Gly Ser Ser Gly Ser Thr Phe Tyr Ala Ser Trp Ala
   1 5 10 15
   Lys Gly
<210> 289
   <211> 10
   <212> PRT
   <213> oryctolagus cuniculus
<400> 289
   Gly Tyr Ser Gly Phe Gly Tyr Phe Lys Leu
   1 5 10
<210> 290
   <211> 360
   <212> DNA
   <213> Oryctolagus cuniculus
<400> 290
   atggacacga gggcccccac tcagctgctg gggctcctgc tgctctggct cccaggtgcc 60
   agatgtgcat tcgaattgac ccagactcca gcctccgtgg aggcagctgt gggaggcaca 120
   gtcaccatca attgccaggc cagtcagaac atttatagat acttagcctg gtatcagcag 180
   aaaccagggc agcctcccaa gttcctgatc tatctggcat ctactctggc atctggggtc 240
   ccatcgcggt ttaaaggcag tggatctggg acagagttca ctctcaccat cagcgacctg 300
   gagtgtgccg atgctgccac ttactactgt caaagttatt atagtagtaa tagtgtcgct 360
<210> 291
   <211> 384
   <212> DNA
   <213> Oryctolagus cuniculus
<400> 291
   atggagactg ggctgcgctg gcttctcctg gtcgctgtgc tcaaaggtgt ccagtgtcag 60
   gagcagctgg tggagtccgg gggagacctg gtccagcctg agggatccct gacactcacc 120
   tgcacagctt ctgagttaga cttcagtagc ggctactgga tatgctgggt ccgccaggtt 180
   ccagggaagg ggctggagtg gatcggatgc atttatactg gtagtagtgg tagcactttt 240
   tacgcgagtt gggcgaaagg ccgattcacc atctccaaaa cctcgtcgac cacggtgact 300
   ctgcaaatga ccagtctgac agccgcggac acggccacct atttctgtgc gagaggttat 360
   agtggctttg gttactttaa gttg 384
<210> 292
   <211> 33
   <212> DNA
   <213> oryctolagus cuniculus
<400> 292
   caggccagtc agaacattta tagatactta gcc 33
<210> 293
   <211> 21
   <212> DNA
   <213> Oryctolagus cuniculus
<400> 293
   ctggcatcta ctctggcatc t 21
<210> 294
   <211> 30
   <212> DNA
   <213> oryctolagus cuniculus
<400> 294
   caaagttatt atagtagtaa tagtgtcgct 30
<210> 295
   <211> 18
   <212> DNA
   <213> oryctolagus cuniculus
<400> 295
   agcggctact ggatatgc 18
<210> 296
   <211> 54
   <212> DNA
   <213> Oryctolagus cuniculus
<400> 296
   tgcatttata ctggtagtag tggtagcact ttttacgcga gttgggcgaa aggc 54
<210> 297
   <211> 30
   <212> DNA
   <213> Oryctolagus cuniculus
<400> 297
   ggttatagtg gctttggtta ctttaagttg 30
<210> 298
   <211> 122
   <212> PRT
   <213> Oryctolagus cuniculus
<400> 298
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Lys Cys Gln Ala Ser
           35                  40                  45
   Glu Asp Ile Tyr Arg Leu Leu Ala Trp Tyr Gln Gln Lys Pro Gly Gln
       50                  55                  60
   Pro Pro Lys Leu Leu Ile Tyr Asp Ser Ser Asp Leu Ala Ser Gly Val
   65                  70                  75                  80
   Pro Ser Arg Phe Lys Gly Ser Gly Ser Gly Thr Glu Phe Thr Leu Ala
                   85                  90                  95
   Ile Ser Gly Val Gln Cys Asp Asp Ala Ala Thr Tyr Tyr Cys Gln Gln
               100                 105                 110
   Ala Trp Ser Tyr Ser Asp Ile Asp Asn Ala
           115                 120
<210> 299
   <211> 123
   <212> PRT
   <213> Oryctolagus cuniculus
<400> 299
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Ser Tyr Tyr Met Ser Trp Val Arg Gln Ala Pro Gly Lys Gly Leu Glu
       50                  55                  60
   Trp Ile Gly Ile Ile Thr Thr Ser Gly Asn Thr Phe Tyr Ala Ser Trp
   65                  70                  75                  80
   Ala Lys Gly Arg Leu Thr Ile Ser Arg Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Thr Ser Asp Ile Phe Tyr Tyr Arg Asn Leu
           115                 120
<210> 300
   <211> 11
   <212> PRT
   <213> Oryctolagus cuniculus
<400> 300
   Gln Ala Ser Glu Asp Ile Tyr Arg Leu Leu Ala
   1               5                   10
<210> 301
   <211> 7
   <212> PRT
   <213> Oryctolagus cuniculus
<400> 301
   Asp Ser Ser Asp Leu Ala Ser
   1               5
<210> 302
   <211> 12
   <212> PRT
   <213> oryctolagus cuniculus
<400> 302
   Gln Gln Ala Trp Ser Tyr Ser Asp Ile Asp Asn Ala
   1               5                   10
<210> 303
   <211> 5
   <212> PRT
   <213> Oryctolagus cuniculus
<400> 303
   Ser Tyr Tyr Met Ser
   1               5
<210> 304
   <211> 16
   <212> PRT
   <213> Oryctolagus cuniculus
<400> 304
   Ile Ile Thr Thr Ser Gly Asn Thr Phe Tyr Ala Ser Trp Ala Lys Gly
   1               5                   10                  15
<210> 305
   <211> 10
   <212> PRT
   <213> oryctolagus cuniculus
<400> 305
   Thr Ser Asp Ile Phe Tyr Tyr Arg Asn Leu
   1               5                   10
<210> 306
   <211> 366
   <212> DNA
   <213> oryctolagus cuniculus
<400> 306
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cagtgaggac atttataggt tattggcctg gtatcaacag 180
   aaaccagggc agcctcccaa gctcctgatc tatgattcat ccgatctggc atctggggtc 240
   ccatcgcggt tcaaaggcag tggatctggg acagagttca ctctcgccat cagcggtgtg 300
   cagtgtgacg atgctgccac ttactactgt caacaggctt ggagttatag tgatattgat 360
   aatgct 366
<210> 307
   <211> 369
   <212> DNA
   <213> oryctolagus cuniculus
<400> 307
   atggagactg ggctgcgctg gcttctcctg gtcgctgtgc tcaaaggtgt ccagtgtcag 60
   tcggtggagg agtccggggg tcgcctggtc acgccgggga cacccctgac actcacctgc 120
   acagcctctg gattctccct cagtagctac tacatgagct gggtccgcca ggctccaggg 180
   aaggggctgg aatggatcgg aatcattact actagtggta atacatttta cgcgagctgg 240
   gcgaaaggcc ggctcaccat ctccagaacc tcgaccacgg tggatctgaa aatcaccagt 300
   ccgacaaccg aggacacggc cacctatttc tgtgccagaa cttctgatat tttttattat 360
   cgtaacttg 369
<210> 308
   <211> 33
   <212> DNA
   <213> Oryctolagus cuniculus
<400> 308
   caggccagtg aggacattta taggttattg gcc 33
<210> 309
   <211> 21
   <212> DNA
   <213> Oryctolagus cuniculus
<400> 309
   gattcatccg atctggcatc t 21
<210> 310
   <211> 36
   <212> DNA
   <213> Oryctolagus cuniculus
<400> 310
   caacaggctt ggagttatag tgatattgat aatgct 36
<210> 311
   <211> 15
   <212> DNA
   <213> Oryctolagus cuniculus
<400> 311
   agctactaca tgagc 15
<210> 312
   <211> 48
   <212> DNA
   <213> Oryctolagus cuniculus
<400> 312
   atcattacta ctagtggtaa tacattttac gcgagctggg cgaaaggc 48
<210> 313
   <211> 30
   <212> DNA
   <213> Oryctolagus cuniculus
<400> 313
   acttctgata ttttttatta tcgtaacttg 30
<210> 314
   <211> 123
   <212> PRT
   <213> Oryctolagus cuniculus
<400> 314
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Ala Ser Pro
   20 25 30
   Val Ser Ala Ala Val Gly Ala Thr Val Thr Ile Asn Cys Gln Ser Ser
   35 40 45
   Gln Ser Val Tyr Asn Asp Met Asp Leu Ala Trp Phe Gln Gln Lys Pro
   50 55 60
   Gly Gln Pro Pro Lys Leu Leu Ile Tyr Ser Ala Ser Thr Leu Ala Ser
   65 70 75 80
   Gly Val Pro Ser Arg Phe Ser Gly Ser Gly Ser Gly Thr Glu Phe Thr
   85 90 95
   Leu Thr Ile Ser Gly Val Gln Cys Asp Asp Ala Ala Thr Tyr Tyr Cys
   100 105 110
   Leu Gly Ala Phe Asp Asp Asp Ala Asp Asn Thr
   115 120
<210> 315
   <211> 129
   <212> PRT
   <213> Oryctolagus cuniculus
<400> 315
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Thr
           35                  40                  45
   Arg His Ala Ile Thr Trp Val Arg Gln Ala Pro Gly Lys Gly Leu Glu
       50                  55                  60
   Trp Ile Gly Cys Ile Trp Ser Gly Gly Ser Thr Tyr Tyr Ala Thr Trp
   65                  70                  75                  80
   Ala Lys Gly Arg Phe Thr Ile Ser Lys Thr Ser Thr Thr Val Asp Leu
                   85                  90                  95
   Arg Ile Thr Ser Pro Thr Thr Glu Asp Thr Ala Thr Tyr Phe Cys Ala
               100                 105                 110
   Arg Val Ile Gly Asp Thr Ala Gly Tyr Ala Tyr Phe Thr Gly Leu Asp
           115                 120                 125
   Leu
<210> 316
   <211> 13
   <212> PRT
   <213> Oryctolagus cuniculus
<400> 316
   Gln Ser Ser Gln Ser Val Tyr Asn Asp Met Asp Leu Ala
   1               5                   10
<210> 317
   <211> 7
   <212> PRT
   <213> oryctolagus cuniculus
<400> 317
   Ser Ala Ser Thr Leu Ala Ser
   1               5
<210> 318
   <211> 11
   <212> PRT
   <213> oryctolagus cuniculus
<400> 318
   Leu Gly Ala Phe Asp Asp Asp Ala Asp Asn Thr
   1 5 10
<210> 319
   <211> 5
   <212> PRT
   <213> oryctolagus cuniculus
<400> 319
   Arg His Ala Ile Thr
   1 5
<210> 320
   <211> 16
   <212> PRT
   <213> oryctolagus cuniculus
<400> 320
   Cys Ile Trp Ser Gly Gly Ser Thr Tyr Tyr Ala Thr Trp Ala Lys Gly
   1 5 10 15
<210> 321
   <211> 16
   <212> PRT
   <213> oryctolagus cuniculus
<400> 321
   Val Ile Gly Asp Thr Ala Gly Tyr Ala Tyr Phe Thr Gly Leu Asp Leu
   1 5 10 15
<210> 322
   <211> 369
   <212> DNA
   <213> oryctolagus cuniculus
<400> 322
   atggacacga gggcccccac tcagctgctg gggctcctgc tgctctggct cccaggtgcc 60
   acgtttgcag ccgtgctgac ccagactgca tcacccgtgt ctgccgctgt gggagccaca 120
   gtcaccatca actgccagtc cagtcagagt gtttataatg acatggactt agcctggttt 180
   cagcagaaac cagggcagcc tcccaagctc ctgatctatt ctgcatccac tctggcatct 240
   ggggtcccat cgcggttcag cggcagtgga tctgggacag agttcactct caccatcagc 300
   ggcgtgcagt gtgacgatgc tgccacttac tactgtctag gcgcttttga tgatgatgct 360
   gataatact 369
<210> 323
   <211> 387
   <212> DNA
   <213> Oryctolagus cuniculus
<400> 323
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtctctg gattctccct cactaggcat gcaataacct gggtccgcca ggctccaggg 180
   aaggggctgg aatggatcgg atgcatttgg agtggtggta gcacatacta cgcgacctgg 240
   gcgaaaggcc gattcaccat ctccaaaacc tcgaccacgg tggatctcag aatcaccagt 300
   ccgacaaccg aggacacggc cacctacttc tgtgccagag tcattggcga tactgctggt 360
   tatgcttatt ttacggggct tgacttg 387
<210> 324
   <211> 39
   <212> DNA
   <213> Oryctolagus cuniculus
<400> 324
   cagtccagtc agagtgttta taatgacatg gacttagcc 39
<210> 325
   <211> 21
   <212> DNA
   <213> Oryctolagus cuniculus
<400> 325
   tctgcatcca ctctggcatc t 21
<210> 326
   <211> 33
   <212> DNA
   <213> Oryctolagus cuniculus
<400> 326
   ctaggcgctt ttgatgatga tgctgataat act 33
<210> 327
   <211> 15
   <212> DNA
   <213> Oryctolagus cuniculus
<400> 327
   aggcatgcaa taacc 15
<210> 328
   <211> 48
   <212> DNA
   <213> oryctolagus cuniculus
<400> 328
   tgcatttgga gtggtggtag cacatactac gcgacctggg cgaaaggc 48
<210> 329
   <211> 48
   <212> DNA
   <213> oryctolagus cuniculus
<400> 329
   gtcattggcg atactgctgg ttatgcttat tttacggggc ttgacttg 48
<210> 330
   <211> 121
   <212> PRT
   <213> oryctolagus cuniculus
<400> 330
   Met Asp Thr Arg Ala Pro Thr Gln Leu Leu Gly Leu Leu Leu Leu Trp
   1 5 10 15
   Leu Pro Gly Ala Arg Cys Ala Tyr Asp Met Thr Gln Thr Pro Ala Ser
   20 25 30
   Val Glu Val Ala Val Gly Gly Thr Val Thr Ile Lys Cys Gln Ala Ser
   35 40 45
   Gln Ser Val Tyr Asn Trp Leu Ser Trp Tyr Gln Gln Lys Pro Gly Gln
   50 55 60
   Pro Pro Lys Leu Leu Ile Tyr Thr Ala Ser Ser Leu Ala Ser Gly Val
   65 70 75 80
   Pro Ser Arg Phe Ser Gly Ser Gly Ser Gly Thr Glu Phe Thr Leu Thr
   85 90 95
   Ile Ser Gly Val Glu Cys Ala Asp Ala Ala Thr Tyr Tyr Cys Gln Gln
   100 105 110
   Gly Tyr Thr Ser Asp Val Asp Asn Val
   115 120
<210> 331
   <211> 130
   <212> PRT
   <213> Oryctolagus cuniculus
<400> 331
   Met Glu Thr Gly Leu Arg Trp Leu Leu Leu Val Ala Val Leu Lys Gly
   1               5                   10                  15
   Val Gln Cys Gln Ser Leu Glu Glu Ala Gly Gly Arg Leu Val Thr Pro
               20                  25                  30
   Gly Thr Pro Leu Thr Leu Thr Cys Thr Val Ser Gly Ile Asp Leu Ser
           35                  40                  45
   Ser Tyr Ala Met Gly Trp Val Arg Gln Ala Pro Gly Lys Gly Leu Glu
       50                  55                  60
   Tyr Ile Gly Ile Ile Ser Ser Ser Gly Ser Thr Tyr Tyr Ala Thr Trp
   65                  70                  75                  80
   Ala Lys Gly Arg Phe Thr Ile Ser Gln Ala Ser Ser Thr Thr Val Asp
                   85                  90                  95
   Leu Lys Ile Thr Ser Pro Thr Thr Glu Asp Ser Ala Thr Tyr Phe Cys
               100                 105                 110
   Ala Arg Gly Gly Ala Gly Ser Gly Gly Val Trp Leu Leu Asp Gly Phe
           115                 120                 125
   Asp Pro
       130
<210> 332
   <211> 11
   <212> PRT
   <213> Oryctolagus cuniculus
<400> 332
   Gln Ala Ser Gln Ser Val Tyr Asn Trp Leu Ser
   1               5                   10
<210> 333
   <211> 7
   <212> PRT
   <213> Oryctolagus cuniculus
<400> 333
   Thr Ala Ser Ser Leu Ala Ser
   1               5
<210> 334
   <211> 11
   <212> PRT
   <213> Oryctolagus cuniculus
<400> 334
   Gln Gln Gly Tyr Thr Ser Asp Val Asp Asn Val
   1 5 10
<210> 335
   <211> 5
   <212> PRT
   <213> Oryctolagus cuniculus
<400> 335
   Ser Tyr Ala Met Gly
   1 5
<210> 336
   <211> 16
   <212> PRT
   <213> Oryctolagus cuniculus
<400> 336
   Ile Ile Ser Ser Ser Gly Ser Thr Tyr Tyr Ala Thr Trp Ala Lys Gly
   1 5 10 15
<210> 337
   <211> 16
   <212> PRT
   <213> oryctolagus cuniculus
<400> 337
   Gly Gly Ala Gly Ser Gly Gly Val Trp Leu Leu Asp Gly Phe Asp Pro
   1 5 10 15
<210> 338
   <211> 363
   <212> DNA
   <213> oryctolagus cuniculus
<400> 338
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cagtcagagt gtttataatt ggttatcctg gtatcagcag 180
   aaaccagggc agcctcccaa gctcctgatc tatactgcat ccagtctggc atctggggtc 240
   ccatcgcggt tcagtggcag tggatctggg acagagttca ctctcaccat cagcggcgtg 300
   gagtgtgccg atgctgccac ttactactgt caacagggtt atactagtga tgttgataat 360
   gtt 363
<210> 339
   <211> 390
   <212> DNA
   <213> Oryctolagus cuniculus
<400> 339
   atggagactg ggctgcgctg gcttctcctg gtcgctgtgc tcaaaggtgt ccagtgtcag 60
   tcgctggagg aggccggggg tcgcctggtc acgcctggga cacccctgac actcacctgc 120
   acagtctctg gaatcgacct cagtagctat gcaatgggct gggtccgcca ggctccaggg 180
   aaggggctgg aatacatcgg aatcattagt agtagtggta gcacatacta cgcgacctgg 240
   gcgaaaggcc gattcaccat ctcacaagcc tcgtcgacca cggtggatct gaaaattacc 300
   agtccgacaa ccgaggactc ggccacatat ttctgtgcca gagggggtgc tggtagtggt 360
   ggtgtttggc tgcttgatgg ttttgatccc 390
<210> 340
   <211> 33
   <212> DNA
   <213> Oryctolagus cuniculus
<400> 340
   caggccagtc agagtgttta taattggtta tcc 33
<210> 341
   <211> 21
   <212> DNA
   <213> Oryctolagus cuniculus
<400> 341
   actgcatcca gtctggcatc t 21
<210> 342
   <211> 33
   <212> DNA
   <213> Oryctolagus cuniculus
<400> 342
   caacagggtt atactagtga tgttgataat gtt 33
<210> 343
   <211> 15
   <212> DNA
   <213> Oryctolagus cuniculus
<400> 343
   agctatgcaa tgggc 15
<210> 344
   <211> 48
   <212> DNA
   <213> Oryctolagus cuniculus
<400> 344
   atcattagta gtagtggtag cacatactac gcgacctggg cgaaaggc 48
<210> 345
   <211> 48
   <212> DNA
   <213> oryctolagus cuniculus
<400> 345
   gggggtgctg gtagtggtgg tgtttggctg cttgatggtt ttgatccc 48
<210> 346
   <211> 123
   <212> PRT
   <213> oryctolagus cuniculus
<400> 346
   Met Asp Thr Arg Ala Pro Thr Gln Leu Leu Gly Leu Leu Leu Leu Trp
   1               5                   10                  15
   Leu Pro Gly Ala Lys Cys Ala Asp Val Val Met Thr Gln Thr Pro Ala
               20                  25                  30
   Ser Val Ser Ala Ala Val Gly Gly Thr Val Thr Ile Asn Cys Gln Ala
           35                  40                  45
   Ser Glu Asn Ile Tyr Asn Trp Leu Ala Trp Tyr Gln Gln Lys Pro Gly
       50                  55                  60
   Gln Pro Pro Lys Leu Leu Ile Tyr Thr Val Gly Asp Leu Ala Ser Gly
   65                  70                  75                  80
   Val Ser Ser Arg Phe Lys Gly Ser Gly Ser Gly Thr Glu Phe Thr Leu
                   85                  90                  95
   Thr Ile Ser Asp Leu Glu Cys Ala Asp Ala Ala Thr Tyr Tyr Cys Gln
               100                 105                 110
   Gln Gly Tyr Ser Ser Ser Tyr Val Asp Asn Val
           115                 120
<210> 347
   <211> 130
   <212> PRT
   <213> Oryctolagus cuniculus
<400> 347
   Met Glu Thr Gly Leu Arg Trp Leu Leu Leu Val Ala Val Leu Lys Gly
   1 5 10 15
   Val Gln Cys Gln Glu Gln Leu Lys Glu Ser Gly Gly Arg Leu Val Thr
   20 25 30
   Pro Gly Thr Pro Leu Thr Leu Thr Cys Thr Val Ser Gly Phe Ser Leu
   35 40 45
   Asn Asp Tyr Ala Val Gly Trp Phe Arg Gln Ala Pro Gly Lys Gly Leu
   50 55 60
   Glu Trp Ile Gly Tyr Ile Arg Ser Ser Gly Thr Thr Ala Tyr Ala Thr
   65 70 75 80
   Trp Ala Lys Gly Arg Phe Thr Ile Ser Ala Thr Ser Thr Thr Val Asp
   85 90 95
   Leu Lys Ile Thr Ser Pro Thr Thr Glu Asp Thr Ala Thr Tyr Phe Cys
   100 105 110
   Ala Arg Gly Gly Ala Gly Ser Ser Gly Val Trp Ile Leu Asp Gly Phe
   115 120 125
   Ala Pro
   130
<210> 348
   <211> 11
   <212> PRT
   <213> Oryctolagus cuniculus
<400> 348
   Gln Ala Ser Glu Asn Ile Tyr Asn Trp Leu Ala
   1 5 10
<210> 349
   <211> 7
   <212> PRT
   <213> Oryctolagus cuniculus
<400> 349
   Thr Val Gly Asp Leu Ala Ser
   1 5
<210> 350
   <211> 12
   <212> PRT
   <213> oryctolagus cuniculus
<400> 350
   Gln Gln Gly Tyr Ser Ser Ser Tyr Val Asp Asn Val
   1 5 10
<210> 351
   <211> 5
   <212> PRT
   <213> oryctolagus cuniculus
<400> 351
   Asp Tyr Ala Val Gly
   1 5
<210> 352
   <211> 16
   <212> PRT
   <213> Oryctolagus cuniculus
<400> 352
   Tyr Ile Arg Ser Ser Gly Thr Thr Ala Tyr Ala Thr Trp Ala Lys Gly
   1 5 10 15
<210> 353
   <211> 16
   <212> PRT
   <213> oryctolagus cuniculus
<400> 353
   Gly Gly Ala Gly Ser Ser Gly Val Trp Ile Leu Asp Gly Phe Ala Pro
   1 5 10 15
<210> 354
   <211> 369
   <212> DNA
   <213> Oryctolagus cuniculus
<400> 354
   atggacacga gggcccccac tcagctgctg gggctcctgc tgctctggct cccaggtgcc 60
   aaatgtgccg atgttgtgat gacccagact ccagcctccg tgtctgcagc tgtgggaggc 120
   acagtcacca tcaattgcca ggccagtgag aacatttata attggttagc ctggtatcag 180
   cagaaaccag ggcagcctcc caagctcctg atctatactg taggcgatct ggcatctggg 240
   gtctcatcgc ggttcaaagg cagtggatct gggacagagt tcactctcac catcagcgac 300
   ctggagtgtg ccgatgctgc cacttactat tgtcaacagg gttatagtag tagttatgtt 360
   gataatgtt 369
<210> 355
   <211> 390
   <212> DNA
   <213> Oryctolagus cuniculus
<400> 355
   atggagactg ggctgcgctg gcttctcctg gtcgctgtgc tcaaaggtgt ccagtgtcag 60
   gagcagctga aggagtccgg gggtcgcctg gtcacgcctg ggacacccct gacactcacc 120
   tgcacagtct ctggattctc cctcaatgac tatgcagtgg gctggttccg ccaggctcca 180
   gggaaggggc tggaatggat cggatacatt cgtagtagtg gtaccacagc ctacgcgacc 240
   tgggcgaaag gccgattcac catctccgct acctcgacca cggtggatct gaaaatcacc 300
   agtccgacaa ccgaggacac ggccacctat ttctgtgcca gagggggtgc tggtagtagt 360
   ggtgtgtgga tccttgatgg ttttgctccc 390
<210> 356
   <211> 33
   <212> DNA
   <213> Oryctolagus cuniculus
<400> 356
   caggccagtg agaacattta taattggtta gcc 33
<210> 357
   <211> 21
   <212> DNA
   <213> Oryctolagus cuniculus
<400> 357
   actgtaggcg atctggcatc t 21
<210> 358
   <211> 36
   <212> DNA
   <213> oryctolagus cuniculus
<400> 358
   caacagggtt atagtagtag ttatgttgat aatgtt 36
<210> 359
   <211> 15
   <212> DNA
   <213> Oryctolagus cuniculus
<400> 359
   gactatgcag tgggc 15
<210> 360
   <211> 48
   <212> DNA
   <213> Oryctolagus cuniculus
<400> 360
   tacattcgta gtagtggtac cacagcctac gcgacctggg cgaaaggc 48
<210> 361
   <211> 48
   <212> DNA
   <213> Oryctolagus cuniculus
<400> 361
   gggggtgctg gtagtagtgg tgtgtggatc cttgatggtt ttgctccc 48
<210> 362
   <211> 121
   <212> PRT
   <213> oryctolagus cuniculus
<400> 362
   Met Asp Thr Arg Ala Pro Thr Gln Leu Leu Gly Leu Leu Leu Leu Trp
   1 5 10 15
   Leu Pro Gly Ala Thr Phe Ala Gln Val Leu Thr Gln Thr Pro Ser Ser
   20 25 30
   Val Ser Ala Ala Val Gly Gly Thr Val Thr Ile Asn Cys Gln Ala Ser
   35 40 45
   Gln Ser Val Tyr Gln Asn Asn Tyr Leu Ser Trp Phe Gln Gln Lys Pro
   50 55 60
   Gly Gln Pro Pro Lys Leu Leu Ile Tyr Gly Ala Ala Thr Leu Ala Ser
   65 70 75 80
   Gly Val Pro Ser Arg Phe Lys Gly Ser Gly Ser Gly Thr Gln Phe Thr
   85 90 95
   Leu Thr Ile Ser Asp Leu Glu Cys Asp Asp Ala Ala Thr Tyr Tyr Cys
   100 105 110
   Ala Gly Ala Tyr Arg Asp Val Asp Ser
   115 120
<210> 363
   <211> 130
   <212> PRT
   <213> Oryctolagus cuniculus
<400> 363
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Gly Ala Ser Leu Thr Leu Thr Cys Thr Ala Ser Gly Phe Ser Phe Thr
           35                  40                  45
   Ser Thr Tyr Tyr Ile Tyr Trp Val Arg Gln Ala Pro Gly Lys Gly Leu
       50                  55                  60
   Glu Trp Ile Ala Cys Ile Asp Ala Gly Ser Ser Gly Ser Thr Tyr Tyr
   65                  70                  75                  80
   Ala Thr Trp Val Asn Gly Arg Phe Thr Ile Ser Lys Thr Ser Ser Thr
                   85                  90                  95
   Thr Val Thr Leu Gln Met Thr Ser Leu Thr Ala Ala Asp Thr Ala Thr
               100                 105                 110
   Tyr Phe Cys Ala Lys Trp Asp Tyr Gly Gly Asn Val Gly Trp Gly Tyr
           115                 120                 125
   Asp Leu
       130
<210> 364
   <211> 13
   <212> PRT
   <213> Oryctolagus cuniculus
<400> 364
   Gln Ala Ser Gln Ser Val Tyr Gln Asn Asn Tyr Leu Ser
   1               5                   10
<210> 365
   <211> 7
   <212> PRT
   <213> Oryctolagus cuniculus
<400> 365
   Gly Ala Ala Thr Leu Ala Ser
   1 5
<210> 366
   <211> 9
   <212> PRT
   <213> Oryctolagus cuniculus
<400> 366
   Ala Gly Ala Tyr Arg Asp Val Asp Ser
   1 5
<210> 367
   <211> 6
   <212> PRT
   <213> Oryctolagus cuniculus
<400> 367
   Ser Thr Tyr Tyr Ile Tyr
   1 5
<210> 368
   <211> 18
   <212> PRT
   <213> Oryctolagus cuniculus
<400> 368
   Cys Ile Asp Ala Gly Ser Ser Gly Ser Thr Tyr Tyr Ala Thr Trp Val
   1 5 10 15
   Asn Gly
<210> 369
   <211> 13
   <212> PRT
   <213> oryctolagus cuniculus
<400> 369
   Trp Asp Tyr Gly Gly Asn Val Gly Trp Gly Tyr Asp Leu
   1 5 10
<210> 370
   <211> 363
   <212> DNA
   <213> Oryctolagus cuniculus
<400> 370
   atggacacga gggcccccac tcagctgctg gggctcctgc tgctctggct cccaggtgcc 60
   acatttgctc aagtgctgac ccagactcca tcctccgtgt ctgcagctgt gggaggcaca 120
   gtcaccatca attgccaggc cagtcagagt gtttatcaga acaactactt atcctggttt 180
   cagcagaaac cagggcagcc tcccaagctc ctgatctatg gtgcggccac tctggcatct 240
   ggggtcccat cgcggttcaa aggcagtgga tctgggacac agttcactct caccatcagc 300
   gacctggagt gtgacgatgc tgccacttac tactgtgcag gcgcttatag ggatgtggat 360
   tct 363
<210> 371
   <211> 390
   <212> DNA
   <213> Oryctolagus cuniculus
<400> 371
   atggagactg ggctgcgctg gcttctcctg gtcgctgtgc tcaaaggtgt ccagtgtcag 60
   tcgttggagg agtccggggg agacctggtc aagcctgggg catccctgac actcacctgc 120
   acagcctctg gattctcctt tactagtacc tactacatct actgggtccg ccaggctcca 180
   gggaaggggc tggagtggat cgcatgtatt gatgctggta gtagtggtag cacttactac 240
   gcgacctggg tgaatggccg attcaccatc tccaaaacct cgtcgaccac ggtgactctg 300
   caaatgacca gtctgacagc cgcggacacg gccacctatt tctgtgcgaa atgggattat 360
   ggtggtaatg ttggttgggg ttatgacttg 390
<210> 372
   <211> 39
   <212> DNA
   <213> oryctolagus cuniculus
<400> 372
   caggccagtc agagtgttta tcagaacaac tacttatcc 39
<210> 373
   <211> 21
   <212> DNA
   <213> oryctolagus cuniculus
<400> 373
   ggtgcggcca ctctggcatc t 21
<210> 374
   <211> 27
   <212> DNA
   <213> Oryctolagus cuniculus
<400> 374
   gcaggcgctt atagggatgt ggattct 27
<210> 375
   <211> 18
   <212> DNA
   <213> Oryctolagus cuniculus
<400> 375
   agtacctact acatctac 18
<210> 376
   <211> 54
   <212> DNA
   <213> Oryctolagus cuniculus
<400> 376
   tgtattgatg ctggtagtag tggtagcact tactacgcga cctgggtgaa tggc 54
<210> 377
   <211> 39
   <212> DNA
   <213> oryctolagus cuniculus
<400> 377
   tgggattatg gtggtaatgt tggttggggt tatgacttg 39
<210> 378
   <211> 120
   <212> PRT
   <213> Oryctolagus cuniculus
<400> 378
   Met Asp Thr Arg Ala Pro Thr Gln Leu Leu Gly Leu Leu Leu Leu Trp
   1               5                   10                  15
   Leu Pro Gly Ala Arg Cys Ala Phe Glu Leu Thr Gln Thr Pro Ser Ser
               20                  25                  30
   Val Glu Ala Ala Val Gly Gly Thr Val Thr Ile Lys Cys Gln Ala Ser
           35                  40                  45
   Gln Ser Ile Ser Ser Tyr Leu Ala Trp Tyr Gln Gln Lys Pro Gly Gln
       50                  55                  60
   Pro Pro Lys Phe Leu Ile Tyr Arg Ala Ser Thr Leu Ala Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Ser
               100                 105                 110
   Tyr Tyr Asp Ser Val Ser Asn Pro
           115                 120
<210> 379
   <211> 127
   <212> PRT
   <213> Oryctolagus cuniculus
<400> 379
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Asp Leu Val Lys Pro
   20 25 30
   Glu Gly Ser Leu Thr Leu Thr Cys Lys Ala Ser Gly Leu Asp Leu Gly
   35 40 45
   Thr Tyr Trp Phe Met Cys Trp Val Arg Gln Ala Pro Gly Lys Gly Leu
   50 55 60
   Glu Trp Ile Ala Cys Ile Tyr Thr Gly Ser Ser Gly Ser Thr Phe Tyr
   65 70 75 80
   Ala Ser Trp Val Asn Gly Arg Phe Thr Ile Ser Lys Thr Ser Ser Thr
   85 90 95
   Thr Val Thr Leu Gln Met Thr Ser Leu Thr Ala Ala Asp Thr Ala Thr
   100 105 110
   Tyr Phe Cys Ala Arg Gly Tyr Ser Gly Tyr Gly Tyr Phe Lys Leu
   115 120 125
<210> 380
   <211> 11
   <212> PRT
   <213> oryctolagus cuniculus
<400> 380
   Gln Ala Ser Gln Ser Ile Ser Ser Tyr Leu Ala
   1 5 10
<210> 381
   <211> 7
   <212> PRT
   <213> Oryctolagus cuniculus
<400> 381
   Arg Ala Ser Thr Leu Ala Ser
   1 5
<210> 382
   <211> 10
   <212> PRT
   <213> Oryctolagus cuniculus
<400> 382
   Gln Ser Tyr Tyr Asp Ser Val Ser Asn Pro
   1 5 10
<210> 383
   <211> 6
   <212> PRT
   <213> Oryctolagus cuniculus
<400> 383
   Thr Tyr Trp Phe Met Cys
   1 5
<210> 384
   <211> 18
   <212> PRT
   <213> Oryctolagus cuniculus
<400> 384
   Cys Ile Tyr Thr Gly Ser Ser Gly Ser Thr Phe Tyr Ala Ser Trp Val
   1 5 10 15
   Asn Gly
<210> 385
   <211> 10
   <212> PRT
   <213> Oryctolagus cuniculus
<400> 385
   Gly Tyr Ser Gly Tyr Gly Tyr Phe Lys Leu
   1 5 10
<210> 386
   <211> 360
   <212> DNA
   <213> Oryctolagus cuniculus
<400> 386
   atggacacga gggcccccac tcagctgctg gggctcctgc tgctctggct cccaggtgcc 60
   agatgtgcat tcgaattgac ccagactcca tcctccgtgg aggcagctgt gggaggcaca 120
   gtcaccatca agtgccaggc cagtcagagc attagtagtt acttagcctg gtatcagcag 180
   aaaccagggc agcctcccaa gttcctgatc tacagggcgt ccactctggc atctggggtc 240
   ccatcgcgat tcaaaggcag tggatctggg acagagttca ctctcaccat cagcgacctg 300
   gagtgtgccg atgctgccac ttactactgt caaagctatt atgatagtgt ttcaaatcct 360
<210> 387
   <211> 381
   <212> DNA
   <213> Oryctolagus cuniculus
<400> 387
   atggagactg ggctgcgctg gcttctcctg gtcgctgtgc tcaaaggtgt ccagtgtcag 60
   tcgttggagg agtccggggg agacctggtc aagcctgagg gatccctgac actcacctgc 120
   aaagcctctg gactcgacct cggtacctac tggttcatgt gctgggtccg ccaggctcca 180
   gggaaggggc tggagtggat cgcttgtatt tatactggta gtagtggttc cactttctac 240
   gcgagctggg tgaatggccg attcaccatc tccaaaacct cgtcgaccac ggtgactctg 300
   caaatgacca gtctgacagc cgcggacacg gccacttatt tttgtgcgag aggttatagt 360
   ggttatggtt attttaagtt g 381
<210> 388
   <211> 33
   <212> DNA
   <213> oryctolagus cuniculus
<400> 388
   caggccagtc agagcattag tagttactta gcc 33
<210> 389
   <211> 21
   <212> DNA
   <213> oryctolagus cuniculus
<400> 389
   agggcgtcca ctctggcatc t 21
<210> 390
   <211> 30
   <212> DNA
   <213> oryctolagus cuniculus
<400> 390
   caaagctatt atgatagtgt ttcaaatcct 30
<210> 391
   <211> 18
   <212> DNA
   <213> Oryctolagus cuniculus
<400> 391
   acctactggt tcatgtgc 18
<210> 392
   <211> 54
   <212> DNA
   <213> Oryctolagus cuniculus
<400> 392
   tgtatttata ctggtagtag tggttccact ttctacgcga gctgggtgaa tggc 54
<210> 393
   <211> 30
   <212> DNA
   <213> Oryctolagus cuniculus
<400> 393
   ggttatagtg gttatggtta ttttaagttg 30
<210> 394
   <211> 124
   <212> PRT
   <213> oryctolagus cuniculus
<400> 394
   Met Asp Thr Arg Ala Pro Thr Gln Leu Leu Gly Leu Leu Leu Leu Trp
   1               5                   10                  15
   Leu Pro Gly Val Thr Phe Ala Ile Glu Met Thr Gln Ser Pro Phe Ser
               20                  25                  30
   Val Ser Ala Ala Val Gly Gly Thr Val Ser Ile Ser Cys Gln Ala Ser
           35                  40                  45
   Gln Ser Val Tyr Lys Asn Asn Gln Leu Ser Trp Tyr Gln Gln Lys Ser
       50                  55                  60
   Gly Gln Pro Pro Lys Leu Leu Ile Tyr Gly Ala Ser Ala Leu Ala Ser
   65                  70                  75                  80
   Gly Val Pro Ser Arg Phe Lys Gly Ser Gly Ser Gly Thr Glu Phe Thr
                   85                  90                  95
   Leu Thr Ile Ser Asp Val Gln Cys Asp Asp Ala Ala Thr Tyr Tyr Cys
               100                 105                 110
   Ala Gly Ala Ile Thr Gly Ser Ile Asp Thr Asp Gly
           115                 120
<210> 395
   <211> 130
   <212> PRT
   <213> Oryctolagus cuniculus
<400> 395
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Gly Ala Ser Leu Thr Leu Thr Cys Thr Thr Ser Gly Phe Ser Phe Ser
           35                  40                  45
   Ser Ser Tyr Phe Ile Cys Trp Val Arg Gln Ala Pro Gly Lys Gly Leu
       50                  55                  60
   Glu Trp Ile Ala Cys Ile Tyr Gly Gly Asp Gly Ser Thr Tyr Tyr Ala
   65                  70                  75                  80
   Ser Trp Ala Lys Gly Arg Phe Thr Ile Ser Lys Thr Ser Ser Thr Thr
                   85                  90                  95
   Val Thr Leu Gln Met Thr Ser Leu Thr Ala Ala Asp Thr Ala Thr Tyr
               100                 105                 110
   Phe Cys Ala Arg Glu Trp Ala Tyr Ser Gln Gly Tyr Phe Gly Ala Phe
           115                 120                 125
   Asp Leu
       130
<210> 396
   <211> 13
   <212> PRT
   <213> oryctolagus cuniculus
<400> 396
   Gln Ala Ser Gln Ser Val Tyr Lys Asn Asn Gln Leu Ser
   1               5                   10
<210> 397
   <211> 7
   <212> PRT
   <213> oryctolagus cuniculus
<400> 397
   Gly Ala Ser Ala Leu Ala Ser
   1               5
<210> 398
   <211> 12
   <212> PRT
   <213> Oryctolagus cuniculus
<400> 398
   Ala Gly Ala Ile Thr Gly Ser Ile Asp Thr Asp Gly
   1 5 10
<210> 399
   <211> 6
   <212> PRT
   <213> oryctolagus cuniculus
<400> 399
   Ser Ser Tyr Phe Ile Cys
   1 5
<210> 400
   <211> 17
   <212> PRT
   <213> Oryctolagus cuniculus
<400> 400
   Cys Ile Tyr Gly Gly Asp Gly Ser Thr Tyr Tyr Ala Ser Trp Ala Lys
   1 5 10 15
   Gly
<210> 401
   <211> 14
   <212> PRT
   <213> Oryctolagus cuniculus
<400> 401
   Glu Trp Ala Tyr Ser Gln Gly Tyr Phe Gly Ala Phe Asp Leu
   1 5 10
<210> 402
   <211> 372
   <212> DNA
   <213> Oryctolagus cuniculus
<400> 402
   atggacacga gggcccccac tcagctgctg gggctcctgc tgctctggct cccaggtgtc 60
   acatttgcca tcgaaatgac ccagagtcca ttctccgtgt ctgcagctgt gggaggcaca 120
   gtcagcatca gttgccaggc cagtcagagt gtttataaga acaaccaatt atcctggtat 180
   cagcagaaat cagggcagcc tcccaagctc ctgatctatg gtgcatcggc tctggcatct 240
   ggggtcccat cgcggttcaa aggcagtgga tctgggacag agttcactct caccatcagc 300
   gacgtgcagt gtgacgatgc tgccacttac tactgtgcag gcgctattac tggtagtatt 360
   gatacggatg gt 372
<210> 403
   <211> 390
   **<212>** DNA
   <213> Oryctolagus cuniculus
<400> 403
   atggagactg ggctgcgctg gcttctcctg gtcgctgtgc tcaaaggtgt ccagtgtcag 60
   tcgttggagg agtccggggg agacctggtc aagcctgggg catccctgac actcacctgc 120
   acaacttctg gattctcctt cagtagcagc tacttcattt gctgggtccg ccaggctcca 180
   gggaaggggc tggagtggat cgcatgcatt tatggtggtg atggcagcac atactacgcg 240
   agctgggcga aaggccgatt caccatctcc aaaacctcgt cgaccacggt gacgctgcaa 300
   atgaccagtc tgacagccgc ggacacggcc acctatttct gtgcgagaga atgggcatat 360
   agtcaaggtt attttggtgc ttttgatctc 390
<210> 404
   <211> 39
   <212> DNA
   <213> Oryctolagus cuniculus
<400> 404
   caggccagtc agagtgttta taagaacaac caattatcc 39
<210> 405
   <211> 21
   <212> DNA
   <213> Oryctolagus cuniculus
<400> 405
   ggtgcatcgg ctctggcatc t 21
<210> 406
   <211> 36
   <212> DNA
   <213> Oryctolagus cuniculus
<400> 406
   gcaggcgcta ttactggtag tattgatacg gatggt 36
<210> 407
   <211> 18
   <212> DNA
   <213> oryctolagus cuniculus
<400> 407
   agcagctact tcatttgc 18
<210> 408
   <211> 51
   <212> DNA
   <213> Oryctolagus cuniculus
<400> 408
   tgcatttatg gtggtgatgg cagcacatac tacgcgagct gggcgaaagg c 51
<210> 409
   <211> 42
   <212> DNA
   <213> Oryctolagus cuniculus
<400> 409
   gaatgggcat atagtcaagg ttattttggt gcttttgatc tc 42
<210> 410
   <211> 124
   <212> PRT
   <213> Oryctolagus cuniculus
<400> 410
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Glu Asp Ile Ser Ser Tyr Leu Ala Trp Tyr Gln Gln Lys Pro Gly Gln
       50                  55                  60
   Pro Pro Lys Leu Leu Ile Tyr Ala Ala Ser Asn Leu Glu Ser Gly Val
   65                  70                  75                  80
   Ser Ser Arg Phe Lys Gly Ser Gly Ser Gly Thr Glu Tyr Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Thr Ile Ser Ile Ser Asp Gly Asn Ala
           115                 120
<210> 411
   <211> 124
   <212> PRT
   <213> Oryctolagus cuniculus
<400> 411
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Ser Tyr Phe Met Thr Trp Val Arg Gln Ala Pro Gly Glu Gly Leu Glu
   50 55 60
   Tyr Ile Gly Phe Ile Asn Pro Gly Gly Ser Ala Tyr Tyr Ala Ser Trp
   65 70 75 80
   Val Lys Gly Arg Phe Thr Ile Ser Lys Ser Ser Thr Thr Val Asp Leu
   85 90 95
   Lys Ile Thr Ser Pro Thr Thr Glu Asp Thr Ala Thr Tyr Phe Cys Ala
   100 105 110
   Arg Val Leu Ile Val Ser Tyr Gly Ala Phe Thr Ile
   115 120
<210> 412
   <211> 11
   <212> PRT
   <213> oryctolagus cuniculus
<400> 412
   Gln Ala Ser Glu Asp Ile Ser Ser Tyr Leu Ala
   1 5 10
<210> 413
   <211> 7
   <212> PRT
   <213> Oryctolagus cuniculus
<400> 413
   Ala Ala Ser Asn Leu Glu Ser
   1 5
<210> 414
   <211> 14
   <212> PRT
   <213> Oryctolagus cuniculus
<400> 414
   Gln Cys Thr Tyr Gly Thr Ile Ser Ile Ser Asp Gly Asn Ala
   1               5                   10
<210> 415
   <211> 5
   <212> PRT
   <213> Oryctolagus cuniculus
<400> 415
   Ser Tyr Phe Met Thr
   1               5
<210> 416
   <211> 16
   <212> PRT
   <213> Oryctolagus cuniculus
<400> 416
   Phe Ile Asn Pro Gly Gly Ser Ala Tyr Tyr Ala Ser Trp Val Lys Gly
   1               5                   10                  15
<210> 417
   <211> 11
   <212> PRT
   <213> Oryctolagus cuniculus
<400> 417
   Val Leu Ile Val Ser Tyr Gly Ala Phe Thr Ile
   1               5                   10
<210> 418
   <211> 372
   <212> DNA
   <213> Oryctolagus cuniculus
<400> 418
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtgaggat attagtagct acttagcctg gtatcagcag 180
   aaaccagggc agcctcccaa gctcctgatc tatgctgcat ccaatctgga atctggggtc 240
   tcatcgcgat tcaaaggcag tggatctggg acagagtaca ctctcaccat cagcgacctg 300
   gagtgtgccg atgctgccac ctattactgt caatgtactt atggtactat ttctattagt 360
   gatggtaatg ct 372
<210> 419
   <211> 372
   <212> DNA
   <213> Oryctolagus cuniculus
<400> 419
   atggagactg ggctgcgctg gcttctcctg gtcgctgtgc tcaaaggtgt ccaatgtcag 60
   tcggtggagg agtccggggg tcgcctggtc acgcctggga cacccctgac actcacctgc 120
   acagtctctg gattctccct cagtagctac ttcatgacct gggtccgcca ggctccaggg 180
   gaggggctgg aatacatcgg attcattaat cctggtggta gcgcttacta cgcgagctgg 240
   gtgaaaggcc gattcaccat ctccaagtcc tcgaccacgg tagatctgaa aatcaccagt 300
   ccgacaaccg aggacacggc cacctatttc tgtgccaggg ttctgattgt ttcttatgga 360
   gcctttacca tc 372
<210> 420
   <211> 33
   <212> DNA
   <213> Oryctolagus cuniculus
<400> 420
   caggccagtg aggatattag tagctactta gcc 33
<210> 421
   <211> 21
   <212> DNA
   <213> Oryctolagus cuniculus
<400> 421
   gctgcatcca atctggaatc t 21
<210> 422
   <211> 42
   <212> DNA
   <213> Oryctolagus cuniculus
<400> 422
   caatgtactt atggtactat ttctattagt gatggtaatg ct 42
<210> 423
   <211> 15
   <212> DNA
   <213> Oryctolagus cuniculus
<400> 423
   agctacttca tgacc 15
<210> 424
   <211> 48
   <212> DNA
   <213> oryctolagus cuniculus
<400> 424
   ttcattaatc ctggtggtag cgcttactac gcgagctggg tgaaaggc 48
<210> 425
   <211> 33
   <212> DNA
   <213> Oryctolagus cuniculus
<400> 425
   gttctgattg tttcttatgg agcctttacc atc 33
<210> 426
   <211> 124
   <212> PRT
   <213> Oryctolagus cuniculus
<400> 426
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Ser Ala Ala Val Gly Gly Thr Val Thr Ile Lys Cys Gln Ala Ser
           35                  40                  45
   Glu Asp Ile Glu Ser Tyr Leu Ala Trp Tyr Gln Gln Lys Pro Gly Gln
       50                  55                  60
   Pro Pro Lys Leu Leu Ile Tyr Gly Ala Ser Asn Leu Glu Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Ile Ile Ser Ile Ser Asp Gly Asn Ala
           115                 120
<210> 427
   <211> 124
   <212> PRT
   <213> oryctolagus cuniculus
<400> 427
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Ser Tyr Phe Met Thr Trp Val Arg Gln Ala Pro Gly Glu Gly Leu Glu
   50 55 60
   Tyr Ile Gly Phe Met Asn Thr Gly Asp Asn Ala Tyr Tyr Ala Ser Trp
   65 70 75 80
   Ala Lys Gly Arg Phe Thr Ile Ser Lys Thr Ser Thr Thr Val Asp Leu
   85 90 95
   Lys Ile Thr Ser Pro Thr Thr Glu Asp Thr Ala Thr Tyr Phe Cys Ala
   100 105 110
   Arg Val Leu Val Val Ala Tyr Gly Ala Phe Asn Ile
   115 120
<210> 428
   <211> 11
   <212> PRT
   <213> Oryctolagus cuniculus
<400> 428
   Gln Ala Ser Glu Asp Ile Glu Ser Tyr Leu Ala
   1 5 10
<210> 429
   <211> 7
   <212> PRT
   <213> oryctolagus cuniculus
<400> 429
   Gly Ala Ser Asn Leu Glu Ser
   1 5
<210> 430
   <211> 14
   <212> PRT
   <213> Oryctolagus cuniculus
<400> 430
   Gln Cys Thr Tyr Gly Ile Ile Ser Ile Ser Asp Gly Asn Ala
   1 5 10
<210> 431
   <211> 5
   <212> PRT
   <213> Oryctolagus cuniculus
<400> 431
   Ser Tyr Phe Met Thr
   1 5
<210> 432
   <211> 16
   <212> PRT
   <213> oryctolagus cuniculus
<400> 432
   Phe Met Asn Thr Gly Asp Asn Ala Tyr Tyr Ala Ser Trp Ala Lys Gly
   1 5 10 15
<210> 433
   <211> 11
   <212> PRT
   <213> oryctolagus cuniculus
<400> 433
   Val Leu Val Val Ala Tyr Gly Ala Phe Asn Ile
   1 5 10
<210> 434
   <211> 372
   <212> DNA
   <213> oryctolagus cuniculus
<400> 434
   atggacacga gggcccccac tcagctgctg gggctcctgc tgctctggct cccaggtgcc 60
   agatgtgatg ttgtgatgac ccagactcca gcctccgtgt ctgcagctgt gggaggcaca 120
   gtcaccatca agtgccaggc cagtgaggac attgaaagct atctagcctg gtatcagcag 180
   aaaccagggc agcctcccaa gctcctgatc tatggtgcat ccaatctgga atctggggtc 240
   tcatcgcggt tcaaaggcag tggatctggg acagagttca ctctcaccat cagcgacctg 300
   gagtgtgccg atgctgccac ttactattgt caatgcactt atggtattat tagtattagt 360
   gatggtaatg ct 372
<210> 435
   <211> 372
   <212> DNA
   <213> oryctolagus cuniculus
<400> 435
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtgtctg gattctccct cagtagctac ttcatgacct gggtccgcca ggctccaggg 180
   gaggggctgg aatacatcgg attcatgaat actggtgata acgcatacta cgcgagctgg 240
   gcgaaaggcc gattcaccat ctccaaaacc tcgaccacgg tggatctgaa aatcaccagt 300
   ccgacaaccg aggacacggc cacctatttc tgtgccaggg ttcttgttgt tgcttatgga 360
   gcctttaaca tc 372
<210> 436
   <211> 33
   <212> DNA
   <213> oryctolagus cuniculus
<400> 436
   caggccagtg aggacattga aagctatcta gcc 33
<210> 437
   <211> 21
   <212> DNA
   <213> oryctolagus cuniculus
<400> 437
   ggtgcatcca atctggaatc t 21
<210> 438
   <211> 42
   <212> DNA
   <213> oryctolagus cuniculus
<400> 438
   caatgcactt atggtattat tagtattagt gatggtaatg ct 42
<210> 439
   <211> 15
   <212> DNA
   <213> Oryctolagus cuniculus
<400> 439
   agctacttca tgacc 15
<210> 440
   <211> 48
   <212> DNA
   <213> Oryctolagus cuniculus
<400> 440
   ttcatgaata ctggtgataa cgcatactac gcgagctggg cgaaaggc 48
<210> 441
   <211> 33
   <212> DNA
   <213> oryctolagus cuniculus
<400> 441
   gttcttgttg ttgcttatgg agcctttaac atc 33
<210> 442
   <211> 124
   <212> PRT
   <213> oryctolagus cuniculus
<400> 442
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Glu Pro Val Gly Gly Thr Val Ser Ile Ser Cys Gln Ser Ser
           35                  40                  45
   Lys Ser Val Met Asn Asn Asn Tyr Leu Ala Trp Tyr Gln Gln Lys Pro
       50                  55                  60
   Gly Gln Pro Pro Lys Leu Leu Ile Tyr Gly Ala Ser Asn Leu Ala Ser
   65                  70                  75                  80
   Gly Val Pro Ser Arg Phe Ser Gly Ser Gly Ser Gly Thr Gln Phe Thr
                   85                  90                  95
   Leu Thr Ile Ser Asp Val Gln Cys Asp Asp Ala Ala Thr Tyr Tyr Cys
               100                 105                 110
   Gln Gly Gly Tyr Thr Gly Tyr Ser Asp His Gly Thr
           115                 120
<210> 443
   <211> 127
   <212> PRT
   <213> Oryctolagus cuniculus
<400> 443
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Lys Pro
               20                  25                  30
   Asp Glu Thr Leu Thr Leu Thr Cys Thr Val Ser Gly Ile Asp Leu Ser
           35                  40                  45
   Ser Tyr Pro Met Asn Trp Val Arg Gln Ala Pro Gly Lys Gly Leu Glu
       50                  55                  60
   Trp Ile Gly Phe Ile Asn Thr Gly Gly Thr Ile Val Tyr Ala Ser Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Gly Ser Tyr Val Ser Ser Gly Tyr Ala Tyr Tyr Phe Asn Val
           115                 120                 125
<210> 444
   <211> 13
   <212> PRT
   <213> oryctolagus cuniculus
<400> 444
   Gln Ser Ser Lys Ser Val Met Asn Asn Asn Tyr Leu Ala
   1               5                   10
<210> 445
   <211> 7
   <212> PRT
   <213> Oryctolagus cuniculus
<400> 445
   Gly Ala Ser Asn Leu Ala Ser
   1               5
<210> 446
   <211> 12
   <212> PRT
   <213> Oryctolagus cuniculus
<400> 446
   Gln Gly Gly Tyr Thr Gly Tyr Ser Asp His Gly Thr
   1 5 10
<210> 447
   <211> 5
   <212> PRT
   <213> Oryctolagus cuniculus
<400> 447
   Ser Tyr Pro Met Asn
   1 5
<210> 448
   <211> 16
   <212> PRT
   <213> Oryctolagus cuniculus
<400> 448
   Phe Ile Asn Thr Gly Gly Thr Ile Val Tyr Ala Ser Trp Ala Lys Gly
   1 5 10 15
<210> 449
   <211> 14
   <212> PRT
   <213> Oryctolagus cuniculus
<400> 449
   Gly Ser Tyr Val Ser Ser Gly Tyr Ala Tyr Tyr Phe Asn Val
   1 5 10
<210> 450
   <211> 372
   <212> DNA
   <213> Oryctolagus cuniculus
<400> 450
   atggacacga gggcccccac tcagctgctg gggctcctgc tgctctggct cccaggtgcc 60
   acatttgccg ccgtgctgac ccagactcca tctcccgtgt ctgaacctgt gggaggcaca 120
   gtcagcatca gttgccagtc cagtaagagt gttatgaata acaactactt agcctggtat 180
   cagcagaaac cagggcagcc tcccaagctc ctgatctatg gtgcatccaa tctggcatct 240
   ggggtcccat cacggttcag cggcagtgga tctgggacac agttcactct caccatcagc 300
   gacgtgcagt gtgacgatgc tgccacttac tactgtcaag gcggttatac tggttatagt 360
   gatcatggga ct 372
<210> 451
   <211> 381
   <212> DNA
   <213> Oryctolagus cuniculus
<400> 451
   atggagactg ggctgcgctg gcttctcctg gtcgctgtgc tcaaaggtgt ccagtgtcag 60
   tcggtggagg agtccggggg tcgcctggtc aagcctgacg aaaccctgac actcacctgc 120
   acagtctctg gaatcgacct cagtagctat ccaatgaact gggtccgcca ggctccaggg 180
   aaggggctgg aatggatcgg attcattaat actggtggta ccatagtcta cgcgagctgg 240
   gcaaaaggcc gattcaccat ctccaaaacc tcgaccacgg tggatctgaa aatgaccagt 300
   ccgacaaccg aggacacggc cacctatttc tgtgccagag gcagttatgt ttcatctggt 360
   tatgcctact attttaatgt c 381
<210> 452
   <211> 39
   <212> DNA
   <213> oryctolagus cuniculus
<400> 452
   cagtccagta agagtgttat gaataacaac tacttagcc 39
<210> 453
   <211> 21
   <212> DNA
   <213> Oryctolagus cuniculus
<400> 453
   ggtgcatcca atctggcatc t 21
<210> 454
   <211> 36
   <212> DNA
   <213> Oryctolagus cuniculus
<400> 454
   caaggcggtt atactggtta tagtgatcat gggact 36
<210> 455
   <211> 15
   <212> DNA
   <213> Oryctolagus cuniculus
<400> 455
   agctatccaa tgaac 15
<210> 456
   <211> 48
   <212> DNA
   <213> Oryctolagus cuniculus
<400> 456
   ttcattaata ctggtggtac catagtctac gcgagctggg caaaaggc 48
<210> 457
   <211> 42
   <212> DNA
   <213> oryctolagus cuniculus
<400> 457
   ggcagttatg tttcatctgg ttatgcctac tattttaatg tc 42
<210> 458
   <211> 121
   <212> PRT
   <213> oryctolagus cuniculus
<400> 458
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Ser Ile Ser Cys Gln Ser Ser
           35                  40                  45
   Gln Ser Val Tyr Asn Asn Asn Trp Leu Ser Trp Phe Gln Gln Lys Pro
       50                  55                  60
   Gly Gln Pro Pro Lys Leu Leu Ile Tyr Lys Ala Ser Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Asp Val Gln Cys Asp Asp Val Ala Thr Tyr Tyr Cys
               100                 105                 110
   Ala Gly Gly Tyr Leu Asp Ser Val Ile
           115                 120
<210> 459
   <211> 126
   <212> PRT
   <213> Oryctolagus cuniculus
<400> 459
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Thr Tyr Ser Ile Asn Trp Val Arg Gln Ala Pro Gly Lys Gly Leu Glu
       50                  55                  60
   Trp Ile Gly Ile Ile Ala Asn Ser Gly Thr Thr Phe Tyr Ala Asn Trp
   65                  70                  75                  80
   Ala Lys Gly Arg Phe Thr Val Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Glu Ser Gly Met Tyr Asn Glu Tyr Gly Lys Phe Asn Ile
           115                 120                 125
<210> 460
   <211> 13
   <212> PRT
   <213> oryctolagus cuniculus
<400> 460
   Gln Ser Ser Gln Ser Val Tyr Asn Asn Asn Trp Leu Ser
   1               5                   10
<210> 461
   <211> 7
   <212> PRT
   <213> Oryctolagus cuniculus
<400> 461
   Lys Ala Ser Thr Leu Ala Ser
   1               5
<210> 462
   <211> 9
   <212> PRT
   <213> Oryctolagus cuniculus
<400> 462
   Ala Gly Gly Tyr Leu Asp Ser Val Ile
   1               5
<210> 463
   <211> 5
   <212> PRT
   <213> Oryctolagus cuniculus
<400> 463
   Thr Tyr Ser Ile Asn
   1               5
<210> 464
   <211> 16
   <212> PRT
   <213> Oryctolagus cuniculus
<400> 464
   Ile Ile Ala Asn Ser Gly Thr Thr Phe Tyr Ala Asn Trp Ala Lys Gly
   1               5                   10                  15
<210> 465
   <211> 13
   <212> PRT
   <213> oryctolagus cuniculus
<400> 465
   Glu Ser Gly Met Tyr Asn Glu Tyr Gly Lys Phe Asn Ile
   1               5                   10
<210> 466
   <211> 363
   <212> DNA
   <213> Oryctolagus cuniculus
<400> 466
   atggacacga gggcccccac tcagctgctg gggctcctgc tgctctggct cccaggtgcc 60
   acatttgccg ccgtgctgac ccagactcca tctcccgtgt ctgcagctgt gggaggcaca 120
   gtcagcatca gttgccagtc cagtcagagt gtttataata acaactggtt atcctggttt 180
   cagcagaaac cagggcagcc tcccaagctc ctgatctaca aggcatccac tctggcatct 240
   ggggtcccat cgcggttcaa aggcagtgga tctgggacac agttcactct caccatcagc 300
   gacgtgcagt gtgacgatgt tgccacttac tactgtgcgg gcggttatct tgatagtgtt 360
   att 363
<210> 467
   <211> 378
   <212> DNA
   <213> Oryctolagus cuniculus
<400> 467
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtctctg gattctccct cagtacctat tcaataaact gggtccgcca ggctccaggg 180
   aagggcctgg aatggatcgg aatcattgct aatagtggta ccacattcta cgcgaactgg 240
   gcgaaaggcc gattcaccgt ctccaaaacc tcgaccacgg tggatctgaa aatcaccagt 300
   ccgacaaccg aggacacggc cacctatttc tgtgccagag agagtggaat gtacaatgaa 360
   tatggtaaat ttaacatc 378
<210> 468
   <211> 39
   <212> DNA
   <213> oryctolagus cuniculus
<400> 468
   cagtccagtc agagtgttta taataacaac tggttatcc 39
<210> 469
   <211> 21
   <212> DNA
   <213> Oryctolagus cuniculus
<400> 469
   aaggcatcca ctctggcatc t 21
<210> 470
   <211> 27
   <212> DNA
   <213> Oryctolagus cuniculus
<400> 470
   gcgggcggtt atcttgatag tgttatt 27
<210> 471
   <211> 15
   <212> DNA
   <213> Oryctolagus cuniculus
<400> 471
   acctattcaa taaac 15
<210> 472
   <211> 48
   <212> DNA
   <213> Oryctolagus cuniculus
<400> 472
   atcattgcta atagtggtac cacattctac gcgaactggg cgaaaggc 48
<210> 473
   <211> 39
   <212> DNA
   <213> Oryctolagus cuniculus
<400> 473
   gagagtggaa tgtacaatga atatggtaaa tttaacatc 39
<210> 474
   <211> 122
   <212> PRT
   <213> Oryctolagus cuniculus
<400> 474
   Met Asp Thr Arg Ala Pro Thr Gln Leu Leu Gly Leu Leu Leu Leu Trp
   1               5                   10                  15
   Leu Pro Gly Ala Arg Cys Ala Ser Asp Met Thr Gln Thr Pro Ser Ser
               20                  25                  30
   Val Ser Ala Ala Val Gly Gly Thr Val Thr Ile Asn Cys Gln Ala Ser
           35                  40                  45
   Glu Asn Ile Tyr Ser Phe Leu Ala Trp Tyr Gln Gln Lys Pro Gly Gln
       50                  55                  60
   Pro Pro Lys Leu Leu Ile Phe Lys Ala Ser Thr Leu Ala Ser Gly Val
   65                  70                  75                  80
   Ser Ser Arg Phe Lys Gly Ser Gly Ser Gly Thr Gln Phe Thr Leu Thr
                   85                  90                  95
   Ile Ser Asp Leu Glu Cys Asp Asp Ala Ala Thr Tyr Tyr Cys Gln Gln
               100                 105                 110
   Gly Ala Thr Val Tyr Asp Ile Asp Asn Asn
           115                 120
<210> 475
   <211> 128
   <212> PRT
   <213> oryctolagus cuniculus
<400> 475
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Ile Asp Leu Ser
           35                  40                  45
   Ala Tyr Ala Met Ile Trp Val Arg Gln Ala Pro Gly Glu Gly Leu Glu
       50                  55                  60
   Trp Ile Thr Ile Ile Tyr Pro Asn Gly Ile Thr Tyr Tyr Ala Asn Trp
   65                  70                  75                  80
   Ala Lys Gly Arg Phe Thr Val Ser Lys Thr Ser Thr Ala Met Asp Leu
                   85                  90                  95
   Lys Ile Thr Ser Pro Thr Thr Glu Asp Thr Ala Thr Tyr Phe Cys Ala
               100                 105                 110
   Arg Asp Ala Glu Ser Ser Lys Asn Ala Tyr Trp Gly Tyr Phe Asn Val
           115                 120                 125
<210> 476
   <211> 11
   <212> PRT
   <213> oryctolagus cuniculus
<400> 476
   Gln Ala Ser Glu Asn Ile Tyr Ser Phe Leu Ala
   1               5                   10
<210> 477
   <211> 7
   <212> PRT
   <213> oryctolagus cuniculus
<400> 477
   Lys Ala Ser Thr Leu Ala Ser
   1               5
<210> 478
   <211> 12
   <212> PRT
   <213> Oryctolagus cuniculus
<400> 478
   Gln Gln Gly Ala Thr Val Tyr Asp Ile Asp Asn Asn
   1 5 10
<210> 479
   <211> 5
   <212> PRT
   <213> oryctolagus cuniculus
<400> 479
   Ala Tyr Ala Met Ile
   1 5
<210> 480
   <211> 16
   <212> PRT
   <213> Oryctolagus cuniculus
<400> 480
   Ile Ile Tyr Pro Asn Gly Ile Thr Tyr Tyr Ala Asn Trp Ala Lys Gly
   1 5 10 15
<210> 481
   <211> 15
   <212> PRT
   <213> oryctolagus cuniculus
<400> 481
   Asp Ala Glu Ser Ser Lys Asn Ala Tyr Trp Gly Tyr Phe Asn Val
   1 5 10 15
<210> 482
   <211> 366
   <212> DNA
   <213> oryctolagus cuniculus
<400> 482
   atggacacga gggcccccac tcagctgctg gggctcctgc tgctctggct cccaggtgcc 60
   agatgtgcct ctgatatgac ccagactcca tcctccgtgt ctgcagctgt gggaggcaca 120
   gtcaccatca attgccaggc cagtgagaac atttatagct ttttggcctg gtatcagcag 180
   aaaccagggc agcctcccaa gctcctgatc ttcaaggctt ccactctggc atctggggtc 240
   tcatcgcggt tcaaaggcag tggatctggg acacagttca ctctcaccat cagcgacctg 300
   gagtgtgacg atgctgccac ttactactgt caacagggtg ctactgtgta tgatattgat 360
   aataat 366
<210> 483
   <211> 384
   <212> DNA
   <213> Oryctolagus cuniculus
<400> 483
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagtttctg gaatcgacct cagtgcctat gcaatgatct gggtccgcca ggctccaggg 180
   gaggggctgg aatggatcac aatcatttat cctaatggta tcacatacta cgcgaactgg 240
   gcgaaaggcc gattcaccgt ctccaaaacc tcgaccgcga tggatctgaa aatcaccagt 300
   ccgacaaccg aggacacggc cacctatttc tgtgccagag atgcagaaag tagtaagaat 360
   gcttattggg gctactttaa cgtc 384
<210> 484
   <211> 33
   <212> DNA
   <213> Oryctolagus cuniculus
<400> 484
   caggccagtg agaacattta tagctttttg gcc 33
<210> 485
   <211> 21
   <212> DNA
   <213> oryctolagus cuniculus
<400> 485
   aaggcttcca ctctggcatc t 21
<210> 486
   <211> 36
   <212> DNA
   <213> Oryctolagus cuniculus
<400> 486
   caacagggtg ctactgtgta tgatattgat aataat 36
<210> 487
   <211> 15
   <212> DNA
   <213> Oryctolagus cuniculus
<400> 487
   gcctatgcaa tgatc 15
<210> 488
   <211> 48
   <212> DNA
   <213> Oryctolagus cuniculus
<400> 488
   atcatttatc ctaatggtat cacatactac gcgaactggg cgaaaggc 48
<210> 489
   <211> 45
   <212> DNA
   <213> Oryctolagus cuniculus
<400> 489
   gatgcagaaa gtagtaagaa tgcttattgg ggctacttta acgtc 45
<210> 490
   <211> 122
   <212> PRT
   <213> oryctolagus cuniculus
<400> 490
   Met Asp Thr Arg Ala Pro Thr Gln Leu Leu Gly Leu Leu Leu Leu Trp
   1               5                   10                  15
   Leu Pro Gly Ala Arg Cys Ala Ser Asp Met Thr Gln Thr Pro Ser Ser
               20                  25                  30
   Val Ser Ala Ala Val Gly Gly Thr Val Thr Ile Asn Cys Gln Ala Ser
           35                  40                  45
   Glu Asn Ile Tyr Ser Phe Leu Ala Trp Tyr Gln Gln Lys Pro Gly Gln
       50                  55                  60
   Pro Pro Lys Leu Leu Ile Phe Arg Ala Ser Thr Leu Ala Ser Gly Val
   65                  70                  75                  80
   Ser Ser Arg Phe Lys Gly Ser Gly Ser Gly Thr Gln Phe Thr Leu Thr
                   85                  90                  95
   Ile Ser Asp Leu Glu Cys Asp Asp Ala Ala Thr Tyr Tyr Cys Gln Gln
   100 105 110
   Gly Ala Thr Val Tyr Asp Ile Asp Asn Asn
   115 120
<210> 491
   <211> 128
   <212> PRT
   <213> oryctolagus cuniculus
<400> 491
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Val Ser Gly Ile Asp Leu Ser
   35 40 45
   Ala Tyr Ala Met Ile Trp Val Arg Gln Ala Pro Gly Glu Gly Leu Glu
   50 55 60
   Trp Ile Thr Ile Ile Tyr Pro Asn Gly Ile Thr Tyr Tyr Ala Asn Trp
   65 70 75 80
   Ala Lys Gly Arg Phe Thr Val Ser Lys Thr Ser Thr Ala Met Asp Leu
   85 90 95
   Lys Ile Thr Ser Pro Thr Thr Glu Asp Thr Ala Thr Tyr Phe Cys Ala
   100 105 110
   Arg Asp Ala Glu Ser Ser Lys Asn Ala Tyr Trp Gly Tyr Phe Asn Val
   115 120 125
<210> 492
   <211> 11
   <212> PRT
   <213> oryctolagus cuniculus
<400> 492
   Gln Ala Ser Glu Asn Ile Tyr Ser Phe Leu Ala
   1 5 10
<210> 493
   <211> 7
   <212> PRT
   <213> oryctolagus cuniculus
<400> 493
   Arg Ala Ser Thr Leu Ala Ser
   1               5
<210> 494
   <211> 12
   <212> PRT
   <213> Oryctolagus cuniculus
<400> 494
   Gln Gln Gly Ala Thr Val Tyr Asp Ile Asp Asn Asn
   1               5                   10
<210> 495
   <211> 5
   <212> PRT
   <213> oryctolagus cuniculus
<400> 495
   Ala Tyr Ala Met Ile
   1               5
<210> 496
   <211> 16
   <212> PRT
   <213> Oryctolagus cuniculus
<400> 496
   Ile Ile Tyr Pro Asn Gly Ile Thr Tyr Tyr Ala Asn Trp Ala Lys Gly
   1               5                   10                  15
<210> 497
   <211> 15
   <212> PRT
   <213> Oryctolagus cuniculus
<400> 497
   Asp Ala Glu Ser Ser Lys Asn Ala Tyr Trp Gly Tyr Phe Asn Val
   1               5                   10                  15
<210> 498
   <211> 366
   <212> DNA
   <213> Oryctolagus cuniculus
<400> 498
   atggacacga gggcccccac tcagctgctg gggctcctgc tgctctggct cccaggtgcc 60
   agatgtgcct ctgatatgac ccagactcca tcctccgtgt ctgcagctgt gggaggcaca 120
   gtcaccatca attgccaggc cagtgagaac atttatagct ttttggcctg gtatcagcag 180
   aaaccagggc agcctcccaa gctcctgatc ttcagggctt ccactctggc atctggggtc 240
   tcatcgcggt tcaaaggcag tggatctggg acacagttca ctctcaccat cagcgacctg 300
   gagtgtgacg atgctgccac ttactactgt caacagggtg ctactgtgta tgatattgat 360
   aataat 366
<210> 499
   <211> 384
   <212> DNA
   <213> Oryctolagus cuniculus
<400> 499
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagtttctg gaatcgacct cagtgcctat gcaatgatct gggtccgcca ggctccaggg 180
   gaggggctgg aatggatcac aatcatttat cctaatggta tcacatacta cgcgaactgg 240
   gcgaaaggcc gattcaccgt ctccaaaacc tcgaccgcga tggatctgaa aatcaccagt 300
   ccgacaaccg aggacacggc cacctatttc tgtgccagag atgcagaaag tagtaagaat 360
   gcttattggg gctactttaa cgtc 384
<210> 500
   <211> 33
   <212> DNA
   <213> Oryctolagus cuniculus
<400> 500
   caggccagtg agaacattta tagctttttg gcc 33
<210> 501
   <211> 21
   <212> DNA
   <213> Oryctolagus cuniculus
<400> 501
   agggcttcca ctctggcatc t 21
<210> 502
   <211> 36
   <212> DNA
   <213> Oryctolagus cuniculus
<400> 502
   caacagggtg ctactgtgta tgatattgat aataat 36
<210> 503
   <211> 15
   <212> DNA
   <213> Oryctolagus cuniculus
<400> 503
   gcctatgcaa tgatc 15
<210> 504
   <211> 48
   <212> DNA
   <213> Oryctolagus cuniculus
<400> 504
   atcatttatc ctaatggtat cacatactac gcgaactggg cgaaaggc 48
<210> 505
   <211> 45
   <212> DNA
   <213> Oryctolagus cuniculus
<400> 505
   gatgcagaaa gtagtaagaa tgcttattgg ggctacttta acgtc 45
<210> 506
   <211> 124
   <212> PRT
   <213> Oryctolagus cuniculus
<400> 506
   Met Asp Thr Arg Ala Pro Thr Gln Leu Leu Gly Leu Leu Leu Leu Trp
   1               5                   10                  15
   Leu Pro Gly Ala Thr Phe Ala Ile Glu Met Thr Gln Thr Pro Ser Pro
               20                  25                  30
   Val Ser Ala Ala Val Gly Gly Thr Val Thr Ile Asn Cys Gln Ala Ser
           35                  40                  45
   Glu Ser Val Phe Asn Asn Met Leu Ser Trp Tyr Gln Gln Lys Pro Gly
       50                  55                  60
   His Ser Pro Lys Leu Leu Ile Tyr Asp Ala Ser Asp Leu Ala Ser Gly
   65                  70                  75                  80
   Val Pro Ser Arg Phe Lys Gly Ser Gly Ser Gly Thr Gln Phe Thr Leu
                   85                  90                  95
   Thr Ile Ser Gly Val Glu Cys Asp Asp Ala Ala Thr Tyr Tyr Cys Ala
               100                 105                 110
   Gly Tyr Lys Ser Asp Ser Asn Asp Gly Asp Asn Val
           115                 120
<210> 507
   <211> 123
   <212> PRT
   <213> Oryctolagus cuniculus
<400> 507
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Phe Ser Leu Asn
           35                  40                  45
   Arg Asn Ser Ile Thr Trp Val Arg Gln Ala Pro Gly Glu Gly Leu Glu
       50                  55                  60
   Trp Ile Gly Ile Ile Thr Gly Ser Gly Arg Thr Tyr Tyr Ala Asn Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Gly His Pro Gly Leu Gly Ser Gly Asn Ile
           115                 120
<210> 508
   <211> 12
   <212> PRT
   <213> oryctolagus cuniculus
<400> 508
   Gln Ala Ser Glu Ser Val Phe Asn Asn Met Leu Ser
   1               5                   10
<210> 509
   <211> 7
   <212> PRT
   <213> Oryctolagus cuniculus
<400> 509
   Asp Ala Ser Asp Leu Ala Ser
   1               5
<210> 510
   <211> 13
   <212> PRT
   <213> oryctolagus cuniculus
<400> 510
   Ala Gly Tyr Lys Ser Asp Ser Asn Asp Gly Asp Asn Val
   1               5                   10
<210> 511
   <211> 5
   <212> PRT
   <213> oryctolagus cuniculus
<400> 511
   Arg Asn Ser Ile Thr
   1               5
<210> 512
   <211> 16
   <212> PRT
   <213> Oryctolagus cuniculus
<400> 512
   Ile Ile Thr Gly Ser Gly Arg Thr Tyr Tyr Ala Asn Trp Ala Lys Gly
   1               5                   10                  15
<210> 513
   <211> 10
   <212> PRT
   <213> Oryctolagus cuniculus
<400> 513
   Gly His Pro Gly Leu Gly Ser Gly Asn Ile
   1               5                   10
<210> 514
   <211> 372
   <212> DNA
   <213> Oryctolagus cuniculus
<400> 514
   atggacacga gggcccccac tcagctgctg gggctcctgc tgctctggct cccaggtgcc 60
   acatttgcca ttgaaatgac ccagactcca tcccccgtgt ctgccgctgt gggaggcaca 120
   gtcaccatca attgccaggc cagtgagagt gtttttaata atatgttatc ctggtatcag 180
   cagaaaccag ggcactctcc taagctcctg atctatgatg catccgatct ggcatctggg 240
   gtcccatcgc ggttcaaagg cagtggatct gggacacagt tcactctcac catcagtggc 300
   gtggagtgtg acgatgctgc cacttactat tgtgcagggt ataaaagtga tagtaatgat 360
   ggcgataatg tt 372
<210> 515
   <211> 369
   <212> DNA
   <213> Oryctolagus cuniculus
<400> 515
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagtctctg gattctccct caacaggaat tcaataacct gggtccgcca ggctccaggg 180
   gaggggctgg aatggatcgg aatcattact ggtagtggta gaacgtacta cgcgaactgg 240
   gcaaaaggcc gattcaccat ctccaaaacc tcgaccacgg tggatctgaa aatgaccagt 300
   ccgacaaccg aggacacggc cacctatttc tgtgccagag gccatcctgg tcttggtagt 360
   ggtaacatc 369
<210> 516
   <211> 36
   <212> DNA
   <213> oryctolagus cuniculus
<400> 516
   caggccagtg agagtgtttt taataatatg ttatcc 36
<210> 517
   <211> 21
   <212> DNA
   <213> Oryctolagus cuniculus
<400> 517
   gatgcatccg atctggcatc t 21
<210> 518
   <211> 39
   <212> DNA
   <213> Oryctolagus cuniculus
<400> 518
   gcagggtata aaagtgatag taatgatggc gataatgtt 39
<210> 519
   <211> 15
   <212> DNA
   <213> Oryctolagus cuniculus
<400> 519
   aggaattcaa taacc 15
<210> 520
   <211> 48
   <212> DNA
   <213> oryctolagus cuniculus
<400> 520
   atcattactg gtagtggtag aacgtactac gcgaactggg caaaaggc 48
<210> 521
   <211> 30
   <212> DNA
   <213> Oryctolagus cuniculus
<400> 521
   ggccatcctg gtcttggtag tggtaacatc 30
<210> 522
   <211> 121
   <212> PRT
   <213> Oryctolagus cuniculus
<400> 522
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Ala Ser Ser
               20                  25                  30
   Val Ser Ala Ala Val Gly Gly Thr Val Thr Ile Asn Cys Gln Ser Ser
           35                  40                  45
   Gln Ser Val Tyr Asn Asn Tyr Leu Ser Trp Tyr Gln Gln Lys Pro Gly
       50                  55                  60
   Gln Pro Pro Lys Leu Leu Ile Tyr Thr Ala Ser Ser Leu Ala Ser Gly
   65                  70                  75                  80
   Val Pro Ser Arg Phe Lys Gly Ser Gly Ser Gly Thr Gln Phe Thr Leu
                   85                  90                  95
   Thr Ile Ser Glu Val Gln Cys Asp Asp Ala Ala Thr Tyr Tyr Cys Gln
               100                 105                 110
   Gly Tyr Tyr Ser Gly Pro Ile Ile Thr
           115                 120
<210> 523
   <211> 122
   <212> PRT
   <213> oryctolagus cuniculus
<400> 523
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Asn
           35                  40                  45
   Asn Tyr Tyr Ile Gln Trp Val Arg Gln Ala Pro Gly Glu Gly Leu Glu
       50                  55                  60
   Trp Ile Gly Ile Ile Tyr Ala Gly Gly Ser Ala Tyr Tyr Ala Thr Trp
   65                  70                  75                  80
   Ala Asn Gly Arg Phe Thr Ile Ala Lys Thr Ser Ser Thr Thr Val Asp
                   85                  90                  95
   Leu Lys Met Thr Ser Leu Thr Thr Glu Asp Thr Ala Thr Tyr Phe Cys
               100                 105                 110
   Ala Arg Gly Thr Phe Asp Gly Tyr Glu Leu
           115                 120
<210> 524
   <211> 12
   <212> PRT
   <213> oryctolagus cuniculus
<400> 524
   Gln Ser Ser Gln Ser Val Tyr Asn Asn Tyr Leu Ser
   1               5                   10
<210> 525
   <211> 7
   <212> PRT
   <213> Oryctolagus cuniculus
<400> 525
   Thr Ala Ser Ser Leu Ala Ser
   1               5
<210> 526
   <211> 10
   <212> PRT
   <213> Oryctolagus cuniculus
<400> 526
   Gln Gly Tyr Tyr Ser Gly Pro Ile Ile Thr
   1               5                   10
<210> 527
   <211> 5
   <212> PRT
   <213> Oryctolagus cuniculus
<400> 527
   Asn Tyr Tyr Ile Gln
   1               5
<210> 528
   <211> 16
   <212> PRT
   <213> Oryctolagus cuniculus
<400> 528
   Ile Ile Tyr Ala Gly Gly Ser Ala Tyr Tyr Ala Thr Trp Ala Asn Gly
   1               5                   10                  15
<210> 529
   <211> 8
   <212> PRT
   <213> Oryctolagus cuniculus
<400> 529
   Gly Thr Phe Asp Gly Tyr Glu Leu
   1               5
<210> 530
   <211> 363
   <212> DNA
   <213> oryctolagus cuniculus
<400> 530
   atggacacga gggcccccac tcagctgctg gggctcctgc tgctctggct cccaggtgcc 60
   acatttgcgc aagtgctgac ccagactgca tcgtccgtgt ctgcagctgt gggaggcaca 120
   gtcaccatca attgccagtc cagtcagagt gtttataata actacttatc ctggtatcag 180
   cagaaaccag ggcagcctcc caagctcctg atctatactg catccagcct ggcatctggg 240
   gtcccatcgc ggttcaaagg cagtggatct gggacacagt tcactctcac catcagcgaa 300
   gtgcagtgtg acgatgctgc cacttactac tgtcaaggct attatagtgg tcctataatt 360
   act 363
<210> 531
   <211> 366
   <212> DNA
   <213> Oryctolagus cuniculus
<400> 531
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagcctctg gattctccct caataactac tacatacaat gggtccgcca ggctccaggg 180
   gaggggctgg aatggatcgg gatcatttat gctggtggta gcgcatacta cgcgacctgg 240
   gcaaacggcc gattcaccat cgccaaaacc tcgtcgacca cggtggatct gaagatgacc 300
   agtctgacaa ccgaggacac ggccacctat ttctgtgcca gagggacatt tgatggttat 360
   gagttg 366
<210> 532
   <211> 36
   <212> DNA
   <213> Oryctolagus cuniculus
<400> 532
   cagtccagtc agagtgttta taataactac ttatcc 36
<210> 533
   <211> 21
   <212> DNA
   <213> Oryctolagus cuniculus
<400> 533
   actgcatcca gcctggcatc t 21
<210> 534
   <211> 30
   <212> DNA
   <213> Oryctolagus cuniculus
<400> 534
   caaggctatt atagtggtcc tataattact 30
<210> 535
   <211> 15
   <212> DNA
   <213> Oryctolagus cuniculus
<400> 535
   aactactaca tacaa 15
<210> 536
   <211> 48
   <212> DNA
   <213> oryctolagus cuniculus
<400> 536
   atcatttatg ctggtggtag cgcatactac gcgacctggg caaacggc 48
<210> 537
   <211> 24
   <212> DNA
   <213> Oryctolagus cuniculus
<400> 537
   gggacatttg atggttatga gttg 24
<210> 538
   <211> 122
   <212> PRT
   <213> Oryctolagus cuniculus
<400> 538
   Met Asp Thr Arg Ala Pro Thr Gln Leu Leu Gly Leu Leu Leu Leu Trp
   1 5 10 15
   Leu Pro Gly Ala Thr Phe Ala Gln Val Leu Thr Gln Thr Pro Ser Pro
   20 25 30
   Val Ser Val Pro Val Gly Asp Thr Val Thr Ile Ser Cys Gln Ser Ser
   35 40 45
   Glu Ser Val Tyr Ser Asn Asn Leu Leu Ser Trp Tyr Gln Gln Lys Pro
   50 55 60
   Gly Gln Pro Pro Lys Leu Leu Ile Tyr Arg Ala Ser Asn Leu Ala Ser
   65 70 75 80
   Gly Val Pro Ser Arg Phe Lys Gly Ser Gly Ser Gly Thr Gln Phe Thr
   85 90 95
   Leu Thr Ile Ser Gly Ala Gln Cys Asp Asp Ala Ala Thr Tyr Tyr Cys
   100 105 110
   Gln Gly Tyr Tyr Ser Gly Val Ile Asn Ser
   115 120
<210> 539
   <211> 124
   <212> PRT
   <213> oryctolagus cuniculus
<400> 539
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Tyr Phe Met Ser Trp Val Arg Gln Ala Pro Gly Glu Gly Leu Glu
       50                  55                  60
   Tyr Ile Gly Phe Ile Asn Pro Gly Gly Ser Ala Tyr Tyr Ala Ser Trp
   65                  70                  75                  80
   Ala Ser Gly Arg Leu Thr Ile Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Ile Leu Ile Val Ser Tyr Gly Ala Phe Thr Ile
           115                 120
<210> 540
   <211> 13
   <212> PRT
   <213> oryctolagus cuniculus
<400> 540
   Gln Ser Ser Glu Ser Val Tyr Ser Asn Asn Leu Leu Ser
   1               5                   10
<210> 541
   <211> 7
   <212> PRT
   <213> Oryctolagus cuniculus
<400> 541
   Arg Ala Ser Asn Leu Ala Ser
   1               5
<210> 542
   <211> 10
   <212> PRT
   <213> Oryctolagus cuniculus
<400> 542
   Gln Gly Tyr Tyr Ser Gly Val Ile Asn Ser
   1 5 10
<210> 543
   <211> 5
   <212> PRT
   <213> Oryctolagus cuniculus
<400> 543
   Ser Tyr Phe Met Ser
   1 5
<210> 544
   <211> 16
   <212> PRT
   <213> oryctolagus cuniculus
<400> 544
   Phe Ile Asn Pro Gly Gly Ser Ala Tyr Tyr Ala Ser Trp Ala Ser Gly
   1 5 10 15
<210> 545
   <211> 11
   <212> PRT
   <213> Oryctolagus cuniculus
<400> 545
   Ile Leu Ile Val Ser Tyr Gly Ala Phe Thr Ile
   1 5 10
<210> 546
   <211> 366
   <212> DNA
   <213> Oryctolagus cuniculus
<400> 546
   atggacacga gggcccccac tcagctgctg gggctcctgc tgctctggct cccaggtgcc 60
   acatttgccc aagtgctgac ccagactcca tcccctgtgt ctgtccctgt gggagacaca 120
   gtcaccatca gttgccagtc cagtgagagc gtttatagta ataacctctt atcctggtat 180
   cagcagaaac cagggcagcc tcccaagctc ctgatctaca gggcatccaa tctggcatct 240
   ggtgtcccat cgcggttcaa aggcagtgga tctgggacac agttcactct caccatcagc 300
   ggcgcacagt gtgacgatgc tgccacttac tactgtcaag gctattatag tggtgtcatt 360
   aatagt 366
<210> 547
   <211> 372
   <212> DNA
   <213> Oryctolagus cuniculus
<400> 547
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtgtctg gattctccct cagtagctac ttcatgagct gggtccgcca ggctccaggg 180
   gaggggctgg aatacatcgg attcattaat cctggtggta gcgcatacta cgcgagctgg 240
   gcgagtggcc gactcaccat ctccaaaacc tcgaccacgg tagatctgaa aatcaccagt 300
   ccgacaaccg aggacacggc cacctatttc tgtgccagga ttcttattgt ttcttatgga 360
   gcctttacca tc 372
<210> 548
   <211> 39
   <212> DNA
   <213> Oryctolagus cuniculus
<400> 548
   cagtccagtg agagcgttta tagtaataac ctcttatcc 39
<210> 549
   <211> 21
   <212> DNA
   <213> oryctolagus cuniculus
<400> 549
   agggcatcca atctggcatc t 21
<210> 550
   <211> 30
   <212> DNA
   <213> Oryctolagus cuniculus
<400> 550
   caaggctatt atagtggtgt cattaatagt 30
<210> 551
   <211> 15
   <212> DNA
   <213> Oryctolagus cuniculus
<400> 551
   agctacttca tgagc 15
<210> 552
   <211> 48
   <212> DNA
   <213> Oryctolagus cuniculus
<400> 552
   ttcattaatc ctggtggtag cgcatactac gcgagctggg cgagtggc 48
<210> 553
   <211> 33
   <212> DNA
   <213> Oryctolagus cuniculus
<400> 553
   attcttattg tttcttatgg agcctttacc atc 33
<210> 554
   <211> 122
   <212> PRT
   <213> Oryctolagus cuniculus
<400> 554
   Met Asp Thr Arg Ala Pro Thr Gln Leu Leu Gly Leu Leu Leu Leu Trp
   1 5 10 15
   Leu Pro Gly Ala Arg Cys Ala Tyr Asp Met Thr Gln Thr Pro Ala Ser
   20 25 30
   Val Glu Val Ala Val Gly Gly Thr Val Thr Ile Lys Cys Gln Ala Thr
   35 40 45
   Glu Ser Ile Gly Asn Glu Leu Ser Trp Tyr Gln Gln Lys Pro Gly Gln
   50 55 60
   Ala Pro Lys Leu Leu Ile Tyr Ser Ala Ser Thr Leu Ala Ser Gly Val
   65 70 75 80
   Pro Ser Arg Phe Lys Gly Ser Gly Ser Gly Thr Gln Phe Thr Leu Thr
   85 90 95
   Ile Thr Gly Val Glu Cys Asp Asp Ala Ala Thr Tyr Tyr Cys Gln Gln
   100 105 110
   Gly Tyr Ser Ser Ala Asn Ile Asp Asn Ala
   115 120
<210> 555
   <211> 128
   <212> PRT
   <213> oryctolagus cuniculus
<400> 555
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Lys Tyr Tyr Met Ser Trp Val Arg Gln Ala Pro Glu Lys Gly Leu Lys
   50 55 60
   Tyr Ile Gly Tyr Ile Asp Ser Thr Thr Val Asn Thr Tyr Tyr Ala Thr
   65 70 75 80
   Trp Ala Arg Gly Arg Phe Thr Ile Ser Lys Thr Ser Thr Thr Val Asp
   85 90 95
   Leu Lys Ile Thr Ser Pro Thr Ser Glu Asp Thr Ala Thr Tyr Phe Cys
   100 105 110
   Ala Arg Gly Ser Thr Tyr Phe Thr Asp Gly Gly His Arg Leu Asp Leu
   115 120 125
<210> 556
   <211> 11
   <212> PRT
   <213> oryctolagus cuniculus
<400> 556
   Gln Ala Thr Glu Ser Ile Gly Asn Glu Leu Ser
   1               5                   10
<210> 557
   <211> 7
   <212> PRT
   <213> Oryctolagus cuniculus
<400> 557
   Ser Ala Ser Thr Leu Ala Ser
   1               5
<210> 558
   <211> 12
   <212> PRT
   <213> Oryctolagus cuniculus
<400> 558
   Gln Gln Gly Tyr Ser Ser Ala Asn Ile Asp Asn Ala
   1               5                   10
<210> 559
   <211> 5
   <212> PRT
   <213> Oryctolagus cuniculus
<400> 559
   Lys Tyr Tyr Met Ser
   1               5
<210> 560
   <211> 17
   <212> PRT
   <213> Oryctolagus cuniculus
<400> 560
   Tyr Ile Asp Ser Thr Thr Val Asn Thr Tyr Tyr Ala Thr Trp Ala Arg
   1               5                   10                  15
   Gly
<210> 561
   <211> 14
   <212> PRT
   <213> oryctolagus cuniculus
<400> 561
   Gly Ser Thr Tyr Phe Thr Asp Gly Gly His Arg Leu Asp Leu
   1 5 10
<210> 562
   <211> 366
   <212> DNA
   <213> oryctolagus cuniculus
<400> 562
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cactgagagc attggcaatg agttatcctg gtatcagcag 180
   aaaccagggc aggctcccaa gctcctgatc tattctgcat ccactctggc atctggggtc 240
   ccatcgcggt tcaaaggcag tggatctggg acacagttca ctctcaccat caccggcgtg 300
   gagtgtgatg atgctgccac ttactactgt caacagggtt atagtagtgc taatattgat 360
   aatgct 366
<210> 563
   <211> 384
   <212> DNA
   <213> oryctolagus cuniculus
<400> 563
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   accgtctctg gattctccct cagtaagtac tacatgagct gggtccgcca ggctccagag 180
   aaggggctga aatacatcgg atacattgat agtactactg ttaatacata ctacgcgacc 240
   tgggcgagag gccgattcac catctccaaa acctcgacca cggtggatct gaagatcacc 300
   agtccgacaa gtgaggacac ggccacctat ttctgtgcca gaggaagtac ttattttact 360
   gatggaggcc atcggttgga tctc 384
<210> 564
   <211> 33
   <212> DNA
   <213> Oryctolagus cuniculus
<400> 564
   caggccactg agagcattgg caatgagtta tcc 33
<210> 565
   <211> 21
   <212> DNA
   <213> oryctolagus cuniculus
<400> 565
   tctgcatcca ctctggcatc t 21
<210> 566
   <211> 36
   <212> DNA
   <213> Oryctolagus cuniculus
<400> 566
   caacagggtt atagtagtgc taatattgat aatgct 36
<210> 567
   <211> 15
   <212> DNA
   <213> Oryctolagus cuniculus
<400> 567
   aagtactaca tgagc 15
<210> 568
   <211> 51
   <212> DNA
   <213> Oryctolagus cuniculus
<400> 568
   tacattgata gtactactgt taatacatac tacgcgacct gggcgagagg c 51
<210> 569
   <211> 42
   <212> DNA
   <213> Oryctolagus cuniculus
<400> 569
   ggaagtactt attttactga tggaggccat cggttggatc tc 42
<210> 570
   <211> 122
   <212> PRT
   <213> Oryctolagus cuniculus
<400> 570
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Lys Cys Gln Ala Thr
           35                  40                  45
   Glu Ser Ile Gly Asn Glu Leu Ser Trp Tyr Gln Gln Lys Pro Gly Gln
       50                  55                  60
   Ala Pro Lys Leu Leu Ile Tyr Ser Ala Ser Thr Leu Ala Ser Gly Val
   65                  70                  75                  80
   Pro Ser Arg Phe Lys Gly Ser Gly Ser Gly Thr Gln Phe Thr Leu Thr
                   85                  90                  95
   Ile Thr Gly Val Glu Cys Asp Asp Ala Ala Thr Tyr Tyr Cys Gln Gln
               100                 105                 110
   Gly Tyr Ser Ser Ala Asn Ile Asp Asn Ala
           115                 120
<210> 571
   <211> 124
   <212> PRT
   <213> Oryctolagus cuniculus
<400> 571
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Thr Tyr Asn Met Gly Trp Val Arg Gln Ala Pro Gly Lys Gly Leu Glu
   50 55 60
   Trp Ile Gly Ser Ile Thr Ile Asp Gly Arg Thr Tyr Tyr Ala Ser Trp
   65 70 75 80
   Ala Lys Gly Arg Phe Thr Val Ser Lys Ser Ser Thr Thr Val Asp Leu
   85 90 95
   Lys Met Thr Ser Leu Thr Thr Gly Asp Thr Ala Thr Tyr Phe Cys Ala
   100 105 110
   Arg Ile Leu Ile Val Ser Tyr Gly Ala Phe Thr Ile
   115 120
<210> 572
   <211> 11
   <212> PRT
   <213> Oryctolagus cuniculus
<400> 572
   Gln Ala Thr Glu Ser Ile Gly Asn Glu Leu Ser
   1 5 10
<210> 573
   <211> 7
   <212> PRT
   <213> oryctolagus cuniculus
<400> 573
   Ser Ala Ser Thr Leu Ala Ser
   1 5
<210> 574
   <211> 12
   <212> PRT
   <213> Oryctolagus cuniculus
<400> 574
   Gln Gln Gly Tyr Ser Ser Ala Asn Ile Asp Asn Ala
   1 5 10
<210> 575
   <211> 5
   <212> PRT
   <213> Oryctolagus cuniculus
<400> 575
   Thr Tyr Asn Met Gly
   1 5
<210> 576
   <211> 16
   <212> PRT
   <213> oryctolagus cuniculus
<400> 576
   Ser Ile Thr Ile Asp Gly Arg Thr Tyr Tyr Ala Ser Trp Ala Lys Gly
   1 5 10 15
<210> 577
   <211> 11
   <212> PRT
   <213> Oryctolagus cuniculus
<400> 577
   Ile Leu Ile Val Ser Tyr Gly Ala Phe Thr Ile
   1 5 10
<210> 578
   <211> 366
   <212> DNA
   <213> Oryctolagus cuniculus
<400> 578
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cactgagagc attggcaatg agttatcctg gtatcagcag 180
   aaaccagggc aggctcccaa gctcctgatc tattctgcat ccactctggc atctggggtc 240
   ccatcgcggt tcaaaggcag tggatctggg acacagttca ctctcaccat caccggcgtg 300
   gagtgtgatg atgctgccac ttactactgt caacagggtt atagtagtgc taatattgat 360
   aatgct 366
<210> 579
   <211> 372
   <212> DNA
   <213> oryctolagus cuniculus
<400> 579
   atggagactg ggctgcgctg gcttctcctg gtcgctgtgc tcaaaggtgt ccagtgtcag 60
   tcgctggagg agtccggggg tcgcctggta acgcctggga cacccctgac actcacctgc 120
   acagtctctg gattctccct cagtacctac aacatgggct gggtccgcca ggctccaggg 180
   aaggggctgg aatggatcgg aagtattact attgatggtc gcacatacta cgcgagctgg 240
   gcgaaaggcc gattcaccgt ctccaaaagc tcgaccacgg tggatctgaa aatgaccagt 300
   ctgacaaccg gggacacggc cacctatttc tgtgccagga ttcttattgt ttcttatggg 360
   gcctttacca tc 372
<210> 580
   <211> 33
   <212> DNA
   <213> Oryctolagus cuniculus
<400> 580
   caggccactg agagcattgg caatgagtta tcc 33
<210> 581
   <211> 21
   <212> DNA
   <213> Oryctolagus cuniculus
<400> 581
   tctgcatcca ctctggcatc t 21
<210> 582
   <211> 36
   <212> DNA
   <213> oryctolagus cuniculus
<400> 582
   caacagggtt atagtagtgc taatattgat aatgct 36
<210> 583
   <211> 15
   <212> DNA
   <213> oryctolagus cuniculus
<400> 583
   acctacaaca tgggc 15
<210> 584
   <211> 48
   <212> DNA
   <213> Oryctolagus cuniculus
<400> 584
   agtattacta ttgatggtcg cacatactac gcgagctggg cgaaaggc 48
<210> 585
   <211> 33
   <212> DNA
   <213> Oryctolagus cuniculus
<400> 585
   attcttattg tttcttatgg ggcctttacc atc 33
<210> 586
   <211> 105
   <212> PRT
   <213> Artificial Sequence
<220>
   <223> Kappa constant domain
<400> 586
<210> 587
   <211> 315
   <212> DNA
   <213> Artificial sequence
<220>
   <223> Kappa constant domain
<400> 587
   gtggctgcac catctgtctt catcttcccg ccatctgatg agcagttgaa atctggaact 60
   gcctctgttg tgtgcctgct gaataacttc tatcccagag aggccaaagt acagtggaag 120
   gtggataacg ccctccaatc gggtaactcc caggagagtg tcacagagca ggacagcaag 180
   gacagcacct acagcctcag cagcaccctg acgctgagca aagcagacta cgagaaacac 240
   aaagtctacg cctgcgaagt cacccatcag ggcctgagct cgcccgtcac aaagagcttc 300
   aacaggggag agtgt 315
<210> 588
   <211> 330
   <212> PRT
   <213> Artificial Sequence
<220>
   <223> Gamma-1 constant domain
<400> 588
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 589
   <211> 990
   <212> DNA
   <213> Artificial sequence
<220>
   <223> Gamma-1 constant domain
<400> 589
   gcctccacca agggcccatc ggtcttcccc ctggcaccct cctccaagag cacctctggg 60
   ggcacagcgg ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagag agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggaggt gcataatgcc aagacaaagc cgcgggagga gcagtacgcc 540
   agcacgtacc gtgtggtcag cgtcctcacc gtcctgcacc aggactggct gaatggcaag 600
   gagtacaagt gcaaggtctc caacaaagcc ctcccagccc ccatcgagaa aaccatctcc 660
   aaagccaaag ggcagccccg agaaccacag gtgtacaccc tgcccccatc ccgggaggag 720
   atgaccaaga accaggtcag cctgacctgc ctggtcaaag gcttctatcc cagcgacatc 780
   gccgtggagt gggagagcaa tgggcagccg gagaacaact acaagaccac gcctcccgtg 840
   ctggactccg acggctcctt cttcctctac agcaagctca ccgtggacaa gagcaggtgg 900
   cagcagggga acgtcttctc atgctccgtg atgcatgagg ctctgcacaa ccactacacg 960
   cagaagagcc tctccctgtc tccgggtaaa 990
<210> 590
   <211> 15
   <212> PRT
   <213> Homo sapiens
<400> 590
   Val Pro Pro Gly Glu Asp Ser Lys Asp Val Ala Ala Pro His Arg
   1 5 10 15
<210> 591
   <211> 15
   <212> PRT
   <213> Homo sapiens
<400> 591
   Gly Glu Asp Ser Lys Asp Val Ala Ala Pro His Arg Gln Pro Leu
   1 5 10 15
<210> 592
   <211> 15
   <212> PRT
   <213> Homo sapiens
<400> 592
   Ser Lys Asp Val Ala Ala Pro His Arg Gln Pro Leu Thr Ser Ser
   1               5                   10                  15
<210> 593
   <211> 15
   <212> PRT
   <213> Homo sapiens
<400> 593
   Val Ala Ala Pro His Arg Gln Pro Leu Thr Ser Ser Glu Arg Ile
   1               5                   10                  15
<210> 594
   <211> 15
   <212> PRT
   <213> Homo sapiens
<400> 594
   Pro His Arg Gln Pro Leu Thr Ser Ser Glu Arg Ile Asp Lys Gln
   1               5                   10                  15
<210> 595
   <211> 15
   <212> PRT
   <213> Homo sapiens
<400> 595
   Gln Pro Leu Thr Ser Ser Glu Arg Ile Asp Lys Gln Ile Arg Tyr
   1               5                   10                  15
<210> 596
   <211> 15
   <212> PRT
   <213> Homo sapiens
<400> 596
   Thr Ser Ser Glu Arg Ile Asp Lys Gln Ile Arg Tyr Ile Leu Asp
   1               5                   10                  15
<210> 597
   <211> 15
   <212> PRT
   <213> Homo sapiens
<400> 597
   Glu Arg Ile Asp Lys Gln Ile Arg Tyr Ile Leu Asp Gly Ile Ser
   1 5 10 15
<210> 598
   <211> 15
   <212> PRT
   <213> Homo sapiens
<400> 598
   Asp Lys Gln Ile Arg Tyr Ile Leu Asp Gly Ile Ser Ala Leu Arg
   1 5 10 15
<210> 599
   <211> 15
   <212> PRT
   <213> Homo sapiens
<400> 599
   Ile Arg Tyr Ile Leu Asp Gly Ile Ser Ala Leu Arg Lys Glu Thr
   1 5 10 15
<210> 600
   <211> 15
   <212> PRT
   <213> Homo sapiens
<400> 600
   Ile Leu Asp Gly Ile Ser Ala Leu Arg Lys Glu Thr Cys Asn Lys
   1 5 10 15
<210> 601
   <211> 15
   <212> PRT
   <213> Homo sapiens
<400> 601
   Gly Ile Ser Ala Leu Arg Lys Glu Thr Cys Asn Lys Ser Asn Met
   1 5 10 15
<210> 602
   <211> 15
   <212> PRT
   <213> Homo sapiens
<400> 602
   Ala Leu Arg Lys Glu Thr Cys Asn Lys Ser Asn Met Cys Glu Ser
   1 5 10 15
<210> 603
   <211> 15
   <212> PRT
   <213> Homo sapiens
<400> 603
   Lys Glu Thr Cys Asn Lys Ser Asn Met Cys Glu Ser Ser Lys Glu
   1 5 10 15
<210> 604
   <211> 15
   <212> PRT
   <213> Homo sapiens
<400> 604
   Cys Asn Lys Ser Asn Met Cys Glu Ser Ser Lys Glu Ala Leu Ala
   1 5 10 15
<210> 605
   <211> 15
   <212> PRT
   <213> Homo sapiens
<400> 605
   Ser Asn Met Cys Glu Ser Ser Lys Glu Ala Leu Ala Glu Asn Asn
   1 5 10 15
<210> 606
   <211> 15
   <212> PRT
   <213> Homo sapiens
<400> 606
   Cys Glu Ser Ser Lys Glu Ala Leu Ala Glu Asn Asn Leu Asn Leu
   1 5 10 15
<210> 607
   <211> 15
   <212> PRT
   <213> Homo sapiens
<400> 607
   Ser Lys Glu Ala Leu Ala Glu Asn Asn Leu Asn Leu Pro Lys Met
   1 5 10 15
<210> 608
   <211> 15
   <212> PRT
   <213> Homo sapiens
<400> 608
   Ala Leu Ala Glu Asn Asn Leu Asn Leu Pro Lys Met Ala Glu Lys
   1 5 10 15
<210> 609
   <211> 15
   <212> PRT
   <213> Homo sapiens
<400> 609
   Glu Asn Asn Leu Asn Leu Pro Lys Met Ala Glu Lys Asp Gly Cys
   1 5 10 15
<210> 610
   <211> 15
   <212> PRT
   <213> Homo sapiens
<400> 610
   Leu Asn Leu Pro Lys Met Ala Glu Lys Asp Gly Cys Phe Gln Ser
   1 5 10 15
<210> 611
   <211> 15
   <212> PRT
   <213> Homo sapiens
<400> 611
   Pro Lys Met Ala Glu Lys Asp Gly Cys Phe Gln Ser Gly Phe Asn
   1 5 10 15
<210> 612
   <211> 15
   <212> PRT
   <213> Homo sapiens
<400> 612
   Ala Glu Lys Asp Gly Cys Phe Gln Ser Gly Phe Asn Glu Glu Thr
   1 5 10 15
<210> 613
   <211> 15
   <212> PRT
   <213> Homo sapiens
<400> 613
   Asp Gly Cys Phe Gln Ser Gly Phe Asn Glu Glu Thr Cys Leu Val
   1 5 10 15
<210> 614
   <211> 15
   <212> PRT
   <213> Homo sapiens
<400> 614
   Phe Gln Ser Gly Phe Asn Glu Glu Thr Cys Leu Val Lys Ile Ile
   1 5 10 15
<210> 615
   <211> 15
   <212> PRT
   <213> Homo sapiens
<400> 615
   Gly Phe Asn Glu Glu Thr Cys Leu Val Lys Ile Ile Thr Gly Leu
   1 5 10 15
<210> 616
   <211> 15
   <212> PRT
   <213> Homo sapiens
<400> 616
   Glu Glu Thr Cys Leu Val Lys Ile Ile Thr Gly Leu Leu Glu Phe
   1 5 10 15
<210> 617
   <211> 15
   <212> PRT
   <213> Homo sapiens
<400> 617
   Cys Leu Val Lys Ile Ile Thr Gly Leu Leu Glu Phe Glu Val Tyr
   1 5 10 15
<210> 618
   <211> 15
   <212> PRT
   <213> Homo sapiens
<400> 618
   Lys Ile Ile Thr Gly Leu Leu Glu Phe Glu Val Tyr Leu Glu Tyr
   1               5                   10                  15
<210> 619
   <211> 15
   <212> PRT
   <213> Homo sapiens
<400> 619
   Thr Gly Leu Leu Glu Phe Glu Val Tyr Leu Glu Tyr Leu Gln Asn
   1               5                   10                  15
<210> 620
   <211> 15
   <212> PRT
   <213> Homo sapiens
<400> 620
   Leu Glu Phe Glu Val Tyr Leu Glu Tyr Leu Gln Asn Arg Phe Glu
   1               5                   10                  15
<210> 621
   <211> 15
   <212> PRT
   <213> Homo sapiens
<400> 621
   Glu Val Tyr Leu Glu Tyr Leu Gln Asn Arg Phe Glu Ser Ser Glu
   1               5                   10                  15
<210> 622
   <211> 15
   <212> PRT
   <213> Homo sapiens
<400> 622
   Leu Glu Tyr Leu Gln Asn Arg Phe Glu Ser Ser Glu Glu Gln Ala
   1               5                   10                  15
<210> 623
   <211> 15
   <212> PRT
   <213> Homo sapiens
<400> 623
   Leu Gln Asn Arg Phe Glu Ser Ser Glu Glu Gln Ala Arg Ala Val
   1 5 10 15
<210> 624
   <211> 15
   <212> PRT
   <213> Homo sapiens
<400> 624
   Arg Phe Glu Ser Ser Glu Glu Gln Ala Arg Ala Val Gln Met Ser
   1 5 10 15
<210> 625
   <211> 15
   <212> PRT
   <213> Homo sapiens
<400> 625
   Ser Ser Glu Glu Gln Ala Arg Ala Val Gln Met Ser Thr Lys Val
   1 5 10 15
<210> 626
   <211> 15
   <212> PRT
   <213> Homo sapiens
<400> 626
   Glu Gln Ala Arg Ala Val Gln Met Ser Thr Lys Val Leu Ile Gln
   1 5 10 15
<210> 627
   <211> 15
   <212> PRT
   <213> Homo sapiens
<400> 627
   Arg Ala Val Gln Met Ser Thr Lys Val Leu Ile Gln Phe Leu Gln
   1 5 10 15
<210> 628
   <211> 15
   <212> PRT
   <213> Homo sapiens
<400> 628
   Gln Met Ser Thr Lys Val Leu Ile Gln Phe Leu Gln Lys Lys Ala
   1 5 10 15
<210> 629
   <211> 15
   <212> PRT
   <213> Homo sapiens
<400> 629
   Thr Lys Val Leu Ile Gln Phe Leu Gln Lys Lys Ala Lys Asn Leu
   1 5 10 15
<210> 630
   <211> 15
   <212> PRT
   <213> Homo sapiens
<400> 630
   Leu Ile Gln Phe Leu Gln Lys Lys Ala Lys Asn Leu Asp Ala Ile
   1 5 10 15
<210> 631
   <211> 15
   <212> PRT
   <213> Homo sapiens
<400> 631
   Phe Leu Gln Lys Lys Ala Lys Asn Leu Asp Ala Ile Thr Thr Pro
   1 5 10 15
<210> 632
   <211> 15
   <212> PRT
   <213> Homo sapiens
<400> 632
   Lys Lys Ala Lys Asn Leu Asp Ala Ile Thr Thr Pro Asp Pro Thr
   1 5 10 15
<210> 633
   <211> 15
   <212> PRT
   <213> Homo sapiens
<400> 633
   Lys Asn Leu Asp Ala Ile Thr Thr Pro Asp Pro Thr Thr Asn Ala
   1 5 10 15
<210> 634
   <211> 15
   <212> PRT
   <213> Homo sapiens
<400> 634
   Asp Ala Ile Thr Thr Pro Asp Pro Thr Thr Asn Ala Ser Leu Leu
   1 5 10 15
<210> 635
   <211> 15
   <212> PRT
   <213> Homo sapiens
<400> 635
   Thr Thr Pro Asp Pro Thr Thr Asn Ala Ser Leu Leu Thr Lys Leu
   1 5 10 15
<210> 636
   <211> 15
   <212> PRT
   <213> Homo sapiens
<400> 636
   Asp Pro Thr Thr Asn Ala Ser Leu Leu Thr Lys Leu Gln Ala Gln
   1 5 10 15
<210> 637
   <211> 15
   <212> PRT
   <213> Homo sapiens
<400> 637
   Thr Asn Ala Ser Leu Leu Thr Lys Leu Gln Ala Gln Asn Gln Trp
   1 5 10 15
<210> 638
   <211> 15
   <212> PRT
   <213> Homo sapiens
<400> 638
   Ser Leu Leu Thr Lys Leu Gln Ala Gln Asn Gln Trp Leu Gln Asp
   1 5 10 15
<210> 639
   <211> 15
   <212> PRT
   <213> Homo sapiens
<400> 639
   Thr Lys Leu Gln Ala Gln Asn Gln Trp Leu Gln Asp Met Thr Thr
   1 5 10 15
<210> 640
   <211> 15
   <212> PRT
   <213> Homo sapiens
<400> 640
   Gln Ala Gln Asn Gln Trp Leu Gln Asp Met Thr Thr His Leu Ile
   1 5 10 15
<210> 641
   <211> 15
   <212> PRT
   <213> Homo sapiens
<400> 641
   Asn Gln Trp Leu Gln Asp Met Thr Thr His Leu Ile Leu Arg Ser
   1 5 10 15
<210> 642
   <211> 15
   <212> PRT
   <213> Homo sapiens
<400> 642
   Leu Gln Asp Met Thr Thr His Leu Ile Leu Arg Ser Phe Lys Glu
   1 5 10 15
<210> 643
   <211> 15
   <212> PRT
   <213> Homo sapiens
<400> 643
   Met Thr Thr His Leu Ile Leu Arg Ser Phe Lys Glu Phe Leu Gln
   1 5 10 15
<210> 644
   <211> 15
   <212> PRT
   <213> Homo sapiens
<400> 644
   His Leu Ile Leu Arg Ser Phe Lys Glu Phe Leu Gln Ser Ser Leu
   1 5 10 15
<210> 645
   <211> 15
   <212> PRT
   <213> Homo sapiens
<400> 645
   Leu Arg Ser Phe Lys Glu Phe Leu Gln Ser Ser Leu Arg Ala Leu
   1 5 10 15
<210> 646
   <211> 15
   <212> PRT
   <213> Homo sapiens
<400> 646
   Phe Lys Glu Phe Leu Gln Ser Ser Leu Arg Ala Leu Arg Gln Met
   1 5 10 15
<210> 647
   <211> 111
   <212> PRT
   <213> Oryctolagus cuniculus
<400> 647
   Ala Tyr Asp Met Thr Gln Thr Pro Ala Ser Val Ser Ala Ala Val Gly
   1 5 10 15
   Gly Thr Val Thr Ile Lys Cys Gln Ala Ser Gln Ser Ile Asn Asn Glu
   20 25 30
   Leu Ser Trp Tyr Gln Gln Lys Pro Gly Gln Arg Pro Lys Leu Leu Ile
   35 40 45
   Tyr Arg Ala Ser Thr Leu Ala Ser Gly Val Ser Ser Arg Phe Lys Gly
   50 55 60
   Ser Gly Ser Gly Thr Glu Phe Thr Leu Thr Ile Ser Asp Leu Glu Cys
   65 70 75 80
   Ala Asp Ala Ala Thr Tyr Tyr Cys Gln Gln Gly Tyr Ser Leu Arg Asn
   85 90 95
   Ile Asp Asn Ala Phe Gly Gly Gly Thr Glu Val Val Val Lys Arg
   100 105 110
<210> 648
   <211> 88
   <212> PRT
   <213> Homo sapiens
<400> 648
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys
                   85
<210> 649
   <211> 88
   <212> PRT
   <213> Homo sapiens
<400> 649
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Tyr
               20                  25                  30
   Leu Ala Trp Tyr Gln Gln Lys Pro Gly Lys Val Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys
                   85
<210> 650
   <211> 88
   <212> PRT
   <213> Homo sapiens
<400> 650
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys
                   85
<210> 651
   <211> 111
   <212> PRT
   <213> Artificial sequence
<220>
   <223> Humanized antibody
<400> 651
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg
               100                 105                 110
<210> 652
   <211> 117
   <212> PRT
   <213> Oryctolagus cuniculus
<400> 652
   Gln Ser Leu Glu Glu Ser Gly Gly Arg Leu Val Thr Pro Gly Thr Pro
   1               5                   10                  15
   Leu Thr Leu Thr Cys Thr Ala Ser Gly Phe Ser Leu Ser Asn Tyr Tyr
               20                  25                  30
   Val Thr Trp Val Arg Gln Ala Pro Gly Lys Gly Leu Glu Trp Ile Gly
           35                  40                  45
   Ile Ile Tyr Gly Ser Asp Glu Thr Ala Tyr Ala Thr Trp Ala Ile Gly
       50                  55                  60
   Arg Phe Thr Ile Ser Lys Thr Ser Thr Thr Val Asp Leu Lys Met Thr
   65                  70                  75                  80
   Ser Leu Thr Ala Ala Asp Thr Ala Thr Tyr Phe Cys Ala Arg Asp Asp
                   85                  90                  95
   Ser Ser Asp Trp Asp Ala Lys Phe Asn Leu Trp Gly Gln Gly Thr Leu
               100                 105                 110
   Val Thr Val Ser Ser
           115
<210> 653
   <211> 97
   <212> PRT
   <213> Homo sapiens
<400> 653
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Val Ile Tyr Ser Gly Gly Ser Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg
<210> 654
   <211> 97
   <212> PRT
   <213> Homo sapiens
<400> 654
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Val Ser Ser Asn
               20                  25                  30
   Tyr Met Ser Trp Val Arg Gln Ala Pro Gly Lys Gly Leu Glu Trp Val
           35                  40                  45
   Ser Val Ile Tyr Ser Gly Gly Ser Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg
<210> 655
   <211> 98
   <212> PRT
   <213> Homo sapiens
<400> 655
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Tyr Ser Gly Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys
<210> 656
   <211> 120
   <212> PRT
   <213> Artificial sequence
<220>
   <223> Humanized antibody
<400> 656
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser
           115                 120
<210> 657
   <211> 120
   <212> PRT
   <213> Artificial sequence
<220>
   <223> Humanized antibody
<400> 657
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser
   115 120
<210> 658
   <211> 166
   <212> PRT
   <213> Oryctolagus cuniculus
<400> 658
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Asn Tyr Tyr Val Thr Trp Val Arg Gln Ala Pro Gly Lys Gly Leu Glu
   50 55 60
   Trp Ile Gly Ile Ile Tyr Gly Ser Asp Glu Thr Ala Tyr Ala Thr Ser
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu Trp Gly Gln
   115 120 125
   Gly Thr Leu Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys
   165
<210> 659
   <211> 16
   <212> PRT
   <213> oryctolagus cuniculus
<400> 659
   Ile Ile Tyr Gly Ser Asp Glu Thr Ala Tyr Ala Thr Ser Ala Ile Gly
   1 5 10 15
<210> 660
   <211> 122
   <212> PRT
   <213> oryctolagus cuniculus
<400> 660
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Ser Ala Ala Val Gly Gly Thr Val Thr Ile Lys Cys Gln Ala Ser
           35                  40                  45
   Gln Ser Ile Asn Asn Glu Leu Ser Trp Tyr Gln Gln Lys Pro Gly Gln
       50                  55                  60
   Arg Pro Lys Leu Leu Ile Tyr Arg Ala Ser Thr Leu Ala Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Gln
               100                 105                 110
   Gly Tyr Ser Leu Arg Asn Ile Asp Asn Ala
           115                 120
<210> 661
   <211> 125
   <212> PRT
   <213> oryctolagus cuniculus
<400> 661
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
   35 40 45
   Asn Tyr Tyr Val Thr Trp Val Arg Gln Ala Pro Gly Lys Gly Leu Glu
   50 55 60
   Trp Ile Gly Ile Ile Tyr Gly Ser Asp Glu Thr Ala Tyr Ala Thr Trp
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu
   115 120 125
<210> 662
   <211> 366
   <212> DNA
   <213> oryctolagus cuniculus
<400> 662
   atggacacga gggcccccac tcagctgctg gggctcctgc tgctctggct cccaggtgcc 60
   agatgtgcct atgatatgac ccagactcca gcctcggtgt ctgcagctgt gggaggcaca 120
   gtcaccatca agtgccaggc cagtcagagc attaacaatg aattatcctg gtatcagcag 180
   aaaccagggc agcgtcccaa gctcctgatc tatagggcat ccactctggc atctggggtc 240
   tcatcgcggt tcaaaggcag tggatctggg acagagttca ctctcaccat cagcgacctg 300
   gagtgtgccg atgctgccac ttactactgt caacagggtt atagtctgag gaatattgat 360
   aatgct 366
<210> 663
   <211> 375
   <212> DNA
   <213> Oryctolagus cuniculus
<400> 663
   atggagactg ggctgcgctg gcttctcctg gtcgctgtgc tcaaaggtgt ccagtgtcag        60
   tcgctggagg agtccggggg tcgcctggtc acgcctggga cacccctgac actcacctgc       120
   acagcctctg gattctccct cagtaactac tacgtgacct gggtccgcca ggctccaggg       180
   aaggggctgg aatggatcgg aatcatttat ggtagtgatg aaacggccta cgcgacctgg       240
   gcgataggcc gattcaccat ctccaaaacc tcgaccacgg tggatctgaa aatgaccagt       300
   ctgacagccg cggacacggc cacctatttc tgtgccagag atgatagtag tgactgggat       360
   gcaaaattta acttg                                                        375
<210> 664
   <211> 450
   <212> PRT
   <213> Oryctolagus cuniculus
<400> 664
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Trp Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 665
   <211> 450
   <212> PRT
   <213> Oryctolagus cuniculus
<400> 665
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 666
   <211> 216
   <212> PRT
   <213> Oryctolagus cuniculus
<400> 666
   Ile Gln Met Thr Gln Ser Pro Ser Ser Leu Ser Ala Ser Val Gly Asp
   1 5 10 15
   Arg Val Thr Ile Thr Cys Gln Ala Ser Gln Ser Ile Asn Asn Glu Leu
   20 25 30
   Ser Trp Tyr Gln Gln Lys Pro Gly Lys Ala Pro Lys Leu Leu Ile Tyr
   35 40 45
   Arg Ala Ser Thr Leu Ala Ser Gly Val Pro Ser Arg Phe Ser Gly Ser
   50 55 60
   Gly Ser Gly Thr Asp Phe Thr Leu Thr Ile Ser Ser Leu Gln Pro Asp
   65 70 75 80
   Asp Phe Ala Thr Tyr Tyr Cys Gln Gln Gly Tyr Ser Leu Arg Asn Ile
   85 90 95
   Asp Asn Ala Phe Gly Gly Gly Thr Lys Val Glu Ile Lys Arg Thr Val
   100 105 110
   Ala Ala Pro Ser Val Phe Ile Phe Pro Pro Ser Asp Glu Gln Leu Lys
   115 120 125
   Ser Gly Thr Ala Ser Val Val Cys Leu Leu Asn Asn Phe Tyr Pro Arg
   130 135 140
   Glu Ala Lys Val Gln Trp Lys Val Asp Asn Ala Leu Gln Ser Gly Asn
   145 150 155 160
   Ser Gln Glu Ser Val Thr Glu Gln Asp Ser Lys Asp Ser Thr Tyr Ser
   165 170 175
   Leu Ser Ser Thr Leu Thr Leu Ser Lys Ala Asp Tyr Glu Lys His Lys
   180 185 190
   Val Tyr Ala Cys Glu Val Thr His Gln Gly Leu Ser Ser Pro Val Thr
   195 200 205
   Lys Ser Phe Asn Arg Gly Glu Cys
   210 215
<210> 667
   <211> 122
   <212> PRT
   <213> Oryctolagus cuniculus
<400> 667
   Met Asp Thr Arg Ala Pro Thr Gln Leu Leu Gly Leu Leu Leu Leu Trp
   1 5 10 15
   Leu Pro Gly Ala Arg Cys Ala Tyr Asp Met Thr Gln Thr Pro Ala Ser
   20 25 30
   Val Glu Val Ala Val Gly Gly Thr Val Thr Ile Asn Cys Gln Ala Ser
   35 40 45
   Glu Thr Ile Tyr Ser Trp Leu Ser Trp Tyr Gln Gln Lys Pro Gly Gln
   50 55 60
   Pro Pro Lys Leu Leu Ile Tyr Gln Ala Ser Asp Leu Ala Ser Gly Val
   65 70 75 80
   Pro Ser Arg Phe Ser Gly Ser Gly Ala Gly Thr Glu Tyr Thr Leu Thr
   85 90 95
   Ile Ser Gly Val Gln Cys Asp Asp Ala Ala Thr Tyr Tyr Cys Gln Gln
   100 105 110
   Gly Tyr Ser Gly Ser Asn Val Asp Asn Val
   115 120
<210> 668
   <211> 126
   <212> PRT
   <213> oryctolagus cuniculus
<400> 668
   Met Glu Thr Gly Leu Arg Trp Leu Leu Leu Val Ala Val Leu Lys Gly
   1 5 10 15
   Val Gln Cys Gln Glu Gln Leu Lys Glu Ser Gly Gly Arg Leu Val Thr
   20 25 30
   Pro Gly Thr Pro Leu Thr Leu Thr Cys Thr Ala Ser Gly Phe Ser Leu
   35 40 45
   Asn Asp His Ala Met Gly Trp Val Arg Gln Ala Pro Gly Lys Gly Leu
   50 55 60
   Glu Tyr Ile Gly Phe Ile Asn Ser Gly Gly Ser Ala Arg Tyr Ala Ser
   65 70 75 80
   Trp Ala Glu Gly Arg Phe Thr Ile Ser Arg Thr Ser Thr Thr Val Asp
   85 90 95
   Leu Lys Met Thr Ser Leu Thr Thr Glu Asp Thr Ala Thr Tyr Phe Cys
   100 105 110
   Val Arg Gly Gly Ala Val Trp Ser Ile His Ser Phe Asp Pro
   115 120 125
<210> 669
   <211> 366
   <212> DNA
   <213> oryctolagus cuniculus
<400> 669
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca attgccaggc cagtgagacc atttacagtt ggttatcctg gtatcagcag 180
   aagccagggc agcctcccaa gctcctgatc taccaggcat ccgatctggc atctggggtc 240
   ccatcgcgat tcagcggcag tggggctggg acagagtaca ctctcaccat cagcggcgtg 300
   cagtgtgacg atgctgccac ttactactgt caacagggtt atagtggtag taatgttgat 360
   aatgtt 366
<210> 670
   <211> 378
   <212> DNA
   <213> Oryctolagus cuniculus
<400> 670
   atggagactg ggctgcgctg gcttctcctg gtcgctgtgc tcaaaggtgt ccagtgtcag 60
   gagcagctga aggagtccgg gggtcgcctg gtcacgcctg ggacacccct gacacttacc 120
   tgcacagcct ctggattctc cctcaatgac catgcaatgg gctgggtccg ccaggctcca 180
   gggaaggggc tggaatacat cggattcatt aatagtggtg gtagcgcacg ctacgcgagc 240
   tgggcagaag gccgattcac catctccaga acctcgacca cggtggatct gaaaatgacc 300
   agtctgacaa ccgaggacac ggccacctat ttctgtgtca gagggggtgc tgtttggagt 360
   attcatagtt ttgatccc 378
<210> 671
   <211> 123
   <212> PRT
   <213> Oryctolagus cuniculus
<400> 671
   Met Asp Thr Arg Ala Pro Thr Gln Leu Leu Gly Leu Leu Leu Leu Trp
   1 5 10 15
   Leu Pro Gly Ala Thr Phe Ala Ala Val Leu Thr Gln Thr Pro Ser Pro
   20 25 30
   Val Ser Ala Ala Val Gly Gly Thr Val Ser Ile Ser Cys Gln Ala Ser
   35 40 45
   Gln Ser Val Tyr Asp Asn Asn Tyr Leu Ser Trp Phe Gln Gln Lys Pro
   50 55 60
   Gly Gln Pro Pro Lys Leu Leu Ile Tyr Gly Ala Ser Thr Leu Ala Ser
   65 70 75 80
   Gly Val Pro Ser Arg Phe Val Gly Ser Gly Ser Gly Thr Gln Phe Thr
   85 90 95
   Leu Thr Ile Thr Asp Val Gln Cys Asp Asp Ala Ala Thr Tyr Tyr Cys
   100 105 110
   Ala Gly Val Tyr Asp Asp Asp Ser Asp Asn Ala
   115 120
<210> 672
   <211> 125
   <212> PRT
   <213> Oryctolagus cuniculus
<400> 672
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Val Tyr Tyr Met Asn Trp Val Arg Gln Ala Pro Gly Lys Gly Leu Glu
   50 55 60
   Trp Ile Gly Phe Ile Thr Met Ser Asp Asn Ile Asn Tyr Ala Ser Trp
   65 70 75 80
   Ala Lys Gly Arg Phe Thr Ile Ser Lys Thr Ser Thr Thr Val Asp Leu
   85 90 95
   Lys Met Thr Ser Pro Thr Thr Glu Asp Thr Ala Thr Tyr Phe Cys Ala
   100 105 110
   Arg Ser Arg Gly Trp Gly Thr Met Gly Arg Leu Asp Leu
   115 120 125
<210> 673
   <211> 369
   <212> DNA
   <213> Oryctolagus cuniculus
<400> 673
   atggacacga gggcccccac tcagctgctg gggctcctgc tgctctggct cccaggtgcc 60
   acatttgccg ccgtgctgac ccagactcca tctcccgtgt ctgcagctgt gggaggcaca 120
   gtcagcatca gttgccaggc cagtcagagt gtttatgaca acaactactt atcctggttt 180
   cagcagaaac cagggcagcc tcccaagctc ctgatctatg gtgcatccac tctggcatct 240
   ggggtcccat cgcggttcgt gggcagtgga tctgggacac agttcactct caccatcaca 300
   gacgtgcagt gtgacgatgc tgccacttac tattgtgcag gcgtttatga tgatgatagt 360
   gataatgcc 369
<210> 674
   <211> 375
   <212> DNA
   <213> Oryctolagus cuniculus
<400> 674
   atggagactg ggctgcgctg gcttctcctg gtggctgtgc tcaaaggtgt ccagtgtcag 60
   tcgctggagg agtccggggg tcgcctggtc acccctggga cacccctgac actcacctgc 120
   acagcctctg gattctccct cagtgtctac tacatgaact gggtccgcca ggctccaggg 180
   aaggggctgg aatggatcgg attcattaca atgagtgata atataaatta cgcgagctgg 240
   gcgaaaggcc gattcaccat ctccaaaacc tcgaccacgg tggatctgaa aatgaccagt 300
   ccgacaaccg aggacacggc cacctatttc tgtgccagga gtcgtggctg gggtacaatg 360
   ggtcggttgg atctc 375
<210> 675
   <211> 123
   <212> PRT
   <213> oryctolagus cuniculus
<400> 675
   Met Asp Thr Arg Ala Pro Thr Gln Leu Leu Gly Leu Leu Leu Leu Trp
   1 5 10 15
   Leu Pro Gly Ala Ile Cys Asp Pro Val Leu Thr Gln Thr Pro Ser Pro
   20 25 30
   Val Ser Ala Pro Val Gly Gly Thr Val Ser Ile Ser Cys Gln Ala Ser
   35 40 45
   Gln Ser Val Tyr Glu Asn Asn Tyr Leu Ser Trp Phe Gln Gln Lys Pro
   50 55 60
   Gly Gln Pro Pro Lys Leu Leu Ile Tyr Gly Ala Ser Thr Leu Asp Ser
   65 70 75 80
   Gly Val Pro Ser Arg Phe Lys Gly Ser Gly Ser Gly Thr Gln Phe Thr
   85 90 95
   Leu Thr Ile Thr Asp Val Gln Cys Asp Asp Ala Ala Thr Tyr Tyr Cys
   100 105 110
   Ala Gly Val Tyr Asp Asp Asp Ser Asp Asp Ala
   115 120
<210> 676
   <211> 126
   <212> PRT
   <213> oryctolagus cuniculus
<400> 676
   Met Glu Thr Gly Leu Arg Trp Leu Leu Leu Val Ala Val Leu Lys Gly
   1 5 10 15
   Val Gln Cys Gln Glu Gln Leu Lys Glu Ser Gly Gly Gly Leu Val Thr
   20 25 30
   Pro Gly Gly Thr Leu Thr Leu Thr Cys Thr Ala Ser Gly Phe Ser Leu
   35 40 45
   Asn Ala Tyr Tyr Met Asn Trp Val Arg Gln Ala Pro Gly Lys Gly Leu
   50 55 60
   Glu Trp Ile Gly Phe Ile Thr Leu Asn Asn Asn Val Ala Tyr Ala Asn
   65 70 75 80
   Trp Ala Lys Gly Arg Phe Thr Phe Ser Lys Thr Ser Thr Thr Val Asp
   85 90 95
   Leu Lys Met Thr Ser Pro Thr Pro Glu Asp Thr Ala Thr Tyr Phe Cys
   100 105 110
   Ala Arg Ser Arg Gly Trp Gly Ala Met Gly Arg Leu Asp Leu
   115 120 125
<210> 677
   <211> 369
   <212> DNA
   <213> Oryctolagus cuniculus
<400> 677
   atggacacga gggcccccac tcagctgctg gggctcctgc tgctctggct cccaggtgcc 60
   atatgtgacc ctgtgctgac ccagactcca tctcccgtat ctgcacctgt gggaggcaca 120
   gtcagcatca gttgccaggc cagtcagagt gtttatgaga acaactattt atcctggttt 180
   cagcagaaac cagggcagcc tcccaagctc ctgatctatg gtgcatccac tctggattct 240
   ggggtcccat cgcggttcaa aggcagtgga tctgggacac agttcactct caccattaca 300
   gacgtgcagt gtgacgatgc tgccacttac tattgtgcag gcgtttatga tgatgatagt 360
   gatgatgcc 369
<210> 678
   <211> 378
   <212> DNA
   <213> Oryctolagus cuniculus
<400> 678
   atggagactg ggctgcgctg gcttctcctg gtggctgtgc tcaaaggtgt ccagtgtcag    60
   gagcagctga aggagtccgg aggaggcctg gtaacgcctg gaggaaccct gacactcacc   120
   tgcacagcct ctggattctc cctcaatgcc tactacatga actgggtccg ccaggctcca   180
   gggaaggggc tggaatggat cggattcatt actctgaata ataatgtagc ttacgcgaac   240
   tgggcgaaag gccgattcac cttctccaaa acctcgacca cggtggatct gaaaatgacc   300
   agtccgacac ccgaggacac ggccacctat ttctgtgcca ggagtcgtgg ctggggtgca   360
   atgggtcggt tggatctc                                                 378
<210> 679 Page 169
   <211> 122
   <212> PRT
   <213> Oryctolagus cuniculus
<400> 679
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Pro Ser Pro
               20                  25                  30
   Val Ser Ala Ala Val Gly Gly Thr Val Thr Ile Asn Cys Gln Ala Ser
           35                  40                  45
   Gln Ser Val Asp Asp Asn Asn Trp Leu Gly Trp Tyr Gln Gln Lys Arg
       50                  55                  60
   Gly Gln Pro Pro Lys Tyr Leu Ile Tyr Ser Ala Ser Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Asp Leu Glu Cys Asp Asp Ala Ala Thr Tyr Tyr Cys
               100                 105                 110
   Ala Gly Gly Phe Ser Gly Asn Ile Phe Ala
           115                 120
<210> 680
   <211> 122
   <212> PRT
   <213> oryctolagus cuniculus
<400> 680
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Tyr Ala Met Ser Trp Val Arg Gln Ala Pro Gly Lys Gly Leu Glu
       50                  55                  60
   Trp Ile Gly Ile Ile Gly Gly Phe Gly Thr Thr Tyr Tyr Ala Thr Trp
   65                  70                  75                  80
   Ala Lys Gly Arg Phe Thr Ile Ser Lys Thr Ser Thr Thr Val Asp Leu
                   85                  90                  95
   Arg Ile Thr Ser Pro Thr Thr Glu Asp Thr Ala Thr Tyr Phe Cys Ala
               100                 105                 110
   Arg Gly Gly Pro Gly Asn Gly Gly Asp Ile
           115                 120
<210> 681
   <211> 366
   <212> DNA
   <213> Oryctolagus cuniculus
<400> 681
   atggacacga gggcccccac tcagctgctg gggctcctgc tgctctggct cccaggtgcc 60
   acatttgccc aagtgctgac ccagactcca tcgcctgtgt ctgcagctgt gggaggcaca 120
   gtcaccatca actgccaggc cagtcagagt gttgatgata acaactggtt aggctggtat 180
   cagcagaaac gagggcagcc tcccaagtac ctgatctatt ctgcatccac tctggcatct 240
   ggggtcccat cgcggttcaa aggcagtgga tctgggacac agttcactct caccatcagc 300
   gacctggagt gtgacgatgc tgccacttac tactgtgcag gcggttttag tggtaatatc 360
   tttgct 366
<210> 682
   <211> 366
   <212> DNA
   <213> Oryctolagus cuniculus
<400> 682
   atggagactg ggctgcgctg gcttctcctg gtcgctgtgc tcaaaggtgt ccagtgtcag        60
   tcggtggagg agtccggggg tcgcctggtc acgcctggga cacccctgac actcacctgc       120
   acagtctctg gcttctccct cagtagctat gcaatgagct gggtccgcca ggctccagga       180
   aaggggctgg agtggatcgg aatcattggt ggttttggta ccacatacta cgcgacctgg       240
   gcgaaaggcc gattcaccat ctccaaaacc tcgaccacgg tggatctgag aatcaccagt       300
   ccgacaaccg aggacacggc cacctatttc tgtgccagag gtggtcctgg taatggtggt       360
   gacatc                                                                  366
<210> 683
   <211> 122
   <212> PRT
   <213> Oryctolagus cuniculus
<400> 683
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Val Pro Val Gly Gly Thr Val Thr Ile Lys Cys Gln Ser Ser
           35                  40                  45
   Gln Ser Val Tyr Asn Asn Phe Leu Ser Trp Tyr Gln Gln Lys Pro Gly
       50                  55                  60
   Gln Pro Pro Lys Leu Leu Ile Tyr Gln Ala Ser Lys Leu Ala Ser Gly
   65                  70                  75                  80
   Val Pro Asp Arg Phe Ser Gly Ser Gly Ser Gly Thr Gln Phe Thr Leu
                   85                  90                  95
   Thr Ile Ser Gly Val Gln Cys Asp Asp Ala Ala Thr Tyr Tyr Cys Leu
               100                 105                 110
   Gly Gly Tyr Asp Asp Asp Ala Asp Asn Ala
           115                 120
<210> 684
   <211> 128
   <212> PRT
   <213> oryctolagus cuniculus
<400> 684
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Ile Asp Leu Ser
   35 40 45
   Asp Tyr Ala Met Ser Trp Val Arg Gln Ala Pro Gly Lys Gly Leu Glu
   50 55 60
   Trp Ile Gly Ile Ile Tyr Ala Gly Ser Gly Ser Thr Trp Tyr Ala Ser
   65 70 75 80
   Trp Ala Lys Gly Arg Phe Thr Ile Ser Lys Thr Ser Thr Thr Val Asp
   85 90 95
   Leu Lys Ile Thr Ser Pro Thr Thr Glu Asp Thr Ala Thr Tyr Phe Cys
   100 105 110
   Ala Arg Asp Gly Tyr Asp Asp Tyr Gly Asp Phe Asp Arg Leu Asp Leu
   115 120 125
<210> 685
   <211> 366
   <212> DNA
   <213> Oryctolagus cuniculus
<400> 685
   atggacacga gggcccccac tcagctgctg gggctcctgc tgctctggct cccaggtgcc 60
   acatttgcag ccgtgctgac ccagacacca tcgcccgtgt ctgtacctgt gggaggcaca 120
   gtcaccatca agtgccagtc cagtcagagt gtttataata atttcttatc gtggtatcag 180
   cagaaaccag ggcagcctcc caagctcctg atctaccagg catccaaact ggcatctggg 240
   gtcccagata ggttcagcgg cagtggatct gggacacagt tcactctcac catcagcggc 300
   gtgcagtgtg acgatgctgc cacttactac tgtctaggcg gttatgatga tgatgctgat 360
   aatgct 366
<210> 686
   <211> 384
   <212> DNA
   <213> Oryctolagus cuniculus
<400> 686
   atggagactg ggctgcgctg gcttctcctg gtcgctgtgc tcaaaggtgt ccagtgtcag        60
   tcggtggagg agtccggggg tcgcctggtc acgcctggga cacccctgac gctcacctgc       120
   acagtctctg gaatcgacct cagtgactat gcaatgagct gggtccgcca ggctccaggg       180
   aaggggctgg aatggatcgg aatcatttat gctggtagtg gtagcacatg gtacgcgagc       240
   tgggcgaaag gccgattcac catctccaaa acctcgacca cggtggatct gaaaatcacc       300
   agtccgacaa ccgaggacac ggccacctat ttctgtgcca gagatggata cgatgactat       360
   ggtgatttcg atcgattgga tctc                                              384
<210> 687
   <211> 122
   <212> PRT
   <213> Oryctolagus cuniculus
<400> 687
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Ser Ala Ala Val Gly Gly Thr Val Thr Ile Lys Cys Gln Ala Ser
           35                  40                  45
   Gln Ser Ile Asn Asn Glu Leu Ser Trp Tyr Gln Gln Lys Ser Gly Gln
       50                  55                  60
   Arg Pro Lys Leu Leu Ile Tyr Arg Ala Ser Thr Leu Ala Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Gln
               100                 105                 110
   Gly Tyr Ser Leu Arg Asn Ile Asp Asn Ala
           115                 120
<210> 688
   <211> 125
   <212> PRT
   <213> Oryctolagus cuniculus
<400> 688
   Met Glu Thr Gly Leu Arg Trp Leu Leu Leu Val Ala Val Leu Ser Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Asn Tyr Tyr Met Thr Trp Val Arg Gln Ala Pro Gly Lys Gly Leu Glu
   50 55 60
   Trp Ile Gly Met Ile Tyr Gly Ser Asp Glu Thr Ala Tyr Ala Asn Trp
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu
   115 120 125
<210> 689
   <211> 366
   <212> DNA
   <213> Oryctolagus cuniculus
<400> 689
   atggacacga gggcccccac tcagctgctg gggctcctgc tgctctggct cccaggtgcc 60
   agatgtgcct atgatatgac ccagactcca gcctcggtgt ctgcagctgt gggaggcaca 120
   gtcaccatca aatgccaggc cagtcagagc attaacaatg aattatcctg gtatcagcag 180
   aaatcagggc agcgtcccaa gctcctgatc tatagggcat ccactctggc atctggggtc 240
   tcatcgcggt tcaaaggcag tggatctggg acagagttca ctctcaccat cagcgacctg 300
   gagtgtgccg atgctgccac ttactactgt caacagggtt atagtctgag gaatattgat 360
   aatgct 366
<210> 690
   <211> 375
   <212> DNA
   <213> Oryctolagus cuniculus
<400> 690
   atggagactg ggctgcgctg gcttctcctg gtcgctgtgc tctcaggtgt ccagtgtcag        60
   tcgctggagg agtccggggg tcgcctggtc acgcctggga cacccctgac actcacctgc       120
   acagcctctg gattctccct cagtaactac tacatgacct gggtccgcca ggctccaggg       180
   aaggggctgg aatggatcgg aatgatttat ggtagtgatg aaacagccta cgcgaactgg       240
   gcgataggcc gattcaccat ctccaaaacc tcgaccacgg tggatctgaa aatgaccagt       300
   ctgacagccg cggacacggc cacctatttc tgtgccagag atgatagtag tgactgggat       360
   gcaaaattta acttg                                                        375
<210> 691
   <211> 450
   <212> PRT
   <213> Oryctolagus cuniculus
<400> 691
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Met Thr Trp Val Arg Gln Ala Pro Gly Lys Gly Leu Glu Trp Val
           35                  40                  45
   Gly Met Ile Tyr Gly Ser Asp Glu Thr Ala Tyr Ala Asn Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 692
   <211> 450
   <212> PRT
   <213> Oryctolagus cuniculus
<400> 692
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Met Thr Trp Val Arg Gln Ala Pro Gly Lys Gly Leu Glu Trp Val
   35 40 45
   Gly Met Ile Tyr Gly Ser Asp Glu Thr Ala Tyr Ala Asn Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 693
   <211> 217
   <212> PRT
   <213> Oryctolagus cuniculus
<400> 693
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 694
   <211> 33
   <212> DNA
   <213> Oryctolagus cuniculus
<400> 694
   caggccagtc agagcattaa caatgagtta tcc 33
<210> 695
   <211> 36
   <212> DNA
   <213> oryctolagus cuniculus
<400> 695
   caacagggtt atagtctgag gaacattgat aatgct 36
<210> 696
   <211> 48
   <212> DNA
   <213> Oryctolagus cuniculus
<400> 696
   atcatctatg gtagtgatga aaccgcctac gctacctccg ctataggc 48
<210> 697
   <211> 36
   <212> DNA
   <213> Oryctolagus cuniculus
<400> 697
   gatgatagta gtgactggga tgcaaagttc aacttg 36
<210> 698
   <211> 336
   <212> DNA
   <213> Oryctolagus cuniculus
<400> 698
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgtacg 336
<210> 699
   <211> 112
   <212> PRT
   <213> Oryctolagus cuniculus
<400> 699
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
<210> 700
   <211> 360
   <212> DNA
   <213> oryctolagus cuniculus
<400> 700
   gaggtgcagc tggtggagtc tgggggaggc ttggtccagc ctggggggtc cctgagactc      60
   tcctgtgcag cctctggatt ctccctcagt aactactacg tgacctgggt ccgtcaggct     120
   ccagggaagg ggctggagtg ggtcggcatc atctatggta gtgatgaaac cgcctacgct     180
   acctccgcta taggccgatt caccatctcc agagacaatt ccaagaacac cctgtatctt     240
   caaatgaaca gcctgagagc tgaggacact gctgtgtatt actgtgctag agatgatagt     300
   agtgactggg atgcaaagtt caacttgtgg ggccaaggga ccctcgtcac cgtctcgagc     360
<210> 701
   <211> 651
   <212> DNA
   <213> Oryctolagus cuniculus
<400> 701
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgtacggtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg t 651
<210> 702
   <211> 217
   <212> PRT
   <213> Oryctolagus cuniculus
<400> 702
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 703
   <211> 1350
   <212> DNA
   <213> Oryctolagus cuniculus
<400> 703
   gaggtgcagc tggtggagtc tgggggaggc ttggtccagc ctggggggtc cctgagactc      60
   tcctgtgcag cctctggatt ctccctcagt aactactacg tgacctgggt ccgtcaggct     120
   ccagggaagg ggctggagtg ggtcggcatc atctatggta gtgatgaaac cgcctacgct     180
   acctccgcta taggccgatt caccatctcc agagacaatt ccaagaacac cctgtatctt     240
   caaatgaaca gcctgagagc tgaggacact gctgtgtatt actgtgctag agatgatagt     300
   agtgactggg atgcaaagtt caacttgtgg ggccaaggga ccctcgtcac cgtctcgagc     360
   gcctccacca agggcccatc ggtcttcccc ctggcaccct cctccaagag cacctctggg     420
   ggcacagcgg ccctgggctg cctggtcaag gactacttcc ccgaaccggt gacggtgtcg     480
   tggaactcag gcgccctgac cagcggcgtg cacaccttcc cggctgtcct acagtcctca     540
   ggactctact ccctcagcag cgtggtgacc gtgccctcca gcagcttggg cacccagacc     600
   tacatctgca acgtgaatca caagcccagc aacaccaagg tggacaagag agttgagccc     660
   aaatcttgtg acaaaactca cacatgccca ccgtgcccag cacctgaact cctgggggga     720
   ccgtcagtct tcctcttccc cccaaaaccc aaggacaccc tcatgatctc ccggacccct     780
   gaggtcacat gcgtggtggt ggacgtgagc cacgaagacc ctgaggtcaa gttcaactgg     840
   tacgtggacg gcgtggaggt gcataatgcc aagacaaagc cgcgggagga gcagtacgcc     900
   agcacgtacc gtgtggtcag cgtcctcacc gtcctgcacc aggactggct gaatggcaag     960
   gagtacaagt gcaaggtctc caacaaagcc ctcccagccc ccatcgagaa aaccatctcc    1020
   aaagccaaag ggcagccccg agaaccacag gtgtacaccc tgcccccatc ccgggaggag    1080
   atgaccaaga accaggtcag cctgacctgc ctggtcaaag gcttctatcc cagcgacatc    1140
   gccgtggagt gggagagcaa tgggcagccg gagaacaact acaagaccac gcctcccgtg    1200
   ctggactccg acggctcctt cttcctctac agcaagctca ccgtggacaa gagcaggtgg    1260
   cagcagggga acgtcttctc atgctccgtg atgcatgagg ctctgcacaa ccactacacg    1320
   cagaagagcc tctccctgtc tccgggtaaa                                     1350
<210> 704
   <211> 450
   <212> PRT
   <213> Oryctolagus cuniculus
<400> 704
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Glu Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 705
   <211> 705
   <212> DNA
   <213> Oryctolagus cuniculus
<400> 705
   atgaagtggg taacctttat ttcccttctg tttctcttta gcagcgctta ttccgctatc        60
   cagatgaccc agtctccttc ctccctgtct gcatctgtag gagacagagt caccatcact       120
   tgccaggcca gtcagagcat taacaatgag ttatcctggt atcagcagaa accagggaaa       180
   gcccctaagc tcctgatcta tagggcatcc actctggcat ctggggtccc atcaaggttc       240
   agcggcagtg gatctgggac agacttcact ctcaccatca gcagcctgca gcctgatgat       300
   tttgcaactt attactgcca acagggttat agtctgagga acattgataa tgctttcggc       360
   ggagggacca aggtggaaat caaacgtacg gtggctgcac catctgtctt catcttcccg       420
   ccatctgatg agcagttgaa atctggaact gcctctgttg tgtgcctgct gaataacttc       480
   tatcccagag aggccaaagt acagtggaag gtggataacg ccctccaatc gggtaactcc       540
   caggagagtg tcacagagca ggacagcaag gacagcacct acagcctcag cagcaccctg       600
   acgctgagca aagcagacta cgagaaacac aaagtctacg cctgcgaagt cacccatcag       660
   ggcctgagct cgcccgtcac aaagagcttc aacaggggag agtgt                       705
<210> 706
   <211> 235
   <212> PRT
   <213> Oryctolagus cuniculus
<400> 706
   Met Lys Trp Val Thr Phe Ile Ser Leu Leu Phe Leu Phe Ser Ser Ala
   1 5 10 15
   Tyr Ser Ala Ile Gln Met Thr Gln Ser Pro Ser Ser Leu Ser Ala Ser
   20 25 30
   Val Gly Asp Arg Val Thr Ile Thr Cys Gln Ala Ser Gln Ser Ile Asn
   35 40 45
   Asn Glu Leu Ser Trp Tyr Gln Gln Lys Pro Gly Lys Ala Pro Lys Leu
   50 55 60
   Leu Ile Tyr Arg Ala Ser Thr Leu Ala Ser Gly Val Pro Ser Arg Phe
   65 70 75 80
   Ser Gly Ser Gly Ser Gly Thr Asp Phe Thr Leu Thr Ile Ser Ser Leu
   85 90 95
   Gln Pro Asp Asp Phe Ala Thr Tyr Tyr Cys Gln Gln Gly Tyr Ser Leu
   100 105 110
   Arg Asn Ile Asp Asn Ala Phe Gly Gly Gly Thr Lys Val Glu Ile Lys
   115 120 125
   Arg Thr Val Ala Ala Pro Ser Val Phe Ile Phe Pro Pro Ser Asp Glu
   130 135 140
   Gln Leu Lys Ser Gly Thr Ala Ser Val Val Cys Leu Leu Asn Asn Phe
   145 150 155 160
   Tyr Pro Arg Glu Ala Lys Val Gln Trp Lys Val Asp Asn Ala Leu Gln
   165 170 175
   Ser Gly Asn Ser Gln Glu Ser Val Thr Glu Gln Asp Ser Lys Asp Ser
   180 185 190
   Thr Tyr Ser Leu Ser Ser Thr Leu Thr Leu Ser Lys Ala Asp Tyr Glu
   195 200 205
   Lys His Lys Val Tyr Ala Cys Glu Val Thr His Gln Gly Leu Ser Ser
   210 215 220
   Pro Val Thr Lys Ser Phe Asn Arg Gly Glu Cys
   225 230 235
<210> 707
   <211> 1404
   <212> DNA
   <213> Oryctolagus cuniculus
<400> 707
   atgaagtggg taacctttat ttcccttctg tttctcttta gcagcgctta ttccgaggtg      60
   cagctggtgg agtctggggg aggcttggtc cagcctgggg ggtccctgag actctcctgt     120
   gcagcctctg gattctccct cagtaactac tacgtgacct gggtccgtca ggctccaggg     180
   aaggggctgg agtgggtcgg catcatctat ggtagtgatg aaaccgccta cgctacctcc     240
   gctataggcc gattcaccat ctccagagac aattccaaga acaccctgta tcttcaaatg     300
   aacagcctga gagctgagga cactgctgtg tattactgtg ctagagatga tagtagtgac     360
   tgggatgcaa agttcaactt gtggggccaa gggaccctcg tcaccgtctc gagcgcctcc     420
   accaagggcc catcggtctt ccccctggca ccctcctcca agagcacctc tgggggcaca     480
   gcggccctgg gctgcctggt caaggactac ttccccgaac cggtgacggt gtcgtggaac     540
   tcaggcgccc tgaccagcgg cgtgcacacc ttcccggctg tcctacagtc ctcaggactc     600
   tactccctca gcagcgtggt gaccgtgccc tccagcagct tgggcaccca gacctacatc     660
   tgcaacgtga atcacaagcc cagcaacacc aaggtggaca agagagttga gcccaaatct     720
   tgtgacaaaa ctcacacatg cccaccgtgc ccagcacctg aactcctggg gggaccgtca     780
   gtcttcctct tccccccaaa acccaaggac accctcatga tctcccggac ccctgaggtc     840
   acatgcgtgg tggtggacgt gagccacgaa gaccctgagg tcaagttcaa ctggtacgtg     900
   gacggcgtgg aggtgcataa tgccaagaca aagccgcggg aggagcagta cgccagcacg     960
   taccgtgtgg tcagcgtcct caccgtcctg caccaggact ggctgaatgg caaggagtac    1020
   aagtgcaagg tctccaacaa agccctccca gcccccatcg agaaaaccat ctccaaagcc    1080
   aaagggcagc cccgagaacc acaggtgtac accctgcccc catcccggga ggagatgacc    1140
   aagaaccagg tcagcctgac ctgcctggtc aaaggcttct atcccagcga catcgccgtg    1200
   gagtgggaga gcaatgggca gccggagaac aactacaaga ccacgcctcc cgtgctggac    1260
   tccgacggct ccttcttcct ctacagcaag ctcaccgtgg acaagagcag gtggcagcag    1320
   gggaacgtct tctcatgctc cgtgatgcat gaggctctgc acaaccacta cacgcagaag    1380
   agcctctccc tgtctccggg taaa                                           1404
<210> 708
   <211> 468
   <212> PRT
   <213> Oryctolagus cuniculus
<400> 708
   Met Lys Trp Val Thr Phe Ile Ser Leu Leu Phe Leu Phe Ser Ser Ala
   1 5 10 15
   Tyr Ser Glu Val Gln Leu Val Glu Ser Gly Gly Gly Leu Val Gln Pro
   20 25 30
   Gly Gly Ser Leu Arg Leu Ser Cys Ala Ala Ser Gly Phe Ser Leu Ser
   35 40 45
   Asn Tyr Tyr Val Thr Trp Val Arg Gln Ala Pro Gly Lys Gly Leu Glu
   50 55 60
   Trp Val Gly Ile Ile Tyr Gly Ser Asp Glu Thr Ala Tyr Ala Thr Ser
   65 70 75 80
   Ala Ile Gly Arg Phe Thr Ile Ser Arg Asp Asn Ser Lys Asn Thr Leu
   85 90 95
   Tyr Leu Gln Met Asn Ser Leu Arg Ala Glu Asp Thr Ala Val Tyr Tyr
   100 105 110
   Cys Ala Arg Asp Asp Ser Ser Asp Trp Asp Ala Lys Phe Asn Leu Trp
   115 120 125
   Gly Gln Gly Thr Leu Val Thr Val Ser Ser Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Arg Val Glu Pro Lys Ser
   225 230 235 240
   Cys Asp Lys Thr His Thr Cys Pro Pro Cys Pro Ala Pro Glu Leu Leu
   245 250 255
   Gly Gly Pro Ser Val Phe Leu Phe Pro Pro Lys Pro Lys Asp Thr Leu
   260 265 270
   Met Ile Ser Arg Thr Pro Glu Val Thr Cys Val Val Val Asp Val Ser
   275 280 285
   His Glu Asp Pro Glu Val Lys Phe Asn Trp Tyr Val Asp Gly Val Glu
   290 295 300
   Val His Asn Ala Lys Thr Lys Pro Arg Glu Glu Gln Tyr Ala Ser Thr
   305 310 315 320
   Tyr Arg Val Val Ser Val Leu Thr Val Leu His Gln Asp Trp Leu Asn
   325 330 335
   Gly Lys Glu Tyr Lys Cys Lys Val Ser Asn Lys Ala Leu Pro Ala Pro
   340 345 350
   Ile Glu Lys Thr Ile Ser Lys Ala Lys Gly Gln Pro Arg Glu Pro Gln
   355 360 365
   Val Tyr Thr Leu Pro Pro Ser Arg Glu Glu Met Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys
   465
<210> 709
   <211> 111
   <212> PRT
   <213> Oryctolagus cuniculus
<400> 709
   Ala Ile Gln Met Thr Gln Ser Pro Ser Ser Leu Ser Ala Ser Val Gly
   1 5 10 15
   Asp Arg Val Thr Ile Thr Cys Gln Ala Ser Gln Ser Ile Asn Asn Glu
   20 25 30
   Leu Ser Trp Tyr Gln Gln Lys Pro Gly Lys Ala Pro Lys Leu Leu Ile
   35 40 45
   Tyr Arg Ala Ser Thr Leu Ala Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Asp Asp Phe Ala Thr Tyr Tyr Cys Gln Gln Gly Tyr Ser Leu Arg Asn
   85 90 95
   Ile Asp Asn Ala Phe Gly Gly Gly Thr Lys Val Glu Ile Lys Arg
   100 105 110
<210> 710
   <211> 11
   <212> PRT
   <213> RASQGIRNDLG
<400> 710
   Arg Ala Ser Gln Gly Ile Arg Asn Asp Leu Gly
   1 5 10
<210> 711
   <211> 11
   <212> PRT
   <213> oryctolagus cuniculus
<400> 711
   Arg Ala Ser Gln Gly Ile Ser Asn Tyr Leu Ala
   1 5 10
<210> 712
   <211> 11
   <212> PRT
   <213> oryctolagus cuniculus
<400> 712
   Arg Ala Ser Gln Ser Ile Ser Ser Trp Leu Ala
   1 5 10
<210> 713
   <211> 7
   <212> PRT
   <213> Oryctolagus cuniculus
<400> 713
   Ala Ala Ser Ser Leu Gln Ser
   1 5
<210> 714
   <211> 7
   <212> PRT
   <213> oryctolagus cuniculus
<400> 714
   Ala Ala Ser Thr Leu Gln Ser
   1 5
<210> 715
   <211> 7
   <212> PRT
   <213> oryctolagus cuniculus
<400> 715
   Lys Ala Ser Ser Leu Glu Ser
   1 5
<210> 716
   <211> 5
   <212> PRT
   <213> Oryctolagus cuniculus
<400> 716
   Ser Asn Tyr Met Ser
   1 5
<210> 717
   <211> 16
   <212> PRT
   <213> oryctolagus cuniculus
<400> 717
   Val Ile Tyr Ser Gly Gly Ser Thr Tyr Tyr Ala Asp Ser Val Lys Gly
   1 5 10 15
<210> 718
   <211> 17
   <212> PRT
   <213> Oryctolagus cuniculus
<400> 718
   Val Ile Tyr Ser Gly Gly Ser Ser Thr Tyr Tyr Ala Asp Ser Val Lys
   1               5                   10                  15
   Gly
<210> 719
   <211> 330
   <212> PRT
   <213> Artificial Sequence
<220>
   <223> Gamma-1 constant domain
<400> 719
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 720
   <211> 297
   <212> DNA
   <213> Oryctolagus cuniculus
<400> 720
   atccagatga cccagtctcc ttcctccctg tctgcatctg taggagacag agtcaccatc      60
   acttgccagg ccagtcagag cattaacaat gagttatcct ggtatcagca gaaaccaggg     120
   aaagccccta agctcctgat ctatagggca tccactctgg catctggggt cccatcaagg     180
   ttcagcggca gtggatctgg gacagacttc actctcacca tcagcagcct gcagcctgat     240
   gattttgcaa cttattactg ccaacagggt tatagtctga ggaacattga taatgct        297
<210> 721
   <211> 333
   <212> DNA
   <213> Oryctolagus cuniculus
<400> 721
   gcctatgata tgacccagac tccagcctcg gtgtctgcag ctgtgggagg cacagtcacc      60
   atcaagtgcc aggccagtca gagcattaac aatgaattat cctggtatca gcagaaacca     120
   gggcagcgtc ccaagctcct gatctatagg gcatccactc tggcatctgg ggtctcatcg     180
   cggttcaaag gcagtggatc tgggacagag ttcactctca ccatcagcga cctggagtgt     240
   gccgatgctg ccacttacta ctgtcaacag ggttatagtc tgaggaatat tgataatgct     300
   ttcggcggag ggaccgaggt ggtggtcaaa cgt                                  333
<210> 722
   <211> 648
   <212> DNA
   <213> oryctolagus cuniculus
<400> 722
   atccagatga cccagtctcc ttcctccctg tctgcatctg taggagacag agtcaccatc 60
   acttgccagg ccagtcagag cattaacaat gagttatcct ggtatcagca gaaaccaggg 120
   aaagccccta agctcctgat ctatagggca tccactctgg catctggggt cccatcaagg 180
   ttcagcggca gtggatctgg gacagacttc actctcacca tcagcagcct gcagcctgat 240
   gattttgcaa cttattactg ccaacagggt tatagtctga ggaacattga taatgctttc 300
   ggcggaggga ccaaggtgga aatcaaacgt acggtggctg caccatctgt cttcatcttc 360
   ccgccatctg atgagcagtt gaaatctgga actgcctctg ttgtgtgcct gctgaataac 420
   ttctatccca gagaggccaa agtacagtgg aaggtggata acgccctcca atcgggtaac 480
   tcccaggaga gtgtcacaga gcaggacagc aaggacagca cctacagcct cagcagcacc 540
   ctgacgctga gcaaagcaga ctacgagaaa cacaaagtct acgcctgcga agtcacccat 600
   cagggcctga gctcgcccgt cacaaagagc ttcaacaggg gagagtgt 648
<210> 723
   <211> 333
   <212> DNA
   <213> Oryctolagus cuniculus
<400> 723
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgt 333
<210> 724
   <211> 327
   <212> DNA
   <213> Oryctolagus cuniculus
<400> 724
   gaggtgcagc tggtggagtc tgggggaggc ttggtccagc ctggggggtc cctgagactc 60
   tcctgtgcag cctctggatt ctccctcagt aactactacg tgacctgggt ccgtcaggct 120
   ccagggaagg ggctggagtg ggtcggcatc atctatggta gtgatgaaac cgcctacgct 180
   acctccgcta taggccgatt caccatctcc agagacaatt ccaagaacac cctgtatctt 240
   caaatgaaca gcctgagagc tgaggacact gctgtgtatt actgtgctag agatgatagt 300
   agtgactggg atgcaaagtt caacttg 327
<210> 725
   <211> 351
   <212> DNA
   <213> Oryctolagus cuniculus
<400> 725
   cagtcgctgg aggagtccgg gggtcgcctg gtcacgcctg ggacacccct gacactcacc    60
   tgcacagcct ctggattctc cctcagtaac tactacgtga cctgggtccg ccaggctcca   120
   gggaaggggc tggaatggat cggaatcatt tatggtagtg atgaaacggc ctacgcgacc   180
   tgggcgatag gccgattcac catctccaaa acctcgacca cggtggatct gaaaatgacc   240
   agtctgacag ccgcggacac ggccacctat ttctgtgcca gagatgatag tagtgactgg   300
   gatgcaaaat ttaacttgtg gggccaaggc accctggtca ccgtctcgag c            351
<210> 726
   <211> 224
   <212> PRT
   <213> Homo sapiens
<400> 726
   Met Glu Lys Leu Leu Cys Phe Leu Val Leu Thr Ser Leu Ser His Ala
   1               5                   10                  15
   Phe Gly Gln Thr Asp Met Ser Arg Lys Ala Phe Val Phe Pro Lys Glu
               20                  25                  30
   Ser Asp Thr Ser Tyr Val Ser Leu Lys Ala Pro Leu Thr Lys Pro Leu
           35                  40                  45
   Lys Ala Phe Thr Val Cys Leu His Phe Tyr Thr Glu Leu Ser Ser Thr
       50                  55                  60
   Arg Gly Tyr Ser Ile Phe Ser Tyr Ala Thr Lys Arg Gln Asp Asn Glu
   65                  70                  75                  80
   Ile Leu Ile Phe Trp Ser Lys Asp Ile Gly Tyr Ser Phe Thr Val Gly
                   85                  90                  95
   Gly Ser Glu Ile Leu Phe Glu Val Pro Glu Val Thr Val Ala Pro Val
               100                 105                 110
   His Ile Cys Thr Ser Trp Glu Ser Ala Ser Gly Ile Val Glu Phe Trp
           115                 120                 125
   Val Asp Gly Lys Pro Arg Val Arg Lys Ser Leu Lys Lys Gly Tyr Thr
       130                 135                 140
   Val Gly Ala Glu Ala Ser Ile Ile Leu Gly Gln Glu Gln Asp Ser Phe
   145                 150                 155                 160
   Gly Gly Asn Phe Glu Gly Ser Gln Ser Leu Val Gly Asp Ile Gly Asn
                   165                 170                 175
   Val Asn Met Trp Asp Phe Val Leu Ser Pro Asp Glu Ile Asn Thr Ile
               180                 185                 190
   Tyr Leu Gly Gly Pro Phe Ser Pro Asn Val Leu Asn Trp Arg Ala Leu
           195                 200                 205
   Lys Tyr Glu Val Gln Gly Glu Val Phe Thr Lys Pro Gln Leu Trp Pro
       210                 215                 220
<210> 727
   <211> 468
   <212> PRT
   <213> Homo sapiens
<400> 727
   Met Leu Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
   275 280 285
   Val Val Gln Leu Arg Ala Gln Glu Glu Phe Gly Gln Gly Glu Trp Ser
   290 295 300
   Glu Trp Ser Pro Glu Ala Met Gly Thr Pro Trp Thr Glu Ser Arg Ser
   305 310 315 320
   Pro Pro Ala Glu Asn Glu Val Ser Thr Pro Met Gln Ala Leu Thr Thr
   325 330 335
   Asn Lys Asp Asp Asp Asn Ile Leu Phe Arg Asp Ser Ala Asn Ala Thr
   340 345 350
   Ser Leu Pro Val Gln Asp Ser Ser Ser Val Pro Leu Pro Thr Phe Leu
   355 360 365
   Val Ala Gly Gly Ser Leu Ala Phe Gly Thr Leu Leu Cys Ile Ala Ile
   370 375 380
   Val Leu Arg Phe Lys Lys Thr Trp Lys Leu Arg Ala Leu Lys Glu Gly
   385 390 395 400
   Lys Thr Ser Met His Pro Pro Tyr Ser Leu Gly Gln Leu Val Pro Glu
   405 410 415
   Arg Pro Arg Pro Thr Pro Val Leu Val Pro Leu Ile Ser Pro Pro Val
   420 425 430
   Ser Pro Ser Ser Leu Gly Ser Asp Asn Thr Ser Ser His Asn Arg Pro
   435 440 445
   Asp Ala Arg Asp Pro Arg Ser Pro Tyr Asp Ile Ser Asn Thr Asp Tyr
   450 455 460
   Phe Phe Pro Arg
   465
<210> 728
   <211> 918
   <212> PRT
   <213> Homo sapiens
<400> 728